# EUROPEAN PATENT SPECIFICATION

(11) **EP 1 147 083 B1**
(45) Date of publication and mention of the grant of the patent: **16.06.2004**
(21) Application number: 00903114.7
(22) Date of filing: 06.01.2000
(51) Int. Cl.: C07D 209/14, A61K 31/40, C07D 401/12, C07D 471/04, C07D 401/08, C07D 405/12, C07D 403/12, C07D 405/14

(54) **ARYLPIPERAZINYL-CYCLOHEXYL INDOLE DERIVATIVES FOR THE TREATMENT OF DEPRESSION**
ARYLPIPERAZINYL-CYCLOHEXYL INDOLDERIVATE ZUR BEHANDLUNG VON DEPRESSIONEN
DERIVES D'INDOLE ARYLPIPERAZINYL-CYCLOHEXYLE POUR LE TRAITEMENT DES DEPRESSIONS

(30) Priority: 07.01.1999 US 226583
(43) Date of publication of application: 24.10.2001
(73) Proprietor: Wyeth, Madison, New Jersey 07940-0874 (US)
(72) Inventor: MEWSHAW, Richard, Eric, King of Prussia, PA 19406 (US); ZHOU, Ping, Plainsboro, NJ 08536 (US); ZHOU, Dahui, East Brunswick, NJ 08816 (US); MEAGHER, Kristin, Lynne, Hightstown, NJ 08520 (US); ASSELIN, Magda, Mahwah, NJ 07430 (US); EVRARD, Deborah, Ann, Hamilton Square, NJ 08690 (US); GILBERT, Adam, Matthew, Congers, NY 10920 (US)
(74) Representative: Wileman, David Francis, Dr.
(86) International application number: PCT/US2000/000223
(87) International publication number: WO 2000/040554

(56) References cited:
- EP-A- 0 345 808
- EP-A- 0 736 525
- US-A- 5 468 767
- DAVID J. WUSTROW ET AL.: "3-(((4-Aryl-1-piperazinyl)alkyl)cyclohexy l)-1H-indoles as dopamine D2 partial agonists and autoreceptor agonists" JOURNAL OF MEDICINAL CHEMISTRY., vol. 40, no. 2, - 1997 pages 250-259, XP002137931 AMERICAN CHEMICAL SOCIETY. WASHINGTON., US ISSN: 0022-2623

## Description

### FIELD OF INVENTION

This invention relates to compounds useful for the treatment of diseases affected by disorders of the serotonin-affected neurological systems, such as depression and anxiety. More specifically the present invention is directed to arylpiperazinyl cyclohexyl derivatives useful for the treatment of such disorders.

### BACKGROUND OF INVENTION

Pharmaceuticals which enhance neurotransmission of serotonin (5-HT) are useful for the treatment of many psychiatric disorders, including depression and anxiety. The first generation of non-selective serotonin-affecting drugs operated through a variety of physiological means which caused them to possess numerous undesired side-effects. The more recently prescribed drugs, the selective serotonin reuptake inhibitors (SSRIs), act predominately by inhibiting 5-HT, which is released at the synapses, from being actively removed from the synaptic cleft via a presynaptic serotonin transport carrier. Since SSRIs require several weeks before they exert their full therapeutic effect, this 5-HT blockade mechanism cannot fully account for their therapeutic activity. It is speculated that this two week induction which occurs before a full antidepressant effect is observed, is due to the involvement of the 5-HT1A autoreceptors which suppress the firing activity of 5-HT neurons, causing a dampening of the therapeutic effect. Studies suggest that after several weeks of SSRI administration, a desensitization of the 5-HT autoreceptors occurs allowing a full antidepressant effect in most patients. (See, e.g., Le Poul et al., Arch. Pharmacol., 352:141 (1995)). Hence, it is believed that overriding this negative feedback by using 5HT1A antagonists would potentially increase and accelerate the clinical antidepressant response. Recent studies by Artigas et al., Trends Neurosci., 19:378-383 (1996), suggest a combination of 5-HT1A activity and inhibition of 5-HT uptake within a single molecular entity can achieve a more robust and fast-acting antidepressant effect.

The present invention relates to a new class of molecules which have the ability to act at the 5-HT1A autoreceptors and concommitantly with the 5-HT transporter. Such compounds are therefore potentially useful for the treatment of depression as well as other serotonin disorders.

U.S. Patent No. 5,468,767 reports a series of substituted indoles of the following formula for the treatment of disorders associated with dysfunction in serotonergic neurotransmission, including depression 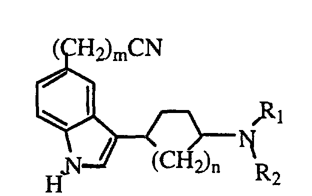 wherein:
R₁ is hydrogen or C₁₋₄ alkyl and R₂ is C₁₋₄ alkyl or (CH₂)pAr.

WO 9415928 discloses a series of piperazine derivatives of the following formula for the treatment of CNS disorders, including depression. 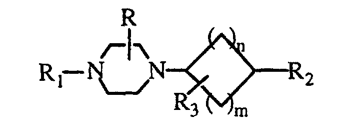 wherein:
R is hydrogen or alkyl;
R₁ and R₂ are each mono- or bicyclic aryl or heteroaryl radicals;
R₃ is hydrogen, alkyl, or a spirocycloalkyl group; and
n is 1 or 2 and m is 1 to 3.

WO 93/10092 discloses a series of cyclohexenes of the following formula for the treatment of dopaminergic disorders. 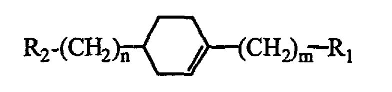 D.J. Wustrow et al., J. Med. Chem., 1997, 40, 250 to 259 disclose compounds having the formula

R-(CH₂)ₙ-NR₁R₂

where R is 4-(2-pyridinyl)-1-piperazinyl, n is 0, 1 or 2 and NR₁R₂ is 4-(1H-3-indolyl)cyclohexyl and teaches that increasing the chain length improved DA D2 receptor binding.
EP 0345806A discloses 1-[indolyl(alkyl, cycloalkyl or cycloalkenyl)]-4-(substituted pyridinyl)piperazines for the treatment of depression.

### SUMMARY OF THE INVENTION

The compounds of this invention are arylpiperazinyl-cyclohexyl indole derivatives represented by Formula I: 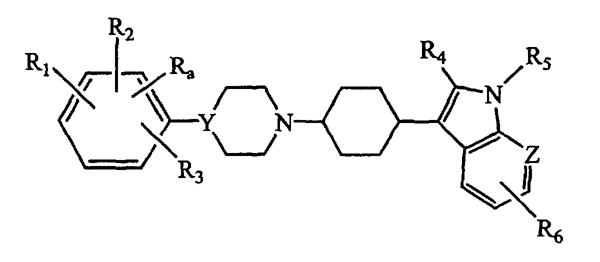 wherein:
Rₐ, R₁, R₂ and R₃ are each, independently, hydrogen, or a substituent selected from halogen, CF₃, alkyl, alkoxy, MeSO₂, amino or aminocarbonyl (each optionally substituted by one or two groups selected from alkyl and benzyl) carboxy, or alkoxycarbonyl ; or two adjacent of Rₐ and R₁₋₃ together can form a 5-7 membered carbocyclic or heterocyclic ring which is optionally substituted by a substituent defined above;
R₄ is hydrogen, halogen, or alkyl;
R₅ is hydrogen, alkyl, arylalkyl, or aryl;
R₆ is hydrogen, halogen, CF₃, CN, carbamide, alkoxy or benzyloxy;
Y is CH or nitrogen; and
Z is carbon or nitrogen; or
pharmaceutically acceptable salts thereof, each alkyl and alkoxy group including the alkyl component of arylalkyl contains 1-6 carbon atoms and each aryl radical including the aryl component of arylalkyl contains 6-12 carbon atoms.

Preferably, the compounds of the present invention are those represented by Formula I, wherein Rₐ, R₁, R₂ and R₃ are each, independently, hydrogen, halogen, alkyl, alkoxy or together form a 5-7 membered carbocyclic or heterocyclic ring;
R₄ is hydrogen or halogen;and.or
R₅ is hydrogen, alkyl or alkylaryl; and/or
R₆ is hydrogen, halogen, CN or alkoxy; and/or
Y and Z are each carbon; or a
pharmaceutically acceptable salt thereof.

More preferably, the compounds of the present invention are selected from the following:
3-[cis-4-[4-(1H-Indol-4-yl)-1-piperazinyl]cyclohexyl]-1H-indole;
3-[trans-4-[4-(1H-indol-4-yl)-1-piperazinyl]cyclohexyl]-1H-indole;
4-Fluoro-3-[cis-4-[4-(1H-indol-4-yl)-1-piperazinyl]cyclohexyl]-1H-indole;
4-Fluoro-3-[trans-4-[4-(1H-indol-4-yl)-1-piperazinyl]cyclohexyl]-1H-indole;
5-Fluoro-3-[cis-4-[4-(1H-indol-4-yl)-1-piperazinyl]cyclohexyl]-1H-indole;
5-Fluoro-3-[trans-4-[4-(1H-indol-4-yl)-1-piperazinyl]cyclohexyl]-1H-indole;
6-Fluoro-3-[cis-4-[4-(1H-indol-4-yl)-1-piperazinyl]cyclohexyl]-1H-indole;
6-Fluoro-3-[trans-4-[4-(1H-indol-4-yl)-1-piperazinyl]cyclohexyl]-1H-indole;
5-Bromo-3-[cis-4-[4-(1H-indol-4-yl)-1-piperazinyl]cyclohexyl]-1H-indole;
5-Bromo-3-[trans-4-[4-(1H-indol-4-yl)-1-piperazinyl]cyclohexyl]-1H-indole;
5-Chloro-3-[cis-4-[4-(1H-indol-4-yl)-1-piperazinyl]cyclohexyl]-1H-indole;
5-Chloro-3-[trans-4-[4-(1H-indol-4-yl)-1-piperazinyl]cyclohexyl]-1H-indole;
3-{4-[(1,4-cis)-4-(1H-indol-4-yl)-piperazinyl-1-yl]cyclohexyl}-1H-indole-5-carbonitrile;
3-{4-[(1,4-trans)-4-(1H-indol-4-yl)-piperazinyl-1-yl]cyclohexyl}-1H-indole-5-carbonitrile;
5-Methoxy-3-[cis-4-[4-(1H-indol-4-yl)-1-piperazinyl]cyclohexyl]-1H-indole;
5-Methoxy-3-[trans-4-[4-(1H-indol-4-yl)-1-piperazinyl]cyclohexyl]-1H-indole;
3-[cis-4-[4-(1H-Indol-4-yl)-1-piperazinyl]cyclohexyl]-2-methyl-1H-indole;
3-[trans-4-[4-(1H-Indol-4-yl)-1-piperazinyl]cyclohexyl]-2-methyl-1H-indole;
3-{(1,4-cis)-4-[4-1H-Indole-4-yl)-piperazin-1-yl]-cyclohexyl}-1H-pyrrolo[2,3-b]pyridine;
3-{(1,4-trans)-4-[4-(1H-Indol-4-yl)-piperazin-1-yl]-cyclohexyl}-1H-pyrrolo[2,3-b]pyridine;
6-Fluoro-1-methyl-3-{cis-4-[4-(1-methyl-1H-indol-4-yl)-1-piperazinyl]cyclohexyl}-1H-indole;
3-{(1,4-cis)-4-[4-(1H-indol-4-yl)-piperazin-1-yl]cyclohexyl}-1-methyl-1H-indole-5-carbonitrile;
3- {(1,4-trans)-4-[4-(1H-indol-4-yl)-piperazin-1-yl]cyclohexyl}-1-methyl-1H-indole-5-carbonitrile;
1-Ethyl-3-{(1,4-cis)-4-[4-(1H-indole-4-yl)-piperazin-1-yl]-cyclohexyl}-1H-indole-5-carbonitrile;
3- {(1,4-cis)-4-[4-(1H-indol-4-yl)-piperazin-1-yl]-cyclohexyl}-1-propyl-1H-indole-5-carbonitrile;
3-{(1,4-trans)-4-[4-(1H-indol-4-yl)-piperazin-1-yl]-cyclohexyl;}-1-propyl-1H-indole-5-carbonitrile;
3-{(1,4-cis)-4-[4-(1H-indol-4-yl)-piperazin-1-yl]-cyclohexyl}-1-isopropyl-1H-indole-5-carbonitrile;
3-{(1,4-trans)-4-[4-(1H-indol-4-yl)-piperazin-1-yl]cyclohexyl}-1-isopropyl-1H-indole-5-carbonitrile;
1-Benzyl-3-{(1,4-cis)-4-[4-(1H-indol-4-yl)-piperazin-1-yl]-cyclohexyl}-1H-indole-5-carbonitrile;
1-Benzyl-3-{(1,4-trans)-4-[4-(1H-indole-4-yl)-piperazin-1-yl]cyclohexyl}-1H-indole-5-carbonitrile;
1-Methyl-3-{(1,4-cis)-4-[4-(1-methyl-1H-indol-4-yl)-piperazine-1-yl]-cyclohexyl}-1 H-indole-5-carbonitrile;
5-Fluoro-3-{(cis)-4-[4-(2-methoxy-phenyl)-piperazin-1-yl]-cyclohexyl}-1H-indole;
5-Fluoro-3-{(1,4-cis)-4-[4-(2-methoxy-phenyl)-piperidin-1-yl]-cyclohexyl}-1H-indole;
5-Fluoro-3- {(1,4-trans)-4-[4-(2-methoxy-phenyl)-piperidin-1-yl]-cyclohexyl}-1H-indole;
5-methoxy-3-{(1,4-cis)-4-[4-(2-methoxy-phenyl)-piperazinyl-1-yl]-cyclohexyl}-1H-indole;
5-Methoxy-3-{(1,4-trans)-4-[4-(2-methoxy-phenyl)-piperidin-1-yl]-cyclohexyl}-1H-indole;
3-{(1,4-cis)-4-[4-(2-methoxy-phenyl)-piperazin-1-yl]-cyclohexyl}-1H-pyrrolo[2,3-b]piperidine;
5-Fluoro-3-{(cis)-4-[4-(5-fluoro-2-methoxy-phenyl)-piperazin-1-yl]-cyclohexyl}-1H-indole;
5-Fluoro-3-{(trans)-4-[4-(5-fluoro-2-methoxy-phenyl)-piperazin-1-yl]-cvyclohexyl}-1H-indole;
3-{(1,4-cis)-4-[4[(2,3-Dihydro-benzo[1,4]dioxin-5-yl)-piperazin-1-yl]-cyclohexyl}-4-fluoro-1H-indole;
3- {(1,4-trans)-4-[4-(2,3-Dihydro-benzo[1,4]dioxin-5-yl)-piperazin-1-yl]-cyclohexyl}-4-fluoro-1H-indole;
3- {(1,4-cis)-4-[4-(2,3-Dihydro-benzo[1,4]dioxin-5-yl)-piperazin-1-yl]-cyclohexyl}-5-fluoro-1H-indole;
3-{(1,4-trans)-4-[4-(2,3-Dihydro-benzo[1,4]dioxin-5-yl)-piperazin-1-yl]-cyclohexyl}-5-fluoro-1H-indole;
3-{(1,4-cis)-4-[4-(2,3-Dihydro-benzo[1,4]dioxin-5-yl)-piperazin-1-yl]-cyclohexyl}-6-fluoro-1H-indole;
3- {(1,4-trans)-4-[4-(2,3-Dihydro-benzo[1,4]dioxin-5-yl)-piperazin-1-yl]-cyclohexyl}-6-fluoro-1H-indole;
3-{(1,4-trans)-4-[4-(2,3-Dihydro-benzo[1,4]dioxin-5-yl)-piperazin-1-yl]-cyclohexyl}-6-fluoro-1H-indole;
3-{(1,4-cis)-4-[4-(2,3-Dihydro-benzo[1,4]dioxin-5-yl)-piperazin-1-yl]-cyclohexyl}-1H-indole-5-carbonitrile;
3-{(1,4-trans)-4-(4-(2,3-Dihydro-benzo[1,4]dioxin-5-yl)-piperazin-1-yl]-cyclohexyl}-1H-indole-5-carbonitrile;
8-{4-[(1,4-cis)-4-(5-Fluoro-1H-indol-3-yl)-cyclohexyl]-piperazin-1-yl}quinoline;
8-{4-[(1,4-trans)-4-(5-Fluoro-1H-indol-3-yl)-cyclohexyl]-piperazin-1-yl}-quinoline;
8-{4-(1,4-cis)-4-[4-(5-Fluoro-1-methyl-1H-indol-3-yl)-cyclohexyl]-piperazin-1-yl}-quinoline;
3-[(1,4-cis)-4-(4-Quinolin-8-yl-piperazin-1-yl)-cyclohexyl]-1H-indole-5-carbonitrile;
3-[(1,4-trans)-4-(4-Quinolin-8-yl-piperazin-1-yl)-cyclohexyl]-1H-indole-5-carbonitrile;
1-Methyl-3-[(1,4-cis)-4-(4-quinolin-8-yl-piperazin-1-yl)-cyclohexyl]-1H-indole-5-carbonitrile;
5-Fluoro-3-{(1,4-cis)-4-[4-(6-fluoro-chroman-8-yl)-piperazin-1-yl]-cyclohexyl}-1H-indole;
5-Fluoro-3-{(1,4-trans)-4-[4-(6-fluoro-chroman-8-yl)-piperazin-1-yl]-cyclohexyl}-1H-indole;
5-Fluoro-3- {(1,4-cis)-4-[4-(5-fluoro-2,3-dihydro-benzofuran-7-yl)-piperazin-1-yl]-cyclohexyl}-1H-indole;
5-Fluoro-3-{(1,4-trans)-4-[4-(5-fluoro-2,3-dihydro-benzofuran-7-yl)-piperazin-1-yl]-cyclohexyl}-1H-indole;
3-{(1,4-cis)-4-[4-(5-Fluoro-2,3-dihydro-benzofuran-7-yl)-piperazin-1-yl]-cyclohexyl}-1H-indole-5-carbonitrile;
3-{(1,4-trans)-4-[4-(5-Fluoro-2,3-dihydro-benzofuran-7-yl)-piperazin-1-yl]-cyclohexyl}-1H-indole-5-carbonitrile;
3-{(1,4-cis)-4-[4-(5-Fluoro-2,3-dihydro-benzofuran-7-yl)-piperazin-1yl]-cyclohexyl}-1-methyl-1H-indole-5-carbonitrile;
3-[(1,4-cis)-4-[4-(Benzofuran-7-yl-piperazin-1-yl)-cyclohexyl]-1H-indole-5-carbonitrile;
3-[(1,4-trans)-4-[4-(Benzofuran-7-yl-piperazin-1-yl)-cyclohexyl]-1H-indole-5-carbonitrile;
5-Fluoro-3-{4-[4-(2-methoxy-phenyl)-piperazin-1-yl]cyclohex-1-enyl}-1H-indole;
3- {4-[4-(1H-Indol-4-yl)-piperazin-1-yl]-cyclohex-1-enyl}-1H-indole-5-carbonitrile;
5-Fluoro-3-{(1,4-cis)-4-[4-(2-methoxy-phenyl)-piperazin-1-yl]-cyclohexyl}-1,3-dihydro-indol-2-one;
5-Fluoro-3-{cis-4-[4-(1H-indol-4-yl)piperazinyl]-cyclohexyl}-1-methyl-1H-indole;
8{(1,4-cis)-4-[4-(5-Fluoro-1H-indol-3-yl)-cyclohexyl]-piperazin-1-yl}-6-methoxy-quinoline;
8-{(1,4-trans)-4-[4-(5-Fluoro-1H-indol-3-yl)-cyclohexyl]piperazin-1-yl}-6-methoxy-quinoline;
3-{(1,4-cis)-4-[4-6-Methoxy-quinoline-8-yl)-piperazin-1-yl]-cyclohexyl}-1H-indole-5-carbonitrile;
3-{(1,4-trans)-4-[4-(6-Methoxy-quinolin-8-yl)-piperazin-1-yl]-cyclohexyl}-1H-indole-5-carbonitrile;
6-Chloro-8-{4-[1,4-cis)-4-(5-fluoro-1H-indol-3-yl)-cyclohexyl]-piperazin-1-yl}quinoline;
6-Chloro-8-{4-[(1,4-trans)-4-(5-fluoro-1H-indol-3-yl)-cyclohexyl]-piperazin-1-yl}quinoline;
3-{(1,4-cis)-4-[(4-(6-Chloro-quinolin-8-yl)-piperazin-1-yl]-cyclohexyl}-1H-indole-5-carbonitrile;
3-{(1,4-trans)-4-[4-(6-Chloro-quinolin-8-yl)-piperazin-1-yl]-cyclohexyl}-1H-indole-5-carbonitrile;
5-Chloro-8-{4-[(1,4-cis)-4-(5-fluoro-1H-indol-3-yl)-cyclohexyl]-piperazin-1-yl}-quinoline;
3-{(1,4-cis)-4-[4-(5-Chloro-quinolin-8-yl)-piperazin-1-yl]-cyclohexyl}-1H-indole-5-carbonitrile;
5-Fluoro-8-{4-[(1,4-cis)-4-(6-fluoro-1H-indole-3-yl)-cyclohexyl]-piperazin-1-yl}-quinoline;
5-Fluoro-8-{4-[(1,4-trans)-4-(6-fluoro-1H-indol-3-yl)-cyclohexyl]-piperazin-1-yl}-quinoline;
3-{(1,4-cis)-4-[4-(2-Methyl-quinolin-8-yl)-piperazin-1-yl]-cyclohexyl}-1H-indole-5-carbonitrile;
3-{(1,4-trans)-4-[4-(2-Methyl-quinolin-8-yl)-piperazin-1-yl]-cyclohexyl}-1H-indole-5-carbonitrile;
4-{4-[(1,4-cis)-4-(5-Fluoro-1H-indol-3-yl)-cyclohexyl]-piperazin-1-yl}-2-trifluoromethyl-quinoline;
4-{4-[(1,4-trans)-4-(5-Fluoro-1H-indol-3-yl)-cyclohexyl]-piperazin-1-yl}-2-trifluoromethyl-quinoline;
3-{(1,4-cis)-4-[4-(2-Trifluoromethyl-quinolin-4-yl)-piperazin-1-yl]-cyclohexyl}-1H-indole-5-carbonitrile
3-{(1,4-trans)-4-[4-(2-Trifluoromethyl-quinolin-4-yl)-piperazin-1-yl]-cyclohexyl}-1H-indole-5-carbonitrile;
4-{4-[(1,4-cis)-4-(5-Fluoro-1H-indol-3-yl)-cyclohexyl]-piperazin-1-yl}-6-methoxy-quinoline;
4-{4[(1,4-trans)-4-(5-Fluoro-1H-indol-3-yl)-cyclohexyl]-piperazin-1-yl}-6-methoxy-quinoline;
3-{(1,4-cis)-4-[4-(6-Methoxy-quinolin-4-yl)-piperazin-1-yl]-cyclohexyl}-1H-indole-5-carbonitrile; and
3-{(1,4-trans)-4-[4-(6-Methoxy-quinolin-4-yl)-piperazin-1-yl]-cyclohexyl}-1H-indole-5-carbonitrile.

As used herein, the terms "alkyl" and "alkoxy" are meant to include both straight and branched carbon chains containing 1-6 carbon atoms. The term "aryl" is meant to include aromatic radicals of 6-12 carbon atoms. The term "halogen" is meant to include fluorine, chlorine, bromine and iodine. Heterocyclic groups have one to three heteroatoms selected from oxygen, nitrogen and sulphur.

The compounds of Formula I also may be used in the form of a pharmaceutically acceptable acid addition salt having the utility of the free base. Such salts, prepared by methods well known to those skilled in the art are formed with both inorganic or organic acids, for example: fumaric, maleic, benzoic, ascorbic, pamoic, succinic, bismethylenesalicylic, methanesulfonic, ethanedisulfonic, acetic, oxalic, propionic, tartaric, salicyclic, citric, gluconic, lactic, malic, mandelic, cinnamic, citraconic, aspartic, stearic, palmitic, itaconic, glycolic, p-aminobenzoic, glutamic, benzene-sulfonic, hydrochloric hydrobromic, sulfuric, cyclohexylsulfamic, phosphoric and nitric acids.

The compounds of the present invention may be prepared by any suitable method which will be recognized by those skilled in the art.

Accordingly this invention provides a process for preparing compounds of formula I: 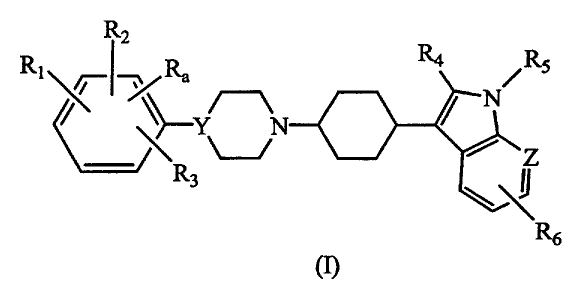 as defined herein or a
pharmaceutically acceptable salt thereof,
which comprises one of the following:
a) reacting a compound of formula 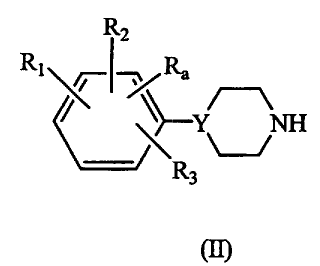 wherein Rₐ, R₁₋₃, Y and X₁₋₃ are as defined above,
   with a compound of formula (IV): 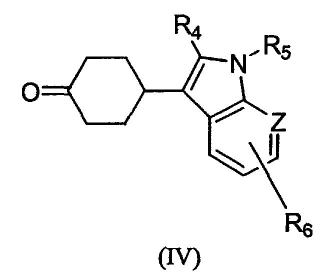 wherein Z, R₄, R₅ and R₆ are as defined above;
   or
b) reducing a compound of formula : 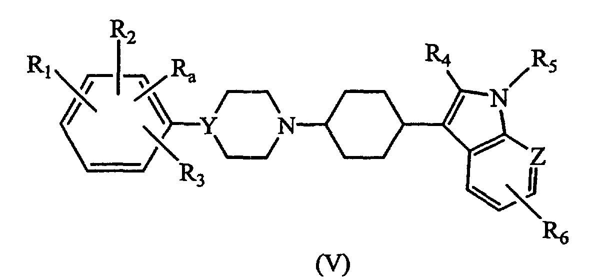 wherein the variables are as defined above to give a compound of formula (I);
   or
c) acidifying a basic compound of formula I with a pharmaceutically acceptable acid to give a pharmaceutically acceptable salt;
   or
d) separating a mixture of cis and trans isomers of a compound of formula (I) to isolate one isomer substantially free from the other isomer.
   or
e) reacting a compound of formula (I) having a reactive substituent group to give a compound of formula (I) having a different substituent group;
f) reacting a compound of formula (I) having a reactive site (e.g. NH) to give a compound of formula (I) having a substituent group on the site;

With regard to process a) the reaction may be carried out by reductive alkylation, e.g. using reducing agent such as sodium triacetoxyborohydride in a suitable solvent e.g. acetic acid.

With regard to process b) the reduction may conveniently carried out using palladium on carbon and hydrogen as exemplified herein.

The compounds of formula I may be isolated in the form of a salt of a pharmaceutically acceptable acid, e.g an organic or inorganic acid by treatment with an acid such as described above.

Geometric (cis and trans) isomers are possible and such isomers can be separated by standard techniques e.g. chromatography.

Examples of process e) involving conversion of substituents to other substituents are conversion of a halo substituent to an amino R₁substituent, esterification of a carboxy to give an ester, hydrolysis of an ester to give a carboxy group; and amination of an ester group to give an amide.

Examples of process f) involving substitution at sites to are alkylation at a NH site in the compound of formula (I) to give N-alkyl or N-benzyl.

The starting materials/reactants used in the processes above are known or can be made by methods known in the art from readily available materials by processes known or readily apparent to those skilled in the art. In any of the processes above reactive substituent groups or sites can be protected with protecting groups before reaction and the protecting group removed thereafter.

However, the present compounds may be advantageously prepared according to any one of Schemes 1-6 set forth below. In the Schemes, the intermediate compounds exemplified hereinafter are identified in parenthesis. The compound produced in each of Schemes 1-6 is identified with reference to the appropriate Example set forth below.

The preparation of such compounds is depicted in Schemes 1-6 below.

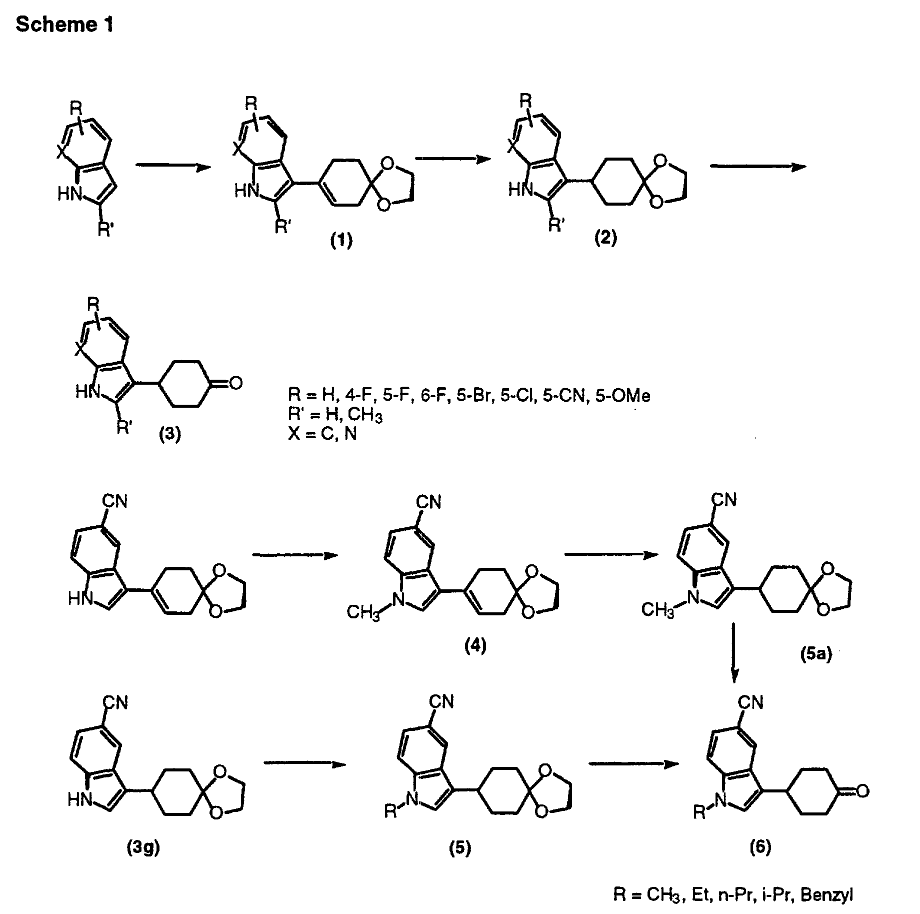

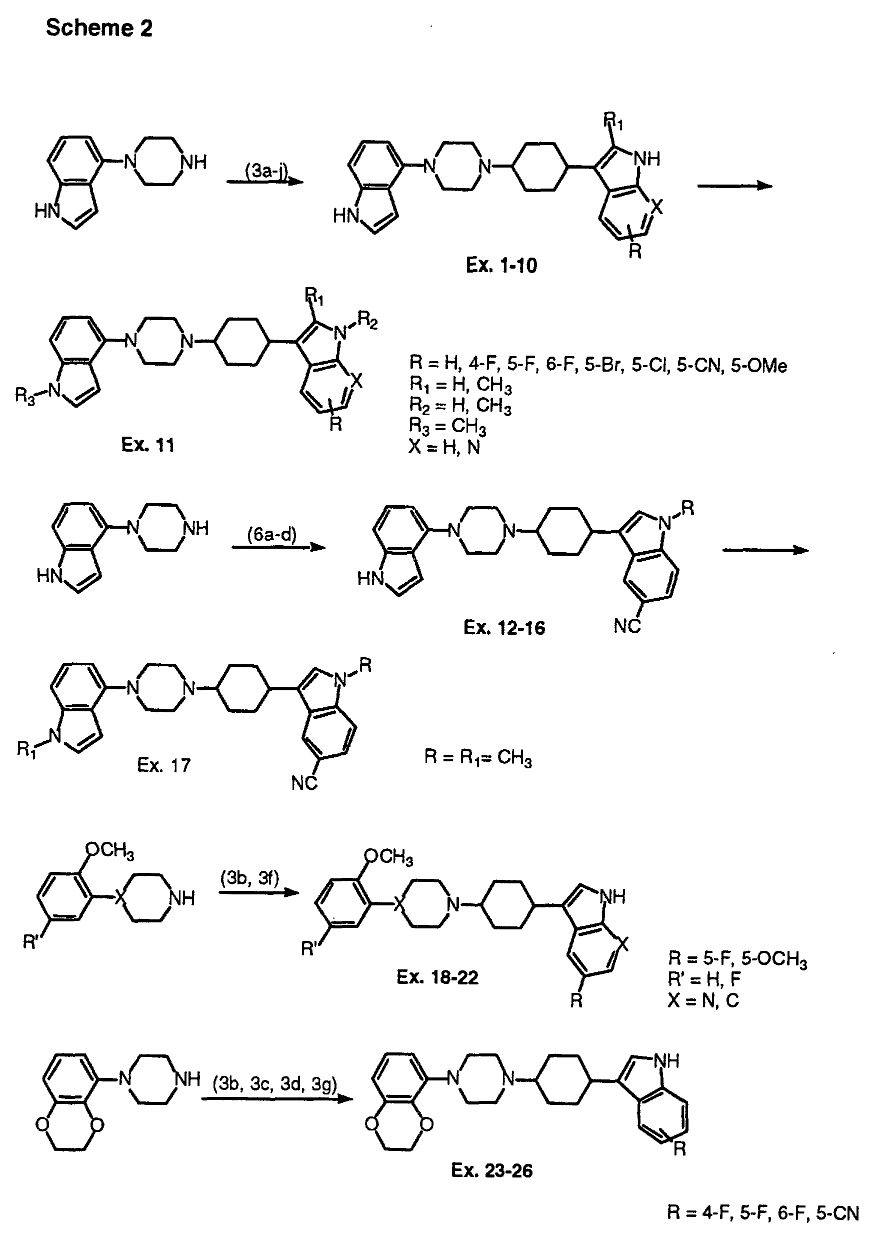

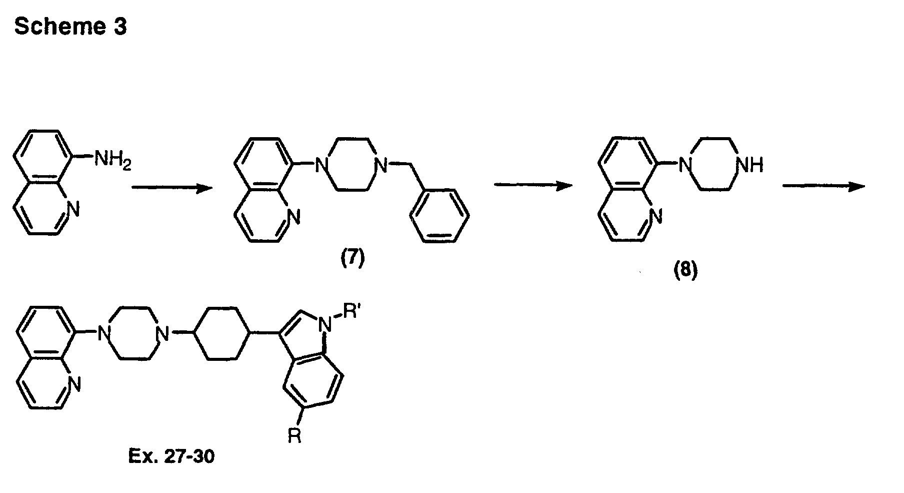

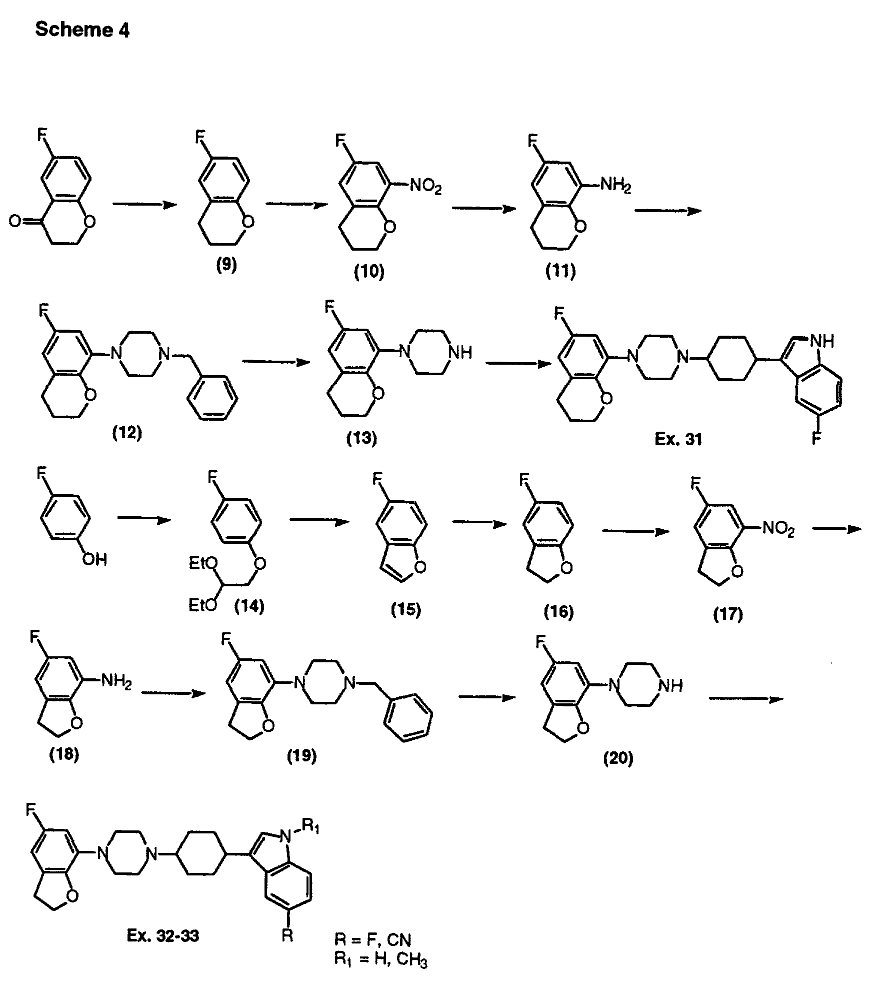

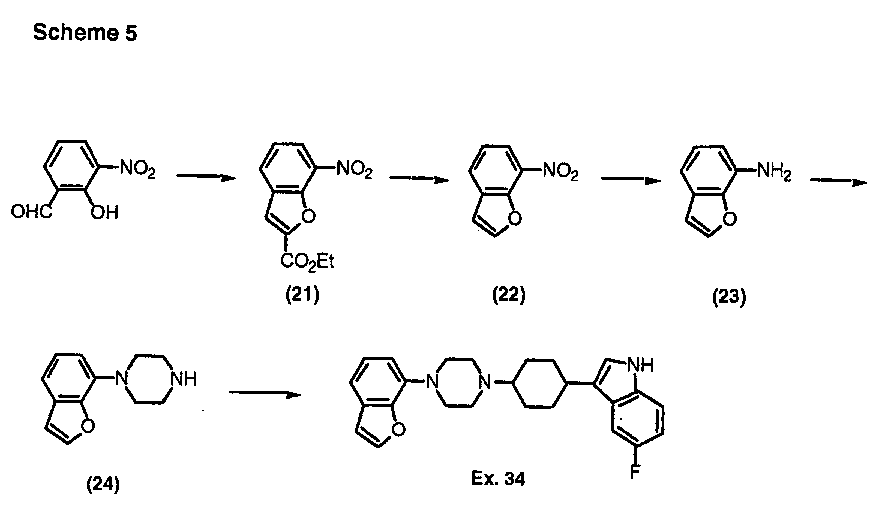

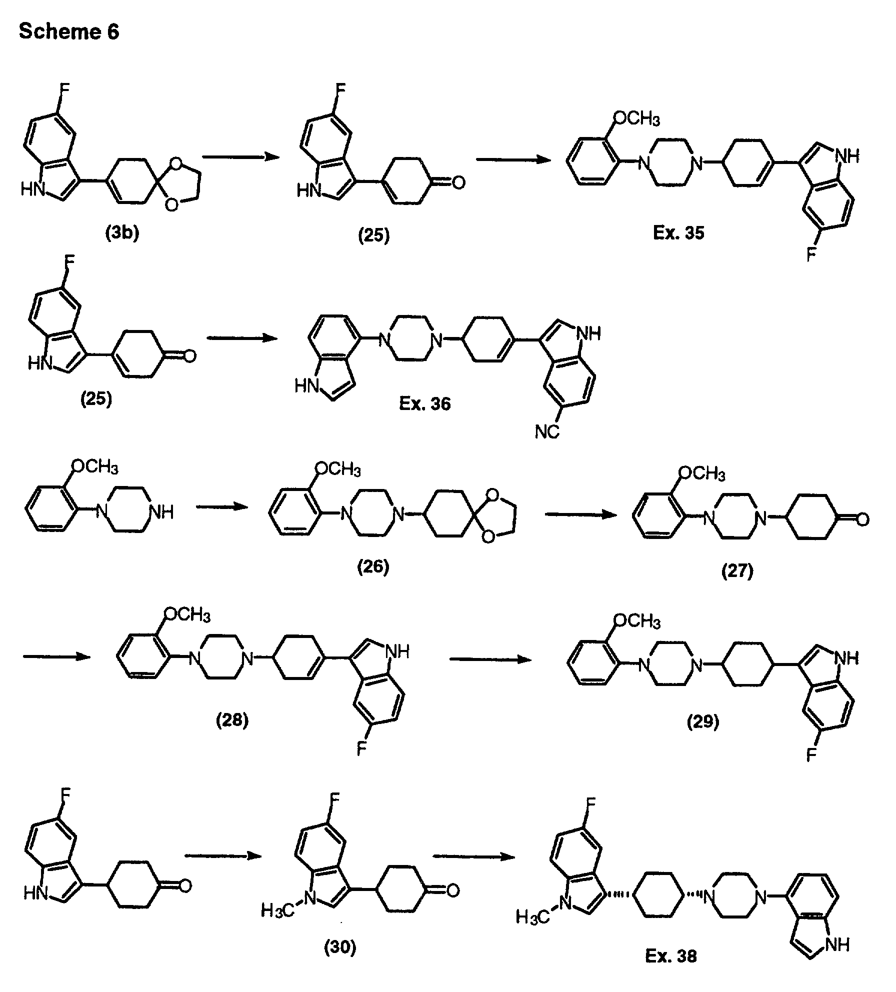

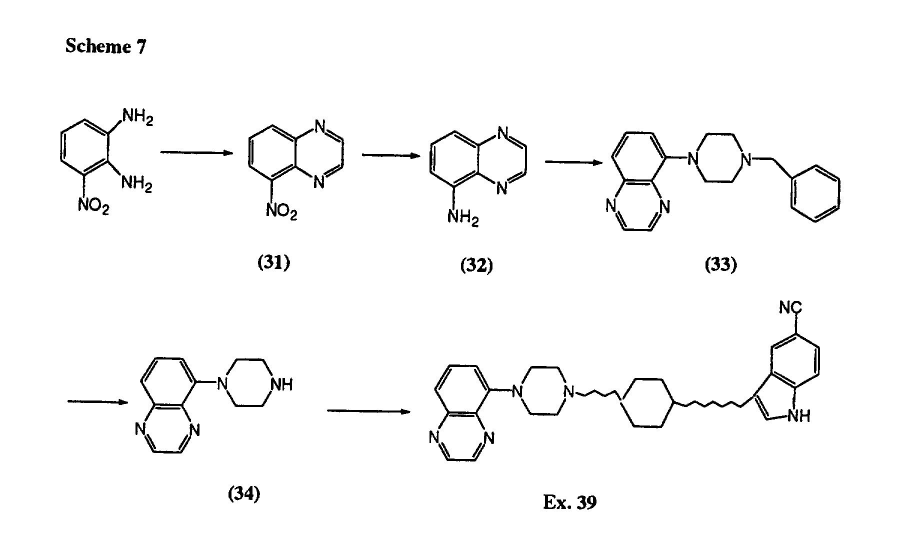

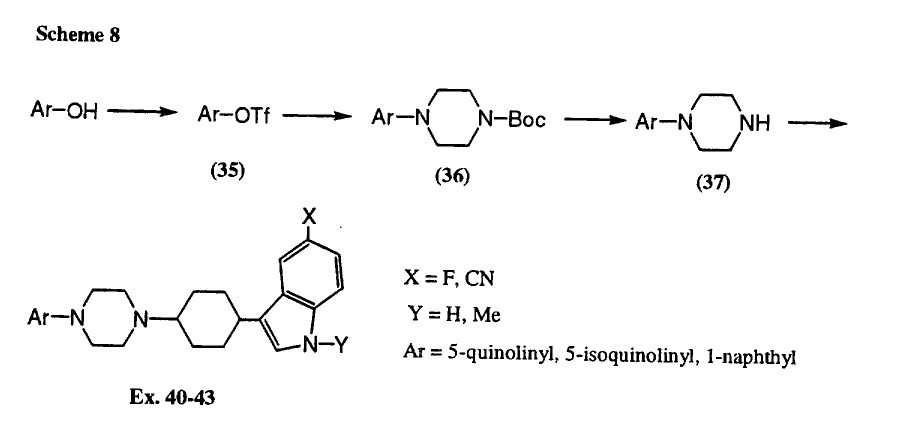

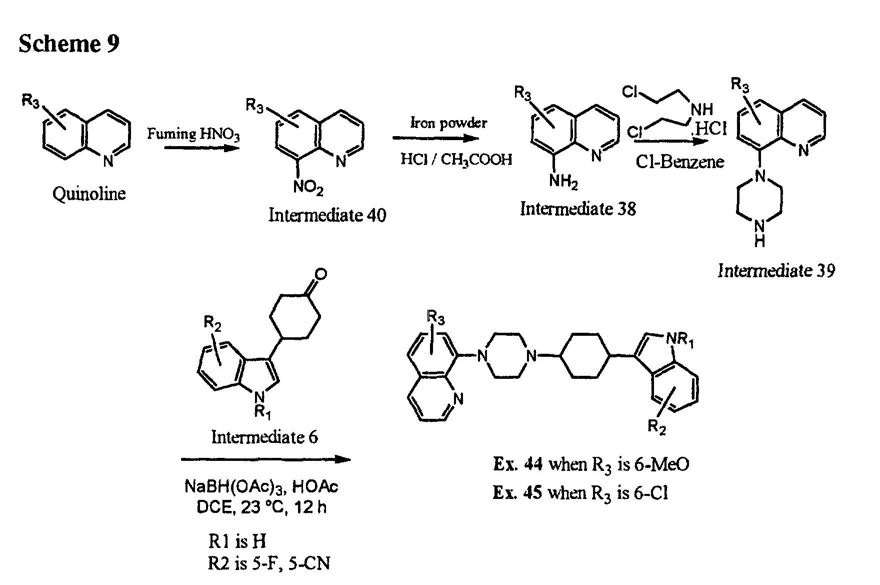

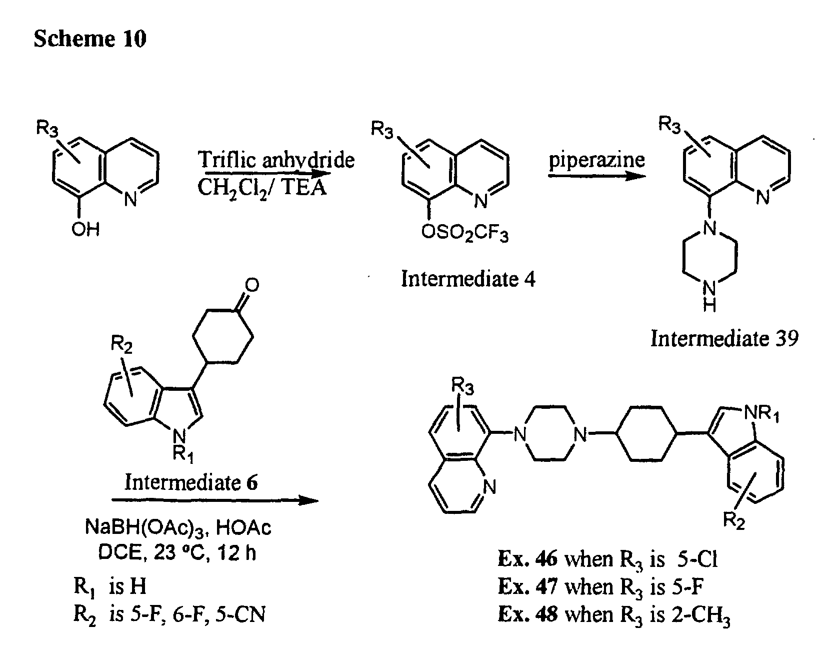

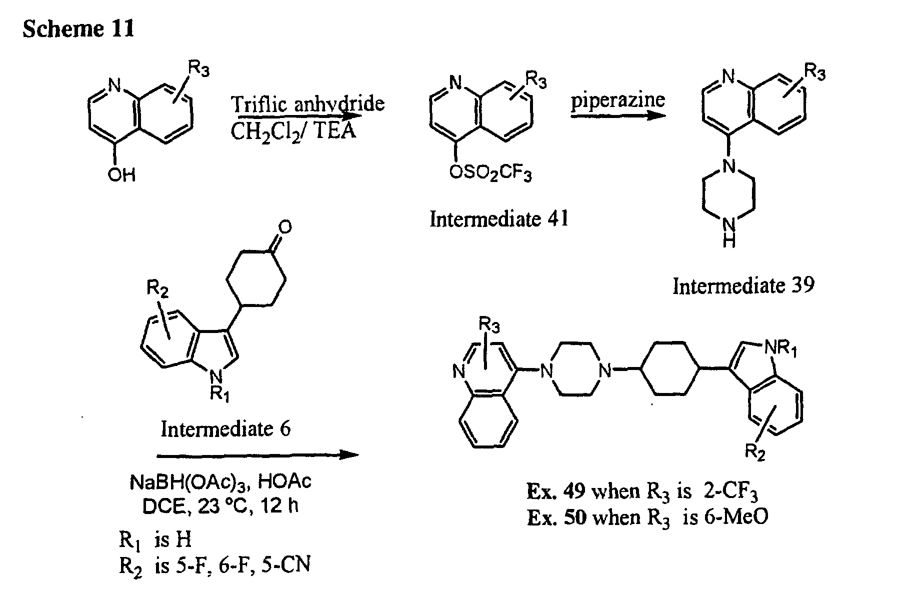

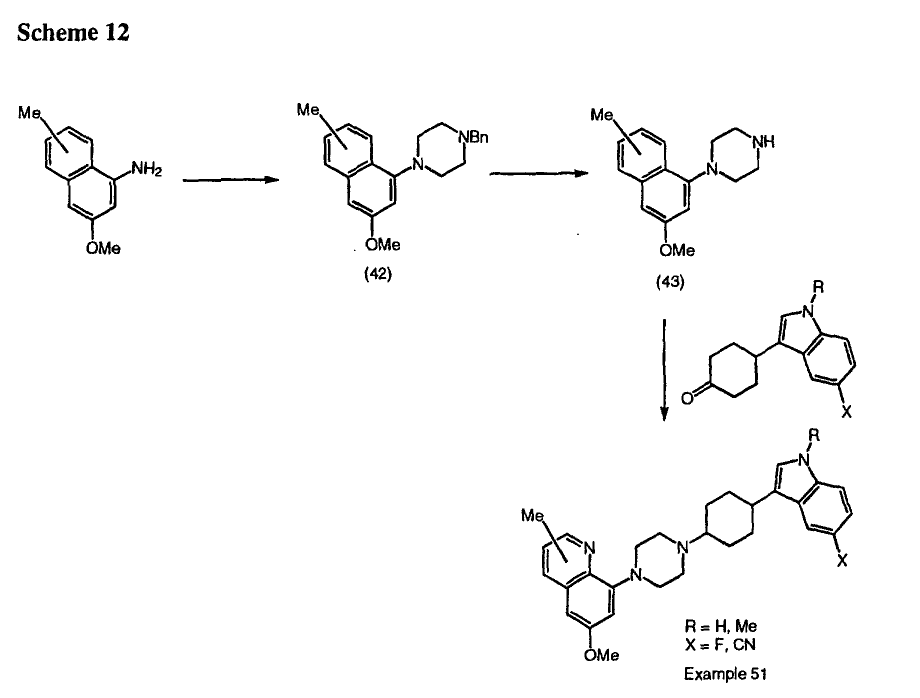

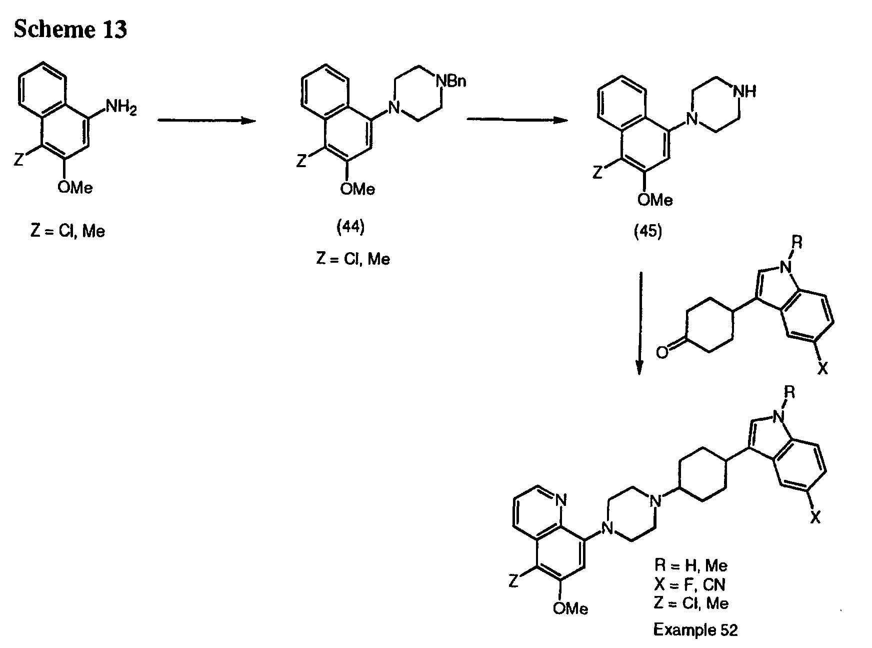

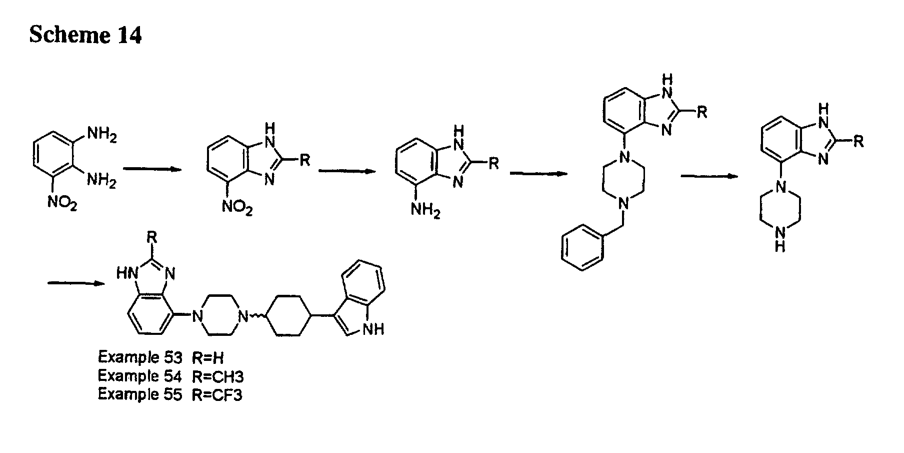

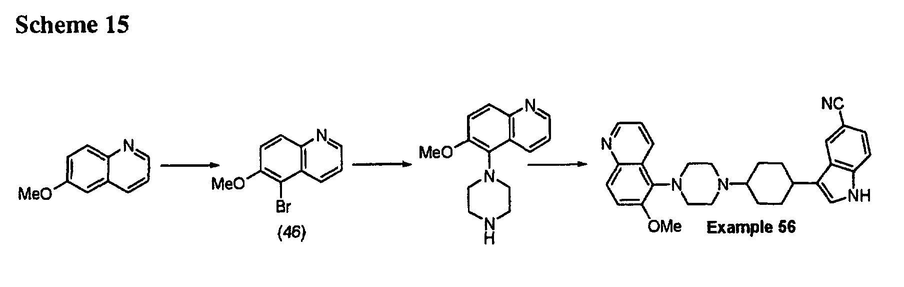

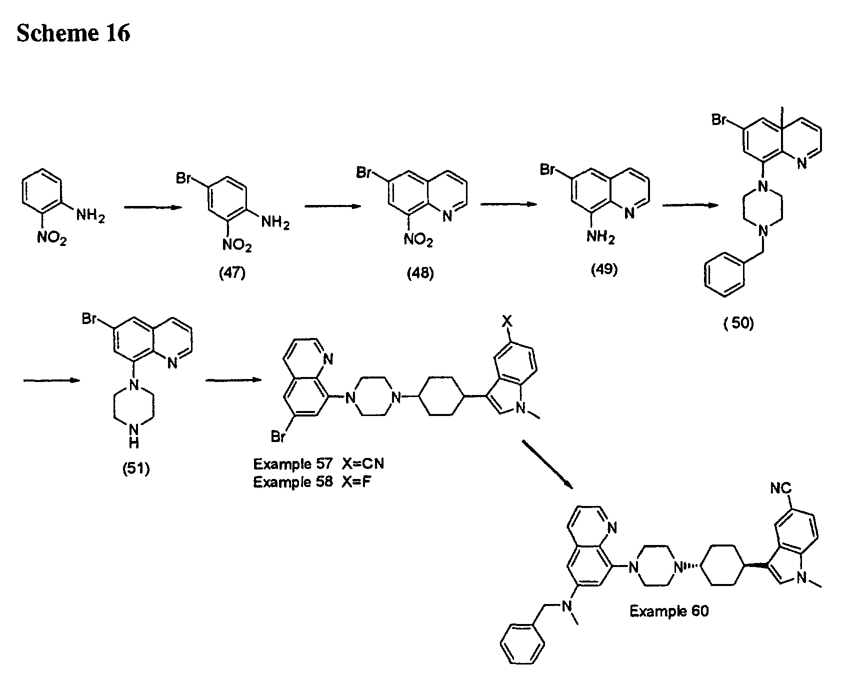

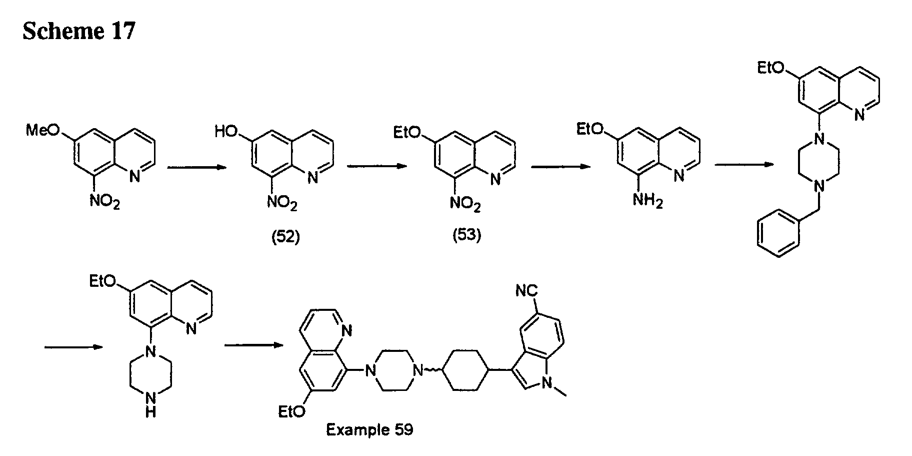

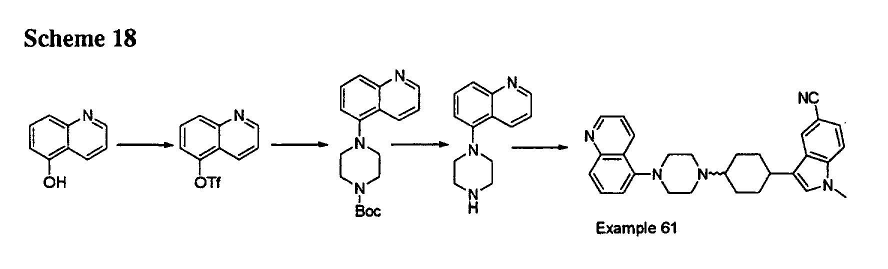

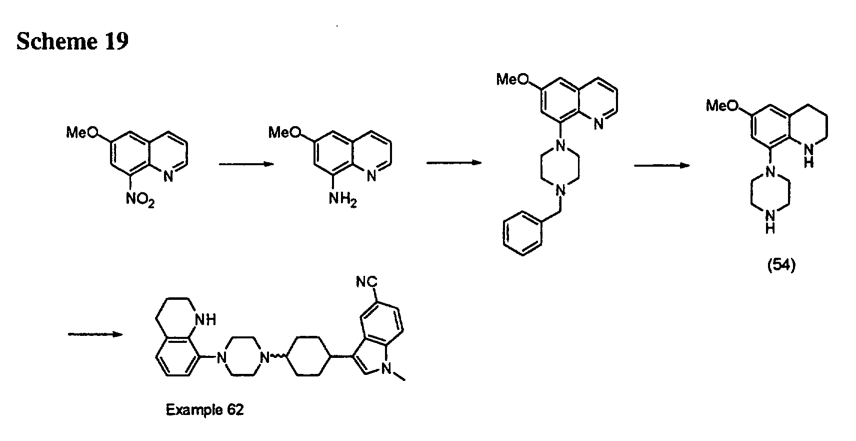

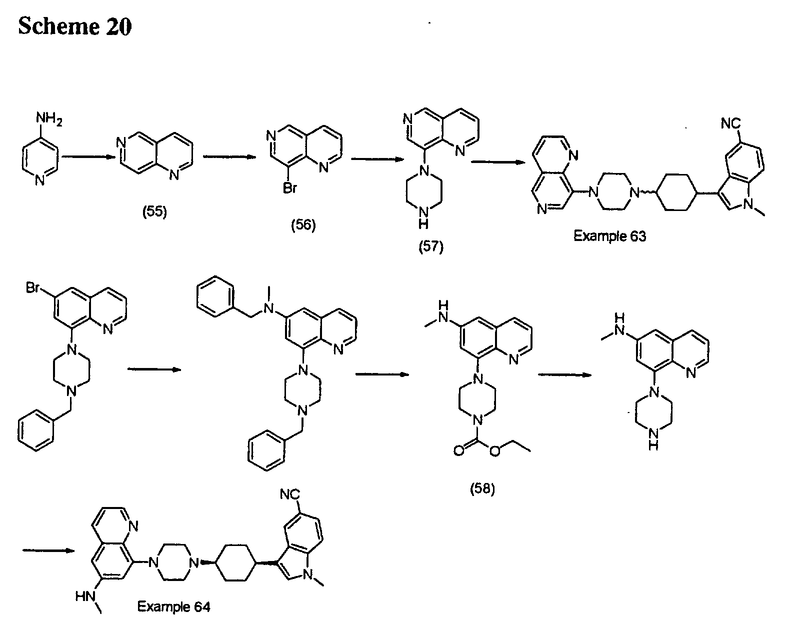

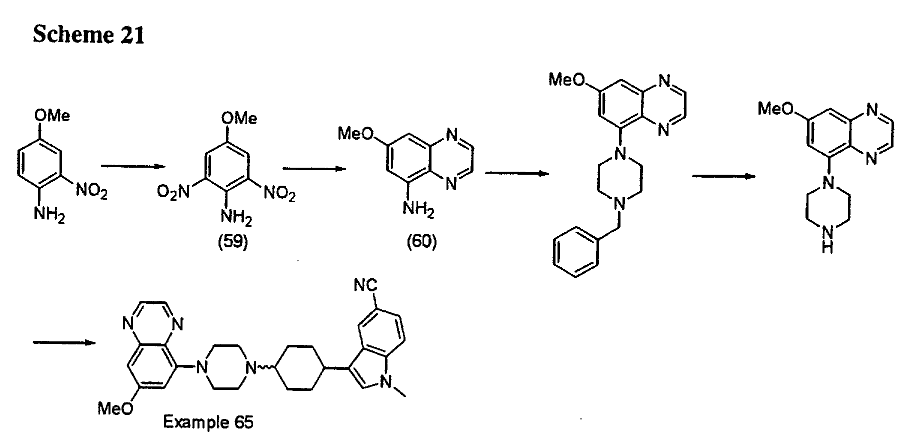

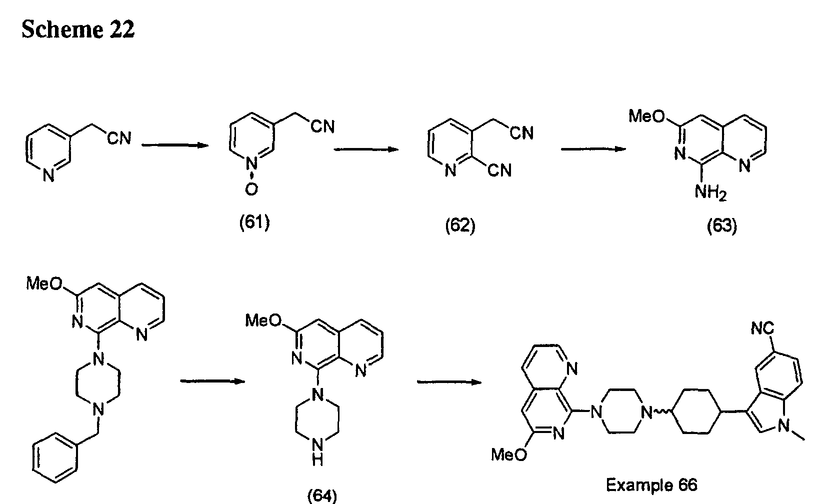

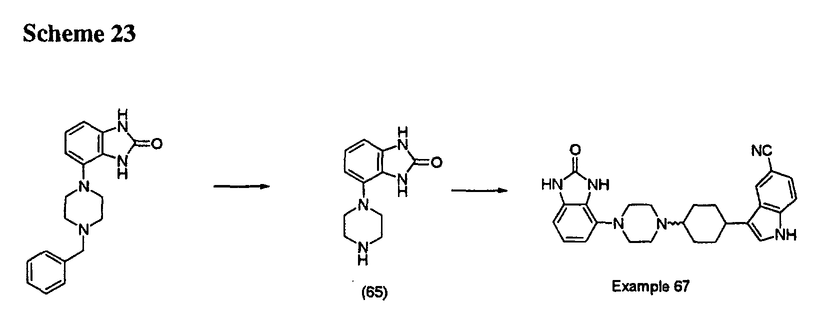

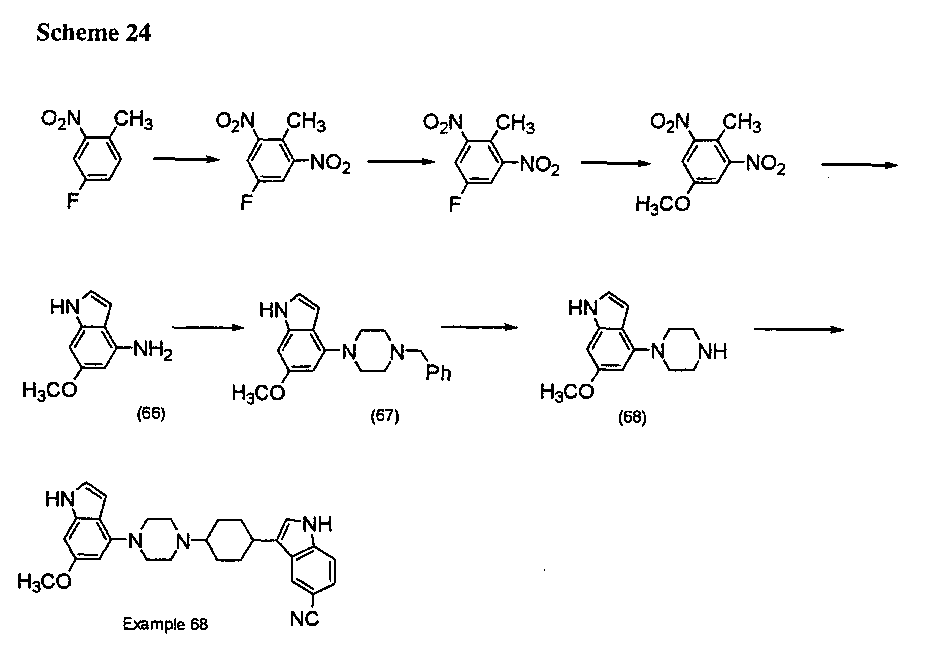

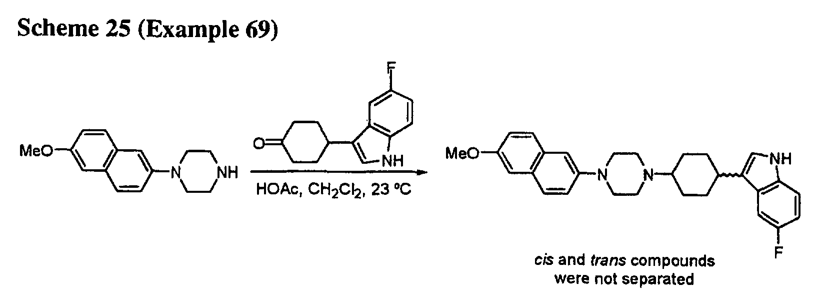

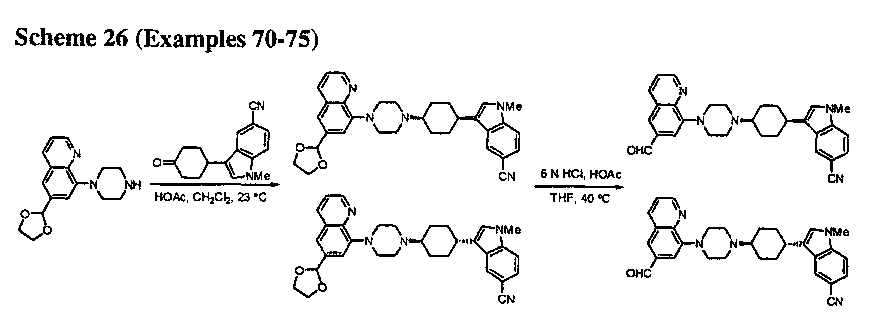

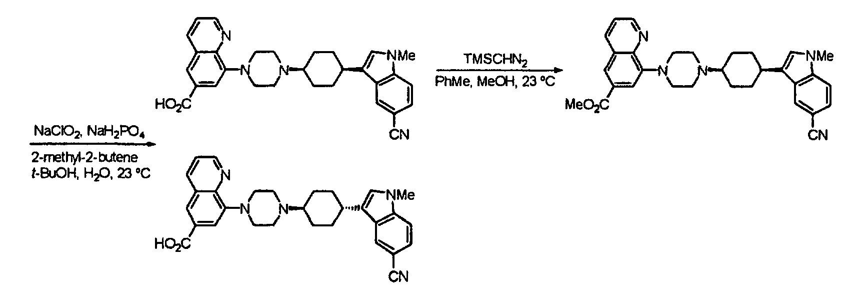

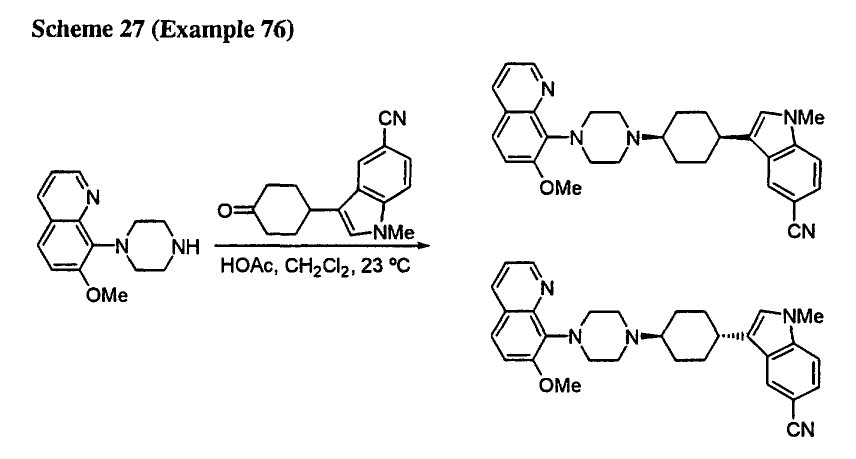

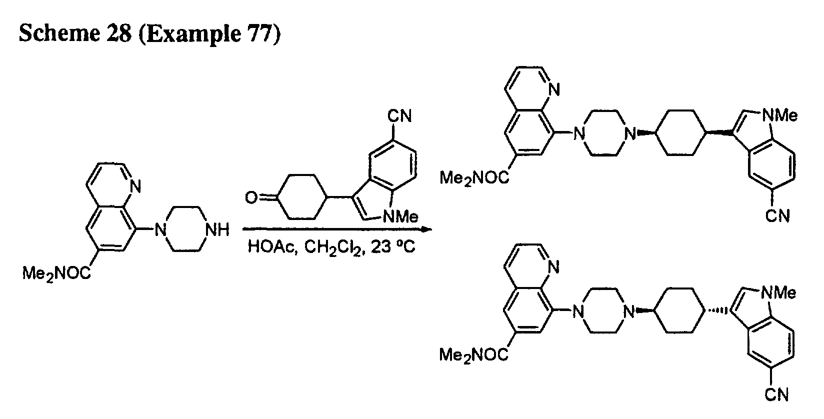

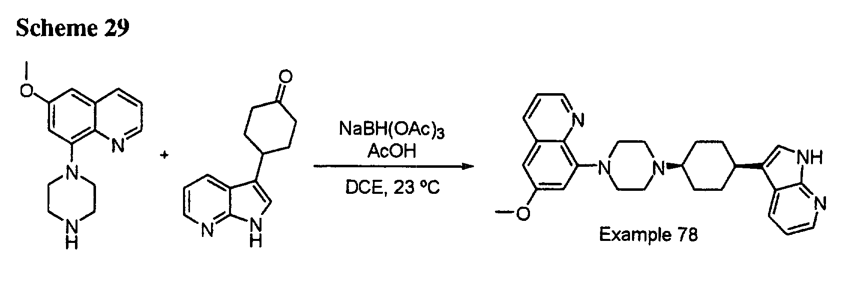

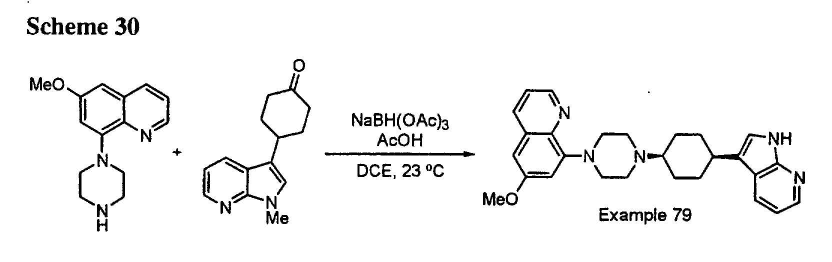

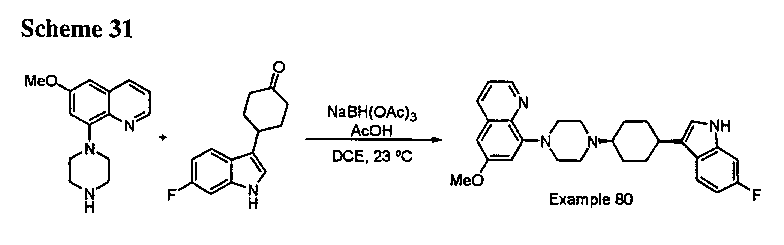

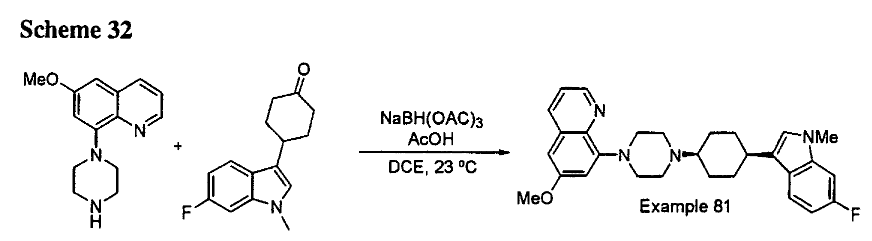

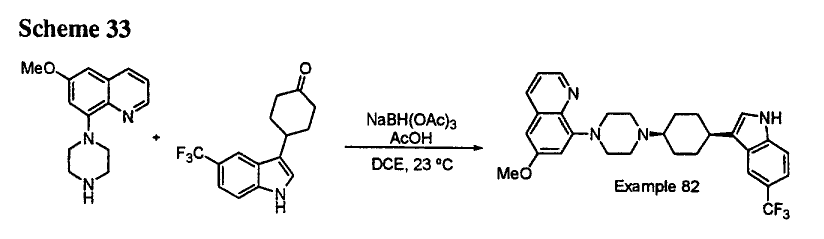

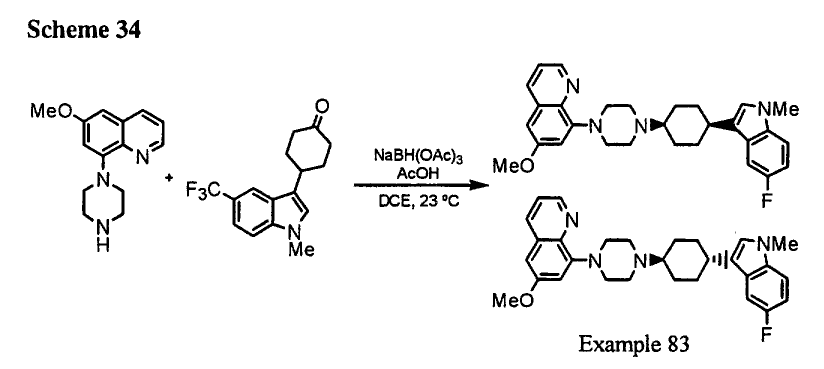

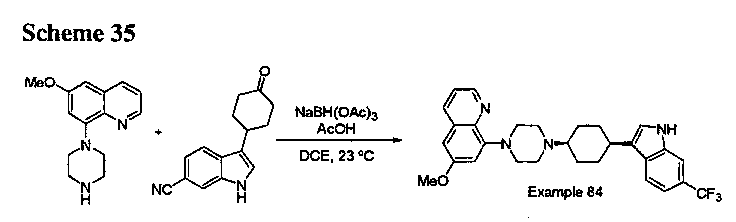

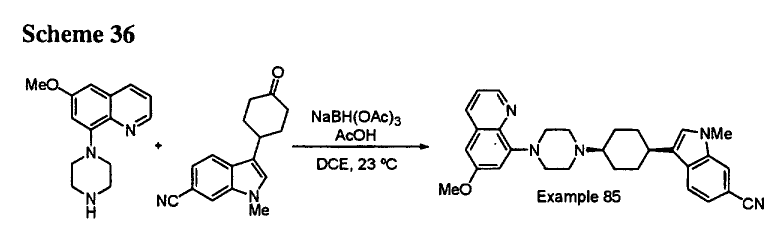

The following Schemes 37-39 were utilized to obtain the compounds of Examples 86-114.

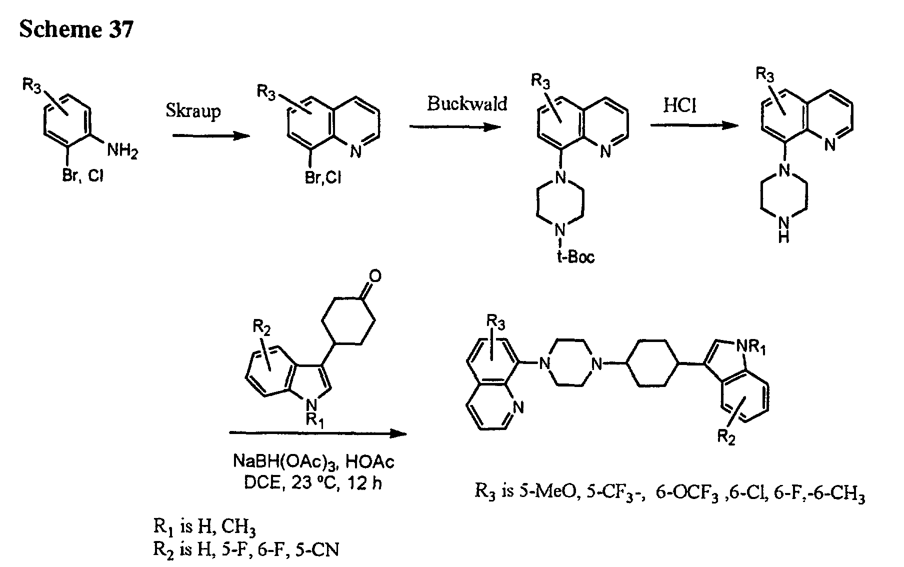

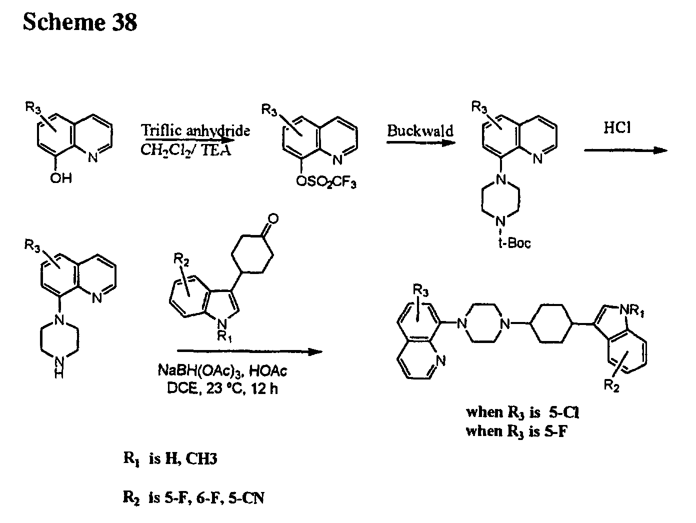

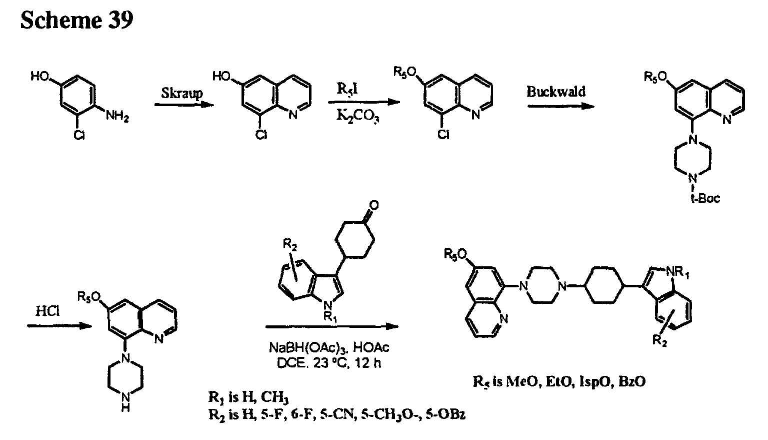

### INTERMEDIATE 1

### 3-(1,4-Dioxa-spiro[4,5]dec-7-en-8-yl)-1H-indole (1a)

Indole (4.69, 40 mmol), 1,4-cyclohexanedione monoethylene ketal (6.3 g, 40 mmol) and potassium hydroxide (13.2 g, 200 mmol) were heated to reflux in 70 ml methanol for 6 hours. The reaction was cooled and the product was isolated by filtration and washed with water to give 9.1 g (89%) of product.

### 3-(1,4-Dioxa-spiro[4,5]dec-7-en-8-yl)-4-fluoro-1H-indole (1b)

This compound was prepared in a similar fashion described above by replacing indole with 4-fluoroindole (3 g, 22 mmol) to afford the title compound in quantitative yield as a white solid: mp at 140°C (sublimated).

### 3-(1,4-Dioxa-spiro[4,5]dec-7-en-8-yl)-5-fluoro-1H-indole (1c)

5-Fluoroindole (4.96 g, 0.036 mol), 1,4-cyclohexanedione monoethylene ketal (7.17 g, 0.046 mol) and potassium hydroxide (6 g, 91 mmol) were heated to reflux in 70 ml methanol for 6 hours. The reaction was cooled and the product was isolated by filtration and washed with water to give 8.59 g (86%) of product as a white solid: mp 153-155°C.

### 3-(1,4-Dioxa-spiro[4,5]dec-7-en-8-yl)-6-fluoro-1H-indole (1d)

This compound was prepared in the manner described for intermediate 1a by replacing indole with 6-fluoroindole (5.14 g, 38 mmol) ) to afford 10 g (96.3 %) of the title compound as a white solid: mp 196-197°C.

| Elemental analysis for C₁₆H₁₆FNO₂ | | | |
|---|---|---|---|
| Calc'd | C, 70.32; | H, 5.90; | N, 5.13 |
| Found | C, 70.62; | H, 5.91; | N, 5.08 |

### 3-(1,4-Dioxa-spiro[4,5]dec-7-en-8-yl)-5-bromo-1H-indole (1e)

This compound was prepared in the manner described above for intermediate 1a by replacing indole with 5-bromoindole (7.84 g, 40 mmol) ) to afford 10.5 g (78 %) of the title compound as a white solid; MS EI *m*/*e* 333 (M⁺).

### 3-(1,4-Dioxa-spiro[4,5]dec-7-en-8-yl)-5-chloro-1H-indole (1f)

This compound was prepared in the manner described above for intermediate 1a by replacing indole with 5-chloroindole (5 g, 33 mmol) ) to afford 9.14 g (96 %) of the title compound as a white solid: mp 178-181°C; MS EI *m*/*e* 273 (M⁺).

### 3-(1,4-Dioxa-spiro[4,5]dec-7-en-8-yl)-5-cyano-1H-indole (1g)

This compound was prepared in the manner described above for intermediate 1a by replacing indole with 5-cyanoindole (29.98 g, 0.21 mol) to afford 29.32 g (50 %) of the title compound as a white solid: mp 158-160°C.

### 3-(1,4-Dioxa-spiro[4,5]dec-7-en-8-yl)-5-methoxy-1H-indole (1h)

This compound was prepared in the manner described above for intermediate 1a by replacing indole with 5 methoxy indole (5 g, 34 mmol) in 82% yield (7.95 g) as a white solid: mp 161-162°C

### 3-(1,4-Dioxa-spiro[4,5]dec-7-en-8-yl)-2-methyl-1H-indole (1i)

A solution of 2-methyl-indole (2.0 g, 15.2 mmol), 1,4-cyclohexanedione monoethylene ketal (4.76 g, 30.4 mmol) and potassium hydroxide (10 g, 0.18 mol) were heated to reflux in 50 ml methanol for 48 hours. The mixture was poured into water (150 ml) and extracted with methylene chloride (2 x 200 ml). The organic layer was dried over anhydrous magnesium sulfate, filtered, and solvent was removed under vacuum. Chromatography (25% ethyl acetate-hexanes) afforded a light tan solid which was washed with ethyl ether (20 ml) to afford 2.35 g (62%) of product as a white solid: mp 136-137°C.

| Elemental analysis for C₁₇H₁₉NO₂ | | | |
|---|---|---|---|
| Calc'd | C, 75.81; | H, 7.11; | N, 5.70 |
| Found | C, 75.47; | H, 7.26; | N, 5.13 |

### 3-(1,4-Dioxa-spiro[4,5]dec-7-en-8-yl) -1H-azaindole (1j)

This compound was prepared in the manner described above for intermediate 1a by replacing indole with 7-azaindole (3.65 g, 31 mmol) in 68% yield (5.42 g) as a white solid: mp 162-165°C; MS EI *m*/*e* 256 (M⁺).

### INTERMEDIATE 2

### 3-(1,4-Dioxa-spiro[4,5]dec-8-yl)-1H-indole (2a)

A mixture of 3-(1,4-dioxa-spiro[4,5]dec-7-en-8-yl)-1H-indole (8.0 g, 31.3 mmol) and 10% palladium on carbon (1.3 g) in ethanol (700 ml) was hydrogenated for 18 hours. The catalyst was filtered off and the solvent removed under vacuum to afford 8.01 g (99 %) of product as a white solid.

### 3-(1,4-Dioxa-spiro[4,5]dec-8-yl)-4-fluoro-1H-indole (2b)

This compound was prepared in the manner described above for intermediate 2a by replacing 3-(1,4-dioxa-spiro[4,5]dec-7-en-8-yl)-1H-indole with 3-(1,4-dioxa-spiro[4,5]dec-7-en-8-yl)-4-fluoro-1H-indole (6.3 g) ) to afford 4.44 g (70 %) of the title compound as a white solid: mp 161-162°C.

| Elemental analysis for C₁₆H₁₈FNO₂ | | | |
|---|---|---|---|
| Calc'd | C, 69.08; | H, 6.59; | N, 5.09 |
| Found | C, 69.05; | H, 6.56; | N, 4.87 |

### 3-(1,4-Dioxa-spiro[4,5]dec-8-yl)-5-fluoro-1H-indole (2c)

A mixture of of 3-(1,4-dioxa-spiro[4,5]dec-7-en-8-yl)-5-fluoro-1H-indole (8.5 g) and 10% palladium on carbon (2.72 g) in ethanol (200 ml) was hydrogenated for 5 hours. The catalyst was filtered off and the solvent removed under vacuum. Chromatography (methanol-methylene chloride) afforded 7.55 g (82 %) of product as a white solid: mp 183-185°C.

### 3-(1,4-Dioxa-spiro[4,5]dec-8-yl)-6-fluoro-1H-indole (2d)

This compound was prepared in the manner described above for intermediate 2a by replacing 3-(1,4-dioxa-spiro[4,5]dec-7-en-8-yl)-1H-indole with 3-(1,4-dioxa-spiro[4,5]dec-7-en-8-yl)-6-fluoro-1H-indole (9.54 g) to afford 5.83 g (60 %) of the title compound as a white solid: mp 158-159°C.

| Elemental analysis for C₁₆H₁₈FNO₂ | | | |
|---|---|---|---|
| Calc'd | C, 69.80; | H, 6.59; | N, 5.09 |
| Found | C, 69.74; | H, 6.48; | N, 5.13 |

### 3-(1,4-Dioxa-spiro[4,5]dec-8-yl)-5-bromo-1H-indole (2e)

A mixture of 3-(1,4-dioxa-spiro[4,5]dec-7-en-8-yl)-5-bromo-1H-indole (6.8 g, 20.34 mmol) and 5% platinum on carbon (5.0 g) in ethanol (500 ml) was hydrogenated overnight. The catalyst was filtered off and the solvent removed under vacuum. Chromatography (30% ethyl acetate-hexanes) afforded 5.0 g (73%) of product as a solid; MS EI *m*/*e* 336 (M⁺).

### 3-(1,4-Dioxa-spiro[4,5]dec-8-yl)-5-chloro-1H-indole (2f)

A mixture of 3-(1,4-dioxa-spiro[4,5]dec-7-en-8-yl)-5-chloro-1H-indole (0.18 g) and platinum oxide (0.02 g) in ethanol (20 ml) with ten drops of acetic acid was hydrogenated overnight. The catalyst was filtered off and the solvent removed under vacuum. Chromatography (25% ethyl acetate-hexanes) afforded 0.16 g (88 %) of product as a white solid: mp 205-206.5°C.

### 3-(1,4-Dioxa-spiro[4,5]dec-8-yl)-5-cyano-1H-indole (2g)

This compound was prepared in the manner described above for intermediate 2a by replacing 3-(1,4-dioxa-spiro[4,5]dec-7-en-8-yl)-1H-indole with 3-(1,4-dioxa-spiro[4,5]dec-7-en-8-yl)-5-cyano-1H-indole (54.6 g) ) to afford 52.12 g (95 %) of the title compound as a white solid in 95% (52.12 g) yield as a white solid: mp 153-155°C.

### 3-(1,4-Dioxa-spiro[4,5]dec-8-yl)-5-methoxy-1H-indole (2h)

This compound was prepared in the manner described above for intermediate 2a by replacing 3-(1,4-dioxa-spiro[4,5]dec-7-en-8-yl)-1H-indole with 3-(1,4-dioxa-spiro[4,5]dec-7-en-8-yl)-5-methoxy-1H-indole to afford 7.18 g (96 %) of the title compound as a white solid: mp 153-155°C.

### 3-(1,4-Dioxa-spiro[4,5]dec-8-yl)-2-methyl-1H-indole (2i)

A mixture of 3-(1,4-dioxa-spiro[4,5]dec-7-en-8-yl)-2-methyl-1H-indole (2.39 g, 8.9 mmol) and 10% palladium on carbon (0.35 g) in ethanol (80 ml) was hydrogenated for 3 hours. The catalyst was filtered off and then a solution of methylene-methanol (80 ml) was used to dissolve any solids within the celite. The solvent removed under vacuum to afford 2.34 g (97 %) of product as an off-white solid, which was triturated with ethyl ether (40 ml) to afford a white solid: mp 166-168°C. The mother liquor was concentrated to afford another 1.2 g of product as a yellow solid.

| Elemental analysis for C₁₇H₂₁NO₂ | | | |
|---|---|---|---|
| Calc'd | C, 75.25; | H, 7.80; | N, 5.16 |
| Found | C, 75.17; | H, 7.99; | N, 5.12 |

### 3-(1,4-Dioxa-spiro[4,5]dec-8-yl)-1H-azaindole (2j)

This compound was prepared in the manner described above for intermediate 2a by replacing 3-(1,4-dioxa-spiro[4,5]dec-7-en-8-yl)-1H-indole (7.18 g) with 3-(1,4-dioxa-spiro[4,5]dec-7-en-8-yl)-1H-azaindole (4.02 g) to afford 2.7 g (67 %) of the title compound as a white solid: mp 204-207°C.

| Elemental analysis for C₁₃H₁₄N₂O | | | |
|---|---|---|---|
| Calc'd | C, 72.87; | H, 6.59; | N, 13.07 |
| Found | C, 72.44; | H, 6.75; | N, 12.81 |

### INTERMEDIATE 3

### 4-(1H-3-Indolyl)-cyclohexanone (3a)

A solution of 3-(1,4-dioxa-spiro[4,5]dec-8-yl)-1H-indale (2.57 g, 10 mmol) in 200 ml (1:1) tetrahydrofuran-hydrochloric acid (1N) was allowed to stir at room temperature for 16 hours. The solvent was evaporated under vacuum. The crude product was dissolved in ethyl acetate, washed with 1N sodium hydroxide (3 x 150 ml). The organic layer was dried over anhydrous sodium sulfate, and filtered. Chromatography (40% ethyl acetate-hexanes) afforded 1.9 g (89%) of product.

### 4-(4-Fluoro-1H-3-indolyl)-cyclohexanone (3b)

This compound was prepared in the manner described above for 3a by replacing 3-(1,4-dioxa-spiro[4,5]dec-8-yl)-1H-indole with 3-(1,4-dioxa-spiro[4,5]ec-8-yl)-4-fluoro-1H-indole (4.0 g) to afford 3.7 g (63 %) of the title compound as a white solid: mp 104-106°C.

### 4-(5-Fluoro-1H-3-indolyl)-cyclohexanone (3c)

A solution of 3-(1,4-dioxa-spiro[4,5]dec-8-yl)-5-fluoro-1H-indole (2.8 g, 10 mmol) in 200 ml(1:1) tetrahydrofuran-hydrochloric acid (1N) was allowed to stir at room temperature for 16 hours. The solvent was evaporated under vacuum. The crude product was dissolved in ethyl acetate, washed with 1N sodium hydroxide (3 x 150 ml). The organic layer was dried over anhydrous sodium sulfate, and filtered. Chromatography (40% ethyl acetate-hexanes) afforded 2.1 g (91%) of product as yellow solid: mp 112-114°C.

### 4-(6-Fluoro-1H-3-indolyl)-cyclohexanone (3d)

This compound was prepared in the manner described above for intermediate 3a by replacing 3-(1,4-dioxa-spiro[4,5]dec-8-yl)-1H-indole with 3-(1,4-dioxa-spiro[4,5]dec-8-yl)-6-fluoro-1H-indole (5.4 g) to afford 19.29 g (99 %) of the title compound as a white solid: mp 102-105°C.

| Elemental analysis for C₁₄H₁₄NOF | | | |
|---|---|---|---|
| Calc'd | C, 72.71; | H, 6.10; | N, 6.06 |
| Found | C, 72.77; | H, 5.98; | N, 5.96 |

### 4-(5-Bromo-1H-3-indolyl)-cyclohexanone (3e)

This compound was prepared in the manner described above for intermediate 3a by replacing 3-(1,4-dioxa-spiro[4,5]dec-8-yl)-1H-indole with 3-(1,4-dioxa-spiro[4,5]dec-8-yl)-5-bromo-1H-indole (4.5 g) to afford 3.3 g (84 %) of the title compound as a white solid: MS EI *m*/*e* 291 (M⁺).

| | | | |
|---|---|---|---|
| Calc'd | C, 75.25; | H, 7.80; | N, 5.16 |
| Found | C, 75.17; | H, 7.99; | N, 5.12 |

### 4-(5-Chloro-1H-3-indolyl)-cyclohexanone (3f)

This compound was prepared in the manner described above for intermediate 3a by replacing 3-(1,4-dioxa-spiro[4,5]dec-8-yl)-1H-indole with 3-(1,4-dioxa-spiro[4,5]dec-8-yl)-5-chloro-1H-indole (2.12 g) to afford 1.13 g (60 %) of the title compound as a clear oil: MS FAB *m*/*e* 248 (M + H)⁺.

### 4-(5-Cyano-1H-3-indolyl)-cyclohexanone (3g)

This compound was prepared in the manner described above for intermediate 3a by replacing 3-(1,4-dioxa-spiro[4,5]dec-8-yl)-1H-indole with 3-(1,4-dioxa-spiro[4,5]dec-8-yl)-5-cyano-1H-indole (6 g) to afford 4.03 g (81 %) of the title compound as a white solid: mp 162.5-164°C.

| Elemental analysis for C₁₅H₁₄N₂O | | | |
|---|---|---|---|
| Calc'd | C, 75.61; | H, 5.92; | N, 11.76 |
| Found | C, 75.82; | H, 6.06; | N, 11.72 |

### 4-(5-Methoxy-1H-3-indolyl)-cyclohexanone (3h)

This compound was prepared in the manner described above for intermediate 3a by replacing 3-(1,4-dioxa-spiro[4,5]dec-8-yl)-1H-indole with 3-(1,4-dioxa-spiro[4,5]dec-8-yl)-5-methoxy-1H-indole (5.85 g) to afford 4.2 g (85 %) of the title compound as a white solid: mp 103-106°C.

### 4-(2-Methyl-1H-3-indolyl)-cyclohexanone (3i)

This compound was prepared in the manner described above for intermediate 3a by replacing 3-(1,4-dioxa-spiro[4,5]dec-8-yl)-1H-indole with 3-(1,4-dioxa-spiro[4,5]dec-8-yl)-2-methyl-1H-indole (2.2 g) to afford 1.62 g (88 %) of the title compound as a yellow thick oil: MS EI *m*/*e* 227 (M⁺).

### 4-(1H-3-pyrrolo[2,3-b]pyridyl)-cyclohexanone (3j)

This compound was prepared in the manner described above for intermediate 3a by replacing 3-(1,4-dioxa-spiro[4,5]dec-8-yl)-1H-indole with 3-(1,4-dioxa-spiro[4,5]dec-8-yl)-1H-azaindole (2.48 g) to afford 1.96 g (95 %) of the title compound as a white solid: mp 162-164°C.

### INTERMEDIATE 4

### 3-(1,4-Dioxa-spiro[4,5]dec-7-en-8-yl)-5-cyano-1-methyl-indole

To a suspension of sodium hydride (60%, 1.74 g, 0.073 mol) in anhydrous N,N-dimethylformamide (100 ml) was added 3-(1,4-dioxa-spiro[4,5]dec-7-en-8-yl)-5-cyano-1H-indole ( 9.9 g, 0.035 mol) at room temperature. The mixture was stirred for 30 minutes at room temperature, then methyl iodide (9 ml, 0.14 mol) was added at room temperature. The reaction was allowed to stir for 1 hour, then quenched with water (50 ml). The mixture was extracted with methylene chloride (3 x 150 ml) and water (3 x 150 ml). The organic layer was dried over anhydrous magnesium sulfate and filtered. The solvent was removed under vacuum. Chromatography (5% methanol-methylene chloride) afforded 2.54 g (24%) of product as a light yellow solid: mp 65-67°C.

| Elemental analysis for C₁₈H₁₈N₂O₂ | | | |
|---|---|---|---|
| Calc'd | C, 73.45; | H, 6.16; N, | 9.52 |
| Found | C, 73.17; | H, 6.24; | N, 9.43 |

### INTERMEDIATE 5

### 3-(1,4-Dioxa-spiro[4,5]dec-8-yl)-5-cyano-1-methyl-indole (5a)

A mixture of 3-(1,4-dioxa-spiro[4,5]dec-7-en-8-yl)-5-bromo-1H-indole (3.77 g) and 10% palladium on carbon (0.99 g) in ethanol-tetrahydrofuran (200 : 80 ml) was hydrogenated for 5 hours. The catalyst was filtered off and the solvent was removed under vacuum to afford a white powder which was washed with ethanol-hexanes (1:1) and dried under vacuum for 4 hours to afford 2.75 g (12%) of product: mp 170-172°C.

| Elemental analysis for C₁₈H₂₀N₂O₂ | | | |
|---|---|---|---|
| Calc'd | C, 72.95; | H, 6.80; | N, 9.45 |
| Found | C, 72.79; | H, 6.82; | N, 9.35 |

### 3-(1,4-Dioxa-spiro[4,5]dec-8-yl)-5-cyano-1-ethyl-indole (5b)

To a suspension of sodium hydride (60%, 1.63 g, 0.068 mol) in anhydrous N,N-dimethylformamide (150 ml) was added 3-(1,4-dioxa-spiro[4,5]dec-8-yl)-5-cyano-1H-indole ( 9.0 g, 0.032 mol) at room temperature. The mixture was stirred for 30 minutes at room temperature then ethylbromide (14.6 g, 0.13 mol) was added at room temperature. The reaction was allowed to stir for overnight, then quenched with water (50 ml). The mixture was extracted with methylene chloride (3 x 150 ml) and water (3 x 150 ml). The organic layer was dried over anhydrous magnesium sulfate and filtered. The solvent was removed under vacuum. Chromatography (hexanes) afforded 5.5 g (69%) of product as a white solid: mp 124-126°C.

| Elemental analysis for C₁₉H₂₂N₂O₂ | | | |
|---|---|---|---|
| Calc'd | C, 73.52; | H, 7.14; | N, 9.02 |
| Found | C, 73.56; | H, 6.93; | N, 8.95 |

### 3-(1,4-Dioxa-spiro[4,5]dec-8-yl)-5-cyano-1-n-propyl-indole (5c)

This compound was prepared in the manner described above for intermediate 5b by replacing ethylbromide with n-propylbromide (13.1 g, 11 mmol) to afford 4.33 g (75 %) of the title compound as a oil: MS EI *m*/*e* 324 (M⁺).

### 3-(1,4-dioxa-spiro[4,5]dec-8-yl)-5-cyano-1-iso-propyl-indole (5d)

This compound was prepared in the manner described above for intermediate 5b by replacing ethylbromide with isopropylbromide (10.2 g, 83 mmol) in 62% yield (6.44 g) as a white solid: mp 114.5-116°C; MS EI *m*/*e* 324 (M⁺).

### 3-(1,4-dioxa-spiro[4,5]dec-8-yl)-5-cyano-1-benzyl-indole (5e)

This compound was prepared in the manner described above for intermediate 5b by replacing ethylbromide with benzylbromide (14.3 g, 84 mmol) to afford 6.04 g (57 %) of the title compound as a white solid: mp 129-130°C.

| Elemental analysis for C₂₃H₂₄N₂O₂ | | | |
|---|---|---|---|
| Calc'd | C, 77.39; | H, 6.50; | N, 7.52 |
| Found | C, 76.59; | H, 6.28; | N, 7.47 |

### INTERMEDIATE 6

### 4-(5-Cyano-1-methyl-3-indolyl)-cyclohexanone (6a)

A solution of 3-(1,4-dioxa-spiro[4,5]dec-8-yl)-5-cyano-1-methyl-indole (5.5 g) in 150 ml (1:1) tetrahydrofuran-hydrochloric acid (1N) was allowed to stir at room temperature for 16 hours, followed by the addition of 4.49 g sodium bicarbonate. The mixture was extracted with methylene chloride (3 x 100 ml), washed with brine (3 x 150 ml). The organic layer was dried over anhydrous magnesium sulfate and filtered. The solvent was removed to afford a light brown solid which was boiled in ethyl acetate-hexanes (1:1). The mixture was cooled to room temperature and solid was collected and dried under vacuum to afford 2.06 g of the title compound as a solid: mp 150-152°C.

| Elemental analysis for C₁₅H₁₅N₂O | | | |
|---|---|---|---|
| Calc'd | C, 76.16; | H, 6.39; | N, 11.10 |
| Found | C, 75.84; | H, 6.34; | N, 10.92 |

### 4-(5-Cyano-1-ethyl-3-indolyl)-cyclohexanone (6b)

This compound was prepared in the manner described above for intermediate 6a by replacing 3-(1,4-dioxa-spiro[4,5]dec-8-yl)-5-cyano-1-methyl-indole with 3-(1,4-dioxa-spiro[4,5]-dec-8-yl)-5-cyano-1-ethyl-indole (6.77 g, 22 mmol) to afford 4.33 g (75 %) of the title compound as a white solid: mp 124°C.

| Elemental analysis for C₁₇H₁₈N₂O | | | |
|---|---|---|---|
| Calc'd | C, 76.66; | H, 6.81; | N, 10.52 |
| Found | C, 76.30; | H, 6.82; | N, 10.25 |

### 4-(5-Cyano-1-n-propyl-3-indolyl)-cyclohexanone (6c)

This compound was prepared in the manner described above for intermediate 6a by replacing 3-(1,4-dioxa-spiro[4,5]dec-8-yl)-5-cyano-1-methyl-indole with 3-(1,4-dioxa-spiro[4,5]-dec-8-yl)-5-cyano-1-n-propyl-indole (2.64 g, 8.2 mmol) to afford 1.67 g (73 %) of the title compound as a white solid: mp 103-104°C; MS EI *m*/*e* 280 (M⁺).

### 4-(5-Cyano-1-benzyl-3-indolyl)-cyclohexanone (6d)

This compound was prepared in the manner described above for intermediate 6a by replacing 3-(1,4-dioxa-spiro[4,5]dec-8-yl)-5-cyano-1-methyl-indole with 3-(1,4-dioxa-spiro[4,5]-dec-8-yl)-5-cyano-1-benzyl-indole (6.43 g, 20 mmol) to afford 3.49 g (63 %) of the title compound as a white solid: mp 115-126°C.

| Elemental analysis for C₂₂H₂₀N₂O | | | |
|---|---|---|---|
| Calc'd | C, 80.46; | H, 6.14; | N, 8.53 |
| Found | C, 80.42; | H, 6.07; | N, 8.49 |

### 4-(5-Cyano-1-isopropyl-3-indolyl)-cyclohexanone (6e)

This compound was prepared in the manner described above for intermediate 6a by replacing 3-(1,4-dioxa-spiro[4,5]dec-8-yl)-5-cyano-1-methyl-indole with 3-(1,4-dioxa-spiro[4,5]-dec-8-yl)-5-cyano-1-isopropyl-indole (5.86 g, 16 mmol) to afford 3.46 g (63 %) of the title compound as a white solid: mp 106-107°C.

| Elemental analysis for C₁₈H₂₀N₂O | | | |
|---|---|---|---|
| Calc'd | C, 77.11; | H, 7.19; | N, 9. |
| Found | C, 76.85; | H, 7.16; | N, 9. |

### INTERMEDIATE 7

### 8-(4-Benzyl-piperazin-1-yl)quinoline

A solution of 8-amino-quinoline (12.91 g, 89 mmol) and bis(2-chloroethyl)-benzylamine (25.95 g, 112 mmol) in n-butanol (65 ml) was allowed to heat at 85°C for 11 hours. The mixture was poured into 50% sodium hydroxide, extracted with methylene chloride and water. The organic layer was dried over anhydrous magnesium sulfate, and filtered. The solvent was removed under vacuum. Chromatography (methanol-methylene chloride) afforded 12.34 g of product as a solid: mp 116.5-118°C.
The HCl salt was prepared in ethyl acetate: mp 249-210°C.

| Elemental analysis for C₂₀H₂₁N₃•2HCl•0.5H₂O | | | |
|---|---|---|---|
| Calc'd | C, 62.34; | H, 6.28; | N, 10.91 |
| Found | C, 62.37; | H, 6.55; | N, 10.80 |

### INTERMEDIATE 8

### 8-(Piperazin-1-yl)-quinoline

To a solution of 8-(4-benzyl-piperazin-1-yl)quinoline (2.63 g, 8.7 mmol) in methylene chloride (30 ml) was added vinyl chloroformate (1.1 ml, 13 mmol) at room temperature slowly. The reaction mixture was refluxed for 2 hours, and then concentrated under vacuum. The residue was dissolved in 12 N hydrochloric acid (20 ml) and stirred at room temperature for 1 hour. The mixture was concentrated, the residue was taken up with 40 ml ethanol and heated up to 50°C for 2 hours. The solvent was removed under vacuum, the residue was dissolved in 1 N sodium hydroxide-ethyl acetate and extracted with ethyl acetate and washed with water. The organic layer was dried over anhydrous sodium sulfate. The solvent was removed under vacuum. Chromatography (10-30% methanol -methylene chloride plus ammonium hydroxide) afforded 1.86 g (90%) yellow oil; MS EI *m*/*e* 213 (M)⁺.

### INTERMEDIATE 9

### 6-Fluorochroman

A mixture of 6-fluoro-4-oxo-chroman (2 g, 12 mmol) and 10% palladium on carbon (1 g) in concentrated hydrochloric acid (20 ml) and ethanol (30 ml) was hydrogenated for 20 hours. The catalyst was filtered and the solvent removed under vacuum. The residue was dissolved in ethyl acetate (100 ml), washed with 1N NaOH (6 x 200 ml) and water (3 x 150 ml), dried over anhydrous sodium sulfate, filtered and the solvent was removed under vacuum. Chromatography (20% ethyl acetate-hexanes) afforded 1.41 g (77%) of product as a clear oil; MS EI *m*/*e* 152 (M⁺).

### INTERMEDIATE 10

### 6-Fluoro-8-nitrochroman

A mixture of nitric acid (100%, 7.8 ml, 0.16 mol) in acetic anhydride was maintained at room temperatue for 0.5 hour. This mixture was added to a solution of 6-fluorochroman (11.9 g, 0.078 mol) in 40 ml acetic anhydride at 0°C. The reaction mixture was stirred at room temperature for 2 hours then poured into ice-water. The mixture was extracted with methylene chloride (3 x 60 ml) and washed with saturated sodium carbonate (8 x 150 ml). The organic layer was dried over anhydrous sodium sulfate and filtered. The solvent was removed under vacuum to afford a yellow solid: mp 48-50°C.

| Elemental analysis for C₉H₈FNO₃ | | | |
|---|---|---|---|
| Calc'd | C, 54.83; | H, 4.09; | N, 7.10. |
| Found | C, 54.78; | H, 3.93; | N, 6.09 |

### INTERMEDIATE 11

### 6-Fluoro-8-aminochroman

A mixture of 6-fluoro-8-nitrochroman (14.4 g) and 10% palladium on carbon (2 g) in ethanol (160 ml) was hydrogenated for 2 hours. The catalyst was filtered off and the solvent removed under vacuum. Chromatography (30% ethyl acetate-hexanes) afforded 12.12 g (100 %) of product as a clear oil; MS EI *m*/*e* 167 (M⁺).

### INTERMEDIATE 12

### 1-Benzyl-4-(6-fluoro-chroman-8-yl)-piperazine

A solution of 6-fluoro-8-aminochroman (1.24 g, 7.4 mmol) and bis(2-chloroethyl)-benzylamine (2.58 g, 11 mmol) in butanol (20 ml) was stirred at 100°C for 10 hours. The mixture was poured into saturated sodium carbonate (950 ml) and extracted with ethyl acetate (3 x 60 ml). The organic layer was dried over anhydrous sodium sulfate and filtered. Chromatography (20% ethyl acetate-hexanes) afforded 1.64 g (68%) of product as an oil; MS EI *m*/*e* 326 (M)⁺.

### INTERMEDIATE 13

### 4-(6-Fluoro-chroman-8-yl)-piperazine

A mixture of 1-benzyl-4-(6-fluoro-chroman-8-yl)-piperazine (1.64 g, 5 mmol), 10% palladium on carbon (0.4 g) and ammonium formate (0.64 g, 10 mmol) in ethanol (20 ml) was allowed to refux for 2 hours. The catalyst was filtered off and the solvent removed under vacuum. Chromatography (10-20% methanol-methylene chloride plus ammonium hydroxide) afforded 1.0 g (84 %) of product as a yellow oil; MS EI *m*/*e* 296 (M⁺).

### INTERMEDIATE 14

### 2-(4-Fluorophenoxy)-acetaldehyde diethyl acetal

To a suspension of sodium hydride (5.4 g, 0.134 mol) in anhydrous N,N-dimethylformamide (100 ml) was added 4-fluorophenol (10 g, 0.089 mol) at 0°C. After hydrogen evolution had ceased, bromo-acetaldehyde diethyl acetal (16 ml, 0.11 mol) was added. The reaction was heated at 160-170°C for 18 hours. The mixture was poured into ice-water, extracted with ethyl acetate (3 x 150 ml), washed with 1N sodium hydroxide (3 x 100 ml), and brine (3 x 100 ml). The organic layer was dried over anhydrous sodium sulfate and filtered. The solvent was removed under vacuum. Chromatography (25% ethyl acetate-hexanes) afforded 16.36 g (80%) of product as a clear oil: MS EI *m*/*e* 228 (M⁺).

### INTERMEDIATE 15

### 5-Fluorobenzofuran

To a mixture of benzene (200 ml) containing polyphosphoric acid (7.9 g, 0.035 mol) was added 2-(4-fluoro-phenoxy)-acetaldehyde diethyl acetal (8 g, 0.035 mol). The mixture was stirred vigorously while being heated to reflux for 2.5 hours. The reaction mixture was cooled to room temperature and decanted from the polyphosphoric acid. The solvent was removed under vacuum. Chromatography (5% ethyl acetate-hexanes) afforded 3.4 g (45%) of product as a clear oil: ¹H NMR (CDCl₃) δ 6.74 (dd, 1H, J = 2.0, 0.6 Hz), 7.01 (td, 1H, J = 9, 2.7 Hz), 7.25 (dd, 1H, J = 8.4, 2.7 Hz), 7.43 ( dd, 1H, J = 9, 3.9 Hz), 7.65 (d, 1H, J = 1.8 Hz).

### INTERMEDIATE 16

### 5-Fluoro-2,3-dihydrobenzofuran

A solution of 5-fluorobenzofuran and 10% palladium on carbon in acetic acid (25 ml) was hydrogenated under 50 psi for 12 hours. The catalyst was filtered through celite and the celite was washed with methylene chloride (200 ml). The organic layer was washed sequentially with 1N NaOH (3 x 100 ml), brine (3 x 100 ml) and dried over anhydrous sodium sulfate and filtered. The solvent was removed under vacuum to afforded 2.59 g (85%) of product as a clear oil: ¹H NMR (300 MHz, CDCl₃): δ 3.12 (t, 2H, J = 8.7 Hz), 4.58 (t, 2H, J = 8.7 Hz), 6.68 (dd, 1H, J = 8.7, 4.2 Hz), 6.79 (tm, 1H, J = 8.7 Hz), 6.89 (dm, 1H, J = 8.1 Hz).

### INTERMEDIATE 17

### 5-Fluoro-7-nitro-2,3-dihydrobenzofuran

A mixture of nitric acid (100%, 1.5 ml, 36 mmol) in acetic anhydride (18 ml) was maintained at room temperatue for 0.5 hour. The mixture was added to a solution of 5-fluoro-2,3-dihydrobenzofuran (2.5 g, 18 mmol) in 10 ml acetic anhydride at 10°C. The reaction mixture was stirred at room temperature for 2 hours then poured into ice-water. The mixture was extracted with methylene chloride (3 x 60 ml), washed with 1N sodium hydroxide (5 x 100 ml) and brine (200 ml). The organic layer was dried over anhydrous sodium sulfate and filtered. The solvent was removed under vacuum to afford a yellow solid: mp 113-114°C.

| Elemental analysis for C₈H₆NO₃ | | | |
|---|---|---|---|
| Calc'd | C, 52.47; | H, 3.30; | N, 7.65 |
| Found | C, 52.40; | H, 3.21; | N, 7.39 |

### INTERMEDIATE 18

### 5-Fluoro-7-amino-2,3-dihydrobenzofuran

A mixture of 5-fluoro-7-nitro-2,3-dihydrobenzofuran (2.65 g) and 10% palladium on carbon (0.5 g) in ethanol (100 ml) was hydrogenated for 3 hours. The catalyst was filtered off and the solvent removed under vacuum. Chromatography (30% ethyl acetate-hexanes) afforded 1.38 g (62 %) of product as a white solid: mp 68-70°C.

| Elemental analysis for C₈H₈NO | | | |
|---|---|---|---|
| Calc'd | C, 62.74; | H, 5.27; | N, 9.15 |
| Found | C, 62.76; | H, 5.32; | N, 9.13 |

### INTERMEDIATE 19

### 1-Benzyl-4-(5-fluoro-2,3-dihydro-benzofuran-7-yl)-piperazine

A solution of 5-fluoro-7-amino-2,3-dihydrobenzofuran (1.38 g, 9 mmol) and bis(2-chloroethyl)-benzylamine (3.14 g, 14 mmol) in butanol (20 ml) was stirred at 100°C for 10 hours. The salt was filtered off, washed with ethyl ether (30 ml) and dried under vacuum: mp 232-233.5°C. The salt was converted to the free base to afford 2.06 g (73 %) of the title compound.

| Elemental analysis for C₁₉H₂₁FN₂O•HCl•0.25H₂O | | | |
|---|---|---|---|
| Calc'd | C, 64.58; | H, 6.42; | N, 7.93 |
| Found | C, 64.43; | H, 6.27; | N, 7.86 |

### INTERMEDIATE 20

### 4-(5-Fluoro-2,3-dihydro-benzofuran-7-yl)-piperazine

A mixture of 1-benzyl-4-(5-fluoro-2,3-dihydro-benzofuran-7-yl)-giperazine (2.06 g, 6.6 mmol), 10% palladium on carbon (0.6 g) and ammonium formate (0.83 g, 13 mmol) in ethanol (20 ml) was allowed to refux for 2 hours. The catalyst was filtered off and the solvent removed under vacuum. Chromatography (10-30% methanol-methylene chloride plus ammonium hydroxide) afforded 1.10 g (75%) of product as a yellow oil; MS EI *m*/*e* 222 (M)⁺.

### INTERMEDIATE 21

### Ethyl 7-nitrobenzofuran-2-carboxylate

A stirred mixture of 2-hydroxy-3-nitrobenzaldehyde (4.8 g, 59 mmol), diethyl bromomalonate (16.8g, 71 mmol), potassium carbonate (12.1 g, 88 mmol) and N,N'-terephthalylidenebis(4-butylaniline) (1.9g, 5.9 mmol) in toluene (100 ml) was refluxed with a Dean-Stark trap for 24 hours. Another 12.1 g potassium carbonate was added to the above reaction mixtuer, and the resulting mixture was allow to reflux for another 3 days. The reaction was quenched with water, extracted with (3 x 200 ml) and washed with 2.0 N sodium hydroxide (100 ml). The organic layer was dried over anhydrous sodium sulfate and filtered. Chromatography (30% ethyl acetate-hexanes) afforded a yellow solid: mp 86.5-87.5°C (lit¹: mp 88-89°C).

### INTERMEDIATE 22

### 7-Nitrobenzofuran

To a suspension of ethyl 7-nitrobenzofuran-2-carboxylate in ethanol was added 2 N potassium hydroxide (60 ml). After being heated at reflux for 0.5 hour, the solution was cooled to room temperature and concentrated to half volume. Concentrated hydrochloric acid was added to the above mixture and filtered. The solid was washed with water and dired under vacuum with phosphorous pentoxide overnight. The dried solid was mixed with quinoline (75 ml) and copper oxide (CuO, 0.4 g). The mixture was heated up to 220°C for 3 hours. The mixture was filtered and the filtrate was concentrated. Chromatography (20% ethyl acetate-hexanes) afforded 5.3 g (91%) of product as a yellow solid: mp 92-94°C. (lit¹: mp 95.5-97°C).

### INTERMEDIATE 23

### 7-Aminobenzofuran hydrochloride

A stirred suspension of 7-nitrobenzofuran (5.3 g, 32 mmol) and Raney nickel (0.1 g) in methanol (70 ml) was heated up to 50°C. Then hydrazine monohydrate (98%, 4.8 ml, 97 mmol) in methanol (10 ml) was slowly added to the above solution at temperature 50-60°C. When the addition was complete, the mixture was allowed to reflux for 2 hours. The Raney nickel was filtered off and the solution was concentrated. The residue was dissolved in ethyl acetate and converted to its HCl salt 3.68 g (66%) (lit¹: mp 212-213°C).

### INTERMEDIATE 24

### 1-(7-Benzofuranyl)piperazine

A solution of 7-aminobenzofuran hydrochloride (3.66 g, 22 mmol) and bis(2-chloroethyl)amine hydrochloride (3.84 g, 22 mmol) in chlorobenzene (80 ml) was heated to reflux for 72 hours. The solvent was removed under vacuum, the residue was dissolved in 2.5 N sodium hydroxide-methylene chloride and extracted with methylene chloride (3 x 100 ml). The organic layer was dried over anhydrous sodium sulfate and filtered. Chromatography (10-20% methanol-methylene chloride plus ammonium hydroxide) afforded 0.66 g (15%) of product as a brown-yellow oil; (lit¹: for HCl salt mp 194.5-195°C).

### INTERMEDIATE 25

### 4-(5-Fluoro-1H-3-indolyl)-cyclohex-3-enone

This compound was prepared in the manner described above for intermediate 3c by replacing 4-(5-fluoro-1H-3-indolyl)-cyclohexanone ethylene ketal with 4-(5-fluoro-1H-3-indolyl)-cyclohex-3-enone-ethylene ketal (1.37 g) to afford 1.01 g (88 %) of the title compound.

### INTERMEDIATE 26

### 1-(2-Methoxy-phenyl)-4-(1,4-dioxa-spiro[4,5]dec-8-yl)-piperazine

A solution of 1,4-cyclohexanedione monoethylene ketal (4.68 g, 30 mmol), 1-(2-methoxy-phenyl)piperazine (5.8 g, 30 mmol), sodium triacetoxyborohydride (9 g, 42 mmol) and acetic acid (1.8 ml, 30 mmol) in 1,2-dichloroethane (8 ml) was allowed to stir at room temperature for 12 hours. The reaction was quenched with 1N sodium hydroxide (pH > 9), and extracted with methylene chloride (3 x 100 ml). The organic layer was dried over anhydrous sodium sulfate and filtered. Chromatography (10% methanol-ethyl acetate) afforded 9.0 g (90%) of product as a semi-solid.

### INTERMEDIATE 27

### 4-[4-(2-Methoxy-phenyl)-piperazin-1-yl]-cyclohexanone

This compound was prepared in the manner described above for intermediate 3a by replacing 3-(1,4-dioxa-spiro[4,5]dec-8-yl)-1H-indole with 1-(2-methoxy-phenyl)-4-(1,4-dioxa-spiro[4,5]dec-8-yl)-piperazine (5.0 g, 15 mmol) to afford 4.0 g (93%) of the title compound.

### INTERMEDIATE 28

### 5-Fluoro-3-{4-[4-(2-methoxy-phenyl)-piperazin-1-yl] cyclohex-1-enyl}-1H-indole

This compound was prepared in the manner described above for intermediate 1c by replacing 1,4-cyclohexanedione monoethylene ketal with 4-[4-(2-methoxy-phenyl)-piperazin-1-yl]-cyclohexanone (1.44 g, 5 mmol). The crude mixture was used in next step without further purification.

### INTERMEDIATE 29

### 5-Fluoro-3-{4-[4-(2-methoxy-phenyl)-piperazin-1-yl] cyclohexyl}-1H-indole

This compound was prepared in the manner described above for intermediate 2c by replacing 4-(5-fluoro-1H-3-indolyl)-cyclohex-3-en- ethylene ketal with 5-fluoro-3-{4-[4-(2-methoxy-phenyl)-piperazin-1-yl] cyclohex-1-enyl}-1H-indole (2.0 g) to afford 1.77 g (84%) of product as a mixture of cis and trans isomer.

### INTERMEDIATE 30

### 4-(5-Fluoro-1-methyl-3-indolyl)-cyclohexanone

To a suspension of sodium hydride (60%, 0.18 g, 4.5 mmol) in anhydrous N, N-dimethylformamide (10 ml) was added 4-(5-fluoro-1H-indol-3-yl)-cyclohexanone (0.7 g, 3.0 mmol) at room temperature. The mixture was stirred for 0.5 hour, then to the above solution was added iodomethane (0.21 ml, 3.3 mmol) at room temperature. The resulting mixture was stirred for another 0.5 hour and quenched with water. The mixture was extracted with methylene chloride (3 x 50 ml) and the organic layer was dried over anhydrous sodium sulfate and filtered. Chromatography (30% ethyl acetate-hexanes) afforded 0.35 g (46%) of product as a yellow oil: MS EI *m*/*e* 245 (M⁺).

### INTERMEDIATE 31

### 5-Nitro-quinoxaline

To a room temperature solution of 3-nitro-*o*-phenylenediamine (10 g, 65.3 mmol) in EtOH (50mL) was added glyoxal (40 % in H₂O, 22.47 mL). The reaction mixture was heated at reflux 1 hour, then diluted with H₂O (100 mL). The cooled mixture was extracted with CH₂Cl₂ (2 x 300 mL) and the organic layers were combined and washed again with H₂O (500 mL), dried over Na₂SO₄ and concentrated yielding a bright orange solid which was recrystallized from EtOAc/Hexanes to give 10.96 (96%) of a tan solid mp 90-92 °C.

| Elemental Analysis for C₈H₅N₃O₂ | | | |
|---|---|---|---|
| Calc'd | C, 54.86; | H, 2.88, | N; 23.99 |
| Found | C, 55.12; | H, 3.05; | N, 24.05. |

### INTERMEDIATE 32

### 5-Amino-quinoxaline

To a three neck 250 mL round bottom flask equipped with a reflux condenser and nitrogen inlet was added 5-nitro-quinoxaline (4 g, 22.8 mmol) dissolved in HOAc (60 mL). The mixture was heated to boiling, removed from heat, and solid Fe powder (3.83 g, 68.6 mmol) was added. Vigorous boiling was observed. The reaction mixture was heated at reflux 10 minutes and then poured into H₂O (100 mL) and ice. The aqueous solution was filtered and basified to pH >10 with 1 M NaOH, and extracted in EtOAc ( 3 x 200 mL). The organic layers were combined, dried over Na₂SO₄, and concentrated. The resulting oil was purified by column chromatography
(40% EtOAc/Hexanes) yielding 2.03 g (61%) of an orange solid: mp 87-90°C.

| Elemental Analysis for C₈H₇N₃ | | | |
|---|---|---|---|
| Calc'd | C, 66.19; | H, 4.86; | N, 28.95 |
| Found | C, 66.25; | H, 4.96; | N, 29.26 |

### INTERMEDIATE 33

### 1-Benzyl-4-(quinoxalin-yl)-piperazine

To a solution of 5-amino-quinoxaline (2.8 g, 19.3 mmol) in BuOH (50 mL) was added bis(2-chloroethyl)-benzlyamine (8.42 g, 38.6 mmol) and Et₃N (5.34 mL, 38.6 mmol). The reaction was stirred at 100°C overnight. A second portion of Et₃N (5.34 mL, 38.6 mmol) was added and the reaction stirred at 100°C an additional 24 hours. The cooled solution was made alkaline with 2.5 N NaOH (500 mL) and extracted into EtOAc (3 x 200 mL). The organic fractions were combined, dried over Na₂SO₄, concentrated and chromatographed (40% EtOAc/Hex) yielding 1.0 g (17%) of a gold oil.

### INTERMEDIATE 34

### 5-(1-Piperazinyl)-quinoxaline

To a room temperature solution of 1-benzyl-4-(quinoxalin-yl)-piperazine (1.0 g, 3.3 mmol) in anhydrous CH₂Cl₂ under nitrogen was added vinyl chloroformate (0.34 mL, 3.9 mmol) drop wise. The reaction mixture was heated at reflux 2 hours. The reaction was cooled, concentrated to dryness and concentrated HCl (25 mL) and 1,4-dioxane (25 mL) were added. The resulting solution was stirred at ambient temperature overnight. The solution was basified with 2.5 N NaOH (300 mL) and extracted into EtOAc (3 x 200 mL). The organic layers were combined, dried over Na₂SO₄, concentrated and chromatographed (10% MeOH/CH₂Cl₂/NH₄OH) to give 450 mg (64%) of an orange solid: mp 106-108°C: MS (+) ESI *m*/*e* 215 [M+H]⁺.

### INTERMEDIATE 35a

### 5-(Trifluoromethylsulfonyloxy)-quinoline

A solution of 5-hydroxy-quinoline (8 g, 55 mmol) and K₂CO₃ (15.2 g, 110 mmol) in anhydrous pyridine (60 mL) under nitrogen was cooled to -20°C. Tf₂O (13.97 mL, 83 mmol) was added drop-wise via syringe. The reaction mixture was stirred 1 hour at -20°C then warmed to 0°C for 1 hour then stirred at ambient temperature for 48 hours. The reaction mixture was then poured into H₂O (200 mL) and extracted in CH₂Cl₂ (2 x 200 mL). The aqueous layer was acidified with 1 N HCl (100 mL) and back extracted with CH₂Cl₂ (2 x 200 mL). The organic fractions were dried over Na₂SO₄, concentrated and purified by column chromatography (40% EtOAc/Hexanes) affording 13.97 g (90%) of the product as a pink oil: MS EI *m*/*e* 277 (M⁺).

### INTERMEDIATE 35b

### 5-(Trifluoromethylsulfonyloxy)-isoquinoline

This compound was prepared in the manner described above for Intermediate 35a by replacing 5-hydroxy-quinoline with 5-hydroxy-isoquinoline (5 g) to afford 7.71 g (79%) of the title compound as a waxy beige solid: MS ESI *m*/*e* 278 (M⁺).

### INTERMEDIATE 35c

### 1-(Trifluoromethylsulfonyloxy)-isoquinoline

This compound was prepared in the manner described for Intermediate 35a by replacing 5-hydroxy-quinoline with isocarbastyril (8 g) to afford 9.74 g (64%) of the title compound as a clear oil: MS EI *m*/*e* 277 (M⁺).

### INTERMEDIATE 36a

### 1-t-butyl-4-(5-Quinolinyl)piperazine carboxylate

To an oven-dried 100 mL flask was added Cs₂CO₃ (19.87 g, 61 mmol), Pd(OAc)₂ (0.49 g, 2.2 mmol), and BINAP (1.183 g, 1.9 mmol). The solids were flushed with N₂ for 10 minutes. A solution of 5-(trifluoromethylsulfonyloxy)-quinoline (12 g, 43 mmol) and 1-*t*-butyl-4-piperazine carboxylate (9.67 g, 52 mmol) in THF was then added slowly to the reaction flask. The reaction mixture was stirred at room temperature for 0.5 hour then at 65°C overnight. The resulting solution was diluted with ether, filtered through a bed of celite, washed with Et₂O (50 mL) and EtOAc (50 mL). The organic fractions were combined, dried over Na₂SO₄, filtered, and chromatographed 3 times (10% MeOH/CH₂Cl₂) yielding 1.57 g (12%) of pure product as a beige solid: mp 116-118°C.

| Elemental Analysis for C₁₈H₂₃N₃O₂: | | | |
|---|---|---|---|
| Calc'd | C, 68.98; | H, 7.40; | N, 13.41 |
| Found | C, 69.09; | H, 7.33; | N, 13.08 |

### INTERMEDIATE 36c

### 1-t-butyl-4-(1-Isoquinolinyl)piperazine carboxylate

This compound was prepared in the manner described above for Intermediate 36a, replacing 5-(trifluoromethylsulfonyloxy)-quinoline with 1-(trifluoromethylulfonyloxy)-isoquinoline (9 g, 32.5 mmol) yielding 2.33 g (25%) of a waxy beige solid: mp 69-71°C.

### INTERMEDIATE 37a

### 5-(1-Piperazinyl)-quinoline

To a solution of 1-*t*-butyl-4-(5-quinolinyl)piperazine carboxylate (1.57 g, 5 mmol) in CH₂Cl₂ (2 mL) at 0°C was added a pre-cooled, premixed, solution of TFA (10 mL), CH₂Cl₂ (20 mL) and MeOH (10 drops). The reaction was warmed slowly to room temperature and allowed to stir overnight. The resulting solution was concentrated, dissolved in H₂O (5 mL) and CH₂Cl₂ (5 mL) and made alkaline with NaHCO₃ to pH 9. The aqueous portion was extracted 6 x 100 mL EtOAc and concentrated yielding 1.0 g (100%) of a yellow oil which solidified upon standing was not purified further.

### INTERMEDIATE 37c

### 1-(1-piperazinyl)-isoquinoline

This compound was prepared in the same manner as intermediate 37a replacing 1-*t*-butyl-4-(5-quinolinyl)piperazine carboxylate with 1-*t*-butyl-4-(1-isoquinolinyl) piperazine carboxylate (2.33 g, 7.4 mmol) affording 1.5 g (95 %) of a beige solid: mp 127-130°C.

### INTERMEDIATE 38a

### 6-Methoxy, 8-Amino-quinoline

To a hot suspension of 6-methoxy, 8-nitro-quinoline in 100 mL of a mixture of ethanol: acetic acid : water (2 : 2 : 1) 3.0 g of iron powder were added in portions. The reaction was refluxed for about 2 1/2 hours, the mixture was cooled, filtered over celite and basified with sodium bicarbonate. The product was extracted with ether, dried and the solvent was removed under vaccum to give 3.2 g of the title compound. MS (ES) m/z (relative intensity): 175 (M+H+ 100).

### INTERMEDIATE 38b

### 8-Amino, 6-Chloro-quinoline

To a hot suspension of (0.800g) 6-chloro, 8-nitro-quinoline in 25 mL of a mixture of ethanol: acetic acid : water (2:2:1) 0.5g of iron powder was added in portions. The reaction was refluxed for about 1 1/2 hours, the mixture was cooled, filtered over celite and basified with sodium carbonate. The product was extracted with ether, dried and the solvent was removed under vaccum to give 0.5g of the title compound. mp 70-73°C. MS (ES) m/z (relative intensity): 179 (M+H+).

| Elemental analysis for C₉ H₇ Cl N₂ | | | |
|---|---|---|---|
| Calculated | C : 60.52; | H : 3.95; | N : 15.68 |
| Found | C : 60.82; | H : 3.77; | N : 15.96 |

### INTERMEDIATE 39a

### 6-Methoxy, 8-piperazino-quinoline

6-Methoxy, 8-amino-quinoline (8.2 g) and bis(chloroethyl)amine hydrohloride (9.0g) were taken in 70 mL chlorobenzene and heated at about 135°C with vigorous stirring for 3 days. The reaction never went to completion. The mixture was cooled. Water was added and extracted with ether. The aqueous phase was basified with sodium carbonate and extracted with ethyl acetate, dried and the solvent removed. The crude product was filtered through 300 mL of silica gel using 10% MeOH/CH₂Cl₂, 20% MeOH/ CH₂Cl₂, then 1% NH4OH / 80% MeOH / 19% CH₂Cl₂, to give 1.5 g of the desired product. MS (ES) m/z (relative intensity): 244 (M+H+, 100).

### INTERMEDIATE 39b

### 6-Chloro-, 8-piperazino-Quinoline

8-amino, 6-chloro-quinoline (0.980g) and bis(chloroethyl)amine hydrohloride (0.980g) were taken in 13 mL chlorobenzene and heated at about 135°C with vigorous stirring for 5 days. The reaction was cooled taken in water and extracted with ether. The aquous phase is basified with sodium carbonate and reextracted with ether, dried and the sovent was removed to give 0.400g of the title compound. MS (ES) m/z (relative intensity): 248 (M+H+).

### INTERMEDIATE 39c

### 5-Chloro-, 8-piperazino-quinoline

To a solution of 5-chloro,8-(trifluoromethylsulfonyloxy)-quinoline (1.0g) in 15 mL chlorobenzene excess piperazine (1.0g) was added. The mixture was heated at 120°C for 2 1/2 days. The reaction was cooled, poured on water and the product was extracted with ether, dried over magnesium sulfate to give 0.480g of product. MS (ES) m/z (relative intensity): 248 (M+H+,100).

### INTERMEDIATE 39d

### 5-Fluoro, 8-piperazino-quinoline

To a solution of 5-Fluoro,8-(trifluoromethylsulfonyloxy)-quinoline (1g) in 5 mL chlorobenzene excess piperazine (2.0g) were added. The mixture was heated at 120°C for 2 1/2 days. The reaction was cooled, poured on water and the product was extracted with ethyl acetate, the organic phase was washed with dilute NaOH, then with water, dried and the solvent was removed. The product was chromatographed on silica gel using 15% methanol / methylene chloride then 79:20:1 methanol : methylene chloride : NH₄OH to give 0.240g of product. MS (ES) m/z (relative intensity): 232 (M+H+,100).

### INTERMEDIATE 39e

### 8-piperazino-quinaldine

To a solution of 8-(trifluoromethylsulfonyloxy)-quinaldine (7g) in 25 mL chlorobenzene, K₂CO₃ (3.3g) and excess piperazine (10.0g) were added. The mixture was heated at 130°C for 3 days. The reaction was cooled, poured on water and the product was extracted with ethyl acetate, dried over magnesium sulfate. The product was chromatographed on silica gel using 20% methanol / methylene chloride then 79:20:1 methanol : methylene chloride : NH₄OH to give 3.2g of product. MS (ES) m/z (relative intensity): 228 (M+H+,100).

### INTERMEDIATE 39f

### 6-MeO, 4-piperazino-quinoline

To a solution of 6-MeO, 4-(trifluoromethylsulfonyloxy)-quinoline (2g) in 10 mL acetonitrile, excess piperazine (2g) was added. The mixture was heated at about 70°C for 1 1/2 hours. Water is added and the product is extracted with ethyl acetate, dried and the solvent was removed to give (2.5g) of product. MS (ES) m/z (relative intensity): 308 (M+H+).

### INTERMEDIATE 40a

### 6-Chloro, 8-Nitro-Quinoline

A solution of 1.0g of 6-Chloro-quinoline in 5 ml fuming nitric acid, was heated to almost reflux for 2 days. The reaction was cooled, poured onto ice water and neutralized with concentrated ammonium hydroxide to about pH 5. The formed precipitate was filtered and dried to give 0.600 g of desired product. mp 149-155°C. MS (ES) m/z (relative intensity): 209 (M+H+).

### INTERMEDIATE 40b

### 5-Cl-8-(trifluoromethylsulfonyloxy)-quinoline

To a suspension of 5-Chloro,8-hydroxy-quinoline (8.95g) in 100 mL CH₂Cl₂, TEA is added (20 mL). The suspension dissolved, then cooled to -15°C. A solution of 21.1g of triflic anhydride in 50 mL of CH₂Cl₂, is added drop by drop with cooling. After complete addition, the reaction was stirred at -15 °C for 1 hour; The reaction was diluted with CH₂Cl₂, washed with a solution of NaHCO₃, then with water dried and the solvent was removed to give 15.0 gr of product. mp 80-83°C. MS (ES) m/z (relative intensity): 312 (M+H+, 100). Elemental analysis for C₁₀ H₅ ClF₃ NO₃ S

| | | | |
|---|---|---|---|
| Calculated | C : 38.54; | H : 1.62; | N : 4.49 |
| Found | C : 38.3; | H : 1.73; | N : 4.5 |

### INTERMEDIATE 40c

### 5-Fluoro-8-(trifluoromethylsulfonyloxy)-quinoline

To a cold solution (-15°C) of 5-Fluoro,8-hydroxy-quinoline (2.5g) in 20 mL CH₂Cl₂, TEA is added (6.3 mL). To the cold mixture a solution of 6.5g of triflic anhydride in 10 mL of CH₂Cl₂, is added drop by drop with cooling. After complete addition, the reaction was stirred at 0°C for 1 hour; The reaction was quenched with water, and the product was extracted with ether, dried and the solvent was removed to give 3.4g of product. MS (ES) m/z (relative intensity): 296 (M+H+,100).

### INTERMEDIATE 40d

### 8-(trifluoromethylsulfonyloxy)-quinaldine

To a cold solution (-15°C) of 8-hydroxy-quinaldine (11.5g) in 50 mL CH₂Cl₂, TEA is added (29 mL). To the cold mixture a solution of 29.6g of triflic anhydride in 50 mL of CH₂Cl₂, were added drop by drop with cooling. After complete addition, the reaction was stirred at -15°C for 1 hour; The reaction was quenched with water, and the product was extracted with ether, dried and the solvent was removed to give 20g of product. MS (ES) m/z (relative intensity): 292 (M+H+).

### INTERMEDIATE 41

### 6-MeO, 4-(trifluoromethylsulfonyloxy)-quinoline

To a cold solution (-15°C) of 6-MeO,4-hydroxy-quinoline (5g) in 30 mL CH₂Cl₂, TEA is added (12 mL). To the cold mixture a solution of 12g of triflic anhydride in 15 mL of CH₂Cl₂, were added drop by drop with cooling. After complete addition, the reaction was stirred at -15°C for 1 hour; The reaction was quenched with water, and the product was extracted with ether, dried and the solvent was removed to give 7g of product. MS (ES) m/z (relative intensity): 308 (M+H+).

### INTERMEDIATE 42a

### 1-benzyl-4-(6-methoxy-2-methylquinolin-8-yl)piperazine

A mixture of 8-amino-6-methoxy-2-methylquinoline (1.75 g, 9.30 mmol), N-benzyl-bis-dichloroethane (8.9 g, 38.3 mmol), and triethylamine (6.5 mL, 46.6 mmol) in 1-butanol (25 mL) was heated at 100°C for 20 hours. After cooling to room temperature, the reaction was diluted with ethyl acetate (50 mL), and poured into saturated aqueous NaHCO₃. The aqueous layer was extracted with ethyl acetate (3 x 50 mL). The combined organic layers were washed with saturated aqueous NaHCO₃ (50 mL) and brine (50 mL), then were dried over anhydrous sodium sulfate, filtered and concentrated in vacuo. Excess 1-butanol was azeotroped with hexane (2 x 500 mL). Flash chromatography on 5.5 x 18 cm SiO₂ (25% EtOAc/hexane) afforded 1.15 g (36%) of a yellow oil, which crystallized on standing. Recrystallization from hexane provided 0.898 g (28%) of analytically pure product as yellow crystals: mp 83-85□C.

| Elemental analysis for C₂₂H₂₅N₃O | | | |
|---|---|---|---|
| Calc'd | C, 76.05; | H, 7.25; | N, 12.09 |
| Found | C, 75.88; | H, 7.37; | N, 12.05 |

### INTERMEDIATE 42b

### 1-benzyl-4-(6-methoxy-3-methylquinolin-8-yl)piperazine

The title compound was prepared by the same method used for 1-benzyl-4-(6-methoxy-2-methylquinolin-8-yl)piperazine, except substituting 8-amino-6-methoxy-3-methylquinoline (2.82 g, 15.0 mmol) for the 8-amino-6-methoxy-2-methyl-quinoline. Flash chromatography on 6 x 20 cm SiO₂ (25-30% EtOAc/hexane), with rechromatography of the mixed fractions, provided 1.13 g (22%) of the title compound as a yellow gum. Crystallization from hexane afforded 0.88 g of analytically pure compound as yellow crystals: mp 112-113°C.

| Elemental analysis for C₂₂H₂₅N₃O | | | |
|---|---|---|---|
| Calc'd | C, 76.05; | H, 7.25; | N, 12.09 |
| Found | C, 75.83; | H, 7.26; | N, 12.07 |

### INTERMEDIATE 42c

### 1-benzyl-4-(6-methoxy-4-methylquinolin-8-yl)piperazine

A mixture of 8-amino-6-methoxy-4-methylquinoline (3.0 g, 15.9 mmol), *N*-benzyl-bis-dichloroethane (11.1g, 48.0 mmol), triethyl amine (4.8 g, 48 mmol) and 1-butanol were heated to 100°C for 24 hours. The reaction mixture was poured into 2.5 N aqueous NaOH and extracted with ethyl acetate (3 x 200 mL). The combined organic layers were washed with water (100 mL) and brine (100 mL), then were dried over anhydrous sodium sulfate, filtered and concentrated to afford 12.0 g of a dark brown oil. Flash chromatography on silica gel (5% methanol/ ethyl acetate) provided 2.3 g (42%) of the title compound as a thick oil, which solidified upon standing: mp 154-155°C.

| Elemental analysis for C₂₂H₂₅N₃O | | | |
|---|---|---|---|
| Calc'd | C, 76.05; | H, 7.25; | N, 12.09 |
| Found | C, 75.92; | H, 7.36; | N, 11.96 |

### INTERMEDIATE 43a

### 4-(6-methoxy-2-methylquinolin-8-yl)piperazine

A mixture of 1-benzyl-4-(6-methoxy-2-methylquinolin-8-yl)piperazine (0.527 g, 1.52 mmol), 10% Pd/C (0.20 g), and ammonium formate (0.96 g, 15.2 mmol) in methanol (10 mL) were heated at reflux under N₂ for 3 hours. TLC analysis (35% EtOAc/hexane) indicated only a trace of starting material remained. After cooling to room temperature, the reaction was filtered through celite, washing with excess methanol. The filtrate was concentrated, diluted with CH₂Cl₂ (50 mL), and washed with saturated aqueous NaHCO₃. The aqueous layer was extracted with CH₂Cl₂ (2 x 50 mL). The combined organic layers were dried over anhydrous sodium sulfate, filtered, and concentrated in vacuo to afford 0.37 g (95%) of the title compound as a yellow oil, which was used in the subsequent reaction without purification.

### INTERMEDIATE 43b

### 4-(6-methoxy-3-methylquinolin-8-yl)piperazine

The title compound was prepared by the same method used for the preparation of 4-(6-methoxy-2-methylquinolin-8-yl)piperazine, except 1-benzyl-4-(6-methoxy-3-methylquinolin-8-yl)piperazine (0.32 g, 0.92 mmol) was substituted for the 1-benzyl-4-(6-methoxy-2-methylquinolin-8-yl)piperazine. The title compound was isolated in nearly quantitative yield and used with purification in the subsequent reaction.

### INTERMEDIATE 43c

### 4-(6-methoxy-4-methylquinolin-8-yl)piperazine

A mixture of 1-benzyl-4-(6-methoxy-4-methylquinolin-8-yl)piperazine (2.0 g, 5.76 mmol), methylene chloride (50 mL) and vinyl chloroformate (0.8 mL, 8.64 mmol) were refluxed for 4 hours. The mixture was concentrated, then dissolved in a 1:1 mixture of dioxane /conc. HCl and stirred at ambient temperature for 18 hours. The reaction mixture was made basic with 2.5 N aqueous NaOH and extracted with ethyl acetate (2 x 200 mL). The combined organic layers were washed with water (100 mL) and brine (100 mL), then were dried over anhydrous sodium sulfate, filtered, and concentrated to give 0.6 g (47%) of the title compound: mp 208-209°C.

| Elemental analysis for C₁₅H₁₉N₃O•HCl•0.5 H₂O | | | |
|---|---|---|---|
| Calc'd | C, 59.50; | H, 6.99; | N, 13.88 |
| Found | C, 59.44; | H, 7.09; | N, 13.57 |

### INTERMEDIATE 44a

### 1-benzyl-4- (6-methoxy-5-methylquinolin-8-yl)piperazine

This compound was prepared in a manner similar to that used for 1-benzyl-4-(6-methoxy-4-methylquinolin-8-yl)piperazine to give 3.0 g (56%) of pure title compound: mp 129-133°C.

| Elemental analysis for C₂₂H₂₅N₃O | | | |
|---|---|---|---|
| Calc'd | C, 76.05; | H, 7.25; | N, 12.09 |
| Found | C, 75.61; | H, 7.35; | N, 11.97 |

### INTERMEDIATE 44b

### 1-benzyl-4- (6-methoxy-5-chloro-quinolin-8-yl)piperazine

This compound was prepared in a manner similar to that used for 1-benzyl-4-(6-methoxy-4-methylquinolin-8-yl)piperazine to give 1.9 g (35%) of pure title compound: mp 138-140°C.

| Elemental analysis for C₂₁H₂₂ClN₃O | | | |
|---|---|---|---|
| Calc'd | C, 68.56; | H, 6.03; | N, 11.42 |
| Found | C, 68.26; | H, 5.98; | N, 11.45 |

### INTERMEDIATE 45a

### 4-(6-methoxy-5-methylquinoline-8-yl)piperazine

A mixture of methanol (15mL), 10% Pd/C (0.12 g), 1-benzyl-4-(6-methoxy-5-methylquinolin-8-yl)piperazine (0.8 g, 2.3 mmol), and ammonium formate (0.88 g, 13.9 mmol) were refluxed for 45 minutes. The reaction mixture was filtered through celite and concentrated. The residue was diluted with 1 N aqueous NaOH (50 mL) and extracted with ethyl acetate (3 x 75 mL). The combined organic layers were washed with water (50 mL) and brine(50 mL), then were dried over anhydrous Na₂SO₄, filtered, and concentrated to give 0.52 g (61 %) of the title compound as a thick oil. MS (ES) m/z: 258 (M+H+).

### INTERMEDIATE 45b

### 4-(6-methoxy-5-chloro-quinolin-8-yl)piperazine

This compound was prepared in a manner as similar to that used 4-(6-methoxy-5-methylquinoline-8-yl)piperazine to give 0.48 g (68%) of pure title compound as a thick oil. MS (ES) m/z: 278 (M+H+).

### INTERMEDIATE 46

### 5-Bromo-6-methoxy-quinoline

To a solution of 6-methoxyquinoline (5 g, 31.4 mmol) in acetic acid (50 mL) was slowly added Br₂ neat (1.62 mL, 31.4 mmol). The reaction mixture was stirred at ambient temperature for 1 hour and then poured onto ice. Saturated aqueous sodium bisulfite was added, and the resulting slurry was extracted into EtOAc (2 x 200 mL). The organic fractions were combined, dried over Na₂SO₄, concentrated, and purified by column chromatography (5% MeOH/CH₂Cl₂) affording 4.39 g of the title compound as the acetate salt. The free base was prepared by washing the salt with 1 N NaOH (50 mL) and H₂O (100 mL) and extracting into CH₂Cl₂ (200 mL). The organic fractions were concentrated affording 3.89 g (52%) of the title compound as a pink solid.

| Elemental analysis for C₁₀H₈BrNO | | | |
|---|---|---|---|
| Calc'd | C, 50.45; | H, 3.39; | N, 5.88 |
| Found | C, 50.34; | H, 3.25; | N, 6.09 |

### INTERMEDIATE 47

### 4-Bromo-2-nitrophenylamine

To a solution of 2-nitro-phenylamine (13.8 g, 100 mmol) in HOAc ( 150 mL) was added NBS (18 g, 101 mmol). The reaction mixture was stirred and heated to reflux over 1 hour. The cooled reaction mixture was poured into H₂O (1000 mL) and stirred for 15 minutes. The resulting orange slurry was filtered and washed with H₂O (300 mL) affording a 20.26 g (93%) of the title compound as a bright orange solid.

| Elemental analysis for C₆H₅BrN₂O₂ | | | |
|---|---|---|---|
| Calc'd | C, 33.21; | H, 2.32; | N, 12.91 |
| Found | C, 33.15; | H, 2.31; | N, 12.75 |

Ref: Montash Chem EN 1994, 125 p. 723-730

### INTERMEDIATE 48

### 6-Bromo-8-nitro-quinoline

A sulfuric acid solution was prepared by adding H₂SO₄ (50 mL) to an 250 mL flask containing H₂O (20 mL) cooled in an ice bath. To this solution was added glycerol (12 mL, 16.5 mmol), m-nitrobenzene sulfonic acid sodium salt (11.4 g, 5.06 mmol), and 4-bromo-2-nitrophenylamine (10 g, 4.6 mmol). The reaction mixture was heated at 135°C for 3 hours. The warm reaction mixture was poured into ice H₂O (200 mL) and extracted into 50% MeOH/EtOAc (2 x 200 mL), dried over Na₂SO₄ and concentrated. The resulting brown solid was triturated with EtOH and filtered affording 3.8 g (33%) of a pink solid: mp 172-174°C.

| Elemental analysis for C₉H₅BrN₂O₂ | | | |
|---|---|---|---|
| Calc'd | C, 42.72; | H, 1.99; | N, 11.07 |
| Found | C, 42.69; | H, 1.85; | N, 11.01 |

Ref: Mantash Chem EN 1994, 125 p. 723-730

### INTERMEDIATE 49

### 6-Bromo-8-amino-quinoline

To a solution of 6-bromo-8-nitro-quinoline (4 g, 1.58 mmol) in EtOH/HOAc/H₂O (50 mL/50mL/25mL) was added iron metal (3.18 g, 5.69 mmol). The resulting solution was heated at reflux for 3 hours. The cooled reaction mixture was neutralized with 2.5 N NaOH, filtered through celite to remove iron solids and washed with EtOAc. The eluent was extracted into EtOAc (3 x 200 mL), combined, dried over Na₂SO₄ and concentrated. The resulting oil was purified by column chromatography (40% EtOAc/hexanes) affording 3.19 g (91%) of a yellow solid: mp 142-145°C.

| Elemental analysis for C₉H₇BrN₂ | | | |
|---|---|---|---|
| Calc'd | C, 48.46; | H, 3.16; | N, 12.56 |
| Found | C, 48.04; | H, 2.93; | N, 12.36 |

### INTERMEDIATE 50

### 8-(4-benzyl-piperazin-1-yl)-6-bromo-quinoline

The free base of bis(2-chloroethyl)-benzlyamine (5.12 g, 19.3 mmol) was prepared by washing the HCl salt with 1 M NaOH (200 mL) and extracting into EtOAc. The resulting organic phases were dried over Na₂SO₄ and concentrated. To this flask was added 6-bromo-8-amino-quinoline (2.15 g, 9.6 mmol), n-BuOH (100 mL), and Et₃N (4 mL, 28.9 mmol). The resulting reaction mixture was stirred at 100°C overnight. TLC analysis showed starting amine was still present, therefore an additional portion of bis(2-chloroethyl)-benzylamine hydrochloride (5 g) was added. The reaction was heated an additional 72 hours. The cooled reaction mixture was quenched with 1 M NaOH (200 mL) and extracted into EtOAc (3 x 200 mL). The organic fractions were combined, dried over Na₂SO₄, and concentrated. The resulting gold oil was purified three times by column chromatography (40% EtOAc/hexanes) affording 1.2 g (33%) of a viscous orange oil which solidified upon standing: mp 65-68°C, MS (+) APCI *m*/*z* 382 [M+H]⁺.

| Elemental analysis for C₂₀H₂₀BrN₃•0.75H₂O | | | |
|---|---|---|---|
| Calc'd | C, 60.69; | H, 5.48; | N, 10.62 |
| Found | C, 60.81; | H, 5.02; | N, 10.88 |

### INTERMEDIATE 51

### 6-Bromo-8-piperazin-1-yl-quinoline

To a solution of 8-(4-benzyl-piperazin-1-yl)-6-bromo-quinoline (1.6 g, 4.2 mmol) in dichloroethane (50 mL) under a N₂ atmosphere was added chloroethylchloroformate (1.26 mL, 12.6 mmol) and the reaction mixture was heated at 80°C for 4 hours, and at ambient temperaure overnight. No reaction was observed by TLC, therefore vinyl chloroformate (0.35 mL, 6.3 mmol) was added and the reaction was heated at 80°C for another 4 hours. The cooled reaction was poured into H₂O and extracted into CH₂Cl₂ (2 x 100 mL) and EtOAc (100 mL). The organic fractions were combined, dried over Na₂SO₄, and left in EtOAc overnight. The organic layer was concentrated and purified by column chromatography (10%MeOH/CH₂Cl₂+NH₄OH) affording 1.03 g (84%) of a brown foam. MS (+) APCI *m*/*z* 292 [M+H]⁺.

### INTERMEDIATE 52

### 6-hydroxy-8-nitro-quinoline

A solution of 6-methoxy-8-nitro-quinoline (9 g, 44.1 mmol) in HBr (100 mL) was heated at 110°C overnight. Additional HBr (80 mL) was added and the reaction continued to heat for an additional 24 hours. The cooled reaction mixture was basified with 2.5 N NaOH (800 mL) and extracted into EtOAc (2 x 300 mL). The organic fractions were combined, dried over Na₂SO₄ and purified by column chromatography (50% EtOAc/hexane) to afford 2.71 g (32%) of the title compound as a white solid: mp discolors above 100°C, MS (-) ESI *m*/*z* 189 [M-H]⁻.

### INTERMEDIATE 53

### 6-Ethoxy-8-nitro-quinoline

A solution of 6-hydroxy-8-nitro-quinoline (2.5 g, 13.2 mmol), ethylbromide (1.08 mL, 14.5 mmol), and K₂CO₃ (4 g, 26.4 mmol) in DMF (50 mL) under a nitrogen atmosphere was heated at 40°C for 5 hours. The cooled reaction mixture was poured into H₂O (200 mL) and extracted into EtOAc (2 x 200 mL). The organic fractions were combined, dried over Na₂SO₄ and concentrated. The resulting beige solid was triturated with 40% EtOAc/hexane to give 2.46 g (85%) of the title compound as beige crystals.

| Elemental analysis for C₁₁H₁₀N₂O₃ | | | |
|---|---|---|---|
| Calc'd | C, 60.55; | H, 4.62; | N, 12.84 |
| Found | C, 60.15; | H, 4.50; | N, 12.75 |

### INTERMEDIATE 54

### 8-(4-benzyl-piperazin-1-yl)-6-methoxy-1,2,3,4-tetrahydroquinoline

A solution of 8-(4-benzyl-piperazin-1-yl)-6-methoxy-quinoline (1 g, 3 mmol) in HOAc (100 mL) was hydrogenated over PtO₂ (300 mg) at 40 psi overnight. The reaction mixture was filtered through a pad of celite and was washed with EtOAc (50 mL). The filtrate was concentrated. The resulting gold oil was purified by column chromatography (10%MeOH/CH₂Cl₂+NH₄OH) affording 330 mg (45%) of a viscous gold oil. An analytical sample was prepared as the HCl salt from EtOAc. MS EI *m*/*z* 247 M⁺.
Ref: J. Chem Soc Perkins I 1980 p. 1933-1939

### INTERMEDIATE 55

### [1,6]naphthyridine

A sulfuric acid solution was prepared by adding H₂SO₄ (100 mL) to H₂O (57 mL) cooled in an ice bath. To this solution was added glycerol (33 mL, 457 mmol), *m*-nitrobenzene sulfonic acid sodium salt (48 g, 212 mmol) and 4-amino-pyridine (10 g, 106 mmol). The reaction mixture was heated at 135°C for 4 hours. The cooled reaction mixture was basicified with 2.5 N NaOH (500 mL) with cooling in an ice bath, and extracted into CH₂Cl₂ (3 x 200 mL). The organic fractions were combined, dried over Na₂SO₄ and concentrated. The resulting oil was purified by column chromatography (5% MeOH/CH₂Cl₂) affording 5.04 g (36%) as a dark orange oil. An analytical sample was prepared as the HCl salt from EtOAc yielding an orange low melting solid. MS EI *m*/*z* 130 M⁺.
Ref: Chem Pharm Bull. 1971, 19, 9, p. 1751-1755

### INTERMEDIATE 56

### 8-Bromo-[1,6]-naphthyridine

To a stirred solution of [1,6]-naphthyridine (4.73 g, 36.4 mmol) in CCl₄ (200 mL) was added Br₂ (2.25 mL, 43.7 mmol) in CCl₄ (35 mL) dropwise via an addition funnel. The resulting solution was heated at reflux for 1 hour. Pyridine (2.94 mL, 36.4 mmol) in CCl₄ (30 mL) was added dropwise to the refluxing solution, and the mixture was refluxed overnight. The cooled reaction mixture was filtered, and the solids were digested with 1 M NaOH (200 mL) for 1 hour. The basic solution was extracted into CH₂Cl₂ (2 x 200 mL), and the organic fractions were combined, dried over Na₂SO₄ and concentrated. The resulting oil was purified by column chromatography (10% EtOAc/CH₂Cl₂) affording 2.03 g (27%) of the title compound as yellow crystals: mp 79-81 °C.

| Elemental analysis for C₈H₅BrN₂ | | | |
|---|---|---|---|
| Calc'd | C, 45.97; | H, 2.41; | N, 13.40 |
| Found | C, 45.72; | H, 2.34; | N, 13.36 |

Ref: JOC 1968, 33, 4, p. 1384-1387

### INTERMEDIATE 57

### 8-piperazin-1-yl-[1,6]-naphthyridine

To an oven-dried 100 mL flask under a nitrogen atmosphere was added 8-bromo-[1,6]-naphthyridine (1.3 g, 6.2 mmol), piperazine (3.21 g, 37.3 mmol), and sodium t-butoxide (900 mg, 9.33 mmol). The solids were suspended in anhydrous o-xylenes (40 mL), and Pd(dba) (285 mg, 5 mol%) and P(*t*-Bu)₃ (0.31 mL, 1.24 mmol) were added. The reaction mixture was heated at 120°C for 3 hours. The cooled reaction mixture was poured into H₂O (100 mL) and extracted into EtOAc (1 x 100 mL) and CH₂Cl₂ (2 x 100 mL). The organic fractions were combined, dried over Na₂SO₄, concentrated, and the resulting oil was chromatographed (10% MeOH/CH₂Cl₂+NH₄OH) affording 470 mg (35%) of the title compound as a dark gold oil. An analytical sample was prepared as the HCl salt from EtOAc giving a brown solid: mp decomposes above 200 °C. MS (+) APCI *m*/*z* 215 [M+H]⁺.
Ref: Tet. Lett. 1998, 39, p. 617-620

### INTERMEDIATE 58

### 4-(6-Methylamino-quinolin-8-yl)-piperazine-1-carboxylic acid ethyl ester

To an oven-dried 25 mL round bottom flask was added Cs₂CO₃ (1.55 g, 4.76 mmol), BINAP (300 mg, 3 mol%), Pd(OAc)₂ (100 mg, 3 mol%) and kept under vacuum overnight. To this reaction vessel under a nitrogen atmosphere was added 8-(4-benzyl-piperazin-1-yl)-6-bromo-quinoline (1.3 g, 3.4 mmol), anhydrous toluene (12 mL) and benzylmethylamine (0.53 mL, 4.1 mmol). The reaction mixture was heated at 100°C overnight. The cooled reaction mixture was diluted with Et₂O (15 mL), filtered to remove solids, washed with EtOAc (10 mL) and concentrated. The resulting oil was purified by column chromatography (40% EtOAc/hexane) affording 830 mg (59%) of benzyl-[8-(4-benzyl-piperazin-1-yl)-quinolin-6-yl]-methyamine as an orange foam.

To a solution of benzyl-[8-(4-benzyl-piperazin-1-yl)-quinolin-6-yl]-methyamine (800 mg, 1.89 mmol) in anhydrous CH₂Cl₂ (100 mL) was added vinyl chloroformate (0.48 mL, 5.68 mmol) and heated at reflux overnight. A second aliquot of vinyl chloroformate (0.48 mL) was added and the reaction refluxed an additional 24 hours. The cooled reaction mixture was diluted with H₂O (50 mL) and extracted into CH₂Cl₂ (2 x 50 mL). The combined organic phases were dried over Na₂SO₄, filtered and concentrated. The resulting oil was purified by column chromatography (40% EtOAc/hexane) affording 600 mg of a monodebenzylated product. This material was dissolved in EtOH (100 mL) and 10% Pd/C (150 mg) and ammonium formate (244 mg, 4.5 mmol) were added. The reaction was heated at reflux overnight. Additional ammonium formate (250 mg) was added and the reaction refluxed for an additional 72 hours. The cooled reaction mixture was filtered through a pad of celite and washed with EtOAc (200 mL), concentrated and purified by column chromatography (10% MeOH/CH₂Cl₂) affording 400 mg of the title compound as a dark gold oil. An analytical sample was prepared as the HCl salt from EtOAc as an orange solid: mp decomposes above 85°C. MS (+) APCI m/z 315 [M+H].

### INTERMEDIATE 59

### 4-methoxy-2,6-dinitro-phenylamine

To a stirred solution of HNO₃ (65 mL) was added 4-methoxy-2-nitro-phenylamine (15 g, 89.3 mmol). The reaction mixture was stirred at room temperature overnight. The dark red precipitate was filtered and washed with H₂O (400 mL) affording 10.01 g (53%) of the title compound.

### INTERMEDIATE 60

### 7-Methoxy-quinoxalin-5-ylamine

A solution of 4-methoxy-2,6-dinitro-phenylamine (5 g, 23.5 mmol) in EtOH (200 mL) was hydrogenated over 10% Pd/C (2 g) at 40 psi for 1 hour. After H₂ uptake had ceased, the reaction was filtered through a pad of celite and washed with EtOAc (50 mL) and concentrated. Glyoxal (8 ml, 704 mmol) and EtOH (50 mL) were immediately added and the reaction was heated at reflux for 2 hours. The cooled reaction was diluted with H₂O (50 mL) and extracted into CH₂Cl₂ (3 x 100 mL). The organic phases were combined, dried over Na₂SO₄, filtered and concentrated. The resulting oil was purified by column chromatography (10% MeOH/CH₂Cl₂) affording 430 mg (10%) as a red oil. An analytical sample was prepared as the HCl salt from EtOAc affording a red solid.

### INTERMEDIATE 61

### (1-Oxy-pyridin-3-yl)-acetonitrile

A solution of 3-pyridylacetonitrile (11 g, 93.1 mmol), HOAc (55 mL), and 30% H₂O₂ (17 mL) was heated at 95°C overnight, and at room temperature for 72 hours. H₂O (50 mL) was added to the reaction mixture and the resulting solution was concentrated. This was repeated with additional H₂O (100 mL). Toluene (2 x 100 mL) was used to remove residual H₂O, and the resulting white solid was dried under vacuum overnight affording a waxy white solid: mp 120-125 °C; MS (+) APCI *m*/*z* 135 [M+H]⁺.
Ref: JACS 1959, 81 p. 740-743

### INTERMEDIATE 62

### 3-Cyanomethyl-pyridine-2-carbonitrile

To a suspension of (1-oxy-pyridin-3-yl)-acetonitrile (10 g, 75 mmol) in anhydrous CH₂Cl₂ under a nitrogen atmosphere was added trimethylsilylcyanide (10.95 mL, 82 mmol) and dimethylcarbamoylchloride (7.55 mL, 82 mmol). The reaction mixture was stirred at room temperature for 72 hours and then concentrated. EtOAc (100 mL) was added to the residue and the organic phase was washed with 1 M NaOH (150 mL), dried over Na₂SO₄, filtered and concentrated. The resulting solid was purified by column chromatography (50% EtOAc/hexanes) affording 7.08 g (66%) of a yellow solid: mp 48-51 °C; MS (+) APCI *m*/*z* 144 [M+H]⁺.
Ref: WO 9818796

### INTERMEDIATE 63

### 6-Methoxy-[1,7]naphthyridin-8-ylamine

To an oven-dried 250 mL flask under a nitrogen atmosphere was added anhydrous MeOH (200 mL). Na metal (1.07 g, 44 mmol) was weighed to a small beaker containing hexane and then transferred to the reaction vessel. After dissolution of the sodium, 3-cyanomethyl-pyridine-2-carbonitrile (5.3 g, 37 mmol) dissolved in anhydrous MeOH (10 mL) was added to the reaction. The resulting solution was heated at 80°C for 3 hours, then stirred at room temperature overnight. The reaction mixture was concentrated to remove MeOH and extracted into CH₂Cl₂ (2 x 200 mL). The organic phases were combined, dried over Na₂SO₄, filtered, concentrated and unsuccessfully chromatographed (2% MeOH/CH₂Cl₂). The mixed fractions were combined and recrystalized from EtOAc/hexane affording 1.16 g (18%) of the title compound as a yellow solid. The mother liquor was rechromatographed (50% EtOAc/hexanes) to afford an additional 560 mg (9%) of product: mp decomposes above 110 °C; MS (+) APCI *m*/*z* 176 [M+H]⁺.
Ref: Tet. Lett. 1975 p. 173-174

### INTERMEDIATE 64

### 6-Methoxy-8-piperazin-1-yl-[1,7]naphthyridine

A solution of 6-methoxy-[1,7]naphthyridin-8-ylamine (2.25 g, 12.8 mmol), bis(2-chloroethyl)-benzlyamine (10.25 g, 38.6 mmol) and Et₃N (5.34 mL, 38.6 mmol) in BuOH (100 mL) was heated at 100°C for 72 hours. The cooled reaction mixture was poured into H₂O (100 mL) and 2.5 N NaOH (100 mL), and extracted into EtOAc (2 x 200 mL). The organic phases were combined, dried over Na₂SO₄, filtered and concentrated. The resulting oil was purified twice by column chromatography (10% MeOH/CH₂Cl₂) affording a dark gold oil with BuOH impurity. This oil was dissolved in EtOH (50 mL) and 10% Pd/C (390 mg) and ammonium formate (730 mg) was added. The reaction mixture was heated at 80°C for 2.5 hours. The cooled reaction mixture was filtered through a pad of celite and washed with EtOAc (50 mL). The organic phase was concentrated and purified by column chromatography (10% MeOH/CH₂Cl₂+NH₄OH) affording 270 mg of the title compound as a dark orange oil. An analytical sample was prepared as the HCl salt from EtOAc.

### INTERMEDIATE 65

### 4-Piperazin-1-yl-1,3-dihydro-benzoimidazol-2-one

To a solution of 4-(4-benzylpiperazin-1-yl)-1,3-dihydro-benzoimidazol-2-one (1 g, 3.2 mmol) in anhydrous CH₂Cl₂ (50 mL) was added vinyl chloroformate (0.41 mL, 4.87 mmol) under a nitrogen atmosphere. The reaction mixture was heated at reflux for 2 hours, and then a second aliquot of vinyl chloroformate (0.41 mL) was added. The reaction was refluxed an additional 3 hours. The cooled reaction mixture was concentrated, and dioxan (25 mL) and conc. HCl (25 mL) were added to the residue. The resulting solution was stirred at room temperature for 72 hours. The reaction was basicified with 2.5 N NaOH (300 mL) and extracted in MeOH/EtOAc (2 x 200 mL). The organic fractions were combined, dried over Na₂SO₄ and concentrated and the resulting oil purified by column chromatography affording 393 mg (46%) as the oxalate salt. MS (+) ESI *m*/*z* 219 [M+H]⁺.

### INTERMEDIATE 66

### 6-Methoxy-1H-indol-4-ylamine

To a solution of 5-methoxy-2-methyl-1,3-dinitrobenzene (3.28 g, 15 mmol) in 15 mL dry N,N-dimethylformamide was added N,N-dimethyformamide dimethyl acetal (6.16 mL, 45 mmol) and pyrrolidine (1.3 mL, 15 mmol). The reaction mixture was heated at 120°C until TLC analysis showed complete consumption of the 5-methoxy-2-methyl-1,3-dinitrobenzene. N,N-Dimethylformamide was removed under the vacuum, affording a dark red material, which was dissolved in dry benzene and hydrogenated at 50 psi with 10% Pd/C (0.1 g) for 4 hours. The catalyst was filtered off and the solvent removed under vacuum. Chromatography (25 % ethyl acetate/hexane) afforded 1.0 g (40%) of the desired product as a yellow solid: mp 83-86 °C; MS EI m/e 162.

### INTERMEDIATE 67

### 4-(4-Benzyl-piperazin-1-yl)-6-methoxy-1H-indole

A solution of 6-methoxy-1*H*-indol-4-ylamine (0.76 g, 4.7 mmol) and bis(2-chloroethyl)-benzylamine (2.72 g, 11.7 mmol) in 1-butanol (20 mL) was stirred at 100°C for 18 hours then poured into aqueous sodium carbonate solution. The mixture was extracted with ethyl acetate (3 × 60 mL). The organic layer was dried over anhydrous sodium sulfate and filtered. The solvent was removed under vacuum. Chromatography (30% ethyl acetate/hexane) afforded 0.60 (40%) of product as a gray oil. MS (+) APCI (M + H)⁺m/e 322.

### INTERMEDIATE 68

### 6-Methoxy-4-piperazin-1-yl-1H-indole

A mixture of 4-(4-benzyl-piperazin-1-yl)-6-methoxy-1*H*-indole (0.37 g, 1.1 mmol), 10% Pd/C (0.05 g) and ammonium formate (0.15 g, 2.2 mmol) in ethanol (20 mL) was allowed to reflux for 2 hours. The catalyst was filtered off and the solvent removed under vacuum. Chromatography (10% methanol/methylene chloride plus ammonium hydroxide) afforded 0.2 g (75%) of product as a yellow foam. MS (EI) m/e 231.

### EXAMPLE 1a

### 3-[cis-4-[4-(1H-Indol-4-yl)-1-piperazinyl]cyclohexyl]-1H-indole

A solution of 4-(1H-indol-3-yl)-cyclohexanone (0.53 g,2.5 mmol), 1-(indol-4-yl)piperazine (0.5g, 2.5 mmol), sodium triacetoxyborohydride (0.78 g, 3.5 mmol) and acetic acid (0.14 ml, 2.5 mmol) in 1,2-dichloroethane (11 ml) was allowed to stir at room temperature overnight. The reaction was quenched with 1N sodium hydroxide (10 ml), extracted with methylene chloride (3 x 60 ml), and washed with brine (3 x 60 ml). The organic layer was dried over anhydrous sodium sulfate and filtered. Chromatography (10% methanol-ethyl acetate) afforded 0.52 g (53%) of product as a white solid: mp 85-87°C.
The HCl salt was prepared in ethyl acetate: mp 198-200°C.

| Elemental analysis for C₂₆H₃₀N₄•HCl | | | |
|---|---|---|---|
| Calc'd | C, 68.25; | H, 7.38; | N, 12.25 |
| Found | C, 68.12; | H, 7.16; | N, 11.93 |

### EXAMPLE 1b

### 3-[trans-4-[4-(1H-indol-4-yl)-1-piperazinyl] cyclohexyl]-1H-indole

The trans compound was isolated at the same time as the cis isomer in 21% yield (0.21 g) as a white solid: mp 105-107°C.
The HCl salt was prepared in ethyl acetate: mp 305°C (decomposed).

| Elemental analysis for C₂₆H₃₀N₄•HCl | | | |
|---|---|---|---|
| Calc'd | C, 68.25; | H, 7.38; | N, 12.25 |
| Found | C, 68.12; | H, 7.16; | N, 11.93 |

### EXAMPLE 2a

### 4-Fluoro-3-[cis-4-[4-(1H-indol-4-yl)-1-piperazinyl] cyclohexyl]-1H-indole

A solution of 4-(4-fluoro-1H-indol-3-yl)-cyclohexanone (0.88 g, 3.8 mmol), 1-(indol-4-yl)piperazine (0.7 g, 3.5 mmol), sodium triacetoxyborohydride (1.1 g, 5.2 mmol) and acetic acid (0.4 ml, 7 mmol) in 1,2-dichloroethane (20 ml) was allowed to stir at room temperature overnight. The reaction was quenched with 1N sodium hydroxide (10 ml), extracted with methylene chloride (3 x 60 ml), and washed with brine (3 x 60 ml). The organic layer was dried over anhydrous sodium sulfate and filtered. Chromatography (5-7% methanol-ethyl acetate) afforded 1.14 g (79%) of product as a white foam.
The HCl salt was prepared in ethanol: mp 283-285°C.

| Elemental analysis for C₂₆H₂₉FN₄•HCl•0.25H₂O | | | |
|---|---|---|---|
| Calc'd | C, 68.26; | H, 6.72; | N, 12.25 |
| Found | C, 68.16; | H, 6.74; | N, 12.04 |

### EXAMPLE 2b

### 4-Fluoro-3-[trans-4-[4-(1H-indol-4-yl)-1-piperazinyl] cyclohexyl]-1H-indole

The trans compound was isolated at the same time as the cis isomer in 17% yield (0.24 g) as a white solid: mp 206-208°C.
The HCl salt was prepared in ethanol: mp 297-299°C.

| Elemental analysis for C₂₆H₂₉FN₄•HCl•H₂O | | | |
|---|---|---|---|
| Calc'd | C, 66.30; | H, 6.85; | N, 11.90 |
| Found | C, 66.17; | H, 6.51; | N, 11.70 |

### EXAMPLE 3a

### 5-Fluoro-3-[cis-4-[4-(1H-indot-4-yl)-1-piperazinyl] cyclohexyl]-1H-indole

This compound was prepared in the manner described above for Example 2 by replacing 4-(4-fluoro-1H-indol-3-yl)-cyclohexanone with 4-(5-fluoro-1H-indol-3-yl)-cyclohexanone (0.56 g, 2.5 mmol) to afford 0.54 g (52%) of product as a white solid: mp 108-110°C.
The HCl salt was prepared in ethyl acetate: mp 215-217°C.

| Elemental analysis for C₂₆H₂₉FN₄•HCl•0.36C₄H₈O₂ | | | |
|---|---|---|---|
| Calc'd | C, 67.37; | H, 6.88; | N, 11.45 |
| Found | C, 67.18; | H, 6.72; | N, 11.18 |

### EXAMPLE 3b

### 5-Fluoro-3-[trans-4-[4-(1H-indol-4-yl)-1-piperazinyl] cyclohexyl]-1H-indole

The trans compound was isolated at the same time as the cis isomer in 30% yield (0.31 g) as a white solid: mp 112-114°C.
The HCl salt was prepared in ethanol: mp 280°C (decomposed).

| Elemental analysis for C₂₆H₂₉FN₄•HCl | | | |
|---|---|---|---|
| Calc'd | C, 66.81; | H, 6.81; | N, 11.99 |
| Found | C, 66.44; | H, 6.66; | N, 11.74 |

### EXAMPLE 4a

### 6-Fluoro-3-[cis-4-[4-(1H-indol-4-yl)-1-piperazinyl] cyclohexyl]-1H-indole

This compound prepared in the manner described above for Example 2 by replacing was 4-(4-fluoro-1H-indol-3-yl)-cyclohexanone with 4-(6-fluoro-1H-indol-3-yl)-cyclohexanone (1.15 g, 5.0 mmol) to afford 1.06 g (51%) of product as a white foam.
The HCl salt was prepared in ethanol: mp 250-252°C (decomposed).

| Elemental analysis for C₂₆H₂₉FN₄•HCl | | | |
|---|---|---|---|
| Calc'd | C, 67.37; | H, 6.88; | N, 11.45 |
| Found | C, 67.18; | H, 6.72; | N, 11.18 |

### EXAMPLE 4b

### 6-Fluoro-3-[trans-4-[4-(1H-indol-4-yl)-1-piperazinyl] cyclohexyl]-1H-indole

The trans compound was isolated at the same time as the cis isomer in 27% yield (0.55 g) as a white foam.
The HCl salt was prepared in ethanol: mp 319-320°C (decomposed).

| Elemental analysis for C₂₆H₂₉FN₄•HCl | | | |
|---|---|---|---|
| Calc'd | C, 66.81; | H, 6.81; | N, 11.99 |
| Found | C, 66.44; | H, 6.66; | N, 11.74 |

### EXAMPLE 5a

### 5-Bromo-3-[cis-4-[4-(1H-indol-4-yl)-1-piperazinyl] cyclohexyl]-1H-indole

This compound was prepared in the manner described above for Example 2 by replacing 4-(4-fluoro-1H-indol-3-yl)-cyclohexanone with 4-(5-bromo-1H-indol-3-yl)-cyclohexanone (0.75 g, 2.5 mmol) to afford 0.81 g (68%) of product.
The HCl salt was prepared in ethyl acetate: mp 276°C.

| Elemental analysis for C₂₆H₂₉BrN₄•HCl | | | |
|---|---|---|---|
| Calc'd | C, 60.23; | H, 5.93; | N, 10.81 |
| Found | C, 59.95; | H, 5.83; | N, 10.64 |

### EXAMPLE 5b

### 5-Bromo-3-[trans-4-[4-(1H-indol-4-yl)-1-piperazinyl] cyclohexyl]-1H-indole

The trans compound was isolated at the same time as the cis isomer in 24% yield (0.29 g).
The HCl salt was prepared in ethyl acetate: mp >300°C.

| Elemental analysis for C₂₆H₂₉BrN₄•HCl | | | |
|---|---|---|---|
| Calc'd | C, 60.75; | H, 5.88; | N, 10.90 |
| Found | C, 60.38; | H, 5.89; | N, 10.61 |

### EXAMPLE 6a

### 5-Chloro-3-[cis-4-[4-(1H-indol-4-yl)-1-piperazinyl] cyclohexyl]-1H-indole

A solution of 4-(5-chloro-1H-indol-3-yl)-cyclohexanone (0.78 g, 3.1 mmol), 1-(indol-4-yl)piperazine (0.6 g, 3 mmol), sodium triacetoxyborohydride (0.95 g, 4.5 mmol) and acetic acid (0.34 ml, 6 mmol) in 1,2-dichloroethane (20 ml) was allowed to stir at room temperature overnight. The reaction was quenched with 1N sodium hydroxide (10 ml), extracted with methylene chloride (3 x 60 ml) and washed with brine (3 x 60 ml). The organic layer was dried over anhydrous sodium sulfate and filtered. Chromatography (5% methanol-ethyl acetate) afforded 0.84 g (65%) of product as a white foam.
The HCl salt was prepared in ethanol: mp 283-285°C.

| Elemental analysis for C₂₆H₂₉ClN₄•HCl•0.25H₂O | | | |
|---|---|---|---|
| Calc'd | C, 65.46; | H, 6.69; | N, 11.45 |
| Found | C, 65.14; | H, 6.73; | N, 11.33 |

### EXAMPLE 6b

### 5-Chloro-3-[trans-4-[4-(1H-indol-4-yl)-1-piperazinyl] cyclohexyl]-1H-indole

The trans compound was isolated at the same time as the cis isomer in 24% yield (0.32 g) as a white foam.
The HCl salt was prepared in ethanol: mp 314-315.5°C.

| Elemental analysis for C₂₆H₂₉ClN₄•HCl•0.25H₂O | | | |
|---|---|---|---|
| Calc'd | C, 65.65; | H, 6.60; | N, 11.62 |
| Found | C, 65.50; | H, 6.50; | N, 11.30 |

### EXAMPLE 7a

### 3-{4-[(1,4-cis)-4-(1H-indol-4-yl)-piperazinyl-1-yl]cyclohexyl} -1H-indole-5-carbonitrile

This compound was prepared in the manner described above for Example 2 by replacing 4-(4-fluoro-1H-indol-3-yl)-cyclohexanone with 4-(5-cyano-1H-indol-3-yl)-cyclohexanone (0.71 g, 3.0 mmol) to afford 0.38 g (30%) of product.
The HCl salt was prepared in ethyl acetate: mp 216-218°C.

| Elemental analysis for C₂₇H₂₉N₅•HCl•0.33C₄H₈O₂ | | | |
|---|---|---|---|
| Calc'd | C, 66.25; | H, 6.94; | N, 13.64 |
| Found | C, 66.05; | H, 6.54; | N, 13.28 |

### EXAMPLE 7b

### 3-{4-[(1,4-trans)-4-(1H-indol-4-yl)-piperazinyl-1-yl]cyclohexyl} -1H-indole-5-carbonitrile

The trans compound was isolated at the same time as the cis isomer in 25% yield (0.32 g).
The HCl salt was prepared in ethyl acetate: mp >310°C.

| Elemental analysis for C₂₇H₂₉N₅•HCl | | | |
|---|---|---|---|
| Calc'd | C, 68.48; | H, 6.71; | N, 14.79 |
| Found | C, 68.43; | H, 6.54; | N, 14.63 |

### EXAMPLE 8a

### 5-Methoxy-3-[cis-4-[4-(1H-indol-4-yl)-1-piperazinyl] cyclohexyl]-1H-indole

A solution of 4-(5-methoxy-1H-indol-3-yl)-cyclohexanone (1.2 g, 5 mmol), 1-(indol-4-yl)piperazine (1 g, 5 mmol), sodium triacetoxyborohydride (1.47 g, 6.2 mmol) and acetic acid (0.28 ml, 4 mmol) in 1,2-dichloroethane (20 ml) was allowed to stir at room temperature overnight. The reaction was quenched with 1N sodium hydroxide (10 ml), extracted with methylene chloride (3 x 60 ml) and washed with brine (3 x 60 ml). The organic layer was dried over anhydrous sodium sulfate and filtered. Chromatography (2.5% methanol-ethyl acetate) afforded 1.18 g (55%) of product as a white solid: mp 105-108°C.
The HCl salt was prepared in ethyl acetate: mp 283-285°C.

| Elemental analysis for C₂₇H₃₂N₄O•HCl•0.5H₂O | | | |
|---|---|---|---|
| Calc'd | C, 68.55; | H, 7.03; | N, 11.85 |
| Found | C, 68.86; | H, 7.29; | N, 11.76 |

### EXAMPLE 8b

### 5-Methoxy-3-[trans-4-[4-(1H-indol-4-yl)-1-piperazinyl] cyclohexyl]-1H-indole

The trans compound was isolated at the same time as the cis isomer in 20% yield (0.43 g) as a white foam.
The HCl salt was prepared in ethyl acetate: mp 194-196°C.

| Elemental analysis for C₂₇H₃₂N₄O•HCl•1.5H₂O | | | |
|---|---|---|---|
| Calc'd | C, 66.65; | H, 7.15; | N, 11.52 |
| Found | C, 66.65; | H, 7.06; | N, 11.44 |

### EXAMPLE 9a

### 3-[cis-4-[4-(1H-Indol-4-yl)-1-piperazinyl]cyclohexyl] -2-methyl-1H-indole

A solution of 4-(1H-indol-3-yl)-cyclohexanone (1.44 g, 6.33 mmol), 1-(indol-4-yl)piperazine (1.27 g, 6.33 mmol), sodium triacetoxyborohydride (1.88 g, 8.86 mmol) and acetic acid (0.76 mg, 12.6 mmol) in 1,2-dichloroethane (100 ml) was allowed to stir at room temperature overnight. The reaction was quenched with 1N sodium hydroxide (80 ml), extracted with methylene chloride (3 x 300 ml), and washed with brine (150 ml). The organic layer was dried over anhydrous sodium sulfate and filtered. The solvent was removed under vacuum to afford an off-white solid. Trituration of the solid with warm methylene chloride (80 ml) followed by filtration afforded 0.88 g of white solid. The mother liquor was concentrated and chromatographed (2% methanol-methylene chloride) to afford another 0.18 g (total yield 40.7%) of product as a white solid: mp 279-280°C.
The HCl salt was prepared in ethanol: mp 200-203°C.

| Elemental analysis for C₂₇H₃₂N₄•2HCl | | | |
|---|---|---|---|
| Calc'd | C, 64.99; | H, 7.17; | N, 11.23 |
| Found | C, 65.05; | H, 7.07; | N, 11.23 |

### EXAMPLE 9b

### 3-[trans-4-[4-(1H-Indol-4-yl)-1-piperazinyl]cyclohexyl] -2-methyl-1H-indole

The trans compound was isolated at the same time as the cis isomer in 25.7% yield (0.67 g) as a white foam.
The HCl salt was prepared in ethanol: mp >310°C.

| Elemental analysis for C₂₇H₃₂N₄•2HCl | | | |
|---|---|---|---|
| Calc'd | C, 66.80; | H, 7.06; | N, 11.54 |
| Found | C, 66.84; | H, 6.87; | N, 11.37 |

### EXAMPLE 10a

### 3-{(1,4-cis)-4-[4-(1H-Indole-4-yl)-piperazin-1-yl]-cyclohexyl}-1H-pyrrolo[2,3-b]pyridine

This compound was prepared in the manner described above for Example 2 by replacing 4-(5-fluoro-1H-indol-3-yl)-cyclohexanone with 4-(1H-3-pyrrolo[2,3-b]pyridyl)-cyclohexanone (1.52 g, 7.1 mmol) in 27 % (0.79 g) yield as a white solid.
•The HCl salt was prepared in ethanol: mp >250°C (dec.)

| Elemental analysis for C₂₅H₂₉N₅•3HCl | | | |
|---|---|---|---|
| Calc'd | C, 58.49; | H, 6.38; | N, 13.64 |
| Found | C, 58.47; | H, 6.52; | N, 12.91 |

### EXAMPLE 10b

### 3-{(1,4-trans)-4-[4-(1H-Indole-4-yl)-piperazin-1-yl]-cyclohexyl}-1H-pyrrolo[2,3-b]pyridine

The trans compound was isolated at the same time as the cis isomer in 9 % yield (0.26 g) as a white solid: mp >228°C.
The HCl salt was prepared in ethanol: mp >250°C (dec.)

| Elemental analysis for C₂₅H₂₉N₅•3HCl | | | |
|---|---|---|---|
| Calc'd | C, 56.50; | H, 6.54; | N, 13.18 |
| Found | C, 56.45; | H, 6.63; | N, 12.98 |

### EXAMPLE 11

### 6-Fluoro-1-methyl-3-{cis-4-[4-(1-methyl-1H-indol-4-yl)-1-piperazinyl]cyclohexyl}-1H-indole

To a solution of 3-[cis-4-[4-(1H-indol-4-yl)-1-piperazinyl] cyclohexyl]-1H-indole (0.27 g, 0.65 mmol) in anhydrous N,N-dimethylformamide (4 ml) was added 60% sodium hydride (33.7 mg, 0.84 mmol) at room temperature. The mixture was allowed to stir for 30 minutes at room temperature, then iodomethane was added to the above solution. The resulting mixture was stirred for another 0.5 hour and then poured into water (80 ml) and extracted with ethyl acetate (2 x 80 ml). The organic layer was dried over anhydrous magnesium sulfate and filtered. Chromatography (20% acetone-hexanes) afforded 0.93 g (55%) of product as an oil which was heated in ethanol to afford a white solid: mp 188-190°C.
The HCl salt was prepared in ethanol: mp 253-254°C.

| Elemental analysis for C₂₈H₃₃N₄F•HCl•0.5H₂O | | | |
|---|---|---|---|
| Calc'd | C, 68.62; | H, 7.20; | N, 11.43 |
| Found | C, 68.98; | H, 6.80; | N, 11.47 |

### EXAMPLE 12a

### 3-{(1,4-cis)-4-[4-(1H-indol-4-yl)-piperazin-1-yl]-cyclohexyl}-1-methyl-1H-indole-5-carbonitrile

A solution of 4-(5-cyano-1-methyl-3-indolyl)-cyclohexanone (0.75 g, 3 mmol), 1-(indol-4-yl)piperazine (0.6 g, 3 mmol), sodium triacetoxyborohydride (0.95 g, 4.5 mmol) and acetic acid (0.34 ml, 6 mmol) in 1,2-dichloroethane (20 ml) was allowed to stir at room temperature overnight. The reaction was quenched with 1N sodium hydroxide (10 ml), extracted with methylene chloride (3 x 60 ml) and washed with brine (3 x 60 ml). The organic layer was dried over anhydrous sodium sulfate and filtered. Chromatography (10% methanol-ethyl acetate) afforded 0.73 g (56%) of product as a white solid: mp 274-275°C.
The HCl salt was prepared in ethyl acetate: mp 285.5-288°C.

| Elemental analysis for C₂₈H₃₁N₅•2HCl•H₂O | | | |
|---|---|---|---|
| Calc'd | C, 68.35; | H, 6.97; | N, 14.23 |
| Found | C, 68.51; | H, 6.65; | N, 14.06 |

### EXAMPLE 12b

### 3-{(1,4-trans)-4-[4-(1H-indol-4-yl)-piperazin-1-yl]-cyclohexyl}-1-methyl-1H-indole-5-carbonitrile

The trans compound was isolated at the same time as the cis isomer in 33% yield (0.42 g) as a white solid: mp 239-240°C.
The HCl salt was prepared in ethyl acetate: mp 286-288°C.

| Elemental analysis for C₂₈H₃₁N₅•2HCl•0.5H₂O | | | |
|---|---|---|---|
| Calc'd | C, 64.73; | H, 6.60; | N, 13.65 |
| Found | C, 64.55; | H, 6.42; | N, 13.41 |

### EXAMPLE 13a

### 1-Ethyl-3-{(1,4-cis)-4-[4-(1H-indol-4-yl)-piperazin-1-yl]-cyclohexyl}-1H-indole-5-carbonitrile

A solution of 4-(5-cyano-1-ethyl-indol-3-yl)-cyclohexanone (1.5 g, 5.6 mmol), 1-(indol-4-yl)piperazine (1.19 g, 5.9 mmol), sodium triacetoxyborohydride (1.73 g, 8.2 mmol) and acetic acid (0.9 ml, 15 mmol) in 1,2-dichloroethane (30 ml) was allowed to stir at room temperature overnight. The reaction was quenched with 1N sodium hydroxide (10 ml), extracted with methylene chloride (3 x 80 ml), and washed with brine (3 x 80 ml). The organic layer was dried over anhydrous sodium sulfate and filtered. Chromatography (2.5% methanol-ethyl acetate) afforded 0.98 g (39%) of product as a white solid: mp 226°C (dec.).
The HCl salt was prepared in ethyl acetate: mp 245°C.

| Elemental analysis for C₂₉H₃₃N₅•2HCl•0.25H₂O | | | |
|---|---|---|---|
| Calc'd | C, 65.84; | H, 6.76; | N, 13.24 |
| Found | C, 65.97; | H, 6.74; | N, 13.40 |

### EXAMPLE 13b

### 1-Ethyl-3-{(1,4-trans)-4-[4-(1H-indol-4-yl)-piperazin-1-yl]-cyclohexyl}-1H-indole-5-carbonitrile

The trans compound was isolated at the same time as the cis isomer in 19% yield (0.48 g) as a light brown solid: mp decomposed at 110°C.
The HCl salt was prepared in ethyl acetate: mp 250°C (decomposed).

| Elemental analysis for C₂₉H₃₃N₅•2HCl | | | |
|---|---|---|---|
| Calc'd | C, 66.40; | H, 6.73; | N, 13.35 |
| Found | C, 66.32; | H, 6.67; | N, 13.10 |

### EXAMPLE 14a

### 3-{(1,4-cis)-4-[4-(1H-indol-4-yl)-piperazin-1-yl]-cyclohexyl}-1-propyl-1H-indole-5-carbonitrile

A solution of 4-(5-cyano-1-n-propyl-indol-3-yl)-cyclohexanone (1.68 g, 6 mmol), 1-(indol-4-yl)piperazine (1.27 g, 6.3 mmol), sodium triacetoxyborohydride (1.84 g, 8.9 mmol) and acetic acid (0.94 ml, 16 mmol) in 1,2-dichloroethane (80 ml) was allowed to stir at room temperature overnight. The reaction was quenched with 1N sodium hydroxide (20 ml), extracted with methylene chloride (3 x 100 ml) and washed with brine (3 x 100 ml). The organic layer was dried over anhydrous sodium sulfate and filtered. Chromatography (10% methanol-ethyl acetate) afforded 0.42 g(15%) of product as a white powder.
The HCl salt was prepared in ethanol: mp 200-206°C.

| Elemental analysis for C₃₀H₃₅N₅•2HCl•0.75H₂O | | | |
|---|---|---|---|
| Calc'd | C, 65.27; | H, 7.03; | N, 12.69 |
| Found | C, 65.18; | H, 6.97; | N, 12.68 |

### EXAMPLE 14b

### 3-{(1,4-trans)-4-[4-(1H-indol-4-yl)-piperazin-1-yl]-cyclohexyl}-1-propyl-1H-indole-5-carbonitrile

The trans compound was isolated at the same time as the cis isomer in 14% yield (0.39 g) as a white foam.
The HCl salt was prepared in ethanol: mp decomposed >245°C.

| Elemental analysis for C₃₀H₃₅N₅•2HCl | | | |
|---|---|---|---|
| Calc'd | C, 66.90; | H, 6.93; | N, 13.00 |
| Found | C, 66.68; | H, 6.97; | N, 12.96 |

### EXAMPLE 15a

### 3-{(1,4-cis)-4-[4-(1H-indol-4-yl)-piperazin-1-yl]-cyclohexyl}-1-isopropyl-1H-indole-5-carbonitrile

A solution of 4-(5-cyano-1-n-propyl-indol-3-yl)-cyclahexanone (1.68 g, 6 mmol), 1-(indol-4-yl)piperazine (1.27 g, 6.3 mmol), sodium triacetoxyborohydride (1.84 g, 8.9 mmol) and acetic acid (0.94 ml, 16 mmol) in 1,2-dichloroethane (80 ml) was allowed to stir at room temperature overnight. The reaction was quenched with 1N sodium hydroxide (20 ml), extracted with methylene chloride (3 x 100 ml), and washed with brine (3 x 100 ml). The organic layer was dried over anhydrous sodium sulfate, and filtered. Chromatography (10% methanol-ethyl acetate) afforded 0.49 g (18 %) of product as a white powder.
The HCl salt was prepared in ethanol: mp 285-286°C.

| Elemental analysis for C₃₀H₃₅N₅•HCl•0.5H₂O | | | |
|---|---|---|---|
| Calc'd | C, 70.50; | H, 7.30; | N, 13.70 |
| Found | C, 70.65; | H, 7.16; | N, 13.45 |

### EXAMPLE 15b

### 3-{(1,4-trans)-4-[4-(1H-indol-4-yl)-piperazin-1-yl]-cyclohexyl}-1-isopropyl-1H-indole-5-carbonitrile

The trans compound was isolated at the same time as the cis isomer in 12% yield (0.34 g) as a white foam.
The HCl salt was prepared in ethanol: mp decomposed > 245°C.

| Elemental analysis for C₃₀H₃₅N₅•HCl | | | |
|---|---|---|---|
| Calc'd | C, 66.90; | H, 6.93; | N, 13.00 |
| Found | C, 66.68; | H, 6.97; | N, 12.96 |

### EXAMPLE 16a

### 1-Benzyl-3-{(1,4-cis)-4-[4-(1H-indol-4-yl)-piperazin-1-yl]-cyclohexyl}-1H-indole-5-carbonitrile

A solution of 4-(5-cyano-1-benzyl-indol-3-yl)-cyclohexanone (2.97 g, 9 mmol), 1-(indol-4-yl)piperazine (1.94 g, 9.6 mmol), sodium triacetoxyborohydride (2.7 g, 13 mmol) and acetic acid (1 ml, 24 mmol) in 1,2-dichloroethane (50 ml) was allowed to stir at room temperature overnight. The reaction was quenched with 1N sodium hydroxide (20 ml), extracted with methylene chloride (3 x 100 ml) and washed with brine (3 x 100 ml). The organic layer was dried over anhydrous sodium sulfate and filtered. Chromatography (25-50% ethyl acetate-hexanes) afforded 1.71 g (37%) of product as a white powder.
The HCl salt was prepared in ethanol: mp dec. > 245°C.

| Elemental analysis for C₃₄H₃₅N₅•HCl•0.5H₂O | | | |
|---|---|---|---|
| Calc'd | C, 68.56; | H, 6.43; | N, 11.76 |
| Found | C, 68.93; | H, 6.55; | N, 11.52 |

### EXAMPLE 16b

### 1-Benzyl-3-{(1,4-trans)-4-[4-(1H-indol-4-yl)-piperazin-1-yl]-cyclohexyl}-1H-indole-5-carbonitrile

The trans compound was isolated at the same time as the cis isomer in 15% yield (0.68 g) as a white foam.
The HCl salt was prepared in ethanol: mp> 240°C (dec.).

| Elemental analysis for C₃₄H₃₅N₅•2HCl•0.25H₂O | | | |
|---|---|---|---|
| Calc'd | C, 69.08; | H, 6.40; | N, 11.85 |
| Found | C, 69.09; | H, 6.17; | N, 11.80 |

### EXAMPLE 17

### 1-Methyl-3-{(1,4-cis)-4-[4-(1-methyl-1H-indol-4-yl)-piperazin-1-yl]-cyclohexyl}-1H-indole-5-carbonitrile

To a suspension of sodium hydride (60%, 95 mg, 2.4 mmol) in anhydrous N, N-dimethylformamide was added a solution 3-{(1,4-cis)-4-[4-(1H-indol-4-yl)-piperazin-1-yl]-cyclohexyl}-1-methyl-1H-indole-5-carbonitrile (0.52 g, 1.2 mmol) in 10 ml N,N-dimethylformide. The mixture was allowed to stir at room temperature for 30 minutes. Then iodomethane (0.17 g, 2.4 mmol) was added to the above reaction mixture. The mixture was allowed to stir at room temperature for another 30 minutes, then quenched with ice-water. The mixture was extracted with methylene chloride (150 ml), and dried over anhydrous sodium sulfate. Chromatography (methanol-methylene chloride-ethyl acetate; 1 : 1 : 8) afforded 0.53 g (99%) of product as a pink foam.
The HCl salt was prepared in ethanol: mp 252-255°C.

| Elemental analysis for C₂₉H₃₃N₅•2HCl | | | |
|---|---|---|---|
| Calc'd | C, 66.40; | H, 6.73; | N, 13.35 |
| Found | C, 66.64; | H, 6.82; | N, 13.21 |

### EXAMPLE 18

### 5-Fluoro-3-{(cis)-4-[4-(2-methoxy-phenyl)-piperazin-1-yl] -cyclohexyl}-1H-indole

A solution of 4-(5-fluoro-1-indol-3-yl)-cyclohexanone (0.35 g, 1.5 mmol), 1-(2-methoxy-phenyl)piperazine (0.29 g, 1.5 mmol), sodium triacetoxyborohydride (0.47 g, 2.1 mmol) and acetic acid (0.05 ml, 1.5 mmol) in 1,2-dichloroethane (8 ml) was allowed to stir at room temperature for 12 hours. The reaction was quenched with 1N sodium hydroxide (pH > 9) and extracted with methylene chloride (3 x 50 ml). The organic layer was dried over anhydrous sodium sulfate and filtered. Chromatography (10% methanol-ethyl acetate) afforded 0.34g (56%) of product as a white solid.
The HCl salt was prepared in ethyl acetate: mp 170-172°C.

| Elemental analysis for C₂₅H₃₀FN₃O•HCl | | | |
|---|---|---|---|
| Calc'd | C, 66.95; | H, 7.08; | N, 9.37 |
| Found | C, 66.93; | H, 7.08; | N, 9.29 |

### EXAMPLE 19a

### 5-Fluoro-3-{(1,4-cis)-4-[4-(2-methoxy-phenyl)-piperidin-1-yl] -cyclohexyl}-1H-indole

This compound was prepared in the manner described above for Example 18 by replacing 1-(2-methoxy-phenyl)piperazine with 1-(2-methoxy-phenyl)piperidine (1.0 g, 5.2 mmol) to afford 1.34 g of product in 63% yield.
The HCl salt was prepared in ethyl acetate: mp 245-250°C.

| Elemental analysis for C₂₆H₃₁FN₂O•HCl•0.09C₄H₈O₂ | | | |
|---|---|---|---|
| Calc'd | C, 69.09; | H, 7.36; | N, 6.20 |
| Found | C, 66.19; | H, 7.18; | N, 6.08 |

### EXAMPLE 19b

### 5-Fluoro-3-{(1,4-trans)-4-[4-(2-methoxy-phenyl)-piperidin-1-yl] -cyclohexyl}-1H-indole

The trans compound was isolated at the same time as the cis isomer in 20% yield (0.43 g).
The HCl salt was prepared in ethyl acetate: mp 297-299°C.

| Elemental analysis for C₂₆H₃₁FN₂O•HCl•0.08C₄H₈O₂ | | | |
|---|---|---|---|
| Calc'd | C, 70.49; | H, 7.28; | N, 6.32 |
| Found | C, 70.17; | H, 7.30; | N, 6.10 |

### EXAMPLE 20a

### 5-Methoxy-3-{(1,4-cis)-4-[4-(2-methoxy-phenyl)-piperazin-1-yl] -cyclohexyl}-1H-indole

This compound was prepared in the manner described above for Example 18 by replacing 4-(5-fluoro-1-indol-3-yl)-cyclohexanone with 4-(5-methoxy-1-indol-3-yl)-cyclohexanone (1.2 g, 5 mmol) to afford 1.18 g (55 %) of the title compound as a white solid: mp 105-108°C.
The HCl salt was prepared in ethyl acetate: mp 198-199°C.

| Elemental analysis for C₂₆H₃₃N₃O₂•HCl | | | |
|---|---|---|---|
| Calc'd | C, 68.48; | H, 7.52; | N, 9.21 |
| Found | C, 68.31; | H, 7.54; | N, 9.08 |

### EXAMPLE 20b

### 5-Methoxy-3-{(1,4-trans)-4-[4-(2-methoxy-phenyl)-piperazin-1-yl] -cyclohexyl}-1H-indole

The trans compound was isolated at the same time as the cis isomer in 20% yield (0.43 g) as a white foam.
The HCl salt was prepared in ethyl acetate: mp 195-197°C.

| Elemental analysis for C₂₆H₃₃N₃O₂•HCl | | | |
|---|---|---|---|
| Calc'd | C, 68.48; | H, 7.52; | N, 9.21 |
| Found | C, 68.18; | H, 7.50; | N, 9.11 |

### EXAMPLE 21

### 3-{(1,4-cis)-4-[4-(2-methoxy-phenyl)-piperazin-1-yl)-cyclohexyl}-1H-pyrrolo[2,3-b]pyridine

This compound was prepared in the manner described above for Example 18 by replacing 4-(5-fluoro-1H-indol-3-yl)-cyclohexanone with 4-(1H-pyrrolo[2,3-b]-3-pyridyl)-cyclohexanone (1.71 g, 7.9 mmol) in 42 % yield (1.34 g) as a white solid:
mp 170-172°C.
The HCl salt was prepared in ethanol: mp 259-261°C.

| Elemental analysis for C₂₄H₃₀ON₄•HCl | | | |
|---|---|---|---|
| Calc'd | C, 65.44; | H, 7.44; | N, 12.72 |
| Found | C, 65.60; | H, 7.36; | N, 12.22 |

### EXAMPLE 22a

### 5-Fluoro-3-{(cis)-4-[4-(5-fluoro-2-methoxy-phenyl)-piperazin-1-yl]-cyclohexyl}-1H-indole

A solution of 4-(5-fluoro-1-indol-3-yl)-cyclohexanone (1.1 g, 4.8 mmol), 1-(2-methoxy-5-fluoro-phenyl)piperazine (1.0 g, 4.8 mmol), sodium triacetoxyborohydride (1.5 g, 7.1 mmol) and acetic acid (0.27 ml, 4.7 mmol) in 1,2-dichloroethane (20 ml) was allowed to stir at room temperature for 12 hours. The reaction was quenched with 1N sodium hydroxide (pH > 9), extracted with methylene chloride (3 x 50 ml). The organic layer was dried over anhydrous sodium sulfate and filtered. Chromatography (10% methanol-ethyl acetate) afforded 1.16 g (53%) of product as a white solid: mp 152-153°C.
The HCl salt was prepared in ethyl acetate: mp 171-174°C.

| Elemental analysis for C₂₅H₂₉F₂N₃O•2HCl | | | |
|---|---|---|---|
| Calc'd | C, 59.17; | H, 6.36; | N, 8.28 |
| Found | C, 59.20; | H, 6.33; | N, 8.09 |

### EXAMPLE 22b

### 5-Fluoro-3-{(trans)-4-[4-(5-fluoro-2-methoxy-phenyl)-piperazin-1-yl]-cyclohexyl}-1H-indole

The trans compound was isolated at same time as the cis isomer in 12% yield (0.25 g) as a white solid: mp 64-67°C.
The HCl salt was prepared in ethyl acetate: mp 272-273.5°C.

| •Elemental analysis for C₂₅H₂₉F₂ON₃•HCl | | | |
|---|---|---|---|
| Calc'd | C, 63.75; | H, 6.64; | N, 8.92 |
| Found | C, 63.77, | H, 6.41; | N, 8.75 |

### EXAMPLE 23a

### 3-{(1,4-cis)-4-[4-(2,3-Dihydro-benzo[1,4]dioxin-5-yl)-piperazin-1-yl]-cyclohexyl}-4-fluoro-1H-indole

A solution of 4-(4-fluoro-1-indol-3-yl)-cyclohexanone (0.71 g, 3.1 mmol), 5-(1-piperazinyl)benzodioxan (0.77 g, 3.5 mmol), sodium triacetoxyborohydride (0.98 g, 4.6 mmol) and acetic acid (0.28 g, 4.6 mmol) in 1,2-dichloroethane (70 ml) was allowed to stir at room temperature for 12 hours. The reaction was quenched with 1N sodium hydroxide (100 ml), extracted with methylene chloride (3 x 100 ml). The organic layer was dried over anhydrous magnesium sulfate and filtered. Chromatography (1% methanol-ethyl acetate) afforded 0.8 g (53%) of product as a white foam which was dissolved in warm ethanol (15 ml) and crystallized to afford a white solid: mp 194-195.5°C.
The HCl salt was prepared in ethanol: mp 215-220°C.

| Elemental analysis for C₂₆H₃₀FN₃O₂•HCl | | | |
|---|---|---|---|
| Calc'd | C, 61.42; | H, 6.34; | N, 8.62 |
| Found | C, 61.15; | H, 6.29; | N, 8.04 |

### EXAMPLE 23b

### 3-{(1,4-trans)-4-[4-(2,3-Dihydro-benzo[1,4]dioxin-5-yl)-piperazin-1-yl]-cyclohexyl}-4-fluoro-1H-indole

The trans compound was isolated at the same time as the cis isomer in 14% yield (0.21 g) as a white foam which was recrystallization in ethanol to afford a white solid: mp 188-190°C.

| Elemental analysis for C₂₆H₃₀FO₂N₃ | | | |
|---|---|---|---|
| Calc'd | C, 71.70; | H, 6.94; | N, 9.65 |
| Found | C, 71.33, | H, 7.03; | N, 9.55 |

### EXAMPLE 24a

### 3-{(1,4-cis)-4-[4-(2,3-Dihydro-benzo[1,4]dioxin-5-yl)-piperazin-1-yl]-cyclohexyl}-5-fluoro-1H-indole

A solution of 4-(5-fluoro-1-indol-3-yl)-cyclohexanone (1.06 g, 4.6 mmol), 5-(1-piperazinyl)benzodioxan (1.14 g, 5.2 mmol), sodium triacetoxyborohydride (1.46 g, 6.9 mmol) and acetic acid (0.41 g, 6.9 mmol) in 1,2-dichloroethane (80 ml) was allowed to stir at room temperature for 12 hours. The reaction was quenched with saturated sodium bicarbonate (100 ml), extracted with methylene chloride (3 x 100 ml). The organic layer was dried over anhydrous magnesium sulfate and filtered. Chromatography (1% methanol-ethyl acetate) afforded 1.06 g (53%) of product as an oil which solidified to afford a white solid: mp 104-106°C.
The HCl salt was prepared in ethanol: mp 222-225°C.

| Elemental analysis for C₂₆H₃₀FN₃O₂•2HCl•0.2H₂O | | | |
|---|---|---|---|
| Calc'd | C, 60.88; | H, 6.39; | N, 8.19 |
| Found | C, 60.85; | H, 6.03; | N, 8.13 |

### EXAMPLE 24b

### 3-{(1,4-trans)-4-[4-(2,3-Dihydro-benzo[1,4]dioxin-5-yl)-piperazin-1-yl]-cyclohexyl}-5-fluoro-1H-indole

The trans compound was isolated at the same time as the cis isomer in 27% yield (0.53 g) as a white solid: mp 206-210°C.
The HCl salt was prepared in ethanol: mp 295-297°C.

| Elemental analysis for C₂₆H₃₀FO₂N₃•2HCl | | | |
|---|---|---|---|
| Calc'd | C, 61.42; | H, 6.34; | N, 8.26 |
| Found | C, 61.22; | H, 6.19; | N, 8.13 |

### EXAMPLE 25a

### 3-{(1,4-cis)-4-[4-(2,3-Dihydro-benzo[1,4]dioxin-5-yl)-piperazin-1-yl]-cyclohexyl}-6-fluoro-1H-indole

A solution of 4-(5-fluoro-1-indol-3-yl)-cyclohexanone (0.77 g, 3.0 mmol), 5-(1-piperazinyl)benzodioxan (0.78 g, 3.0 mmol), sodium cyanoborohydride (0.2 g, 3.0 mmol) in methanol (100 ml) was allowed to stir at room temperature for 48 h. The reaction was quenched with potassium hydroxide (0.4 g). The methanol was removed under vacuum, the residue was extracted with ethyl acetate (3 x 100 ml) and washed with water. The organic layer was dried over anhydrous magnesium sulfate and filtered. Chromatography (1% methanol-ethyl acetate) afforded 0.24 g (18%) of product as a yellow solid.
The HCl salt was prepared in ethanol: mp 228-230°C.

| Elemental analysis for C₂₆H₃₀FN₃O₂•2HCl•0.6C₂H₆O | | | |
|---|---|---|---|
| Calc'd | C, 61.37; | H, 6.38; | N, 8.22 |
| Found | C, 61.19; | H, 6.32; | N, 8.29 |

### EXAMPLE 25b

### 3-{(1,4-trans)-4-[4-(2,3-Dihydro-benzo[1,4]dioxin-5-yl)-piperazin-1-yl]-cyclohexyl}-6-fluoro-1H-indole

The trans compound was isolated at the same time as the cis isomer in 8% yield (0.11 g) as an oil.
The HCl salt was prepared in ethanol: mp 309-310°C.

| Elemental analysis for C₂₆H₃₀FO₂N₃•2HCl•0.08C₄H₈O₂ | | | |
|---|---|---|---|
| Calc'd | C, 61.42; | H, 6.34; | N, 8.26 |
| Found | C, 61.22; | H, 6.19; | N, 8.13 |

### EXAMPLE 26a

### 3-{(1,4-cis)-4-[4-(2,3-Dihydro-benzo[1,4]dioxin-5-yl)-piperazin-1-yl]-cyclohexyl}-1H-indole-5-carbonitrile

A solution of 4-(5-cyano-1-indol-3-yl)-cyclohexanone (0.60 g, 2.5 mmol), 5-(1-piperazinyl)benzodioxane (0.55 g, 2.5 mmol), sodium triacetoxyborohydride (0.78 g, 3.5 mmol) and acetic acid (0.14 g, 2.5 mmol) in 1,2-dichloroethane (11 ml) was allowed to stir at room temperature for 12 hours. The reaction was quenched with 1N sodium (100 ml), extracted with methylene chloride (3 x 100 ml). The organic layer was dried over anhydrous magnesium sulfate, and filtered. Chromatography (1% methanol-ethyl acetate) afforded 0.46 g (41%) of product.
The HCl salt was prepared in ethyl acetate: mp 300°C.

| Elemental analysis for C₂₇H₃₀N₄O₂•HCl•0.07C₄H₈O₂ | | | |
|---|---|---|---|
| Calc'd | C, 65.84; | H, 6.65; | N, 11.38 |
| Found | C, 65.65; | H, 6.47; | N, 11.11 |

### EXAMPLE 26b

### 3-{(1,4-trans)-4-[4-(2,3-Dihydro-benzo[1,4]dioxin-5-yl)-piperazin-1-yl]-cyclohexyl}-1H-indole-5-carbonitrile

The trans compound was isolated at the same time as the cis isomer in 31% yield (0.34 g).
The HCl salt was prepared in ethyl acetate: mp 300°C (decomposed).

| Elemental analysis for C₂₇H₃₀O₂N₄•HCl•0.08C₄H₈O₂ | | | |
|---|---|---|---|
| Calc'd | C, 66.43; | H, 6.69; | N, 11.34 |
| Found | C, 66.57; | H, 7.02; | N, 10.85 |

### EXAMPLE 27a

### 8-{4-[(1,4-cis)-4-(5-Fluoro-1H-indol-3-yl)-cyclohexyl] -piperazin-1-yl}-quinoline

A solution of 4-(5-fluoro-1-indol-3-yl)-cyclohexanone (0.54 g, 2.3 mmol), 8-(piperazin-1-yl)-quinoline (0.5 g, 2.3 mmol), sodium triacetoxyborohydride (0.75 g, 3.5 mmol) and acetic acid (0.27 ml, 4.7 mmol) in 1,2-dichloroethane (20 ml) was allowed to stir at room temperature for overnight. The reaction was quenched with 1N sodium hydroxide (20 ml), extracted with methylene chloride (3 x 100 ml), and washed with brine (3 x 100 ml). The organic layer was dried over anhydrous sodium sulfate and filtered. Chromatography (5% methanol-ethyl acetate) afforded 0.46 g (46%) of product as a white solid: mp 122-125°C.
The HCl salt was prepared in ethanol: mp 209-212°C.

| Elemental analysis for C₂₇H₂₉FN₄•3HCl | | | |
|---|---|---|---|
| Calc'd | C, 66.28; | H, 6.00; | N, 10.42 |
| Found | C, 60.23; | H, 6.29; | N, 10.21 |

### EXAMPLE 27b

### 8-{4-[(1,4-trans)-4-(5-Fluoro-1H-indol-3-yl)-cyclohexyl] -piperazin-1-yl}-quinoline

The trans compound was isolated at the same time as the cis isomer in 25% yield (0.25 g) as a white solid: mp 207.5-209°C.
The HCl salt was prepared in ethanol: mp 286-288°C.

| Elemental analysis for C₂₇H₂₉FN₄•HCl | | | |
|---|---|---|---|
| Calc'd | C, 64.67; | H, 6.23; | N, 11.17 |
| Found | C, 64.74; | H, 6.27; | N, 11.06 |

### EXAMPLE 28

### 8-{4-(1,4-cis)-4-[4-(5-Fluoro-1-methyl-1H-indol-3-yl)-cyclohexyl] -piperazin-1-yl}-quinoline

To a suspension of sodium hydride (60%, 0.03 g, 0.76 mmol) in anhydrous N, N-dimethylformamide (4 ml) was added 8-{4-[(1,4-cis)-4-(5-fluoro-1H-indol-3-yl)-cyclohexyl]-piperazin-1-yl}-quinoline (0.25 g, 0.58 mmol) in 6 ml anhydrous N, N-dimethylformamide at room temperature. The mixture was stirred at room temperature for 30 minutes, then iodomethane (0.044 ml, 0.7 mmol) was added to the above solution. The resulting mixture was stirred at room temperature for 30 minutes, and quenched with water. The mixture was extracted with ethyl acetate and the organic layer was dried over anhydrous sodium sulfate. The solvent was removed under vacuum. Chromatography (50% methylene-ethylactate plus 5% methanol) afforded 0.22 g (85%) of product as a yellow solid: mp >200°C.
The HCl salt was prepared in ethanol: mp 261-263.5°C.

| Elemental analysis for C₂₈H₃₁FN₄•2HCl•H₂O | | | |
|---|---|---|---|
| Calc'd | C, 63.03; | H, 6.61; | N, 10.50 |
| Found | C, 63.39; | H, 6.43; | N, 10.21 |

### EXAMPLE 29a

### 3-[(1,4-cis)-4-(4-Quinolin-8-yl-piperazin-1-yl)-cyclohexyl] -1H-indole-5-carbonitrile

A solution of 4-(5-cyano-1-indol-3-yl)-cyclohexanone (1.47 g, 6.2 mmol), 8-(piperazin-1-yl)-quinoline (1.32 g, 6.2 mmol), sodium triacetoxyborohydride (2.0 g, 7.2 mmol) and acetic acid (0.71 ml, 12 mmol) in 1,2-dichloroethane (40 ml) was allowed to stir at room temperature overnight. The reaction was quenched with 1N sodium hydroxide (20 ml), extracted with methylene chloride (3 x 100 ml), and washed with brine (3 x 100 ml). The organic layer was dried over anhydrous sodium sulfate and filtered. Chromatography (5% methanol-ethyl acetate) afforded 1.48 g(55%) of product as a white solid: mp 149-151°C.
The HCl salt was prepared in ethanol: mp 209-212°C.

| Elemental analysis for C₂₇H₂₉FN₄•2HCl•0.75H₂O | | | |
|---|---|---|---|
| Calc'd | C, 64.43; | H, 6.28; | N, 13.58 |
| Found | C, 64.46; | H, 6.29; | N, 13.37 |

### EXAMPLE 29b

### 3-[(1,4-trans)-4-(4-Quinolin-8-yl-piperazin-1-yl)-cyclohexyl] -1H-indole-5-carbonitrile

The trans compound was isolated at the same time as the cis isomer in 26% yield (0.55 g) as a white solid: mp 276-278°C.
The HCl salt was prepared in ethanol: mp 286-288°C.

| Elemental analysis for C₂₇H₂₉FN₄•2HCl•0.5H₂O | | | |
|---|---|---|---|
| Calc'd | C, 64.98; | H, 6.23; | N, 13.53 |
| Found | C, 65.28; | H, 5.96; | N, 13.30 |

### EXAMPLE 30

### 1-Methyl-3-[(1,4-cis)-4-(4-quinolin-8-yl-piperazin-1-yl)-cyclohexyl]-1H-indole-5-carbonitrile

To a suspension of sodium hydride (60%, 0.06 g, 1.4 mmol) in anhydrous N, N-dimethylformamide (8 ml) was added 3-[(1,4-cis)-4-(4-quinolin-8-yl-piperazin-1-yl)-cyclohexyl]-1H-indole-5-carbonitrile (0.30 g, 0.69 mmol) in 4 ml anhydrous N, N-dimethylformamide at room temperature. The mixture was stirred at room temperature for 30 minutes, followed by the addition of iodomethane (0.051 ml, 0.83 mmol) to the above solution. The resulting mixture was stirred at room temperature for 30 minutes and quenched with water. The mixture was extracted with ethyl acetate, dried over anhydrous sodium sulfate, and the solvent removed under vacuum.
Chromatography (50% methylene-ethyl acetate plus 5% methanol) afforded 0.27 g (90%) of product as a light yellow solid: mp 208-209°C.
The HCl salt was prepared in ethanol: mp 288-289°C.

| Elemental analysis for C₂₉H₃₁N₅•2HCl•0.15C₄H₁₀O | | | |
|---|---|---|---|
| Calc'd | C, 66.62; | H, 6.52; | N, 13.12 |
| Found | C, 66.79; | H, 6.74; | N, 12.81 |

### EXAMPLE 31a

### 5-Fluoro-3-{(1,4-cis)-4-[4-(6-fluoro-chroman-8-yl)-piperazin-1-yl]-cyclohexyl}-1H-indole

A solution of 4-(5-fluoro-1-indol-3-yl)-cyclohexanone (0.49 g, 2.1 mmol), 4-(6-fluoro-chroman-8-yl)-piperazine (0.5 g, 2.1 mmol), sodium triacetoxyborohydride (0.67 g, 3.2 mmol) and acetic acid (0.24 ml, 4.2 mmol) in 1,2-dichloroethane (20 ml) was allowed to stir at room temperature overnight. The reaction was quenched with 1N sodium hydroxide (20 ml), extracted with methylene chloride (3 x 100 ml), and washed with brine (3 x 100 ml). The organic layer was dried over anhydrous sodium sulfate and filtered. Chromatography (5% methanol-ethyl acetate) afforded 0.42 g (44%) of product as a white foam.
The HCl salt was prepared in ethanol: mp 199-200.5°C.

| Elemental analysis for C₂₇H₃₁F₂ON₃•HCl•0.5H₂O | | | |
|---|---|---|---|
| Calc'd | C, 65.25; | H, 6.69; | N, 8.45 |
| Found | C, 65.04; | H, 6.61; | N, 8.29 |

### EXAMPLE 31b

### 5-Fluoro-3-{(1,4-trans)-4-[4-(6-fluoro-chroman-8-yl)-piperazin-1-yl]-cyclohexyl}-1H-indole

The trans compound was isolated at the same time as the cis isomer in 35% yield (0.33 g) as a clear oil.
The HCl salt was prepared in ethanol: mp 286-288°C.

| Elemental analysis for C₂₇H₃₁F₂ON₃•HCl•0.5H₂O | | | |
|---|---|---|---|
| Calc'd | C, 65.25; | H, 6.69; | N, 8.45 |
| Found | C, 65.09; | H, 6.63; | N, 8.29 |

### EXAMPLE 32a

### 5-Fluoro-3-{(1,4-cis)-4-[4-(5-fluoro-2,3-dihydro-benzofuran-7-yl)-piperazin-1-yl]-cyclohexyl}-1H-indole

A solution of 4-(5-fluoro-1-indol-3-yl)-cyclohexanone (0.52 g, 2.2 mmol), 4-(5-fluoro-2,3-dihydro-benzofuran-7-yl)-piperazine (0.5 g, 2.2 mmol), sodium triacetoxyborohydride (0.72 g, 3.4 mmol) and acetic acid (0.26 ml, 4.5 mmol) in 1,2-dichloroethane (20 ml) was allowed to stir at room temperature overnight. The reaction was quenched with 1N sodium hydroxide (20 ml), extracted with methylene chloride (3 x 100 ml), and washed with brine (3 x 100 ml). The organic layer was dried over anhydrous sodium sulfate and filtered. Chromatography (5% methanol-ethyl acetate) afforded 0.37 g (38%) of product as a white solid: mp 182-183.5°C.
The HCl salt was prepared in ethanol: mp 196-198°C.

| Elemental analysis for C₂₆H₂₉F₂ON₃•HCl•0.5H₂O | | | |
|---|---|---|---|
| Calc'd | C, 64.65; | H, 6.47; | N, 8.70 |
| Found | C, 64.45; | H, 6.20; | N, 8.60 |

### EXAMPLE 32b

### 5-Fluoro-3-{(1,4-trans)-4-[4-(5-fluoro-2,3-dihydro-benzofuran-7-yl)-piperazin-1-yl]-cyclohexyl}-1H-indole

The trans compound was isolated at the same time as the cis isomer in 34% yield (0.34 g) as a clear oil.
The HCl salt was prepared in ethanol: mp 303-305°C.

| Elemental analysis for C₂₆H₂₉F₂ON₃•HCl•0.5H₂O | | | |
|---|---|---|---|
| Calc'd | C, 64.65; | H, 6.47; | N, 8.70 |
| Found | C, 64.86; | H, 6.40; | N, 8.36 |

### EXAMPLE 33a

### 3-{(1,4-cis)-4-[4-(5-Fluoro-2,3-dihydro-benzofuran-7-yl)-piperazin-1-yl]-cyclohexyl}-1H-indole-5-carbonitrile

A solution of 4-(5-cyano-1-indol-3-yl)-cyclohexanone (0.46 g, 1.9 mmol), 4-(5-fluoro-2,3-dihydro-benzofuran-7-yl)-piperazine (0.43 g, 1.9 mmol), sodium triacetoxy-borohydride (0.62 g, 2.9 mmol) and acetic acid (0.22 ml, 3.9 mmol) in 1,2-dichloroethane (20 ml) was allowed to stir at room temperature overnight. The reaction was quenched with 1N sodium hydroxide (20 ml), extracted with methylene chloride (3 x 100 ml), and washed with brine (3 x 100 ml). The organic layer was dried over anhydrous sodium sulfate and filtered. Chromatography (5% methanol-ethyl acetate) afforded 0.35 g (41 %) of product as a white foam.
The HCl salt was prepared in ethanol: mp 298-301°C.

| Elemental analysis for C₂₇H₂₉FON₄•HCl•0.75H₂O | | | |
|---|---|---|---|
| Calc'd | C, 65.58; | H, 6.42; | N, 11.33 |
| Found | C, 65.38; | H, 6.22; N, 11.14 | |

### EXAMPLE 33b

### 3-{(1,4-trans)-4-[4-(5-Fluoro-2,3-dihydro-benzofuran-7-yl)-piperazin-1-yl]-cyclohexyl}-1H-indole-5-carbonitrile

The trans compound was isolated at the same time as the cis isomer in 23% yield (0.20 g) as a white foam.
The HCl salt was prepared in ethanol: mp 330-331°C.

| Elemental analysis for C₂₇H₂₉FON₄•HCl•0.75H₂O | | | |
|---|---|---|---|
| Calc'd | C, 65.58; | H, 6.42; | N, 11.33 |
| Found | C, 65.17; | H, 6.14; | N, 10.97 |

### EXAMPLE 33c

### 3-{(1,4-cis)-4-[4-(5-Fluoro-2,3-dihydro-benzofuran-7-yl)-piperazin-1-yl]-cyclohexyl}-1-methyl-1H-indole-5-carbonitrile

To a suspension of sodium hydride (60%, 0.036 g, 0.9 mmol) in anhydrous N, N-dimethylformamide (2 ml) was added 3-{(1,4-cis)-4-[4-(5-fluoro-2,3-dihydro-benzofuran-7-yl)-piperazin-1-yl]-cyclohexyl}-1H-indole-5-carbonitrile (0.2 g, 0.45 mmol) in 6 ml anhydrous N, N-dimethylformamide at room temperature. The mixture was stirred at room temperature for 30 minutes, followed by the addition of iodomethane (0.034 ml, 0.54 mmol) to the above solution. The resulting mixture was stirred at room temperature for 30 minutes, and quenched with water. The mixture was extracted with ethyl acetate, and the organic layer was dried over anhydrous sodium sulfate. The solvent was removed under vacuum. Chromatography (5% methanol-ethyl acetate) afforded 0.18 g (87%) of product as a white solid: mp 207-208°C.
The HCl salt was prepared in ethanol: mp 282-284°C.

| Elemental analysis for C₂₈H₃₁FON₄•HCl | | | |
|---|---|---|---|
| Calc'd | C, 67.94; | H, 6.52; | N, 11.32 |
| Found | C, 67.61; | H, 6.39; | N, 10.98 |

### EXAMPLE 34a

### 3-[(1,4-cis)-4-[4-(Benzofuran-7-yl-piperazin-1-yl)-cyclohexyl]-1H-indole-5-carbonitrile

A solution of 4-(5-fluoro-1-indol-3-yl)-cyclohexanone (0.72 g, 3.1 mmol), 1-(7-benzofuranyl)piperazine (0.55 g, 2.8 mmol), sodium triacetoxyborohydride (0.84 g, 3.9 mmol) and acetic acid (0.18 g, 2.8 mmol) in 1,2-dichloroethane (80 ml) was allowed to stir at room temperature overnight. The reaction was quenched with 0.5 N sodium hydroxide (100 ml), extracted with methylene chloride (2 x 100 ml. The organic layer was dried over anhydrous magnesium sulfate and filtered. The solvent was removed, crystals appeared after 1 hour. The crystals were triturated with ethyl ether (80 ml) to afford 0.47 g (35%) of product as a white solid: mp 158-159°C.
The HCl salt was prepared in ethanol: mp 295-296°C.

| Elemental analysis for C₂₇H₂₈ON₄•HCl•0.25H₂O | | | |
|---|---|---|---|
| Calc'd | C, 69.66; | H, 6.39; | N, 12.04 |
| Found | C, 69.56; | H, 6.38; | N, 12.12 |

### EXAMPLE 34b

### 3-[(1,4-trans)-4-[4-(Benzofuran-7-yl-piperazin-1-yl)-cyclohexyl] -1H-indole-5-carbonitrile

The remaining residue of the above reaction was purified by chromatography (acetone- methanol-hexanes: 3 : 5 : 3) to afford 0.17 g (12%) of product as a glass.
The HCl salt was prepared in ethanol: mp 330-331°C

| Elemental analysis for C₂₇H₂₈FON₄•HCl•0.75H₂O | | | |
|---|---|---|---|
| Calc'd | C, 65.58; | H, 6.42; | N, 11.33 |
| Found | C, 65.17; | H, 6.14; | N, 10.97 |

### EXAMPLE 35

### 5-Fluoro-3-{4-[4-(2-methoxy-phenyl)-piperazin-1-yl]cyclohex-1-enyl}-1H-indole

This compound was prepared in the manner described above for Example 18 by replacing 4-(5-fluoro-1H-indol-3-yl)-cyclohexanone (1.71 g, 7.9 mmol) with 4-(5-fluoro-1H-3-indolyl)-cyclohex-3-enone in 32 % (0.26 g) yield.
The HCl salt was prepared in ethyl acetate: mp 250°C.

| Elemental analysis for C₂₅H₂₈OFN₃•HCl | | | |
|---|---|---|---|
| Calc'd | C, 67.94; | H, 6.61; | N, 9.51 |
| Found | C, 66.47; | H, 6.58; | N, 9.38 |

### EXAMPLE 36

### 3-{4-[4-(1H-Indol-4-yl)-piperazin-1-yl]-cyclohex-1-enyl}-1H-indole-5-carbonitrile

This compound was prepared in the manner described above for Example 18 by replacing with 4-(5-fluoro-1H-3-indolyl)-cyclohex-3-enone with 4-(5-cyano-1H-3-indolyl)-cyclohex-3-enone in (0.7 g, 2.96 mmol) in 62 % (0.78 g) yield.
The HCl salt was prepared in ethyl acetate: mp 199-201 °C.

| Elemental analysis for C₂₇H₂₇N₅•2HCl | | | |
|---|---|---|---|
| Calc'd | C, 66.25; | H, 6.49; | N, 14.31 |
| Found | C, 66.43; | H, 6.24; | N, 14.27 |

### EXAMPLE 38

### 5-Fluoro-3-{cis-4-[4-(1H-indol-4-yl)-piperazinyl]-cyclohexyl}-1-methyl-1H-indole

This compound was prepared in the manner described above for Example 2 by replacing with 4-(5-fluoro-1H-3-indolyl)-cyclohexone with 4-(5-fluoro-1-methyl-3-indolyl)-cyclohexone in (0.34 g, 1.4 mmol) in 34 % (0.24 g) yield as a clear oil.
The HCl salt was prepared in ethanol: mp 247-249°C.

| Elemental analysis for C₂₇H₃₁FN₄•2HCl•0.25H₂O | | | |
|---|---|---|---|
| Calc'd | C, 63.84; | H, 6.65; | N, 11.03 |
| Found | C, 63.88; | H, 6.51; | N, 10.77 |

### EXAMPLE 39a

### 3-{(1,4-cis)-4-[4-(Quinoxalin-yl)-piperazin-1-yl]-cyclohexyl}-1-methyl-1H-indole-5-carbonitrile

A solution of 4-(5-cyano-1H-3-indolyl)-cyclohexanone (443 mg, 1.87 mmol), Intermediate 34 (400 mg, 1.87 mmol), acetic acid (0.22 mL, 3.7 mmol), and sodium triacetoxyborohydride (590 mg, 2.8 mmol) in dichloroethane (50 mL) was stirred at room temperature overnight. The reaction was quenched with 1 M NaOH (100 mL) and extracted into CH₂Cl₂ (3 x 100 mL). The organic fractions were combined, dried over Na₂SO₄, and filtered. The resulting oil was purified by column chromatography (5% MeOH/EtOAc) yielding 130 mg (16%) of the product as a yellow solid: mp 223-225°C.

| Elemental Analysis for C₂₇H₂₈N₆•1H₂O | | | |
|---|---|---|---|
| Calc'd | C, 71.34; | H, 6.65; | N, 18.49 |
| Found | C, 71.02; | H, 6.33; | N, 18.03 |

### EXAMPLE 39b

### 3-{(1,4-trans)-4-[4-(Quinoxalin-yl)-piperazin-1-yl]-cyclohexyl}-1-methyl-1H-indole-5-carbonitrile

The trans isomer was isolated at the same time as the cis isomer affording 240 mg (29%) of a pale yellow solid: mp 257-259°C.

| Elemental Analysis for C₂₇H₂₈N₆•1H₂O | | | |
|---|---|---|---|
| Calc'd | C, 71.34; | H, 6.65; | N, 18.49 |
| Found | C, 71.63; | H, 6.38; | N, 18.39 |

### EXAMPLE 40a

### 3-[(1,4-cis)-4-(4-Quinolin-5-yl-piperazin-1-yl)-cyclohexyl]-1H-indole-5-carbonitrile

To a solution of 5-(1-piperazinyl)-quinoline (500 mg, 2.35 mmol), 4-(5-cyano-1H-3-indolyl)-cyclohexanone (540 mg, 2.35 mmol), and sodium triacetoxyborohydride (740 mg, 3.5 mmol) in dichloroethane (20 mL) was added acetic acid (0.27 mL, 4.7 mmol) and stirred overnight at room temperature. The reaction was quenched with 1 M NaOH ( 50 mL) and extracted in CH₂Cl₂ (3 x 100 mL). The organic fractions were combined, dried over Na₂SO₄, concentrated, filtered and chromatographed (5% MeOH/EtOAc) yielding 410 mg (41%) of the cis isomer as a white solid. The HCl salt was generated from EtOAc yielding a white solid: mp 220-223°C.

| Elemental Analysis for C₂₈H₂₉N₅•HCl•1H₂O | | | |
|---|---|---|---|
| Calc'd | C, 68.62; | H, 6.58; | N, 14.29 |
| Found | C, 68.99; | H, 6.54; | N, 14.06 |

### EXAMPLE 40b

### 3-[(1,4-trans)-4-(4-Quinolin-5-yl)-piperazin-1-yl)-cyclohexyl]-1H-indole-5-carbonitrile

The trans isomer was isolated at the same time as the cis isomer in Example 40a affording 180 mg (18%) as a beige solid. The HCl salt was generated from EtOAc yielding a white solid: mp 210-211°C.

| Elemental Analysis for C₂₈H₂₉N₅•HCl•0.4H₂O | | | |
|---|---|---|---|
| Calc'd | C, 70.17; | H, 6.48; | N, 14.62 |
| Found | C, 70.23; | H, 6.21; | N, 14.45 |

### EXAMPLE 40c

### 5-{4-[(1,4-cis)-4-(5-Fluoro-1H-indol-3-yl)-cyclohexyl]-piperazin-1-yl}-quinoline

This compound was prepared in the same manner as the compound in Example 40a replacing 4-(5-cyano-1H-3-indolyl)-cyclohexanone with 4-(5-fluoro-1H-3-indolyl)-cyclohexanone (540 mg, 2.35 mmol) to afford 410 mg (41%) of a pale yellow solid: mp 220-223°C; MS (+) ESI *m*/*e* 429 [M+H]⁺.

### EXAMPLE 40d

### 5-{4-[(1,4-trans)-4-(5-Fluoro-1H-indol-3-yl)-cyclohexl]-piperazin-1-yl}-isoquinoline

The trans isomer was isolated at the same time as the cis isomer of Example 40c as the cis isomer of Example 40c affording 180 mg (18%) as a white solid: mp 210-211°C; MS (+) ESI *m*/*e* 429 [M+H]⁺.

### EXAMPLE 40e

### 5-{4-[(1,4-cis)-4-(5-Fluoro-1-methyl-1H-indol-3-yl)-cyclohexyl]-piperazin-1-yl}-quinoline

To a solution of NaH (38 mg, 0.94 mmol) in anhydrous DMF (4 mL) under nitrogen atmosphere was added a solution of 5-{4-[(1,4-cis)-4-(5-fluoro-1H-indol-3-yl)-cyclohexyl]-piperazin-1-yl}-quinoline (200 mg, 0.47 mmol) in DMF (6 mL). The mixture was stirred at room temperature 0.5 hour after which Mel (0.035 mL, 0.56 mmol) was added via syringe. The reaction mixture was stirred an additional 0.5 hour and then quenched with H₂O (50 mL) and extracted with EtOAc (3 x 50 mL). The organic fractions were combined, dried over Na₂SO₄ and concentrated yielding 190 mg (92 %) of a clear oil. The HCl salt was made from EtOAc affording a pale yellow solid: mp decomposes > 270°C.

| Elemental Analysis for C₂₈H₃₁FN₄•HCl•0.75H₂O | | | |
|---|---|---|---|
| Calc'd | C, 68.28; | H, 6.86; | N, 11.37 |
| Found | C, 68.34; | H, 6.56; | N, 11.26 |

### EXAMPLE 41a

### 5-Fluoro-3-[(1,4-cis)-4-(4-naphthalen-1-yl-piperazine-1-yl)-cyclohexyl]-1H-indole

This compound was prepared in the same manner as the compound of Example 40a replacing 4-(5-cyano-1H-3-indolyl)-cyclohexanone with 4-(5-fluoro-1H-3-indolyl)-cyclohexanone (437 mg, 1.9 mmol) and 5-(1-piperazinyl)-quinoline with 1-(1-naphthyl)piperazine (410 mg, 1.9 mmol) affording 240 mg (29 %) of the product as a white solid: mp 195-197°C.

| Elemental Analysis for C₂₈H₃₀FN₃ | | | |
|---|---|---|---|
| Calc'd | C, 78.66; | H, 7.07; | N, 9.83 |
| Found | C, 78.24; | H, 7.06; | N, 9.59 |

### EXAMPLE 41b

### 5-Fluoro-3-[(1,4-trans)-4-(4-naphthalen-1-yl-piperazine-1-yl)-cyclohexyl]-1H-indole

The trans isomer was isolated at the same time as the cis isomer of Example 41a affording 70 mg (9 %) of a white solid: mp 179-181°C.

| Elemental Analysis for C₂₈H₃₀FN₃ | | | |
|---|---|---|---|
| Calc'd | C, 78.66; | H, 7.07; | N, 9.83 |
| Found | C, 78.28; | H, 7.05; | N, 9.79 |

### EXAMPLE 42a

### 5-{4-[(1,4-cis)-4-(5-fluoro-1H-indol-3-yl)-cyclohexyl]piperazin-1-yl}-isoquinoline

This compound was prepared in the same manner as described for the compound of Example 36a replacing 5-(trifluoromethylsulfonyloxy)-quinoline with 5-(trifluoromethylsulfonyloxy)-isoquinoline (12 g, 43.3 mmol) to afford an inseparable mixture of the desired product and impurities. The mixture was treated with TFA (10 mL), MeOH (10 drops), and CH₂Cl₂ (20 mL) at 0°C and warmed to room temperature overnight. The resulting solution was concentrated and the redissolved in CH₂Cl₂ and neutralized with NaHCO₃. The aqueous layer was extracted in CH₂Cl₂ (3 X 100 mL) and EtOAc (3 X 100 mL), dried over Na₂SO₄, filtered and concentrated giving a bright orange oil. The oil was purified twice by column chromatography (10% MeOH/CH₂Cl₂/NH₄OH) but a highly colored impurity persisted. The 5-(1-piperazinyl)-isoquinoline (450 mg, 2.1 mmol), 4-(5-fluoro-1H-3-indolyl)-cyclohexanone (485 mg, 2.1 mmol) and sodium triacetoxyborohydride (672 mg, 3.2 mmol) were dissolved in dichloroethane (30 mL). Acetic acid (0.25 mL, 4.2 mmol) was added and the resulting solution stirred at ambient temperature overnight. The reaction mixture was quenched with 1 M NaOH (40 mL) and extracted in CH₂Cl₂ (4 X 100 mL). The organic fractions were combined, dried over Na₂SO₄ and concentrated yielding a yellow oil which was purified by column chromatography (5% MeOH/EtOAc) affording 300 mg (33 % from 5-(1-piperazinyl)isoquinoline)of the title compound as a beige solid: mp 209-211°C.

| Elemental Analysis for C₂₇H₂₉FN₄ | | | |
|---|---|---|---|
| Calc'd | C, 75.67; | H, 6.82; | N, 13.07 |
| Found | C, 75.40; | H, 6.83; | N, 12.89 |

### EXAMPLE 42b

### 5-{4-[(1,4-trans)-4-(5-fluoro-1H-indol-3-yl)-cyclohexyl]piperazin-1-yl}-isoquinoline

The trans isomer was isolated at the same time as the cis isomer of Example 42a affording 110 mg (12 %) of a pink solid: mp 218-221°C.

| Elemental Analysis for C₂₇H₂₉FN₄•0.25H₂O | | | |
|---|---|---|---|
| Calc'd | C, 74.89; | H, 6.87; | N, 12.94 |
| Found | C, 74.79; | H, 6.79; | N, 12.85 |

### EXAMPLE 43a

### 1{4-[(1,4-cis)-4-(5-Fluoro-1H-indol-3-yl)-cyclohexl]-piperazin-1-yl}-isoquinoline

This compound was prepared in the same manner as the compound of Example 40a replacing 4-(5-cyano-1H-3-indolyl)-cyclohexanone with 4-(5-fluoro-1H-3-indolyl)-cyclohexanone (530 mg, 2.3 mmol) and 5-(1-piperazinyl)-quinoline with 1-(1-piperazinyl)-isoquinoline (500 mg, 2.3 mmol) affording 260 mg (27 %) of the product as a pale yellow solid: mp 180-183°C.

| Elemental Analysis for C₂₇H₂₉FN₄•0.5H₂O | | | |
|---|---|---|---|
| Calc'd | C, 74.11; H, | 6.91; | N, 12.81 |
| Found | C, 74.13; | H, 6.58; | N, 12.60 |

### EXAMPLE 43b

### 1{4-[(1,4-trans)-4-(5-Fluoro-1H-indol-3-yl)-cyclohexl]-piperazin-1-yl}-isoquinoline

The trans isomer was isolated at the same time as the cis isomer of Example 43a affording 180 mg (18 %) of a white solid: mp 232-235°C.

| Elemental Analysis for C₂₇H₂₉FN₄•0.25H₂O | | | |
|---|---|---|---|
| Calc'd | C, 74.89; | H, 6.87; | N, 12.94 |
| Found | C, 74.68; | H, 6.88; | N, 12.64 |

### EXAMPLE 43c

### 1{4-[(1,4-cis)-4-(5-Cyano-1H-indol-3-yl)-cyclohexl]-piperazin-1-yl}-isoquinoline

This compound was prepared in the same manner as the compound of Example 40a replacing 5-(1-piperazinyl)-quinoline with 1-(1-piperazinyl)-isoquinoline (500 mg, 2.3 mmol) affording 230 mg (23 %) of the product as a pale yellow solid: mp 107-109°C; HRMS EI *m*/*e* 435.2431 (M⁺).

### EXAMPLE 43d

### 1{4-[(1,4-trans)-4-(5-Cyano-1H-indol-3-yl)-cyclohexl]-piperazin-1-yl}-isoquinoline

The trans isomer was isolated at the same time as the cis isomer of Example 43c affording 170 mg (17 %) of a white solid: mp 252-255°C; MS (+)APCI *m*/*e* 436 (M+H)⁺.

### EXAMPLE 44a

### 8-{(1,4-cis)-4-[4-(5-Fluoro-1H-indol-3-yl)-cyclohexyl]-piperazin-1-yl}-6- methoxy-quinoline

To a solution of 0.360 g of 6-Methoxy, 8-piperazino-Quinoline in 10 mL of CH₂Cl₂, was added 0.285g of 4-(5-fluoro-1-H-3-indolyl)-cyclohexanone followed by 0.625 g of sodium triacetoxyborohydride and 0.09 mL acetic acid. The reaction was stirred at room temperature overnight. It was quenched with 1N NaOH, and the product was extracted with CH₂Cl₂. The organic phase was washed with water and dried over magnesium sulfate. The product was filtered through 75 mL of silica gel using 50% ethyl acetate/hexanes, 75% ethyl acetate/hexanes, and finally 100% ethyl acetate to give 0.053 g of the desired product: mp 226-227"C; MS (ES) m/z (relative intensity): 459 (M+H+, 100).

### EXAMPLE 44b

### 8-{(1,4-trans)-4-[4-(5-Fluoro-1H-indol-3-yl)-cyclohexyl]-piperazin-1-yl}-6-methoxy-quinoline

The trans isomer of the compound of Example 44a was isolated at the same time as the cis isomer as an off white solid (0.013 g).mp 207-215°C. MS (ES) m/z (relative intensity): 459 (M+H+, 100).

### EXAMPLE 44c

### 3-{(1,4-cis)-4-[4-(6-Methoxy-quinolin-8-yl)-piperazin-1-yl]-cyclohexyl}-1H- indole-5-carbonitrile

To a solution of 1.0 g of 6-Methoxy, 8-piperazino-quinoline in 20 mL of CH₂Cl₂, was added 0.979g of 3-(4-oxo-cyclohexyl)-1H-indole-5-carbonitrile followed by 1.3 g of sodium triacetoxyborohydride and .246 mL acetic acid. The reaction was stirred at room temperature overnight. It was quenched with 1N NaOH, and the product was extracted with CH₂Cl₂. The organic phase was washed with water and dried over magnesium sulfate. The product was filtered through 300 mL of silica gel using 2.5% MeOH / CH₂Cl₂ to give 0.550 g of the desired product: mp 183-185°C; MS (ES) m/z (relative intensity): 466 (M+H+, 100). The hydrochloride was also prepared to give a yellow solid mp 183-185°C.

### EXAMPLE 44d

### 3-{(1,4-trans)-4-[4-(6-Methoxy-quinolin-8-yl)-piperazin-1-yl]-cyclohexyl}-1H- indole-5-carbonitrile

The trans isomer of the compound of Example 44c was isolated at the same time as the cis isomer as an off white solid (0.170 g) mp 148-152°C. MS (ES) m/z (relative intensity): 466 (M+H+, 100). The maleic acid salt was prepared to give an off white solid (0.129g). mp 160-165°C.

### EXAMPLE 45a

### 6-Chloro-8-{4-[(1,4-cis)-4-(5-fluoro-1H-indol-3-yl)-cyclohexyl]-piperazin-1-yl}-quinoline

To a solution of 0.200 g of 6-Chloro, 8-piperazino-quinoline in 10 mL of CH₂Cl₂, was added 0.266g of 4-(5-fluoro-1-H-3-indolyl)-cyclohexanone followed by 0.430 g of sodium triacetoxyborohydride and 0.09 mL acetic acid. The reaction was stirred at room temperature overnight. It was quenched with 1N NaOH, and the product was extracted with CH₂Cl₂. The organic phase was washed with water and dried over magnesium sulfate. The product was filtered through 75 mL of silica gel using 50% ethyl acetate/hexanes, and then 75% ethyl acetate/hexanes, to give 0.119 g of the desired product: mp 166-176°C; MS (ES) m/z (relative intensity): 464 (M+H+, 100).

| Elemental analysis for C₂₇ H₂₈ Cl F N₄ | | | |
|---|---|---|---|
| Calculated | C : 70.04; | H : 6.1; | N : 12.1 |
| Found | C : 70.07; | H : 6.33; | N : 11.87 |

### EXAMPLE 45b

### 6-Chloro-8-{4-[(1,4-trans)-4-(5-fluoro-1H-indol-3-yl)-cyclohexyl]-piperazin-1-yl}-quinoline

The trans isomer of the compound of Example 45a was isolated at the same time as the cis isomer as an off white solid (0.026g) mp 209-210°C. MS (ES) m/z (relative intensity): 464 (M+H+100). Elemental analysis for C₂₇ H₂₈ Cl F N₄

| | | | |
|---|---|---|---|
| Calculated | C : 70.04; | H : 6.1; | N : 12.1 |
| Found | C : 70.23; | H : 6.33; | N: 11.94 |

### EXAMPLE 45c

### 3-{(1,4-cis)-4-[4-(6-Chloro-quinolin-8-yl)-piperazin-1-yl]-cyclohexyl}-1H-indole-5-carbonitrile

To a solution of 0.250 g of 6-chloro, 8-piperazino-quinoline in 10 mL of CH₂Cl₂, was added 0.240g of 3-(4-oxo-cyclohexyl)-1H-indole-5-carbonitrile followed by 0.532g of sodium triacetoxyborohydride and 0.09 mL acetic acid. The reaction was stirred at room temperature overnight. It was quenched with 1N NaOH, and the product was extracted with ether. The organic phase was washed with water and dried. The product was filtered through 75 mL of silica gel using 25% ethyl acetate/hexanes, and then 75% ethyl acetate/hexanes, to give 0.123 g of the desired product: mp 152-160°C; MS (ES) m/z (relative intensity): 471 (M+H+, 100).

### EXAMPLE 45d

### 3-{(1,4-trans)-4-[4-(6-Chloro-quinolin-8-yl)-piperazin-1-yl]-cyclohexyl}-1H-indole-5-carbonitrile

The trans isomer of the compound of Example 45c was isolated at the same time as the cis isomer as an off white solid (0.032g) mp 144-152°C. MS (ES) m/z (relative intensity): 471 (M+H+,100).

### EXAMPLE 46a

### 5-Chloro-8-{4-[(1,4-cis)-4-(5-fluoro-1H-indol-3-yl)-cyclohexyl]-piperazin-1-yl}-quinoline

To a solution of 0.250 g of 5-chloro, 8-piperazino-quinoline in 10 mL of CH₂Cl₂, was added 0.200g of 4-(5-fluoro-1-H-3-indolyl)-cyclohexanone followed by 0.533 g of sodium triacetoxyborohydride and 0.09 mL acetic acid. The reaction was stirred at room temperature overnight. It was quenched with 1N NaOH, and the product was extracted with ether. The organic phase was washed with water and dried over magnesium sulfate. The product was filtered through 75 mL of silica gel using 25% ethyl acetate/hexanes, 75% ethyl acetate/hexanes, to give 0.074 g of the desired product: mp 101-104°C; MS (ES) m/z (relative intensity): 464 (M+H+, 100).

### EXAMPLE 46b

### 3-{(1,4-cis)-4-[4-(5-Chloro-quinolin-8-yl)-piperazin-1-yl]-cyclohexyl}-1H-indole-5-carbonitrile

To a solution of 0.300 g of 5-chloro, 8-piperazino-quinoline in 10 mL of CH₂Cl₂, was added 0.230g of 3-(4-oxo-cyclohexyl)-1H-indole-5-carbonitrile followed by 0.550 g of sodium triacetoxyborohydride and 0.09 mL acetic acid. The reaction was stirred at room temperature overnight. It was quenched with 1N NaOH, and the product was extracted with CH₂Cl₂. The organic phase was washed with water and dried over magnesium sulfate. The product was filtered through 75 mL of silica gel using 50% ethyl acetate/hexanes, 75% ethyl acetate/hexanes, and finally 100% ethyl acetate to give 0.051 g of the desired product: mp 135-144°C; MS (ES) m/z (relative intensity): 471 (M+H+, 100).

### EXAMPLE 47a

### 5-Fluoro-8-{4-[(1,4-cis)-4-(6-fluoro-1H-indol-3-yl)-cyclohexyl]-piperazin-1-yl}-quinoline

To a solution of 0.231 g of 5-fluoro, 8-piperazino-quinoline in 10 mL of CH₂Cl₂, was added 0.230g of 4-(6-fluoro-1-H-3-indolyl)-cyclohexanone followed by 0.530 g of sodium triacetoxyborohydride and 0.09 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 100% ethyl acetate, then 6% MeOH / ethyl acetate to give 0.049 g of the desired product: mp 172-174°C; MS (ES) m/z (relative intensity): 447 (M+H+,100).

### EXAMPLE 47b

### 5-Fluoro-8-{4-[(1,4-trans-4-(6-fluoro-1H-indol-3-yl)-cyclohexyl]-piperazin-1-yl}-quinoline

The trans isomer of the compound of Example 47a was isolated at the same time as the cis isomer as an off white solid (0.055 g) mp173-175 °C. MS (ES) m/z (relative intensity): 447 (M+H+,100).

### EXAMPLE 48a

### 3-{(1,4-cis)-4-[4-(2-Methyl-quinolin-8-yl)-piperazin-1-yl]-cyclohexyl}-1H-indole-5-carbonitrile

To a solution of 0.230 g of 8-piperazino-quinaldine in 10 mL of CH₂Cl₂, was added 0.238g of 3-(4-oxo-cyclohexyl)-1H-indole-5-carbonitrile followed by 0.527 g of sodium triacetoxyborohydride and 0.09 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 100% ethyl acetate and finally 10% MeOH / ethyl acetate to give 0.089 g of the desired product: mp 197-199°C; MS (ES) m/z (relative intensity): 450 (M+H+, 100).

### EXAMPLE 48b

### 3-{(1,4-trans)-4-[4-(2-Methyl-quinolin-8-yl)-piperazin-1-yl]-cyclohexyl}-1H-indole-5-carbonitrile

The trans isomer of the compound of Example 48a was isolated at the same time as the cis isomer as an off white solid (0.058 g) mp 268-280°C. MS (ES) m/z (relative intensity): 450 (M+H+,100).

### EXAMPLE 49a

### 4-{4-[(1,4-cis)-4-(5-Fluoro-1H-indol-3-yl)-cyclohexyl]-piperazin-1-yl}-2-trifluoromethyl-quinoline

To a solution of 0.281g of 1-[2-(trifluoromethyl)quinol-4yl]piperazine in 10 mL CH₂Cl₂, was added 0.231g of 4-(5-fluoro-1-H-3-indolyl)-cyclohexanone followed by 0.528 g of sodium triacetoxyborohydride and 0.09 mL acetic acid. The reaction was stirred at room temperature overnight. It was quenched with 1N NaOH, and the product was extracted with ether. The organic phase was washed with water and dried over magnesium sulfate. The product was filtered through 100 mL of silica gel using 25% ethyl acetate/hexanes,then 50% ethyl acetate/hexanes, to give 0.089 g of the desired product: mp 235-239°C; MS (ES) m/z (relative intensity): 497 (M+H+,100).

### EXAMPLE 49b

### 4-{4-[(1,4-trans)-4-(5-Fluoro-1H-indol-3-yl)-cyclohexyl]-piperazin-1-yl}-2-trifluoromethyl-quinoline

The trans isomer of the compound of Example 49A was isolated at the same time as the cis isomer as an off white solid (0.110 g) mp218-223°C. MS (ES) m/z (relative intensity): 497 (M+H+,100).

### EXAMPLE 49c

### 3-{(1,4-cis)-4-[4-(2-Trifluoromethyl-quinolin-4-yl)-piperazin-1-yl]-cyclohexyl}-1H-indole-5-carbonitrile

This compound was prepared in the same manner as in Example 49a replacing 4-(5-fluoro-1-H-3-indolyl)-cyclohexanone with 3-(4-oxo-cyclohexyl)-1H-indole-5-carbonitrile tto afford 0.137g of a white solid. mp 235-239°C; MS (ES) m/z (relative intensity): 504 (M+H+,100).

| Elemental analysis for C₂₉ H₂₈ F₃ N₅ | | | |
|---|---|---|---|
| Calculated | C : 69.17; | H : 5.6; | N : 13.91 |
| Found | C : 68.96; | H : 5.37; | N : 13.8 |

### EXAMPLE 49d

### 3-{(1,4-trans)-4-[4-(2-Trifluoromethyl-quinolin-4-yl)-piperazin-1-yl]-cyclohexyl}-1H-indole-5-carbonitrile

The trans isomer of the compound of Example 49C was isolated at the same time as the cis isomer as an off white solid (0.036g) mp 259-264°C. MS (ES) m/z (relative intensity): 504 (M+H+, 100).

### EXAMPLE 50a

### 4-{4-[(1,4-cis)-4-(5-Fluoro-1H-indol-3-yl)-cyclohexyl]-piperazin-1-yl}-6-methoxy-quinoline

To a solution of 0.280g of 6-methoxy-4-piperazino-quinoline in 10 mL CH₂Cl₂, was added 0.230g of 4-(5-fluoro-1-H-3-indolyl)-cyclohexanone followed by 0.530 g of sodium triacetoxyborohydride and 0.09 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 100% ethyl acetate, then 10% MeOH / ethyl acetate, to give 0.036 g of the desired product: mp 222-227°C; MS (ES) m/z (relative intensity): 459 (M+H+,100).

### EXAMPLE 50b

### 4-{4-[(1,4-trans)-4-(5-Fluoro-1H-indol-3-yl)-cyclohexyl]-piperazin-1-yl}-6-methoxy-quinoline

The trans isomer of the compound of Example 50a was isolated at the same time as the cis isomer as an off white solid (0.027g) mp 249-251°C. MS (ES) m/z (relative intensity): 459 (M+H+,100).

### EXAMPLE 50c

### 3-{(1,4-cis)-4-[4-(6-Methoxy-quinolin-4-yl)-piperazin-1-yl]-cyclohexyl}-1H-indole-5-carbonitrile

This compound was prepared in the same manner as in Example 50a replacing 4-(5-fluoro-1-H-3-indolyl)-cyclohexanone with 3-(4-oxo-cyclohexyl)-1H-indole-5-carbonitrile to afford 0.016g of a white solid. mp 271-272°C; MS (ES) m/z (relative intensity): 466 (M+H+,100).

### EXAMPLE 50d

### 3-{(1,4-trans)-4-[4-(6-Methoxy-quinolin-4-yl)-piperazin-1-yl]-cyclohexyl}-1H-indole-5-carbonitrile

The trans isomer of the compound of Example 50c was isolated at the same time as the cis isomer as an off white solid (0.014g) mp 288-292°C.MS (ES) m/z (relative intensity): 466 (M+H+,100).

### EXAMPLE 51a

### (cis)-3-{4-[4-(6-methoxy-2-methylquinolin-8-yl)piperazin-1-yl]cyclohexyl}-1-methyl-1H-indole-5-carbonitrile

To a mixture of 4-(6-methoxy-2-methylquinolin-8-yl)piperazine (300 mg, 1.16 mmol), 3-(1-methyl-1H-indole-5-carbonitrile)cyclohexane-4-one (440 mg, 1.75 mmol), and sodium triacetoxyborohydride (495 mg, 2.34 mmol) in 5 mL of anhydrous THF was added 70 µL (73 mg, 1.22 mmol) glacial acetic acid. The resulting mixture was stirred at ambient temperature under N₂ for 24 hours. The reaction was treated with saturated aqueous sodium bicarbonate (50 mL), and aqueous mixture was extracted with CH₂CL₂ (3 x 50 mL). The organic layers were combined, dried over anhydrous Na₂SO₄, filtered, and concentrated in vacuo. Flash chromatography on 4 x 15 cm SiO₂ (gradient elution, 50% EtOAc/hex to 100% EtOAc then 5% MeOH/EtOAc) afforded still impure title compound. A second chromatography using the same eluent on 2 x 20 cm SiO₂ afforded 190 mg (33%) of clean product and 140 mg of still impure product. Recrystallization of the clean product from EtOAc/hexane afforded 100 mg (17%) of the title compound: mp 201-203 °C

| Elemental analysis for C₃₁H₃₅N₅O•0.1 C₄H₈O₂ | | | |
|---|---|---|---|
| Calc' d | C, 75.06; | H, 7.18; | N, 13.94 |
| Found | C, 75.00; | 7.32; | N, 13.83 |

### EXAMPLE 51b

### (cis)-3-{4-[4-(6-methoxy-3-methylquinolin-8-yl)piperazin-1-yl]cyclohexyl}-1-methyl-1H-intlole-5-carbonitrile

To a mixture of 4-(6-methoxy-3-methylquinolin-8-yl)piperazine (210 mg, 0.82 mmol), 3-(1-methyl-1H-indole-5-carbonitrile)cyclohexane-4-one (330 mg, 1.31 mmol), and sodium triacetoxyborohydride (435 mg, 2.05 mmol) in 5 mL of anhydrous THF was added 55 µL (68 mg, 0.96 mmol) glacial acetic acid. The resulting mixture was stirred at ambient temperature under N₂ for 24 hours. The reaction was treated with saturated aqueous sodium bicarbonate (50 mL), and the aqueous mixture was extracted with CH₂Cl₂ (3 x 50 mL). The organic layers were combined, dried over anhydrous Na₂SO₄, filtered, and concentrated in vacuo. Flash chromatography on 2 x 20 cm SiO₂ (5% MeOH/EtOAc) Afforded the title compound, which was slightly impure. Recrystallization from EtOAc/hexane afforded 0.26 g (64%) of the title compound: mp 190-191.5°C

| Elemental analysis for C₃₁H₃₅N₅O | | | |
|---|---|---|---|
| Calc'd | C, 75.43; | H, 7.15; | N, 14.19 |
| Found | C, 75.13; | 7.25; | N, 14.01 |

### EXAMPLE 51c

### (cis)-3-{4-[4-(6-methoxy-4-methylquinolin-8-yl)piperazin-1-yl]cyclohexyl}-1-methyl-1H-indole-5-carbonitrile

To a mixture of 4-(6-methoxy-4-methylquinolin-8-yl)piperazine (0.2 g, 0.78 mmol), 3-(1-methyl-1H-indole-5-carbonitrile)cyclohexane-4-one (0.215 g, 0.85 mmol), dichloroethane (10 mL) and glacial acetic acid (0.12 mL) was addded sodium triacetoxyborohydride (0.25 g, 1.16 mmol). The reaction mixture was stirred at ambient temperature for 24 hours. The reaction mixture was diluted with dichloromethane (60 ml), washed with 1N aqueous sodium hydroxide (2 x 50 mL), water (50 mL), and brine (50 mL). The organic layer was dried over anhydrous sodium sulfate, filtered and concentrated to give 0.43 g of crude product. Flash chromatography on 50 g of silica gel (5% methanol/ethyl acetate) afforded 0.15g (40%) of the title compound. Recrystallization from ethyl acetate/hexane yielded 0.085 g (23%) of pure product: mp 210-212°C.

| Elemental analysis for C₃₁H₃₅N₅O•0.25 H₂O | | | |
|---|---|---|---|
| Calc'd | C, 74.74; | H, 7.18; | N, 14.06 |
| Found | C, 74.82; | H, 7.12; | N, 14.11 |

### EXAMPLE 51d

### (trans)-3-{4-[4-(6-methoxy-4-methylquinolin-8-yl)piperazin-1-yl]cyclohexyl}-1-methyl-1H-indole-5-carbonitrile

The trans isomer was isolated at the same time as the cis isomer in 16% yield (0.062 g). Trituration with ethyl acetate/hexane afforded 0.058 g (15%) of pure title compound: mp 230-232°C.

| Elemental analysis for C₃₁H₃₅N₅O•0.5 H₂O | | | |
|---|---|---|---|
| Calc'd | C, 74.07; | H, 7.22; | N, 13.93 |
| Found | C, 74.12; | H, 7.10; | N, 13.95 |

### EXAMPLE 52a

### (cis)-3-{4-[4-(6-methaxy-5-methylquinolin-8-yl)piperazin-1-yl]cyclohexyl}-1-methyl-1H-indole-5-carbonitrile

The above compound was prepared utilizing the same method as that used for the preparation of (cis)-3-{4-[4-(6-methoxy-4-methylquinolin-8-yl)piperazin-1-yl]-cyclohexyl}-1-methyl-1H-indole-5-carbonitrile to give 0.25 g of the title compound. Recrystallization from ethyl acetate afforded 0.125 g (20%) of pure product: mp 227-228°C.

| Elemental analysis for C₃₁H₃₅N₅O•0.25 H₂O | | | |
|---|---|---|---|
| Calc'd | C, 74.74; | H, 7.18; | N, 14.06 |
| Found | C, 74.61; | H, 7.20; | N, 13.71 |

### EXAMPLE 52b

### (trans)-3-{4-[4-(6-methoxy-5-methylquinolin-8-yl)piperazin-1-yl]cyclohexyl}-1-methyl-1H-indole-5-carbonitrile

The trans isomer (0.15 g) was isolated at the same time as the cis compound. Trituration from ethyl acetate afforded 0.110 g (18%) of pure product: mp 212-213°C.

| Elemental analysis for C₃₁H₃₅N₅O•0.25 H₂O | | | |
|---|---|---|---|
| Calc'd | C, 75.43; | H, 7.15; | N, 14.19 |
| Found | C, 75.09; | H, 7.10; | N, 13.96 |

### EXAMPLE 52c

### (cis)-5-chloro-8-{4-[-(5-fluoro-1-methyl-1H-indol-3-yl)cyclohexyl]piperazin-1-yl}-6-methoxyquinoline

The above compound was prepared utilizing the same method as that used for the preparation of (cis)-3-{4-[4-(6-methoxy-4-methylquinolin-8-yl)piperazin-1-yl]-cyclohexyl}-1-methyl-1H-indole-5-carbonitrile to give 0.13 g of the title compound. Trituration from ethyl acetate afforded 0.120 g (29%) of pure product.

| Elemental analysis for C₂₉H₃₂ClFN₄O | | | |
|---|---|---|---|
| Calc'd | C, 68.70; | H, 6.36; | N, 11.05 |
| Found | C, 68.45; | H, 6.24; | N, 10.89 |

### EXAMPLE 52d

### (trans)-5-chloro-8-{4-[-(5-fluoro-1-methyl-1H-indol-3-yl)cyclohexyl]piperazin-1-yl}-6-methoxyquinoline

The trans isomer was isolated in 19% yield (0.075 g) at the same time as the cis compound. Trituration from ethyl acetate afforded 0.070 g (17%) of pure product: mp 170-171 °C

| Elemental analysis for C₂₉H₃₂ClFN₄O | | | |
|---|---|---|---|
| Calc'd | C, 68.70; | H, 6.36; | N, 11.05 |
| Found | C, 68.44; | H, 6.32; | N, 11.02 |

### EXAMPLE 52e

### (cis)-3-{4-[4-(5-chloro-6-methoxyquinolin-8-yl)piperazin-1-yl]cyclohexyl}-1-methyl-1H-indole-5-carbonitrile

The above compound was prepared utilizing the same method as that used for the preparation of (cis)-3-{4-[4-(6-methoxy-4-methylquinolin-8-yl)piperazin-1-yl]-cyclohexyl}-1-methyl-1H-indole-5-carbonitrile to give 0.1 g (24%) of title compound. Recrystallizion from ethyl acetate afforded 0.080 g (20%) of pure product: mp 231-231°C.

| Elemental analysis for C₃₀H₃₂ClN₅O•0.25 H₂O | | | |
|---|---|---|---|
| Calc'd | C, 69.48; | H, 6.32; | N, 13.50 |
| Found | C, 69.49; | H, 6.31; | N, 13.29 |

### EXAMPLE 52f

### (trans)-3-{4-[4-(5-chloro-6-methoxyquinolin-8-yl)piperazin-1-yl]cyclohexyl}-1-methyl-1H-indole-5-carbonitrile

The trans isomer was isolated in 22% yield (0.095 g) at the same time as the cis compound. Trituration from ethyl acetate afforded 0.070 g (17%) of pure product: mp 215-216°C.

| Elemental analysis for C₃₀H₃₂ClN₅O•0.25 H₂O | | | |
|---|---|---|---|
| Calc'd | C, 69.48; | H, 6.32; | N, 13.50 |
| Found | C, 69.36; | H, 6.28; | N, 13.27 |

### EXAMPLE 53a

### 4-{4-[(1,4-cis)-4-(1H-indol-3-yl)cyclohexyl]piperazin-1-yl}-2-(trifluoromethyl)-1H-benzimidazole

To a solution of 4-piperazin-1-yl-2-trifluoromethyl-1H-benzoimidazole (400 mg, 1.48 mmol), 4-(1H-3-indolyl)-cyclohexanone (315 mg, 1.48 mmol), and sodium triacetoxyborohydride (470 mg, 2.22 mmol) in dichloroethane (30 mL) was added acetic acid (0.20 mL, 2.96 mmol) and stirred overnight at room temperature. The reaction was quenched with 1 M NaOH (50 mL) and extracted in CH₂Cl₂ (2 x 100 mL) and 50% EtOAc/MeOH (3 x 100 mL). The organic fractions were combined, dried over Na₂SO₄, concentrated, filtered and chromatographed twice (5% MeOH/EtOAc) yielding 170 mg (25%) of the cis isomer as a white solid. The HCl salt was generated from EtOAc yielding a white solid: mp foams above 207°C.

| Elemental analysis for C₂₆H₂₈F₃N₅•HCl•H₂O | | | |
|---|---|---|---|
| Calc'd | C, 59.82; | H, 5.99; | N, 13.42 |
| Found | C, 60.18; | H, 5.84; | N, 13.29 |

### EXAMPLE 53b

### 4-{4-[(1,4-trans)-4-(1H-indol-3-yl)cyclohexyl]piperazin-1-yl}-2-(trifluoromethyl)-1H-benzimidazole

The trans isomer was isolated at the same time affording 180 mg (9%) as a beige solid. The HCl salt was generated from EtOAc yielding a white solid: mp decomposes above 200°C.

| Elemental analysis for C₂₆H₂₈F₃N₅•HCl•0.75H₂O | | | |
|---|---|---|---|
| Calc'd | C, 60.34; | H, 5.94; | N, 13.53 |
| Found | C, 60.37; | H, 5.68; | N, 13.43 |

### EXAMPLE 54a

### 4-{4-[(1,4-cis)-4-(1H-indol-3-yl)cyclohexyl]piperazin-1-yl}-1H-benzimidazole

This compound was prepared as described for 1a replacing 4-piperazin-1-yl-2-trifluoromethyl-1H-benzoimidazole with 4-piperazin-1-yl-1H-benzoimidazole (510 mg, 2.5 mmol) to afford 350 mg (34%) of the title compound as a yellow foam which was triturated with Et₂O to give a white solid: mp 217-219°C.

| Elemental analysis for C₂₅H₂₉N₅ | | | |
|---|---|---|---|
| Calc'd | C, 75.16; | H, 7.32, | N, 17.53 |
| Found | C, 74.82; | H, 7.21; | N, 17.05 |

### EXAMPLE 54b

### 4-{4-[(1,4-trans)-4-(1H-indol-3-yl)cyclohexyl]piperazin-1-yl}-1H-benzimidazole

The trans isomer was isolated at the same time affording 200 mg (20%) as a white solid. The HCl salt was generated from Et₂O/EtOH to give a white solid: mp decomposes above 215°C.

| Elemental analysis for C₂₅H₂₉N₅•2HCl•H₂O | | | |
|---|---|---|---|
| Calc'd | C, 61.22; | H, 6.78; | N, 14.28 |
| Found | C, 61.24; | H, 6.97; | N, 14.09 |

### EXAMPLE 55a

### 4-{4-[(1,4-cis)-4-(1H-indol-3-yl)cyclohexyl]piperazin-1-yl}-2-methyl-1H-benzimidazole

This compound was prepared as described for Example 53a replacing 4-piperazin-1-yl-2-trifluoromethyl-1H-benzoimidazole with 4-piperazin-1-yl-2-methyl-1H-benzoimidazole (340 mg, 1.57 mmol) to afford 350 mg (54%) of the title compound as a white foam. The HCl salt was generated from EtOAc to give a white solid: mp decomposes above 190°C.

| Elemental analysis for C₂₆H₃₁N₅•2HCl•H₂O | | | |
|---|---|---|---|
| Calc'd | C, 61.90; | H, 6.99; | N, 13.88 |
| Found | C, 62.26; | H, 7.18; | N, 13.46 |

### EXAMPLE 55b

### 4-{4-[(1,4-trans)-4-(1H-indol-3-yl)cyclohexyl]piperazin-1-yl}-2-methyl-1H-benzimidazole

The trans isomer was isolated at the same time affording 110 mg (17%) as a white solid. The HCl salt was generated from EtOH/Et₂O to give a white solid: mp decomposes above 220°C.

| Elemental Analysis for C₂₅H₂₉N₅•2HCl•1.5H₂O | | | |
|---|---|---|---|
| Calc'd | C, 60.81; | H, 7.07; | N, 13.64 |
| Found | C, 60.84; | H, 7.04; | N, 13.31 |

### EXAMPLE 56

### 3-{4-[(1,4-cis)-4-(6-methoxyquinolin-5-yl)piperazin-1-yl]cyclohexyl}-1H-indole-5-carbonitrile

To an oven-dried 100 mL flask under N₂ atmosphere was added 5-bromo-6-methoxyquinoline (3 g, 12.6 mmol), piperazine (6.5 g. 75.6 mmol), Pd(dba)₂ (570 mg, 5 mol%), P(*t*-Bu)₃ (0.628 mL, 5 mol%) and sodium *t*-butoxide (1.82 g, 18.9 mmol). 50 mL dry *o*-xylene was added and the reaction mixture stirred and heated at 120 °C for 3 hours, then at room temperature overnight. The reaction mixture was poured into H₂O (100 mL) and extracted into EtOAc (3 x 100 mL). The organic fractions were combined, dried over Na₂SO₄, concentrated and purified by column chromatography (10% MeOH/CH₂Cl₂+NH₄OH) affording 170 mg (6%) of 6-methoxy-5-piperazin-1-yl-quinoline. This material was used without further purification (combined with another batch) in the next step. (Ref: Tet Lett. 1998, 39, p. 617-620).

To a solution of 6-methoxy-5-piperazin-1-yl-quinoline (220 mg, 0.9 mmol), 4-(5-cyano-1H-3-indolyl)-cyclohexanone (215 mg, 0.9 mmol), and sodium triacetoxyborohydride (288 mg, 1.36 mmol) in dichloroethane (20 mL) was added acetic acid (0.10 mL, 1.75 mmol) and stirred overnight at room temperature. The reaction was quenched with 2.5 M NaOH (20 mL) and H₂O (150 mL) then extracted in CH₂Cl₂ (2 x 100 mL) and 5% MeOH/EtOAc (3 x 100 mL). The organic fractions were combined, dried over Na₂SO₄, concentrated, filtered and chromatographed (5% MeOH/EtOAc) yielding 140 mg (33%) of the cis isomer as a yellow glass. The HCl salt was generated from EtOAc yielding a yellow solid: mp discolors above 85°C.

| Elemental analysis for C₂₆H₃₁N₅•3HCl•H₂O | | | |
|---|---|---|---|
| Calc'd | C, 58.74; | H, 6.12; | N, 11.81 |
| Found | C, 58.67; | H, 6.34; | N, 11.47 |

### EXAMPLE 57

### 2-{4-(1,4-trans)-[4-(6-Bromoquinolin-8-yl)piperazin-1-yl]cyclohexyl}-1-methyl-1H-indole-5-carbonitrile

To a solution of 6-bromo-8-piperazin-1-yl-quinoline (1 g, 3.4 mmol), 4-(5-cyano-1-methyl-1H-3-indolyl)-cyclohexanone (857 mg, 3.4 mmol), and sodium triacetoxyborohydride (1.08 g, 5.1 mmol) in dichloroethane (40 mL) was added acetic acid (0.40 mL, 6.8 mmol) and stirred overnight at room temperature. The reaction was quenched with 2.5 M NaOH (20 mL) and H₂O (150 mL) then extracted in CH₂Cl₂ (2 x 100 mL) and 5% MeOH/EtOAc (3 x 100 mL). The organic fractions were combined, dried over Na₂SO₄, concentrated, filtered and chromatographed (5% MeOH/EtOAc) yielding 360 mg (20%) of the trans isomer as a white foam. The HCl salt was generated from EtOAc to give a white solid: mp decomposes above 85°C.

| Elemental analysis for C₂₉H₃₀BrN₅•HCl•0.75H₂O | | | |
|---|---|---|---|
| Calc'd | C, 60.21; | H, 5.66; | N, 12.11 |
| Found | C, 60.17; | H, 5.44; | N, 11.99 |

### EXAMPLE 58a

### (Cis)-6-bromo-8-{4-[4-(5-fluoro-1-methyl-1H-indol-3-yl)cyclohexyl]piperazin-1-yl}quinoline

To a solution of 6-bromo-8-piperazin-1-yl-quinoline (610 mg, 2.09 mmol), 4-(5-fluoro-1-methyl-1H-indol-3-yl)-cyclohexanone (510 mg, 2.09 mmol), and sodium triacetoxyborohydride (660 mg, 3.14 mmol) in dichloroethane (40 mL) was added acetic acid (0.24 mL, 4.18 mmol) and stirred overnight at room temperature. The reaction was quenched with 1 M NaOH (50 mL) and H₂O (100 mL) then extracted in CH₂Cl₂ (100 mL) and EtOAc (100 mL). The organic fractions were combined, dried over Na₂SO₄, concentrated, filtered and chromatographed (5% MeOH/EtOAc). The majority of the cis compound precipitated out of 5% MeOH/EtOAc before application to the column and was purified by filtration affording 510 mg (47%) of the cis isomer as a pale yellow solid: mp 215-217°C.

| Elemental analysis for C₂₈H₃₀BrFN₄•0.5H₂O | | | |
|---|---|---|---|
| Calc'd | C, 60.21; | H, 5.66; | N, 12.11 |
| Found | C, 60.17; | H, 5.44; | N, 11.99 |

### EXAMPLE 58b

### (Trans)-6-bromo-8-{4-[4-(5-fluoro-1-methyl-1H-indol-3-yl)cyclohexyl]piperazin-1-yl}quinoline

The trans isomer was isolated by chromatography affording 210 mg (19%) as a pale yellow foam. The HCl salt was generated from EtOAc to give a gray solid: mp decomposes above 225°C.

| Elemental analysis for C₂₈H₃₀BrFN₄•HCl•0.5H₂O | | | |
|---|---|---|---|
| Calc'd | C, 59.32; | H, 5.69; | N, 9.88 |
| Found | C, 59.36; | H, 5.47; | N, 9.79 |

### EXAMPLE 59a

### 3-{4-(1,4-cis)-4-(6-ethoxyquinolin-8-yl)piperazine-1-yl]cyclohexyl}-1-methyl-1H-indole-5-carbonitrile

To a solution of 6-ethoxy-8-piperazin-1-yl-quinoline (500 mg, 1.95 mmol), 4-(5-cyano-1-methyl-1H-indol-3-yl)-cyclohexanone (490 mg, 1.95 mmol), and sodium triacetoxyborohydride (620 mg, 2.93 mmol) in dichloroethane (40 mL) was added acetic acid (0.25 mL, 3.9 mmol) and stirred overnight at room temperature. The reaction was quenched with 1 M NaOH (100 mL) and H₂O (50 mL) then extracted in CH₂Cl₂ (50 mL) and 5% MeOH/EtOAc (2 x 100 mL). The organic fractions were combined, dried over Na₂SO₄, concentrated, filtered and chromatographed (5% MeOH/EtOAc). The majority of the cis compound precipitated out of 5% MeOH/EtOAc before application to the column and was purified by filtration and combined with the column fractions affording 450 mg (47%) of the cis isomer as an off-white solid: mp decomposes above 215°C.

| Elemental analysis for C₃₁H₃₅N₅O•1.25H₂O | | | |
|---|---|---|---|
| Calc'd | C, 72.14; | H, 7.32; | N, 13.57 |
| Found | C, 72.23; | H, 7.06; | N, 13.35 |

### EXAMPLE 59b

### 3-{4-(1,4-trans)-4-(6-ethoxyquinolin-8-yl)piperazine-1-yl]cyclohexyl}-1-methyl-1H-indole-5-carbonitrile

The trans isomer was isolated at the same time by chromatography affording 210 mg (22%) as a yellow foam which was triturated with Et₂O to afford a pale yellow solid: mp 225-228°C.

| Elemental Analysis for C₃₁H₃₅N₅O•H₂O | | | |
|---|---|---|---|
| Calc'd | C, 72.77; | H, 7.29; | N, 13.69 |
| Found | C, 72.79; | H, 7.07; | N, 13.41 |

### EXAMPLE 60

### 3-[4-(4-{6-[benzyl(methyl)amino]quinolin-8-yl}piperazin-1-yl)cyclohexyl]-1-methyl-1H-indole-5-carbonitrile

To an oven-dried 10 mL round bottom flask under a N₂ atmosphere was added Cs₂CO₃ (173 mg, 0.53 mmol), BINAP (15 mg, 3 mol %), Pd(OAc)₃ (5 mg, 3 mol %) and 2-{4-(1,4-trans)-[4-(6-bromoquinolin-8-yl)piperazin-1-yl]cyclohexyl}-1-methyl-1H-indole-5-carbonitrile (200 mg, 0.38 mmol). Toluene (1 mL) and benzylmethylamine (0.06 mL, 0.45 mmol) were added via syringe, and the reaction mixture was heated at 100 °C overnight. The cooled reaction mixture was diluted with Et₂O (15 mL), filtered to remove solids, and concentrated. The resulting oil was purified by column chromatography (5%MeOH/EtOAc + NH₄OH) to give 60 mg of the title compound as a brown solid. The HCl salt was generated from EtOAc/Et₂O affording an orange solid: mp decomposes above 90°C.

| Elemental analysis for C₃₇H₄ON₆•3HCl | | | |
|---|---|---|---|
| Calc'd | C, 65.53; | H, 6.39; | N, 12.39 |
| Found | C, 65.36; | H, 6.71; | N, 12.39 |

### EXAMPLE 61a

### 1-Methyl-3-[(1,4-cis)-4-(4-quinolin-5-ylpiperazin-1-yl)cyclohexyl]-1H-indole-5-carbonitrile

To a solution of 5-piperazin-1-yl-quinoline (300 mg, 1.4 mmol), 4-(5-cyano-1-methyl-1H-indol-3-yl)-cyclohexanone (350 mg, 1.4 mmol), and sodium triacetoxyborohydride (450 mg, 2.1 mmol) in dichloroethane (40 mL) was added acetic acid (0.2 mL, 3.4 mmol) and stirred overnight at room temperature. The reaction was quenched with 1 M NaOH (25 mL) and H₂O (100 mL) then extracted into CH₂Cl₂ (100 mL) and EtOAc (2 x 100 mL). The organic fractions were combined, dried over Na₂SO₄, concentrated, filtered and chromatographed (5% MeOH/EtOAc) affording 190 mg (30%) of the cis isomer as a white foam. The HCl salt was generated from EtOAc to give a white solid: mp decomposes above 235°C.

| Elemental analysis for C₂₉H₃₁N₅•HCl•0.5H₂O | | | |
|---|---|---|---|
| Calc'd | C, 70.36; | H, 6.72; | N, 14.15 |
| Found | C, 70.43; | H, 6.57; | N, 13.83 |

### EXAMPLE 61b

### 1-Methyl-3-[(1,4-trans)-4-(4-quinolin-5-ylpiperazin-1-yl)cyclohexyl]-1H-indole-5-carbonitrile

The trans isomer was isolated at the same time affording 140 mg (22%) as a pale yellow solid: mp discolors above 200°C.

| Elemental analysis for C₂₉H₃₁N₅•0.5H₂O | | | |
|---|---|---|---|
| Calc'd | C, 75.95; | H, 7.03; | N, 15.27 |
| Found | C, 75.82; | H, 6.72; | N, 15.09 |

### EXAMPLE 62a

### 3-{(1,4-cis)-4-[4-(6-methoxy-1,2,3,4-tetrahydroquinolin-8-yl)piperazin-1-yl]cyclohexyl}-1-methyl-1H-indole-5-carbonitrile

To a solution of 6-methoxy-5-piperazin-1-yl-1,2,3,4-tetrahydroquinoline (300 mg, 1.2 mmol), 4-(5-cyano-1-methyl-1H-indol-3-yl)-cyclohexanone (306 mg, 1.2 mmol), and sodium triacetoxyborohydride (254 mg, 1.8 mmol) in dichloroethane (50 mL) was added acetic acid (0.15 mL, 2.4 mmol) and stirred overnight at room temperature. The reaction was quenched with 1 M NaOH (50 mL) and H₂O (50 mL) then extracted in CH₂Cl₂ (100 mL) and EtOAc (2 x 100 mL). The organic fractions were combined, dried over Na₂SO₄, concentrated, filtered and chromatographed twice (5% MeOH/EtOAc) affording 140 mg (24%) of the cis isomer as a white foam. The HCl salt was generated from EtOAc to give a white solid: mp decomposes above 170°C.

| Elemental analysis for C₃₀H₃₇N₅O•HCl•H₂O | | | |
|---|---|---|---|
| Calc'd | C, 66.96; | H, 7.49; | N, 13.01 |
| Found | C, 66.71; | H, 7.28; | N, 12.50 |

### EXAMPLE 62b

### 3-{(1,4-trans)-4-[4-(6-methoxy-1,2,3,4-tetrahydroquinolin-8-yl)piperazin-1-yl]cyclohexyl}-1-methyl-1H-indole-5-carbonitrile

The trans isomer was isolated at the same time affording 80 mg (22%) as a white foam. The HCl salt was generated from EtOAc affording a white solid: mp decomposes above 225°C.

| Elemental analysis for C₃₀H₃₇N₅O•HCl•0.5H₂O | | | |
|---|---|---|---|
| Calc'd | C, 68.10; | H, 7.43; | N, 13.24 |
| Found | C, 68.17; | H, 7.30; | N, 13.17 |

### EXAMPLE 63a

### 1-Methyl-3-[(1,4-cis)-4-(4-[1,6]naphthyridine-8-ylpiperazin-1-yl)cyclohexyl]-1H-indole-5-carbonitrile

To a solution of 8-piperazin-1-yl-naphthyridine (470 mg, 2.19 mmol), 4-(5-cyano-1-methyl-1H-indol-3-yl)-cyclohexanone (550 mg, 2.19 mmol), and sodium triacetoxyborohydride (700 mg, 3.28 mmol) in dichloroethane (40 mL) was added acetic acid (0.25 mL, 4.38 mmol) and stirred overnight at room temperature. The reaction was quenched with 1 M NaOH (40 mL) and H₂O (20 mL) then extracted in CH₂Cl₂ (50 mL) and EtOAc (2 x 100 mL). The organic fractions were combined, dried over Na₂SO₄, concentrated, filtered and chromatographed three times (5% MeOH/EtOAc) affording 490 mg (50%) of the cis isomer as a pale yellow solid: mp decomposes above 215°C, then melts 227-230°C.

| Elemental analysis for C₂₈H₃₀N₆•0.5H₂O | | | |
|---|---|---|---|
| Calc'd | C, 73.90; | H, 6.76; | N, 18.47 |
| Found | C, 73.90; | H, 6.76; | N, 18.61 |

### EXAMPLE 63b

### 1-Methyl-3-[(1,4-trans)-4-(4-[1,6]naphthyridine-8-yl-piperazin-1-yl)cyclohexyl]-1H-indole-5-carbonitrile

The trans isomer was isolated at the same time affording 120 mg (12%) as a pale yellow solid: mp decomposes above 195°C.

| Elemental analysis for C₃₀H₃₇N₅O•0.5H₂O | | | |
|---|---|---|---|
| Calc'd | C, 73.90; | H, 6.76; | N, 18.47 |
| Found | C, 73.87; | H, 6.75; | N, 18.66 |

### EXAMPLE 64

### 1-Methyl-3-((1,4-cis)-4-{4-[6-(methylamino)quinolin-8-yl]piperazin-1-yl}cyclohexyl)-1H-indole-5-carbonitrile

To a solution of 6-(methylamino)-8-piperazin-1-yl-quinoline (100 mg, 0.43 mmol), 4-(5-cyano-1-methyl-1H-indol-3-yl)-cyclohexanone (100 mg, 0.43 mmol), and sodium triacetoxyborohydride (130 mg, 0.62 mmol) in dichloroethane (30 mL) was added acetic acid (0.1 mL, 0.86 mmol) and stirred overnight at room temperature. The reaction was quenched with 1 M NaOH (50 mL) and H₂O (50 mL) then extracted in CH₂Cl₂ (100 mL) and EtOAc (2 x 100 mL). The organic fractions were combined, dried over Na₂SO₄, concentrated, filtered and chromatographed (10% MeOH/EtOAc) affording 60 mg (30%) of the cis isomer as a gold oil. The HCl salt was generated from EtOAc affording a yellow solid: mp decomposes above 170°C.

| Elemental analysis for C₃₀H₃₄N₆•HCl•H₂O | | | |
|---|---|---|---|
| Calc'd | C, 67.59; | H, 7.00; | N, 15.76 |
| Found | C, 67.58; | H, 6.86; | N, 15.65 |

### EXAMPLE 65a

### (Cis)-3-{4-[4-(7-methoxyquinoxalin-5-yl)piperazin-1-yl]cyclohexyl}-1-methyl-1H-indole-5-carbonitrile

To a solution of 7-methoxy-5-piperazin-1-yl-quinoxaline (160 mg, 0.66 mmol), 4-(5-cyano-1-methyl-1H-indol-3-yl)-cyclohexanone (170 mg, 0.66 mmol), and sodium triacetoxyborohydride (210 mg, 0.98 mmol) in dichloroethane (30 mL) was added acetic acid (0.1 mL, 1.3 mmol) and stirred overnight at room temperature. The reaction was quenched with 1 M NaOH (100 mL) then extracted in CH₂Cl₂ (75 mL) and EtOAc (100 mL). The organic fractions were combined, dried over Na₂SO₄, concentrated, filtered and chromatographed (5% MeOH/EtOAc) affording 120 mg (38%) of the cis isomer as a bright yellow solid: mp 226-229°C.

| Elemental analysis for C₂₉H₃₂N₆O•H₂O | | | |
|---|---|---|---|
| Calc'd | C, 69.86; | H, 6.87; | N, 16.85 |
| Found | C, 69.94; | H, 6.71; | N, 16.60 |

### EXAMPLE 65b

### (Trans)-3-{4-[4-(7-methoxyquinoxalin-5-yl)piperazin-1-yl]cyclohexyl}-1-methyl-1H-indole-5-carbonitrile

The trans isomer was isolated at the same time affording 80 mg (12%) as a yellow solid: mp 230-233°C.

| Elemental analysis for C₂₉H₃₂N₆O•0.5H₂O | | | |
|---|---|---|---|
| Calc'd | C, 71.14; | H, 6.79; | N, 17.16 |
| Found | C, 71.29; | H, 6.69; | N, 17.16 |

### EXAMPLE 66a

### (Cis)-3-{4-[4-(6-methoxy[1,7]naphthyridin-8-yl)piperazin-1-yl]cyclohexyl]-1-methyl-1H-indole-5-carbonitrile

To a solution of 6-methoxy-8-piperazin-1-yl-[1,7]naphthyridine (250 mg, 1.02 mmol), 4-(5-cyano-1-methyl-1H-indol-3-yl)-cyclohexanone (260 mg, 1.02 mmol), and sodium triacetoxyborohydride (320 mg, 1.53 mmol) in dichloroethane (50 mL) was added acetic acid (0.12 mL, 2.04 mmol) and stirred overnight at room temperature. The reaction was quenched with 1 M NaOH (50 mL) then extracted in CH₂Cl₂ (1 x 50 mL) and EtOAc (75 mL). The organic fractions were combined, dried over Na₂SO₄, concentrated, filtered and chromatographed (5% MeOH/EtOAc + NH₄OH) affording 160 mg (33%) of the cis isomer as a yellow foam. The HCl salt was generated form EtOAc affording a pale yellow solid: mp 235-238°C.

| Elemental analysis for C₂₉H₃₂N₆O•HCl•H₂O | | | |
|---|---|---|---|
| Calc'd | C, 65.10; | H, 6.59; | N, 15.71 |
| Found | C, 65.09; | H, 6.77; | N, 15.60 |

### EXAMPLE 66b

### (Cis)-3-{4-[4-(6-methoxy[1,7]naphthyridin-8-yl)piperazin-1-yl]cyclohexyl}-1-methyl-1H-indole-5-carbonitrile

The trans isomer was isolated at the same time affording 90 mg (18%) as a yellow foam. The HCl salt was generated from EtOAc affording a pale yellow solid: mp 230-233°C.

| Elemental analysis for C₂₉H₃₂N₆O•HCl•0.5H₂O | | | |
|---|---|---|---|
| Calc'd | C, 66.21; | H, 6.51; | N, 15.97 |
| Found | C, 66.26; | H, 6.37; | N, 15.91 |

### EXAMPLE 67a

### 3-{(1,4-cis)4-[4-(2-Oxo-2,3-dihydro-1H-benzimidoazol-4-yl)-piperazin-1-yl]-cyclohexyl}-1H-indole-5-carbonitrile

To a solution of 4-piperazin-1-yl-1,3-dihydro-benzoimidazol-2-one (400 mg, 1.8 mmol), 4-(5-cyano-1H-indol-3-yl)-cyclohexanone (430 mg, 1.8 mmol), and sodium triacetoxyborohydride (590 mg, 2.8 mmol) in dichloroethane (50 mL) was added acetic acid (0.21 mL, 3.7 mmol) and stirred overnight at room temperature. The reaction was quenched with 2.5 M NaOH (100 mL) then extracted in MeOH/CH₂Cl₂ (2 x 100 mL). The organic fractions were combined, dried over Na₂SO₄, concentrated, filtered and chromatographed two times (10% MeOH/EtOAc) affording 185 mg (23%) of the cis isomer as a beige solid. The HCl salt was generated form EtOAc affording an off-white solid: mp decomposes above 235°C.

| Elemental analysis for C₂₆H₂₈N₆O•HCl•1.5H₂O | | | |
|---|---|---|---|
| Calc'd | C, 61.96; | H, 6.40; | N, 16.67 |
| Found | C, 61.97; | H, 6.26; | N, 16.28 |

### EXAMPLE 67b

### 3-{(1,4-trans)4-[4-(2-Oxo-2,3-dihydro-1H-benzimidoazol-4-yl)-piperazin-1-yl]-cyclohexyl}-1H-indole-5-carbonitrile

The trans isomer was isolated at the same time affording 90 mg (18%) as a white solid. The HCl salt was generated from EtOAc affording a white solid: mp decomposes above 265°C.

| Elemental analysis for C₂₆H₂₈N₆O•HCl•1.5H₂O | | | |
|---|---|---|---|
| Calc'd | C, 61.96; | H, 6.40; | N, 16.67 |
| Found | C, 61.98; | H, 6.25; | N, 16.38 |

### EXAMPLE 68a

### 3-[cis-4-[4-(6-Methoxy-1H-dinole-4-yl)-1-piperazinyl] cyclohexyl]1H-indole-5-carbonitrile

A solution of 4-(5-cyano-1-methyl-3-indolyl)-cyclohexanone (0.43 g, 1.8 mmol), 6-methoxy-4-piperazin-1-yl-1*H*-indole (0.4 g, 1.8 mmol), sodium triacetoxy-borohydride (0.77 g, 2.7 mmol) and acetic acid (0.21 mL, 3.6 mmol) in 1,2-dichloroethane (20 mL) was allowed to stir at room temperature overnight. The reaction was quenched with 1 N aqueous sodium hydroxide (10 mL), and extracted with methylene chloride (3 x 50 mL). The combined organic layers were washed with brine (2 x 50 mL), then dried over anhydrous sodium sulfate and filtered. Chromatography (5% methanol/ethyl acetate) afforded 0.38 g (48%) of the title compound as a white solid: mp 182-185°C.
The HCl salt was prepared in ethyl acetate: mp 225-226 °C.

| Elemental analysis for C₂₈H₃₁N₅O•2HCl•0.25H₂O•0.40C₄H₈O₂ | | | |
|---|---|---|---|
| Calc'd | C, 62.79; | H, 6.53; | N, 12.37 |
| Found | C, 62.28; | H, 6.44; | N, 12.97 |

### EXAMPLE 68b

### 3-[trans-4-[4-(6-Methoxy-1H-indole-4-yl)-1-piperazinyl] cyclohexyl]1H-indole-5-carbonitrile

The trans compound was isolated at same time as the cis isomer in 33% yield (0.26 g) as a white solid: mp 157-160 °C. The HCl salt was prepared in ethyl acetate: mp > 210 °C.

| Elemental analysis for C₂₈H₃₁N₅O•HCl•1.5H₂O | | | |
|---|---|---|---|
| Calc'd | C, 64.82; | H, 6.58; | N, 13.94 |
| Found | C, 65.04; | H, 6.82; | N, 13.54 |

### EXAMPLE 69

### 5-Fluoro-3-{4-[4-(6-methoxy-naphthalen-2-yl)-piperazin-1-yl]-cyclohexyl}-1H-indole

To 400 mg (1.66 mmol) of 1-(6-methoxy-naphthalen-2-yl)-piperazine in 40 mL of CH₂Cl₂ and 100 mg of glacial HOAc at 23 °C was added 384 mg (1.66 mmol) of 4-(5-fluoro-1H-indol-3-yl)-cyclohex-3-enone followed by 216 mg, (1.89 mmol) of Na(OAc)₃BH. After stirring at 23 °C for 12 hours, the reaction mixture was transferred to a separatory funnel and partitioned between water and CH₂Cl₂. The organics were washed with brine, dried over MgSO₄, and chromatographed on silica gel eluting with 20:1 EtOAc:2 M NH₃ in MeOH. The product fractions were pooled, stripped, and treated with 115 mg (1.3 mmol) of (CO₂H)₂ in absolute EtOH to give 640 mg (1.40 mmol, an 84% yield) of the oxalate salt of the title compound as a white crystalline solid. mp: 200-203°C; MS (ES) *m*/*z* 458 (MH)⁺.

| Elemental Analysis for C₂₉H₃₂FN₃O | | | |
|---|---|---|---|
| Calc'd. | C, 67.95; | H, 6.25, | N, 7.67. |
| Found | C, 66.64; | H, 6.71; | N, 7.11. |

### EXAMPLE 70a

### 3-[4-[(Cis)-4-(6-[1,3]dioxolan-2-yl-quinolin-8-yl)-piperazin-1-yl]-cyclohexyl-1H-indole-5-carbonitrile

6-[1,3]Dioxolan-2-yl-8-piperazinyl-quinoline 1.36 g (4.8 mmol) was combined with 1-methyl-3-(4-oxo-cyclohexyl)-1H-indole-5-carbonitrile, 1.53 g (7.2 mmol), 0.43 g (7.2 mmol) CH₃CO₂H, and 100 mL CH₂Cl₂ by the process described for Example 1. The crude was chromatographed on silica gel in a gradient of CH₂Cl₂ to 10:1 CH₂Cl₂:MeOH, and the cis compound was isolated, (R_{f}=0.39, 10:1 CH₂Cl₂:MeOH).. The product fractions were pooled, stripped, and treated with 0.09 g (1.0 mmol) (CO₂H)₂ in absolute EtOH to give 1.0 g (1.9 mmol, a 40% yield) of the oxalate salt of the *cis* isomer of the title compound as a yellow crystalline solid. mp: 105°C; MS (ES) *m*/*z* 522 (MH)⁺.

| Elemental Analysis for C₃₂H₃₅N₅O₂ | | | |
|---|---|---|---|
| Calc'd. | C, 73.68; | H, 6.76, | N, 13.43. |
| Found | C, 73.67; | H, 6.82; | N, 13.23. |

### EXAMPLE 70b

### 3-[4-[(Trans)-4-(6-[1,3]dioxolan-2-yl-quinolin-8-yl)-piperazin-1-yl]-cyclohexyl-1H-indole-5-carbonitrile

The *trans* compound was also obtained, (R_{f}=0.24, 10:1 CH₂Cl₂:MeOH). The product fractions were pooled, stripped, and treated with 0.07 g (0.8 mmol) of (CO₂H)₂ in absolute EtOH to give 0.80 g (1.5 mmol, a 31% yield) mp: 160°C; MS ES *m*/*z* 522 (MH)⁺.

| Elemental Analysis for C₃₂H₃₅N₅O₂ | | | |
|---|---|---|---|
| Calc'd. | C, 73.68; | H, 6.76, | N, 13.43. |
| Found | C, 67.05; | H, 6.27; | N, 12.03. |

### EXAMPLE 71

### 8-[4-[(Cis)-4-(5-Cyano-1-methyl-1H-indole-3-yl)-cyclohexyl]-piperazin-1-yl]-6-quinolinecarbaldehyde

To 920 mg (1.8 mmol) of 3-[4-[(*cis*)-4-(6-[1,3]dioxolan-2-yl-quinolin-8-yl)-piperazinyl]cyclohexyl-1H-indole-5-carbonitrile in 7 mL of THF and 14 mL of glacial HOAc at 23 °C was added 0.8 ml of 6N HCl. The reaction was heated at 40°C for 5 hours. The volatiles were removed by rotary evaporation and the aqueous was neutralized with 5 N NaOH. The organics were extracted into CH₂Cl₂ and washed with brine, dried over MgSO₄, and chromatographed on silica gel eluting with 10:1 CH₂Cl₂:MeOH. ). The product fractions were pooled, stripped, and treated with 147 mg (1.6 mmol) (CO₂H)₂ in absolute EtOH to give 780 mg (1.3 mmol, a 72% yield) oxalate salt of the title compound as a pale yellow crystalline solid. mp: 172-174°C; MS (ES) *m*/*z* 478 (MH)⁺.

| Elemental Analysis for C₃₀H₃₁N₅O | | | |
|---|---|---|---|
| Calc'd. | C, 75.44; | H, 6.54, | N, 14.66. |
| Found | C, 73.27; | H, 6.66; | N, 13.98 |

### EXAMPLE 72

### 8-[4-[(Trans)-4-(5-Cyano-1-methyl-1H-indole-3-yl)-cyclohexyl]-piperazin-1-yl]-6-quinolinecarbaldehyde

The trans compound was obtained by the process described for Example 4 by combining .750 mg (1.4 mmol) 3-[4-[(*trans*)-4-(6-[1,3]dioxolan-2-yl-quinolin-8-yl)-piperazinyl]cyclohexyl-1H-indole-5-carbonitrile, .6 ml 6N HCl, 7 ml THF, 7 ml glacial HOAc. The product fractions were pooled, striped, and treated with 85 mg (0.9 mmol) (CO₂H)₂ in absolute EtOH to give 450 mg (.76 mmol, a 42% yield) Mp: 201-203°C; MS (ES *m*/*z* 478 (MH)⁺.

| Elemental Analysis for C₃₀H₃₁N₅O | | | |
|---|---|---|---|
| Calc'd. | C, 75.44; | H, 6.54, | N, 14.66. |
| Found | C, 72.10; | H, 6.80; | N, 12.64. |

### EXAMPLE 73

### 8-[4-[(Cis)-4-(5-cyano-1-methyl-1H-indole-3-yl)cyclohexyl]-1-piperazinyl]-6-quinolinecarboxylic acid

To 750 mg (1.6 mmol) of 8-[4-[(*Cis*)-4-(5-cyano-1-methyl-1H-indole-3-yl)cyclohexyl]-1-piperazinyl]-6-quinolinecarbaldehyde in 60 mL of *t*-BuOH and 8 mL of CH₃CHC(CH₃)₂ at 23°C was added a solution of 1.3 mg (14.4 mmol) NaClO₂, 1.3 g (10.8 mmol) NaH₂PO₄ in 3 ml water. After stirring at 23°C for 12 hours, the volatiles were removed by rotary evaporation. The reaction mixture was transferred to a separatory funnel and partitioned between water and CH₂Cl₂. The organics were washed with brine, dried over MsSO₄, and chromatographed on silica gel eluting with 20:1 CH₂Cl₂:MeOH containing 5% glacial HOAc. The product fractions were pooled, stripped, and treated with 75 mg (0.83 mmol) of (CO₂H)₂ in absolute EtOH to give 390 mg (0.6 mmol, a 38% yield) of the oxalate salt of the title compound as a tan crystalline solid. mp: 230°C; MS (ES) *m*/*z*: 494 (MH)⁺.

| Elemental Analysis for C₃₀H₃₁N₅O₂ | | | |
|---|---|---|---|
| Calc'd. | C, 73.00; | H, 6.33, | N, 14.19. |
| Found | C, 50.91; | H, 4.92; | N, 7.70 |

### EXAMPLE 74

### 8-[4-[(Trans)-4-(5-cyano-1-methyl-1H-indole-3-yl)cyclohexyl]-1-piperazinyl]-6-quinolinecarboxylic acid

The trans was obtained by the process described for Example 73 by combining .30 g (.60 mmol) 8-[4-[(*Trans*)-4-(5-cyano-1-methyl-1H-indole-3-yl)cyclohexyl]-1-piperazinyl]-6-quinolinecarbaldehyde, .48 g (5.5 mmol) NaClO₂, .48 g (4.1mmol) NaH₂PO₄, 24 mL *t*-BuOH, 3 mLCH₃CHC(CH₃)₂, and 6 ml water. The product fractions were pooled, striped, and treated with 54 mg (0.60 mmol) of (CO₂H)₂ in absolute EtOH to give 97mg (.16 mmol, a 10% yield) mp: 275°C MS (ES) *m*/*z*: 494 (MH)⁺.

| Elemental Analysis for C₃₀H₃₁N₅O | | | |
|---|---|---|---|
| Calc'd. | C, 75.44; | H, 6.54, | N, 14.66. |
| Found | C, 50.42; | H, 4.66; | N, 8.82. |

### EXAMPLE 75

### Methyl 8-[4-[(Cis)-4-(5-cyano-1-methyl-1H-indol-3-yl)cyclohexyl]-1-piperazinyl]-6-quinolinecarboxylate

To 50 mg (0.1 mmol) of 8-[4-[(*Cis*)-4-(5-cyano-1-methyl-1H-indole-3-yl)cyclohexyl]-1-piperazinyl]-6-quinolinecarboxylic acid in 1 mL of MeOH and 3 mL of C₆H₅CH₃ at 23°C was added 0.9 mL (.39 mmol) of a 10% solution of (CH₃)₃SiCHN₂ in hexanes. After stirring at 23°C for 12 hours, the volatiles were removed by rotary evaporation. The crude product was chromatographed on silica gel eluting with 20:1 CH₂Cl₂:MeOH. The product fractions were pooled, stripped, and treated with 5 mg (0.05 mmol) of (CO₂H)₂ in absolute EtOH to give 20 mg (0.04 mmol, a 40% yield) of the oxalate salt of the title compound as a tan crystalline solid. mp: 153-155°C; MS (ES) *m*/*z*: 599 (MH)⁺.

| Elemental Analysis for C₃₁H₃₃N₅O₂ | | | |
|---|---|---|---|
| Calc' d. | C, 66.28; | H, 5.90, | N, 11.71. |
| Found | C, 61.49; | H, 5.85; | N, 10.35. |

### EXAMPLE 76a

### 3-[4-[(Cis)-4-(7-methoxy-8-quinolinyl)-1-piperazinyl]cyclohexyl]-1-methyl-1H-indole-5-carbonitrile

7-Methoxy-8-(1-piperazinyl)quinoline 400 mg (1.6 mmol) was combined with 404 mg (1.6 mmol) of 1-methyl-3-(4-oxo-cyclohexyl)-1H-indole-5-carbonirile, 510 mg (2.4 mmol) of Na(OAc)₃BH, 143 mg (2.4 mmol) of glacial HOAc, in 30 mL CH₂Cl₂ by the process described for Example 69. The crude was chromatographed on silica gel eluting with 20:1 CH₂Cl₂:MeOH, the *cis* compound was isolated (R_{f}=0.34, 10:1 EtOAc:MeOH). The product fractions were pooled, stripped, and treated with 27 mg (0.30 mmol) of (CO₂H)₂ in absolute EtOH to give 179 mg (0.37 mmol, a 23% yield) of the oxalate salt of the title compound as a yellow crystalline solid. mp: 183-186°C; MS (ES) *m*/*z*: 480 (MH)⁺.

| Elemental Analysis for C₃₀H₃₃N₅O | | | |
|---|---|---|---|
| Calc'd. | C, 67.43; | H, 6.19, | N, 12.29. |
| Found | C, 65.38; | H, 6.34; | N, 11.83. |

### EXAMPLE 76b

### 3-[4-[(Trans)-4-(7-methoxy-8-quinolinyl)-1-piperazinyl]cyclohexyl]-1-methyl-1H-indole-5-carbonitrile

The *trans* compound was obtained at the same time (R_{f}=0.17, 10:1 EtOAc:MeOH). The product fractions were pooled, stripped, and treated with 12 mg (0.13 mmol) of (CO₂H)₂ in absolute EtOH to give 80 mg (.17 mmol, an 11% yield) mp: 144-148°C: MS (ES) *m*/*z*: 480 (MH)⁺.

| Elemental Analysis for C₃₀H₃₃N₅O | | | |
|---|---|---|---|
| Calc'd. | C, 67.43; | H, 6.19, | N, 12.29. |
| Found | C, 64.17; | H, 6.37; | N, 11.68. |

### EXAMPLE 77a

### 8-[4-[(Cis)-4-(5-cyano-1-methyl-1H-indol-3-yl)cyclohexyl]-1-piperazinyl]-N,N-dimethyl-6-quinolincarboxamide

N,N-dimethyl-8-(1-piperazinyl)-6-quinolinecarboxamide 300 mg (1.1 mmol) was combined with 267 mg (1.1 mmol) of 1-methyl-3-(4-oxo-cyclohexyl)-1H-indole-5-carbonirile, 339 mg (1.6 mmol) of Na(OAc)₃BH, 96 mg (1.6 mmol) of glacial HOAc in 20 mL CH₂Cl₂ by the process described for Example 69. The crude product was chromatographed on silica gel with a gradient of EtOAc to 10:1 EtOAc:MeOH, and the *cis* compound was isolated (R_{f}=0.43, 10:1 EtOAc:2 M NH₃ in MeOH). The product fractions were pooled, striped, and treated with 35 mg (0.39 mmol) of (CO₂H)₂ in absolute EtOH to give 210 mg (0.40 mmol, a 36% yield) of the oxalate salt of the title compound as a pale yellow crystalline solid. mp: 163-165°C; MS (ES) *m*/*z*: 521 (MH)⁺.

| Elemental Analysis for C₃₂H₃₆N₆O | | | |
|---|---|---|---|
| Calc'd. | C, 66.83; | H, 6.27, | N, 13.75. |
| Found | C, 59.62; | H, 6.15; | N, 11.33. |

### EXAMPLE 77b

### 8-[4-[(Trans)-4-(5-cyano-1-methyl-1H-indol-3-yl)cyclohexyl]-1-piperazinyl]-N,N-dimethyl-6-quinolincarboxamide

The *trans* compound was obtained at the same time, (R_{f}=0.33, 10:1 EtOAc:2M NH₃ in MeOH). The product fractions were pooled, striped, and treated with 15 mg (0.17 mmol) of (CO₂H)₂ in absolute EtOH to give 80 mg (0.15 mmol, a 14% yield) mp: 160-163°C; MS (ES) *m*/*z*: 521 (MH)⁺.

| Elemental Analysis for C₃₂H₃₆N₆O | | | |
|---|---|---|---|
| Calc'd. | C, 66.83; | H, 6.27, | N, 13.75. |
| Found | C, 62.7; | H, 6.52; | N, 12.33. |

### EXAMPLE 78

### 6-Methoxy-8-{cis-4-[4-(1H-pyrrolo[2,3-b]pyridin-3-yl)cyclohexyl]-1-piperazinyl}quinoline

To a stirred solution of 195 mg (0.80 mmol) of 6-methoxy-8-(1-piperazinyl)quinoline in 10 mL of 1,2-dichloroethane at 23 °C was added 177.9 mg (0.83 mmol) of 4-(1H-pyrrolo[2,3-b]pyridin-3-yl)cyclohexanone, 254 mg (1.2 mmol) of sodium triacetoxyborohydride, and 78 mg (1.3 mmol) of glacial acetic acid. The reaction was monitored by TLC on a silica gel plate eluted with CH₂Cl₂/ MeOH (10:1). After stirring at 23 °C for 64 hours, the reaction was quenched with 10 mL of 1 N NaOH, and extracted with CH₂Cl₂ (2 x 25 mL). The aqueous layer was adjusted to pH 10 with AcOH, and further extracted with CH₂Cl₂ (2 x 75 mL). The combined organic layers were washed with brine (2 x 75 mL), dried over MgSO₄, filtered, and evaporated to a tan solid.

The crude product was purified by flash chromatography on silica gel using a gradient elution of CH₂Cl₂/ MeOH (40:1 to 10:1 to 4:1). The appropriate fractions were combined and evaporated to afford 94.8 mg (0.21 mmol, a 27% yield) of the title compound as a tan crystalline solid.

The oxalate salt of the title compound was prepared by adding 19 mg (0.21 mmol) of oxalic acid to 92 mg (0.21 mmol) of the title compound in 1 mL of ethanol at 23°C. After stirring at 23°C for 64 hours, a solid precipitated out of solution. Diethyl ether (5 mL) was added to the suspension and cooled to 0°C, to further crystallize the product. The precipitated solid was collected and washed with ether to afford 79.5 mg (15 mmol, a 71% yield) of the oxalate salt. mp: 216-220°C; MS (ES) *m*/*z*: 442.3 (MH)⁺, 221.6 (M/2+ H)⁺.

| Elemental Analysis For C₂₉H₃₃N₅O₅ | | | |
|---|---|---|---|
| Calc'd. | C, 65.48; | H, 6.25; | N, 13.17. |
| Found | C, 62.66; | H, 5.95; | N, 11.67. |

### EXAMPLE 79

### 6-Methoxy-8-{cis-4-[4-(1-methyl-1H-pyrrolo[2,3,-b]pyridin-3-yl)cyclohexyl]-1-piperazinyl}quinoline

The title compound was prepared by the procedure described in Example 78 using 4-(1-methyl-1H-pyrrolo[2,3-b]pyridin-3-yl)-cyclohexanone (204.7 mg, 0.89 mmol) in place of 4-(1H-pyrrolo[2,3-b]pyridin-3-yl)cyclohexanone. Yield: 30% (108.5 mg, 0.24 mmol); viscous yellow oil.

The oxalate salt was prepared in the manner previously described in Example 78 using 108.5 mg (0.24 mmol) of the title compound. Yield: 30% (39.2 mg, 0.072 mmol). mp: 105-110°C; MS (ES) *m*/*z*: 456.3 (MH)⁺, 228.8 (M/2 + H)⁺.

| Elemental Analysis for C₃₀H₃₅N₅O₅ | | | |
|---|---|---|---|
| Calc'd. | C, 66.00; | H, 6.46; | N, 12.83. |
| Found | C, 58.43; | H, 6.31; | N, 10.57. |

### EXAMPLE 80

### 8-{Cis-4-[4-(6-fluoro-1H-indol-3-yl)cyclohexyl]-1-piperazinyl}-6-methoxyquinoline

The title compound was prepared by the procedure described in Example 78 using 4-(6-fluoro-1H-indol-3-yl)-cyclohexanone (401 mg, 1.87 mmol) in place of 4-(1H-pyrrolo[2,3-b]pyridin-3-yl)cyclohexanone. Yield: 28% (243 mg, 0.53 mmol); white crystalline solid.

The oxalate salt was prepared in the manner previously described in Example 78 using 78.0 mg (0.24 mmol) of the title compound. Yield: 66% (61.1 mg, 0.11 mmol) as a white solid. mp: 239-243°C; MS (ES) *m*/*z*: 459.3 (MH)⁺, 230.1 (M/2+ H).

| Elemental Analysis for C₃₀H₃₃FN₄O₅ | | | |
|---|---|---|---|
| Calc' d. | C, 65.64; | H, 6.06; | N, 10.21. |
| Found | C, 65.16; | H, 6.40; | N, 9.86. |

### EXAMPLE 81

### 8-{Cis-4-[4-(6-fluoro-1-methyl-1H-indol-3-yl)cyclohexyl]-1-piperazinyl}-6-methoxyquinoline

The title compound was prepared by the procedure described in Example 78 using 4-(6-fluoro-1-methyl-1H-indol-3-yl)-cyclohexanone (230 mg, 0.94 mmol) in place of 4-(1H-pyrrolo[2,3-b]pyridin-3-yl)cyclohexanone. Yield: 30% (131.6 mg, 0.28 mmol); white crystalline solid.

The oxalate salt was prepared in the manner previously described in Example 78 using 127.9 mg (0.27 mmol) of the title compound. Yield: 20% (30.1 mg, 0.054 mmol). mp: 219-223 °C; MS (ES) *m*/*z*: 473.2 (MH)⁺.

| Elemental Analysis for C₃₁H₃₅FN₄O₅ | | | |
|---|---|---|---|
| Calc'd. | C, 66.14; | H, 6.27; | N, 9.95. |
| Found | C, 66.26; | H, 6.16; | N, 7.49. |

### EXAMPLE 82

### 6-Methoxy-8-(4-{(cis)-4-[5-(trifluoromethyl)-1H-indol-3-yl]cyclohexyl}-1-piperazinyl)quinoline

The title compound was prepared by the procedure described in Example 78 using cyclohexanone 4-(5-trifluoromethyl-1H-indol-3-yl)-cyclohexanone (271.5 mg, 0.97 mmol) in place of 4-(1H-pyrrolo[2,3-b]pyridin-3-yl)cyclohexanone. Yield: 12% (57 mg, 0.12 mmol); off-white solid.

The oxalate salt was prepared in the manner previously described in Example 78 using 25.6 mg (0.050 mmol) of the title compound. Yield: 67% (20 mg, 0.033 mmol). mp: 143-147°C; MS (ES) *m*/*z*: 509.4 (MH)⁺.

| Elemental Analysis for C₃₁H₃₃F₃N₄O₅ | | | |
|---|---|---|---|
| Calc'd. | C, 62.17; | H, 5.55; | N, 9.35. |
| Found | C, 57.55; | H, 5.84; | N, 8.63. |

### EXAMPLE 83a

### (Cis)-6-methoxy-8-(4-{4-[1-methyl-5-(trifluoromethyl)-1H-indol-3-yl]cyclohexyl}piperazinyl)quinoline

The title compound was prepared by the procedure described in Example 78 using 4-(1-methyl-5-trifluoromethyl-1H-indol-3-yl)-cyclohexanone (750.3 mg, 2.54 mmol) in place of 4-(1H-pyrrolo[2,3-b]pyridin-3-yl)cyclohexanone. Flash chromatography was performed using a gradient elution of ethyl acetate/ MeOH (40:1 to 10:1 to 4:1) in place of CH₂Cl₂/ MeOH; R_{f} = 0.36. Yield: 12% (162.1 mg, 0.30 mmol); tan solid.

The oxalate salt was prepared in the manner previously described in Example 78 using 93.5 mg (0.18 mmol) of the title compound. Yield: 29% (31.4 mg, 0.051 mmol). mp: 101-104°C; MS (ES) *m*/*z*: 523.2 (MH)⁺.

| Elemental Analysis for C₃₂H₃₅F₃N₄O₅ | | | |
|---|---|---|---|
| Calc'd. | C, 62.70; | H, 5.76; | N, 9.14. |
| Found | C, 55.43; | H, 6.21; N, 7.75. | |

### EXAMPLE 83b

### (Trans)-6-methoxy-8-(4-{4-[1-methyl-5-(trifluoromethyl)-1H-indol-3-yl]cyclohexyl}piperazinyl)quinoline

The trans compound (R_{f} = 0.26) was isolated at the same time as the cis isomer in 11% yield (140 mg, 0.27 mmol) as a tan solid. The oxalate salt was prepared in the manner previously described in Example 78 using 100 mg (0.19 mmol) of the title compound. Yield: 86% (101 mg, 0.16 mmol). mp: 111-115°C; MS (ES) *m*/*z*: 523.3 (MH)⁺.

| Elemental Analysis for C₃₂H₃₅F₃N₄O₅ | | | |
|---|---|---|---|
| Calc' d. | C, 62.70; | H, 5.76; | N, 9.14. |
| Found | C, 59.47; | H, 5.80; | N, 7.93. |

### EXAMPLE 84

### 3-{(Cis)-4-[4-(6-methoxy-8-quinolinyl)-1-piperazinyl]cyclohexyl}-1-methyl-1H-carbonitrile

The title compound was prepared in a similar manner described in Example 781 using 1-methyl-3-(4-oxo-cyclohexyl)-1H-indole-6-carbonitrile (164 mg, 0.69 mmol) in place of 4-(1H-pyrrolo[2,3-b]pyridin-3-yl)cyclohexanone. Flash chromotography was performed using a gradient elution of ethyl acetate/ MeOH (40:1 to 10:1 to 4:1) in place of CH₂Cl₂/ MeOH. Yield: 20% (80.4 mg, 0.17 mmol); yellow solid.

The oxalate salt was prepared in the manner previously described in Example 78 using 80.4 mg (0.17 mmol) of the title compound and DMF in place of EtOH. Yield: 56% (53.4 mg, 0.094 mmol). mp: 111-114°C; MS (ES) *m*/*z*: 480.2 (MH)⁺, 240.7 (M/2 + H)⁺.

| Elemental Analysis for C₃₂H₃₅N₅O₅ | | | |
|---|---|---|---|
| Calc' d. | C, 67.43; | H, 6.19; | N, 12.29. |
| Found | C, 62.99; | H, 5.98; | N, 11.16. |

### EXAMPLE 85

### 3-{4-[4-(6-Methoxy-8-quinolinyl)-1-piperazinyl]cyclohexyl}-1H-indole-6-carbonitrile

The title compound was prepared by the procedure described in Example 78 using 3-(4-oxo-cyclohexyl)-1H-indole-6-carbonitrile (404.8 mg, 1.7 mmol) in place of 4-(1H-pyrrolo[2,3-b]pyridin-3-yl)cyclohexanone. Flash chromotography was performed using a gradient elution of ethyl acetate/ MeOH (40:1 to 10:1 to 4:1) in place of CH₂Cl₂/ MeOH. Yield: 63% (493.7 mg, 1.06 mmol); tan solid.

The oxalate salt was prepared in the manner previously described in Example 78 using 183.5 mg (0.39 mmol) of title compound and DMF in place of EtOH. Yield: 43% (93 mg, 0.20 mmol). mp: 242-244°C; MS (ES) *m*/*z*: 466.2 (MH)⁺.

| Elemental Analysis for C₃₁H₃₃N₅O₅ | | | |
|---|---|---|---|
| Calc'd. | C, 66.97; | H, 5.98; | N, 12.60. |
| Found | C, 67.56; | H, 6.09; | N, 13.15. |

### EXAMPLE 86a

### 8-{4-[(1,4-cis)-4-(5-Fluoro-1-methyl-1H-indol-3-yl)-cyclohexyl]-piperazin-1-'yl}-6-methoxy-quinoline

To a solution of 0.270 g of 6-Methoxy, 8-piperazino-quinoline in 20 mL of CH₂Cl₂, was added 0.245g of 4-(5-fluoro-1-methyl-1H-3-indolyl)-cyclohexanone followed by 0.530 g of sodium triacetoxyborohydride and 0.09 mL acetic acid. The reaction was stirred at room temperature overnight. It was quenched with 1N NaOH, and the product was extracted with CH₂Cl₂. The organic phase was washed with water and dried over magnesium sulfate. The product was filtered through 75 mL of silica gel using 50% ethyl acetate/hexanes, 75% ethyl acetate/hexanes, and finally 100% ethyl acetate to give 0.115 g of the desired product: mp 216-218°C; MS (ES) m/z (relative intensity): 473 (M⁺+H, 100).

### EXAMPLE 86b

### 8-{4-[(1,4-trans)-4-(5-Fluoro-1-methyl-1H-indol-3-yl)-cyclohexyl]-piperazin-1-'yl}-6-methoxy-quinoline

The trans isomer was isolated at the same time as the cis isomer as an off white solid (0.013020 g).mp 198-200°C. MS (ES) m/z (relative intensity): 473 (M⁺+H, 100).

### EXAMPLE 87a

### 8-{4-[4-((1,4-cis)-1H-Indol-3-yl)-cyclohexyl]-piperazin-1-yl}-6-methoxy-quinoline

To a solution of 0.350 g of 6-Methoxy, 8-piperazino-quinoline in 10 mL of CH₂Cl₂, was added 0.335g of 4-(1-H-3-indolyl)-cyclohexanone followed by 0.840 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 125 mL of silica gel using 50% ethyl acetate/hexanes, 75% ethyl acetate/hexanes, and finally 100% ethyl acetate to give 0.041 g of the desired product: mp 165-171°C; MS (ES) m/z (relative intensity): 441 (M⁺+H, 100).

### EXAMPLE 87b

### 8-{4-[4-((1,4-trans)-1H-Indol-3-yl)-cyclohexyl]-piperazin-1-yl}-6-methoxy-quinoline

The trans isomer was isolated at the same time as the cis isomer as an off white solid (0.023 g).mp 118-122°C. MS (ES) m/z (relative intensity): 441 (M⁺+H, 100).

### EXAMPLE 88a

### 3-{(1,4-cis)-4-[4-(6-Methoxy-quinolin-8-yl)-piperazin-1-yl]-cyclohexyl}-1-methyl-1H-indole-5-carbonitrile

To a solution of 0.243 g of 6-Methoxy, 8-piperazino-quinoline in 10 mL of CH₂Cl₂, was added 0.252g of 3-(4-oxo-cyclohexyl)-1-methyl-1H-indole-5-carbonitrile followed by 0.527 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.085 g of the desired product: mp 239-240°C; MS (ES) m/z (relative intensity): 480 (M⁺+H, 100).

### EXAMPLE 88b

### 3-{(1,4-trans)-4-[4-(6-Methoxy-quinolin-8-yl)-piperazin-1-yl]-cyclohexyl}-1-methyl-1H-indole-5-carbonitrile

The trans isomer was isolated at the same time as the cis isomer as an off white solid (0.029 g).mp 225-228°C. MS (ES) m/z (relative intensity): 480 (M⁺+H, 100).

### EXAMPLE 89a

### 6-Methoxy-8-{4(1,4-cis)-[4-(1-methyl-1H-indol-3-yl)-cyclohexyl]-piperazin-1-'yl}-quinoline

To a solution of 0.243 g of 6-Methoxy, 8-piperazino-quinoline in 10 mL of CH₂Cl₂, was added 0.250g of 4-(1-methyl-1-H-3-indolyl)-cyclohexanone followed by 0.527 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.120 g of the desired product: mp 190-191°C; MS (ES) m/z (relative intensity): 455 (M⁺+H, 100).

### EXAMPLE 89b

### 6-Methoxy-8-{4-(1,4-trans)[4-(1-methyl-1H-indol-3-yl)-cyclohexyl]-piperazin-1-'yl}-quinoline

The trans isomer was isolated at the same time as the cis isomer as an off white solid (0.027 g).mp 208-210°C. MS (ES) m/z (relative intensity): 455 (M⁺+H, 100).

### EXAMPLE 90a

### 8-{4-(1,4-cis)[4-(5-Fluoro-1-methyl-1H-indol-3-yl)-cyclohexyl]-piperazin-1-'yl}-6-methyl-quinoline

To a solution of 0.275 g of 6-Methyl, 8-piperazino-quinoline in 10 mL of CH₂Cl₂, was added 0.326g of 4-(5-fluoro-1-methyl-3-indolyl)-cyclohexanone followed by 0.639 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 75 mL of silica gel using 50% ethyl acetate/hexanes, 75% ethyl acetate/hexanes, and finally 100% ethyl acetate to give 0.145 g of the desired product: mp 179-181°C; MS (ES) m/z (relative intensity): 457 (M⁺+H, 100).

### EXAMPLE 90b

### 8-{4-(1,4-trans)[4-(5-Fluoro-1-methyl-1H-indol-3-yl)-cyclohexyl]-piperazin-1-'yl}-6-methyl-quinoline

The trans isomer was isolated at the same time as the cis isomer as an off white solid (0.043 g).mp 98-103°C. MS (ES) m/z (relative intensity): 457 (M⁺+H, 100).

### EXAMPLE 91a

### 8-{(1,4-cis)-4-[4-(5-cyano-1H-indol-3-yl)-cyclohexyl]-piperazin-1-yl}-6-methyl-quinoline

To a solution of 0.300 g of 6-Methyl, 8-piperazino-quinoline in 10 mL of CH₂Cl₂, was added 0.280g of 4-(1-H-3-indolyl)-cyclohexanone followed by 0.700 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.125 g of the desired product: mp 132-135°C; MS (ES) m/z (relative intensity): 425 (M⁺+H, 100).

### EXAMPLE 91b

### 8-{(1,4-cis)-4-[4-(5-cyano-1H-indol-3-yl)-cyclohexyl]-piperazin-1-yl}-6- methyl-quinoline

To a solution of 0.275 g of 6-Methyl, 8-piperazino-quinoline in 10 mL of CH₂Cl₂, was added 0.315g of 3-(4-oxo-cyclohexyl)-1H-indole-5-carbonitrile followed by 0.639 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.175 g of the desired product: mp 142-147°C; MS (ES) m/z (relative intensity): 450 (M⁺+H, 100).

### EXAMPLE 92

### 8-{(1,4-cis)-4-[4-(1-ethyl-5-Fluoro-1H-indol-3-yl)-cyclohexyl]-piperazin-1-yl}-6-methoxy-quinoline

To a solution of 0.400 g of 6-Methoxy, 8-piperazino-quinoline in 20 mL of CH₂Cl₂, was added 0.300 g of 4-(5-fluoro-1-ethyl-3-indolyl)-cyclohexanone followed by 0.651 g of sodium triacetoxyborohydride and 0.4 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.041 g of the desired product: mp 203-205°C; MS (ES) m/z (relative intensity): 487 (M⁺+H, 100).

### EXAMPLE 93a

### 8-{(1,4-cis)-4-[4-(5-methoxy-1-methyl-1H-indol-3-yl)-cyclohexyl]-piperazin-1-yl}-6- methoxy-quinoline

To a solution of 0.500 g of 6-Methoxy, 8-piperazino-quinoline in 20 mL of CH₂Cl₂, was added 0.565g of 4-(5-methoxy-1-methyl-3-indolyl)-cyclohexanone followed by 1.1 g of sodium triacetoxyborohydride and 0.4 mL acetic acid. The reaction was stirred at room temperature overnight. It was quenched with 1N NaOH, and the product was extracted with CH₂Cl₂. The organic phase was washed with water and dried over magnesium sulfate. The product was filtered through 200 mL of silica gel using 50% ethyl acetate/hexanes, 75% ethyl acetate/hexanes, and finally 100% ethyl acetate to give 0.077 g of the desired product: mp 170-172°C; MS (ES) m/z (relative intensity): 485 (M⁺+H, 100).

### EXAMPLE 93b

### 8-{(1,4-trans)-4-[4-(5-methoxy-1H-indol-3-yl)-cyclohexyl]-piperazin-1-yl}-6-methoxy-quinoline

The trans isomer was isolated at the same time as the cis isomer as an off white solid (0.039 g).mp 185-186°C. MS (ES) m/z (relative intensity): 485 (M⁺+H, 100).

### EXAMPLE 94a

### 3-{(1,4-cis)-4-[4-(6-isopropoxy-quinolin-8-yl)-piperazin-1-yl]-cyclohexyl}-1-methyl-1H-indole-5-carbonitrile

To a solution of 0.350 g of 6-Isopropoxy, 8-piperazino-quinoline in 10 mL of CH₂Cl₂, was added 0.356g of 3-(4-oxo-cyclohexyl)-1-methyl-1H-indole-5-carbonitrile followed by 0.405 g of sodium triacetoxyborohydride and 0.08 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.141 g of the desired product: mp 223 -226°C; MS (ES) m/z (relative intensity): 508 (M⁺+H, 100).

### EXAMPLE 94b

### 3-{(1,4-trans)-4-[4-(6-isopropoxy-quinolin-8-yl)-piperazin-1-yl]-cyclohexyl}-1-methyl-1H-indole-5-carbonitrile

The trans isomer was isolated at the same time as the cis isomer as an off white solid (0.087 g).mp 221 -223°C. MS (ES) m/z (relative intensity): 508 (M⁺+H, 100).

### EXAMPLE 95a

### 3-{(1,4-cis)-4-[4-(6-fluoro-quinolin-8-yl)-piperazin-1-yl]-cyclohexyl}-1-methyl-1H-indole-5-carbonitrile

To a solution of 0.300 g of 6-Fluoro, 8-piperazino-quinoline in 10 mL of CH₂Cl₂, was added 0.411 g of 3-(4-oxo-cyclohexyl)-1-methyl-1H-indole-5-carbonitrile followed by 0.359 g of sodium triacetoxyborohydride and 0.1 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.187 g of the desired product: mp 230°C; MS (ES) m/z (relative intensity): 468 (M⁺+H, 100).

### EXAMPLE 95b

### 3-{(1,4-trans)-4-[4-(6-fluoro-quinolin-8-yl)-piperazin-1-yl]-cyclohexyl}-1-methyl-1H-indole-5-carbonitrile

The trans isomer was isolated at the same time as the cis isomer as an off white solid (0.039 g).mp 214 - 216°C. MS (ES) m/z (relative intensity): 468 (M⁺+H, 100).

### EXAMPLE 96a

### 3-{(1,4-cis)-4-[4-(6-trifluoromethoxy-quinolin-8-yl)-piperazin-1-yl]-cyclohexyl}-1-methyl-1H-indole-5-carbonitrile

To a solution of 0.297 g of 6-Trifluoromethoxy, 8-piperazino-quinoline in 10 mL of DCE was added 0.272 g of 3-(4-oxo-cyclohexyl)-1-methyl-1H-indole-5-carbonitrile followed by 0.316 g of sodium triacetoxyborohydride and 0.1 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.166 g of the desired product: mp 206°C; MS (ES) m/z (relative intensity): 534 (M⁺+H, 100).

### EXAMPLE 96b

### 3-{(1,4-trans)-4-[4-(6-trifluoromethoxy-quinolin-8-yl)-piperazin-1-yl]-cyclohexyl}-1-methyl-1H-indole-5-carbonitrile

The trans isomer was isolated at the same time as the cis isomer as an off white solid (0.064 g).mp 170°C. MS (ES) m/z (relative intensity): 534 (M⁺+H, 100).

### EXAMPLE 97a

### 3-{(1,4-cis)-4-[4-(5-methoxy-quinolin-8-yl)-piperazin-1-yl]-cyclohexyl}-1-methyl-1H-indole-5-carbonitrile

To a solution of 0.500 g of 5-Methoxy, 8-piperazino-quinoline in 10 mL of DCE, was added 0.544 g of 3-(4-oxo-cyclohexyl)-1-methyl-1H-indole-5-carbonitrile followed by 0.633 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.310 g of the desired product: mp 221°C; MS (ES) m/z (relative intensity): 480 (M⁺+H, 100).

### EXAMPLE 97b

### 3-{(1,4-trans)-4-[4-(5-methoxy-quinolin-8-yl)-piperazin-1-yl]-cyclohexyl}-1-methyl-1H-indole-5-carbonitrile

The trans isomer was isolated at the same time as the cis isomer as an off white solid (0.118 g).mp 206°C. MS (ES) m/z (relative intensity): 480 (M⁺+H, 100).

### EXAMPLE 98a

### 8-{(1,4-cis)-4-[4-(5-Fluoro-1H-indol-3-yl)-cyclohexyl]-piperazin-1-yl}-6-Fluoro-quinoline

To a solution of 0.300 g of 6-Fluoro, 8-piperazino-quinoline in 10 mL of DCE, was added 0.411g of 4-(5-fluoro-1-methyl-3-indolyl)-cyclohexanone followed by 0.349 g of sodium triacetoxyborohydride and 0.1 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.190 g of the desired product: mp 194.5°C; MS (ES) m/z (relative intensity): 461 (M⁺+H, 100).

### EXAMPLE 98b

### 8-{(1,4-trans)-4-[4-(5-Fluoro-1H-indol-3-yl)-cyclohexyl]-piperazin-1-yl}-6-Fluoro-quinoline

The trans isomer was isolated at the same time as the cis isomer as an off white solid (0.062 g).mp 171°C. MS (ES) m/z (relative intensity): 461 (M⁺+H, 100).

### EXAMPLE 99a

### 3-{(1,4-cis)-4-[4-(6-Benzyloxy-quinolin-8-yl)-piperazin-1-yl]-cyclohexyl}-1-methyl-1H-indole-5-carbonitrile

To a solution of 0.300 g of 6-Benzyloxy, 8-piperazino-quinoline in 10 mL of DCE, was added 0.252 g of 3-(4-oxo-cyclohexyl)-1-methyl-1H-indole-5-carbonitrile followed by 0.297 g of sodium triacetoxyborohydride and 0.1 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.172 g of the desired product: mp 171°C; MS (ES) m/z (relative intensity): 556 (M⁺+H, 100).

### EXAMPLE 99b

### 3-{(1,4-trans)-4-[4-(6-Benzyloxy-quinolin-8-yl)-piperazin-1-yl]-cyclohexyl}-1-methyl-1H-indole-5-carbonitrile

The trans isomer was isolated at the same time as the cis isomer as an off white solid (0.083 g).mp 118.5°C. MS (ES) m/z (relative intensity): 556 (M⁺+H, 100).

### EXAMPLE 100

### 3-{(1,4-cis)-4-[4-(6-Hydroxy-quinolin-8-yl)-piperazin-1-yl]-cyclohexyl}-1-methyl-1H-indole-5-carbonitrile

A solution of .100 g of 3-{(1,4-cis)-4-[4-(6-Benzyloxy-quinolin-8-yl) piperazin-1-yl]-cyclohexyl}-1-methyl-1H-indole-5-carbonitrile in THF is added to a suspension of 0.015 gr 10 % Pd/C in MeOH and hydrogenated for ½ hour. Filtered and the solvent was evaporated to give 0.045 g of the desired product. mp 144°C. MS (ES) m/z (relative intensity): 466 (M⁺+H, 100).

### EXAMPLE 101

### 3-{(1,4-cis)4-[4-(6-fluoro-8-quinolinyl)-1-piperazinyl]-cyclohexyl}-1-methyl-1H-indole-5-'carboxamide

To a solution of .100 g of 6-fluoro-8-{4-[4-(5-fluoro-1-methyl-1H-indol-3-yl)cyclohexyl]-1-'piperazinyl}quinoline in 5 ml ( THF: MeOH ), 1 ml of 5N NaOH was added followed by 2 ml 30 % H₂O₂. The mixture was stirred at ROOM TEMPERATURE for 24 hours. Wate was added and the product was filtered to give 0.035 g of the desired product. mp 289°C. MS (ES) m/z (relative intensity): 486 (M⁺+H, 100).

### EXAMPLE 102a

### 3-{(1,4-cis)-4-[4-(5-trifluoromethyl-quinolin-8-yl)-piperazin-1-yl]-cyclohexyl}-1-methyl-1H-indole-5-carbonitrile

To a solution of 0.250 g of 5-Trifluoromethyl, 8-piperazino-quinoline in 10 mL of DCE, was added 0.224 g of 3-(4-oxo-cyclohexyl)-1-methyl-1H-indole-5-carbonitrile followed by 0.287 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.057 g of the desired product: mp 231°C; MS (ES) m/z (relative intensity): 518 (M⁺+H, 100).

### EXAMPLE 102b

### 3-{(1,4-trans)-4-[4-(5-trifluoromethyl-quinolin-8-yl)-piperazin-1-yl]-cyclohexyl}-1-methyl-1H-indole-5-carbonitrile

The trans isomer was isolated at the same time as the cis isomer as an off white solid (0.044 g).mp 194-197°C. MS (ES) m/z (relative intensity): 518 (M⁺+H, 100).

### EXAMPLE 103a

### 3-{(1,4-cis)-4-[4-(6-chloro-quinolin-8-yl)-piperazin-1-yl]-cyclohexyl}-1-methyl-1H-indole-5-carbonitrile

To a solution of 0.300 g of 6-Chloro, 8-piperazino-quinoline in 10 mL of DCE, was added 0.305 g of 3-(4-oxo-cyclohexyl)-1-methyl-1H-indole-5-carbonitrile followed by 0.274 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.057 g of the desired product: mp 222°C; MS (ES) m/z (relative intensity): 485 (M⁺+H, 100).

### EXAMPLE 103b

### 3-{(1,4-trans)-4-[4-(6-chloro-quinolin-8-yl)-piperazin-1-yl]-cyclohexyl}-1-methyl-1H-indole-5-carbonitrile

The trans isomer was isolated at the same time as the cis isomer as an off white solid (0.044 g).mp 229°C. MS (ES) m/z (relative intensity): 485 (M⁺+H, 100).

### EXAMPLE 104a

### 8-{(1,4-cis)-4-[4-(5-Fluoro-1-methyl-1H-indol-3-yl)-cyclohexyl]-piperazin-1-yl}-6- chloro-quinoline

To a solution of 0.247 g of 6-Chloro, 8-piperazino-quinoline in 10 mL of DCE, was added 0.245 g of 4-(5-fluoro-1-methyl-3-indolyl)-cyclohexanone followed by 0.274 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.070 g of the desired product: mp 219°C; MS (ES) m/z (relative intensity): 478 (M⁺+H, 100).

### EXAMPLE 104b

### 8-{(1,4-trans)-4-[4-(5-Fluoro-1-methyl-1H-indol-3-yl)-cyclohexyl]-piperazin-1-yl}-6- chloro-quinoline

The trans isomer was isolated at the same time as the cis isomer as an off white solid (0.049 g).mp 193 °C. MS (ES) m/z (relative intensity): 478 (M⁺+H, 100).

### EXAMPLE 105a

### 3-{(1,4-Cis)-4-[4-(5-chloro-8-quinolinyl)-1-piperazinyl]-cyclohexyl}-1-methyl-1H-indole-5-'carbonitrile

To a solution of 0.250 g of 5-Chloro, 8-piperazino-quinoline in 10 mL of DCE, was added 0.260 g of 3-(4-oxo-cyclohexyl)-1-methyl-1H-indole-5-carbonitrile followed by 0.274 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.080 g of the desired product: mp 243-248°C; MS (ES) m/z (relative intensity): 485 (M⁺+H, 100).

### EXAMPLE 105b

### 3-{(1,4-trans)-4-[4-(5-chloro-8-quinolinyl)-1-piperazinyl]-cyclohexyl}-1-methyl-1H-indole-5-'carbonitrile

The trans isomer was isolated at the same time as the cis isomer as an off white solid (0.034 g).mp 192-196°C. MS (ES) m/z (relative intensity): 485 (M⁺+H, 100).

### EXAMPLE 106a

### 8-{(1,4-cis)-4-[4-(5-Fluoro-1-methyl-1H-indol-3-yl)-cyclohexyl]-piperazin-1-yl}-5- chloro-quinoline

To a solution of 0.250 g of 5-Chloro, 8-piperazino-quinoline in 10 mL of DCE, was added 0.224 g of 4-(5-fluoro-1-methyl-3-indolyl)-cyclohexanone followed by 0.274 g of sodium triacetoxyborohydride and 0.1 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.053 g of the desired product: mp 196°C; MS (ES) m/z (relative intensity): 478 (M⁺+H, 100).

### EXAMPLE 106b

### 8-{(1,4-trans)-4-[4-(5-Fluoro-1-methyl-1H-indol-3-yl)-cyclohexyl]-piperazin-1-yl}-5- chloro-quinoline

The trans isomer was isolated at the same time as the cis isomer as an off white solid (0.025 g).mp 196°C. MS (ES) m/z (relative intensity): 478 (M⁺+H, 100).

### EXAMPLE 107a

### 8-{(1,4-cis)-4-[4-(6-Fluoro-1-methyl-1H-indol-3-yl)-cyclohexyl]-piperazin-1-yl}-5- chloro-quinoline

To a solution of 0.250 g of 5-Chloro, 8-piperazino-quinoline in 10 mL of DCE, was added 0.250 g of 4-(6-fluoro-1-methyl-3-indolyl)-cyclohexanone followed by 0.274 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.030 g of the desired product: mp 107-110°C; MS (ES) m/z (relative intensity): 478 (M⁺+H, 100).

### EXAMPLE 107b

### 8-{(1,4-trans)-4-[4-(6-Fluoro-1-methyl-1H-indol-3-yl)-cyclohexyl]-piperazin-1-yl}-5-chloro-quinoline

The trans isomer was isolated at the same time as the cis isomer as an off white solid (0.014 g).mp 228°C. MS (ES) m/z (relative intensity): 478 (M⁺+H, 100).

### EXAMPLE 108a

### 8-{(1,4-cis)-4-[4-(5-benzyloxy-1-methyl-1H-indol-3-yl)-cyclohexyl]-piperazin-1-yl}-6-methoxy-quinoline

To a solution of 0.650 g of 6-Methoxy, 8-piperazino-quinoline in 15 mL of DCE, was added 0.959 g of 4-(5-benzyloxy-1-methyl-3-indolyl)-cyclohexanone followed by 0.790 g of sodium triacetoxyborohydride and 0.5 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.175 g of the desired product: mp 168°C; MS (ES) m/z (relative intensity): 561 (M⁺+H, 100).

### EXAMPLE 108b

### 8-{(1,4-trans)-4-[4-(5-benzyloxy-1-methyl-1H-indol-3-yl)-cyclohexyl]-piperazin-1-yl}-6-methoxy-quinoline

The trans isomer was isolated at the same time as the cis isomer as an off white solid (0.055 g).mp 228°C. MS (ES) m/z (relative intensity): 561 (M⁺+H, 100).

### EXAMPLE 109a

### 8-{(1,4-cis)-4-[4-(6-fluoro -1-methyl-1H-indol-3-yl)-cyclohexyl]-piperazin-1-yl}-5-fluoro-quinoline

To a solution of 0.231 g of 5-Fluoro, 8-piperazino-quinoline in 10 mL of DCE, was added 0.245 g of 4-(6-fluoro-1-methyl-3-indolyl)-cyclohexanone followed by 0.274 g of sodium triacetoxyborohydride and 0.1 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.030 g of the desired product: mp 112-115 °C; MS (ES) m/z (relative intensity): 461 (M⁺+H, 100).

### EXAMPLE 109b

### 8-{(1,4-trans)-4-[4-(6-fluoro-1-methyl-1H-indol-3-yl)-cyclohexyl]-piperazin-1-yl}-5-fluoro-quinoline

The trans isomer was isolated at the same time as the cis isomer as an off white solid (0.010 g). MS (ES) m/z (relative intensity): 461 (M⁺+H, 100).

### EXAMPLE 110

### 3-{(1,4-cis)-4-[4-(6-methoxy-8-quinolinyl)-1-piperazinyl]-cyclohexyl}-1-methyl-1H-indol-5-ol

A solution of .120 g of 8-{(1,4-cis)-4-[4-(5-benzyloxy-1-methyl-1H-indol-3-yl)-cyclohexyl]-piperazin-1-yl}-6-methoxy-quinoline in 10 ml THF is added to a suspension of 0.100 g 10 % Pd/C in MeOH and hydrogenated for 1 hour. Filtered and the solvent was evaporated to give 0.036 g of the desired product. mp 250°C. MS (ES) m/z (relative intensity): 471 (M⁺+H, 100).

### EXAMPLE 111a

### 8-{(1,4-cis)-4-[4-(5-fluoro-1-methyl-1H-indol-3-yl)-cyclohexyl]-piperazin-1-yl}-5-fluoro-quinoline

To a solution of 0.200 g of 5-Fluoro, 8-piperazino-quinoline in 10 mL of DCE, was added 0.245 g of 4-(5-fluoro-1-methyl-3-indolyl)-cyclohexanone followed by 0.274 g of sodium triacetoxyborohydride and 0.1 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.040 g of the desired product: mp 199-202°C; MS (ES) m/z (relative intensity): 461 (M⁺+H, 100).

### EXAMPLE 111b

### 8-{(1,4-trans)-4-[4-(5-fluoro-1-methyl-1H-indol-3-yl)-cyclohexyl]-piperazin-1-yl}-5-fluoro-quinoline

The trans isomer was isolated at the same time as the cis isomer as an off white solid (0.021 g). mp 197°C; MS (ES) m/z (relative intensity): 461 (M⁺+H, 100).

### EXAMPLE 112a

### 8-Chloro-7-{(1,4-cis)-4-[4-(5-fluoro-1-methyl-1H-indol-3-yl)-cyclohexyl]-1-'piperazinyl}quinoline

To a solution of 0.247 g of 8-Chloro, 7-piperazino-quinoline in 10 mL of DCE, was added 0.245 g of 4-(5-fluoro-1-methyl-3-indolyl)-cyclohexanone followed by 0.274 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.085 g of the desired product: mp 182-184°C; MS (ES) m/z (relative intensity): 478 (M⁺+H, 100).

### EXAMPLE 112b

### 8-Chloro-7-{(1,4-trans)-4-[4-(5-fluoro-1-methyl-1H-indol-3-yl)cyclohexyl]-1-piperazinyl}quinoline

The trans isomer was isolated at the same time as the cis isomer as an off white solid (0.025 g). mp 181-182°C; MS (ES) m/z (relative intensity): 478 (M⁺+H, 100).

### EXAMPLE 113a

### 3-{(1,4-cis)4-[4-(8-chloro-7-quinolinyl)-1-piperazinyl]-cyclohexyl}-1-methyl-1H-indole-5-'carbonitrile

To a solution of 0.247 g of 8-Chloro, 7-piperazino-quinoline in 10 mL of DCE, was added 0.252 g of 4-(5-fluoro-1-methyl-1-H-3-indolyl)-cyclohexanone followed by 0.274 g of sodium triacetoxyborohydride and 0.2 mL acetic acid. The reaction was stirred at room temperature overnight. It was quenched with 1N NaOH, and the product was extracted with CH₂Cl₂. The organic phase was washed with water and dried over magnesium sulfate. The product was filtered through 100 mL of silica gel using 50% ethyl acetate/hexanes, 75% ethyl acetate/hexanes, and finally 100% ethyl acetate to give 0.075 g of the desired product: mp 240-242°C; MS (ES) m/z (relative intensity): 485 (M⁺+H, 100).

### EXAMPLE 113b

### 3-{(1,4-trans)-4-[4-(8-chloro-7-quinolinyl)-1-piperazinyl]-cyclohexyl}-1-methyl-1H-indole-5-'carbonitrile

The trans isomer was isolated at the same time as the cis isomer as an off white solid (0.015 g). mp 233-237°C; MS (ES) m/z (relative intensity): 485 (M⁺+H, 100).

### EXAMPLE 114a

### 3-{(1,4-cis)-4-[4-(4-fluoro-quinolin-8-yl)-piperazin-1-yl]-cyclohexyl}-1H-indole-5-carbonitrile

To a solution of 0.310 g (1.34 mmol) of 4-fluoro-8-piperazino-quinoline in 50 mL of CH₂Cl₂, was added 0.319 g (1.34 mmol) of 3-(4-oxo-cyclohexyl)-1H-indole-5-carbonitrile followed by 0.402 g (1.5 eq) of sodium triacetoxyborohydride and 0.076 mL acetic acid. The reaction was stirred at room temperature overnight. It was quenched with 1 N NaOH, and the product was extracted with ether. The organic phase was washed with water and dried. The product was filtered through 75 mL of silica gel using 25% ethyl acetate/hexanes, 75% ethyl acetate/hexanes, to give 0.185 g of the cis product: mp 152-160°C; MS (ES) m/z (relative intensity): 454.3 (M⁺+H, 100).

### EXAMPLE 114b

### 3-{(1,4-trans)-4-[4-(4-fluoro-quinolin-8-yl)-piperazin-1-yl]-cyclohexyl}-1H-indole-5-carbonitrile

The trans isomer (0.065 g) was isolated at the same time as the cis compound, as an off-white solid: mp 144-152 °C. MS (ES) m/z (relative intensity): 454.4 (M⁺+H, 100).

The activity of the present compounds is demonstrated by the following standard pharmacological test procedures.

The PCR cloning of the human 5-HT_{1A} receptor subtype from a human genomic library has been described previously Chanda et al., Mol. Pharmacol., 43:516 (1993). A stable Chinese hamster ovary cell line expressing the human 5-HT_{1A} receptor subtype (5-HT_{1A}.CHO cells) was employed throughout this study. Cells were maintained in DMEM supplemented with 10% foetal calf serum, non-essential amino acids and penicillin/ streptomycin.

Cells were grown to 95-100% confluency as a monolayer before membranes were harvested for binding studies. Cells were gently scraped from the culture plates, transferred to centrifuge tubes, and washed twice by centrifugation (2000 rpm for 10 min., 4°C) in buffer (50 mM Tris; pH 7.5). The resulting pellets were aliquoted and placed at -80°C. On the day of assay, the cells were thawed on ice, and resuspended in buffer. Studies were conducted using [³H]8-OH-DPAT as the radioligand. The binding assay was performed in 96 well microtiter plates in a final total volume of 250 µL of buffer. Competition experiments were performed by using 7 concentrations of unlabelled drug and a final ligand concentration of 1.5 nM. Nonspecific binding was determined in the presence of 10 µM 5HT. Saturation analysis was conducted by using [³H]8-OH-DPAT at concentrations ranging from 0.3-30 nM. Following a 30 minute incubation at room temperature, the reaction was terminated by the addition of ice cold buffer and rapid filtration using a M-96 Brandel Cell Harvester (Gaithersburg, MD) through a GF/B filter presoaked for 30 minutes in 0.5% polyethyleneimine.

A protocol similar to that used by Cheetham et al., Neuropharmacol. , 32:737 (1993) was used to determine the affinity of compounds for the serotonin transporter. Briefly, frontal cortical membranes prepared from male Sprague-Dawley rats were incubated with ³H-paroxetine (0.1 nM) for 60 minutes at 25°C. All tubes also contained either vehicle, test compound (one to eight concentrations), or a saturating concentration of fluoxetine (10 µM) to define specific binding. All reactions are terminated by the addition of ice cold Tris buffer followed by rapid filtration using a Tom Tech filtration device to separate bound from free ³H-paroxetine. Bound radioactivity was quantitated using a Wallac 1205 Beta Plate® counter. Nonlinear regression analysis was used to determine IC₅₀ values which were converted to Ki values using the method of Cheng and Prusoff, Biochem. Pharmacol., 22:3099 (1973); Ki = IC50/((Radioligand conc.)/(1 + KD)).

The [³⁵S]-GTPγS binding assay was similar to that used by Lazareno and Birdsall, Br. J. Pharmacol. 109:1120 (1993). Briefly, 5-HT_{1A} cloned receptor membrane fragments (as used for 5-HT_{1A} receptor binding assays) were stored at -70°C until needed. When needed, membranes were rapidly thawed, centrifuged at 40,000 x g for 10 minutes and resuspended at 4 °C for 10 minutes in assay buffer (25 mM HEPES, 3 mM MgCl₂, 100 mM NaCl, 1 mM EDTA, 10 uM GDP, 500 mM DTT, pH 8.0). These membranes were then incubated for 30 minutes at 30°C with [³⁵S]GTPgS (1 nM) in the presence of vehicle, test compound (one to eight concentrations), or excess 8-OH-DPAT to define maximum agonist response. All reactions are terminated by the addition of ice cold Tris buffer followed by rapid filtration using a Tom Tech® filtration device to separate bound from free [³⁵S]GTPgS. Agonists produce an increase in the amount of [³⁵S]GTPgS bound whereas antagonists produce no increase in binding. Bound radioactivity was counted and analyzed as above.

The following assays were performed by incubating the cells with DMEM containing 25 mM HEPES, 5 mM theophylline and 10 µM pargyline for a period of 20 minutes at 37°C. Functional activity was assessed by treating the cells with forskolin (1 uM final concentration) followed immediately by test compound (6 concentrations) for an additional 10 minutes at 37°C. In separate experiments, 6 concentrations of antagonist were preincubated for 20 minutes prior to the addition of 10 nM 8-OH-DPAT and forskolin. The reaction was terminated by removal of the media and addition of 0.5 ml ice cold assay buffer. Plates were stored at -20°C prior to assessment of cAMP formation by a cAMP SPA assay (Amersham).

The compounds tested correspond to those prepared in Examples 1-13 above. The results of the procedures are set forth in Table 1.

| Example No. | 5-HT_{1A} (Ki, nM) | ST (Kⱼ, nM,) | GTPγS ED50 (%EMax) | cAMP ED50 (EMax) |
|---|---|---|---|---|
| 1a | 32.0 | 38.0 | 327 (0%) | 631 (0%) |
| 1b | 5.29 | 155 | 176 (32%) | 17 (77%) |
| 2a | 117.3 | 27% | | |
| 2b | 22.3 | 0% | | |
| 3a | 36.7 | 5.4 | 650 (10%) | 400 (0%) |
| 3b | 4.62 | 10.07 | 42.6 (51%) | 155 (0%) |
| 4a | 33.5 | 12.7 | 278 (0%) | 580 (0%) |
| 4b | 5.45 | 35% | | 85 (7.5%) |
| 5a | 0% | 34% | | |
| 5b | 78.7% | 14% | | |
| 6a | 325.7 | 28 | 84.6 (53%) | 4.72 (80%) |
| 6b | 58.3 | 20% | | |
| 7a | 69.6 | 1.62 | 539 (0%) | 87 (0%) |
| 7b | 3.51 | 4.19 | | 8.9 (83%) |
| 8a | 60.3 | 25% | 0% | 357 (0%) |
| 8b | 2.87 | 0% | 38.6 (32%) | 8.9 (77%) |
| 9a | 87.1 | 4% | | |
| 9b | 13.0 | 12% | | |
| 10a | 15.81 | 18% | 0% | 209 (0%) |
| 10b | 7.78 | 0% | 16.3 (14%) | 3.9 (79%) |
| 11 | 0% | 40 | | |
| 12a | 234 | 0.76 | | |
| 12b | 53.2 | 35% | | |
| 13a | 563.5 | 8.9 | | |
| 13b | 827 | 40 | | |
| 14a | 819.9 | 17 | | |
| 14b | 0% | 40 | | |
| 15a | 694.2 | 28 | | |
| 15b | 0% | 16% | | |
| 16a | 0% | 29.0 | | |
| 16b | 0%@100nM | 25%100nM | | |
| 17 | 0% | 2.5 | | |
| 18 | 129.4 | 1.36 | | |
| 19a | 264.4 | 5.72 | | |
| 19b | 26.2 | 24% | 418(74%) | 14.9(92%) |
| 20a | 631.2 | 29% | | |
| 20b | 14.9 | 0% | 35.5 (33%) | 3.05 (75.5%) |
| 21 | 110.4 | 11% | | |
| 22a | 80.7 | 4.96 | 0% | 101.3 (0%) |
| 22b | 11.6 | 36.5 | 4.5% | 357(0%) |
| 23a | 103.2 | 22% | | |
| 23b | 14.9 | 32% | | |
| 24a | 65.7 | 6.90 | 15.4% | 52.1(81%) |
| 24b | 11.3 | 36.0 | 73% | |
| 25a | 67.7 | 63.0 9% 16.0(0%) | | |
| 25b | 9.66 | 58.0 | 24(46%) | |
| 26a | 59.1 | 4.1 | 3960(18%) | 59.6(0%) |
| 26b | 8.5 | 23.0 | 15 (39%) | |
| 27a | 69.7 | 8.6 | 139 (20%) | 212 (0%) |
| 27b | 6.54 | 28.0 | 26 (66%) | |
| 28 | 25.1 | 2.02 | 25(0%) | 95(0%) |
| 29a | 43.9 | 2.25 | 23% | 9.05 (0%) |
| 29b | 2.91 | 46.0% | 34 (70%) | |
| 30 | 24.5 | 1.25 | | 29.5(95%) |
| 31a | 142.2 | 13 | | |
| 31b | 32.4 | 17% | | |
| 32a | 245.6 | 14 | | |
| 32b | 49.1 | 22% | | |
| 33a | 98.9 | 1.9 | | |
| 33b | 19.2 | 45.0 | | |
| 33c | 431.0 | 7.1 | | |
| 34a | 185.4 | 1.49 | | |
| 34b | 8.37 | 17.0 | | |
| 35 | 70.1 | 91 | | |
| 36 | 12.34 | 28 | 84.6(53%) | 4.72(80%) |
| 38 | 124 | 7.22 | | |
| 44c | 21.0 | 1.5 | 556(0%) | 521(0%) |

As demonstrated by the results set forth above, the compounds of the present invention are active towards 5HT1A receptors and generally elevate serotonin levels by inhibiting 5-HT transport. Accordingly, the present compounds should be useful in treating disorders related to defects in serotonin concentration.

The compounds of this invention may be administered orally or parenterally, neat or in combination with conventional pharmaceutical carriers. Applicable solid carriers can include one or more substances which may also act as flavoring agents, lubricants, solubilizers, suspending agents, fillers, glidants, compression aids, binders or tablet-disintegrating agents or an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Any of the solid carriers known to those skilled in the art may be used with the compounds of this invention. Particularly suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidone, low melting waxes and ion exchange resins.

Liquid carriers may be used in preparing solutions, suspensions, emulsions, syrups and elixirs of the compounds of this invention. The compounds of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above, e.g., cellulose derivatives, preferably sodium carboxymethyl cellulose solution), alcohols (including monohydric alcohols and polyhydric alcohols, e.g., glycols) and their derivatives and oils (e.g., fractionated coconut oil and arachis oil). For parenteral administration, the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. Compositions for oral administration may be either liquid or solid composition form.

Preferably, the pharmaceutical compositions containing the compounds of this invention are in unit dosage form, e.g., tablets or capsules. In such form, the compositions may be sub-divided in unit doses containing appropriate quantities of the present compounds. The unit dosage forms can be packaged compositions, for example, packeted powders, vials, ampoules, prefilled syringes or sachets containing liquids. Alternatively, the unit dosage form can be, for example, a capsule or tablet itself, or it can be the appropriate number of any such compositions in package form.

The therapeutically effective amount of the compounds of this invention that is administered and the dosage regimen depends on a variety of factors, including the weight, age, sex, and medical condition of the subject, the severity of the disease, the route and frequency of administration, and the specific compound employed, and thus may vary widely. However, it is believed that the pharmaceutical compositions may contain the compounds of this invention in the range of about 0.1 to about 2000 mg, preferably in the range of about 0.5 to about 500 mg and more preferably between about 1 and about 100 mg. Projected daily dosages of active compound are about 0.01 to about 100 mg/kg body weight. The daily dose can be conveniently administered two to four times per day.

## Claims

1. A compound of the formula: 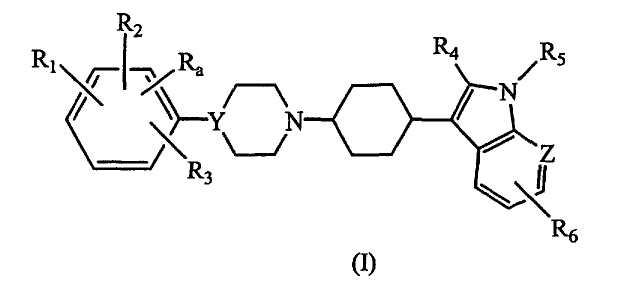 wherein:
Rₐ, R₁, R₂ and R₃ are each, independently, hydrogen, or a substituent selected from halogen, CF₃, alkyl, alkoxy, MeSO₂, amino or aminocarbonyl ( each optionally substituted by one or two groups selected from alkyl and benzyl) carboxy, or alkoxycarbonyl ; or two adjacent of Rₐ and R₁₋₃ together can form a 5-7 membered carbocyclic or heterocyclic ring which is optionally substituted by a substituent defined above;
R₄ is hydrogen, halogen, or alkyl;
R₅ is hydrogen, alkyl, alkylaryl, or aryl;
R₆ is hydrogen, halogen, CF₃, CN, carbamide, alkoxy or benzyloxy;
Y is CH or nitrogen; and
Z is carbon or nitrogen; or a
pharmaceutically acceptable salts thereof, each alkyl and alkoxy group including the alkyl component of arylalkyl contains 1-6 carbon atoms and each aryl radical including the aryl component of arylalkyl contains 6-12 carbon atoms.

2. A compound as in claim 1, wherein:
Rₐ, R₁, R₂ and R₃ are each, independently, hydrogen, halogen, alkyl, alkoxy, or together can form a 5-7 membered carbocyclic or heterocyclic ring;

3. A compound as claimed in claim 1 or claim 2, wherein R₄ is hydrogen or halogen.

4. A compound as in any one of claims I to 3 wherein R₅ is hydrogen, alkyl or alkylaryl.

5. A compound as in any one of claims 1 to 4 wherein R₆ is hydrogen, halogen, CN or alkoxy.

6. A compound as claimed in any one of claim 1 to 5 wherein Y and Z are each carbon.

7. A compound of claim 1 which is selected from the following:
3-[cis-4-[4-(1H-Indol-4-yl)-1-piperazinyl]cyclohexyl]-1H-indole;
3-[trans-4-[4-(1H-indol-4-yl)-1-piperazinyl]cyclohexyl]-1H-indole;
4-Fluoro-3-[cis-4-[4-(1H-indol-4-yl)-1-piperazinyl]cyclohexyl]-1H-indole;
4-Fluoro-3-[trans-4-[4-(1H-indol-4-yl)-1-piperazinyl]cyclohexyl]-1H-indole;
5-Fluoro-3-[cis-4-[4-(1H-indol-4-yl)-1-piperazinyl]cyclohexyl]-1H-indole;
5-Fluoro-3-[trans-4-[4-(1H-indol-4-yl)-1-piperazinyl]cyclohexyl]-1H-indole;
6-Fluoro-3-[cis-4-[4-(1H-indol-4-yl)-1-piperazinyl]cyclohexyl]-1H-indole;
6-Fluoro-3-[trans-4-[4-(1H-indol-4-yl)-1-piperazinyl]cyclohexyl]-1H-indole;
5-Bromo-3-[cis-4-[4-(1H-indol-4-yl)-1-piperazinyl]cyclohexyl]-1H-indole;
5-Bromo-3-[trans-4-[4-(1H-indol-4-yl)-1-piperazinyl]cyclohexyl]-1H-indole;
5-Chloro-3-[cis-4-[4-(1H-indol-4-yl)-1-piperazinyl]cyclohexyl]-1H-indole;
5-Chloro-3-[trans-4-[4-(1H-indol-4-yl)-1-piperazinyl]cyclohexyl]-1H-indole;
3-{4-[(1,4-cis)-4-(1H-indol-4-yl)-piperazinyl-1-yl]cyclohexyl}-1H-indole-5-carbonitrile;
3-{4-[(1,4-trans)-4-(1H-indol-4-yl)-piperazinyl-1-yl]cyclohexyl}-1H-indole-5-carbonitrile;
5-Methoxy-3-[cis-4-[4-(1H-indol-4-yl)-1-piperazinyl]cyclohexyl]-1H-indole;
5-Methoxy-3-[trans-4-[4-(1H-indol-4-yl)-1-piperazinyl]cyclohexyl]-1H-indole;
3-[cis-4-[4-(1H-Indol-4-yl)-1-piperazinyl]cyclohexyl]-2-methyl-1H-indole;
3-[trans-4-[4-(1H-Indol-4-yl)-1-piperazinyl]cyclohexyl]-2-methyl-1H-indole;
3-{(1,4-cis)-4-[4-1H-Indole-4-yl)-piperazin-1-yl]-cyclohexyl}-1H-pyrrolo[2,3-b]pyridine;
3-{(1,4-trans)-4-[4-(1H-Indol-4-yl)-piperazin-1-yl]-cyclohexyl}-1H-pyrrolo[2,3-b]pyridine;
6-Fluoro-1-methyl-3-{cis-4-[4-(1-methyl-1H-indol-4-yl)-1-piperazinyl]cyclohexyl}-1H-indole;
3-{(1,4-cis)-4-[4-(1H-indol-4-yl)-piperazin-1-yl]cyclohexyl}-1-methyl-1H-indole-5-carbonitrile;
3-{(1,4-trans)-4-[4-(1H-indol-4-yl)-piperazin-1-yl]cyclohexyl}-1-methyl-1H-indole-5-carbonitrile;
1-Ethyl-3-{(1,4-cis)-4-[4-(1H-indole-4-yl)-piperazin-1-yl]-cyclohexyl}-1H-indole-5-carbonitrile;
3-{(1,4-cis)-4-[4-(1H-indol-4-yl)-piperazin-1-yl]-cyclohexyl}-1-propyl-1H-indole-5-carbonitrile;
3-{(1,4-trans)-4-[4-(1H-indol-4-yl)-piperazin-4-yl]-cyclohexyl}-1-propyl-1H-indole-5-carbonitrile;
3-{(1,4-cis)-4-[4-(1H-indol-4-yl)-piperazin-1-yl]-cyclohexyl}-1-isopropyl-1H-indole-5-carbonitrile;
3-{(1,4-trans)-4-[4-(1H-indol-4-yl)-piperazin-1-yl]cyclohexyl}-1-isopropyl-1H-indole-5-carbonitrile;
1-Benzyl-3-{(1,4-cis)-4-[4-(1H-indol-4-yl)-piperazin-1-yl]-cyclohexyl}-1H-indole-5-carbonitrile;
1-Benzyl-3-{(1,4-trans)-4-[4-(1H-indole-4-yl)-piperazin-1-yl]cyclohexyl}-1H-indole-5-5-carbonitrile;
1-Methyl-3-{(1,4-cis)-4-[4-(1-methyl-1H-indol-4-yl)-piperazine-1-yl]-cyclohexyl}-1H-indole-5-carbonitrile;
5-Fluoro-3-{(cis)-4-[4-(2-methoxy-phenyl)-piperazin-1-yl]-cyclohexyl}-1H-indole;
5-Fluoro-3-{(1,4-cis)-4-[4-(2-methoxy-phenyl)-piperidin-1-yl]-cyclohexyl}-1H-indole;
5-Fluoro-3-{(1,4-trans)-4-[4-(2-methoxy-phenyl)-piperidin-1-yl]-cyclohexyl}-1H-indole;
5-methoxy-3-{(1,4-cis)-4-[4-(2-methoxy-phenyl)-piperazin-1-yl]-cyclohexyl}-1H-indole;
5-Methoxy-3-{(1,4-trans)-4-[4-(2-methoxy-phenyl)- piperazin-1-yl]-cyclohexyl}-1H-indole;
3-{(1,4-cis)-4-[4-(2-methoxy-phenyl)-piperazin-1-yl]-cyclohexyl}-1H-pyrrolo[2,3-b]pyridine.
5-Fluoro-3-{(cis)-4-[4-(5-fluoro-2-methoxy-phenyl)-piperazin-1-yl]-cyclohexyl}-1H-indole;
5-Fluoro-3-{(trans)-4-[4-(5-fluoro-2-methoxy-phenyl)-piperazin-1-yl]-cvyclohexyl}-1H-indole;
3-{(1,4-cis)-4-[4[(2,3-Dihydro-benzo[1,4]dioxin-5-yl)-piperazin-1-yl]-cyclohexyl}-4-fluoro-1H-indole;
3-{(1,4-cis)-4-[4-(2,3-Dihydro-benzo[1,4]dioxin-5-yl)-piperazin-1-yl]-cyclohexyl}-4-fluoro-1H-indole;
3-{(1,4-trans)-4-[4-(2,3-Dihydro-benzo[1,4]dioxin-5-yl)-piperazin-1-yl]-cyclohexyl}-5-fluoro-1H-indole;
3-{(1,4-trans)-4-[4-(2,3-Dihydro-benzo[1,4]dioxin-5-yl)-piperazin-1-yl]-cyclohexyl}-5-fluoro-1H-indole;
3-{(1,4-cis)-4-[4-(2,3-Dihydro-benzo[1,4]dioxin-5-yl)-piperazin-1-yl]-cyclohexyl}-6-fluoro-1H-indole;
3-{(1,4-trans)-4-[4-{2,3-Dihydro-benzo[1,4]dioxin-5-yl)-piperazin-1-yl]-cyclohexyl}-6-fluoro-1H-indole;
3-{(1,4-cis)-4-[4-(2,3-Dihydro-benzo[1,4]dioxin-5-yl)-piperazin-1-yl]-cyclohexyl}-1H-indole-5-carbonitrile;
3-{(1,4-cis)-4-[4-(2,3-Dihydro-benzo[1,4]dioxin-5-yl)-piperazin-1-yl]-cyclohexyl}-1H-indole-5-carbonitrile;
3-{(1,4-trans)-4-(4-(2,3-Dihydro-benzo[1,4]dioxin-5-yl)-piperazin-1-yl]-cyclohexyl}-1H-indole-5-carbonitrile;
8-{4-[(1,4-cis)-4-(5-Fluoro-1H-indol-3-yl)-cyclohexyl]-piperazin-1-yl}quinoline;
8-{4-[(1,4-trans)-4-(5-Fluoro-1H-indol-3-yl)-cyclohexyl]-piperazin-1-yl}-quinoline;
8-{4-(1,4-cis)-4-[4-(5-Fluoro-1-methyl-1H-indol-3-yl)-cyclohexyl]-piperazin-1-yl}-quinoline;
3-[(1,4-cis)-4-(4-Quinolin-8-yl-piperazin-1-yl)-cyclohexyl]-1H-indole-5-carbonitrile;
3-[(1,4-trans)-4-(4-Quinolin-8-yl-piperazin-1-yl)-cyclohexyl]-1H-indole-5-carbonitrile;
1-Methyl-3-[(1,4-cis)-4-(4-quinolin-8-yl-piperazin-1-yl)-cyclohexyl]-1H-indole-5-carbonitrile;
5-Fluoro-3-{(1,4-cis)-4-[4-(6-fluoro-chroman-8-yl)-piperazin-1-yl]-cyclohexyl}-1H-indole;
5-Fluoro-3-{(1,4-trans)-4-[4-(6-fluoro-chroman-8-yl)-piperazin-1-yl]-cyclohexyl}-1H-indole;
5-Fluoro-3-{(1,4-cis)-4-[4-(5-fluoro-2,3-dihydro-benzofuran-7-yl)-piperazin-1-yl]-cyclohexyl}-1H-indole;
5-Fluoro-3-{(1,4-trans)-4-[4-(5-fluoro-2,3-dihydro-benzofuran-7-yl)-piperazin-1-yl]-cyclohexyl}-1H-indole;
3-{(1,4-cis)-4-[4-(5-Fluoro-2,3-dihydro-benzofuran-7-yl)-piperazin-1-yl]-cyclohexyl}-1H-indole-5-carbonitrile;
3-{(1,4-trans)-4-[4-(5-Fluoro-2,3-dihydro-benzofuran-7-yl)-piperazin-1-yl]-cyclohexyl}-1H-indole-5-carbonitrile;
3-{(1,4-trans)-4-[4-(5-Fluoro-2,3-dihydro-benzofuran-7-yl)-piperazin-1yl]-cyclohexyl}-1-methyl-1H-indole-5-carbonitrile;
3-[(1,4-cis)-4-[4-(Benzofuran-7-yl-piperazin-1-yl)-cyclohexyl]-1H-indole-5-carbonitrile;
3-[(1,4-trans)-4-[4-(Benzofuran-7-yl-piperazin-1-yl)-cyclohexyl]-1H-indole-5-carbonitrile;
5-Fluoro-3-{cis-4-[4-(1H-indol-4-yl)piperazinyl]-cyclohexyl}-1-methyl-1H-indole;
3-{(1,4-cis)-4-[4-(6-Methoxy-quinolin-8-yl)-piperazin-1-yl]-cyclohexyl}-1H-indole-5-carbonitrile;
or a pharmaceutically acceptable salt thereof

8. A pharmaceutical composition comprising a compound of the formula (I) as claimed in any one of claims 1 to 7 or a pharmaceutically acceptable salt thereof and a pharmaceutically acceptable carrier..

9. Use of a compound of the formula (I) as claimed in any one of claims 1 to 7 or a pharmaceutically acceptable salt thereof for the manufacture of a medicament for treating depression.

10. A process for preparing a compound of formula (I) as defined in claim 1 which comprises one of the following;
a) reacting a compound of formula 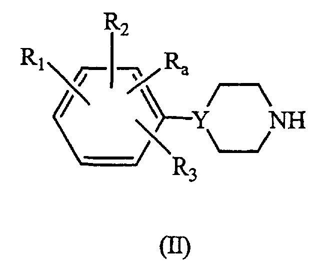 wherein Rₐ, R₁₋₃, and Y are as defined above, with a compound of formula (IV): 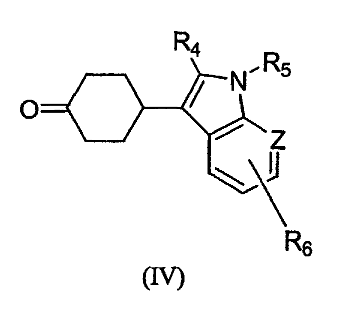 wherein Z, R₄, R₅ and R₆ are as defined above;
or
b) reducing a compound of formula : 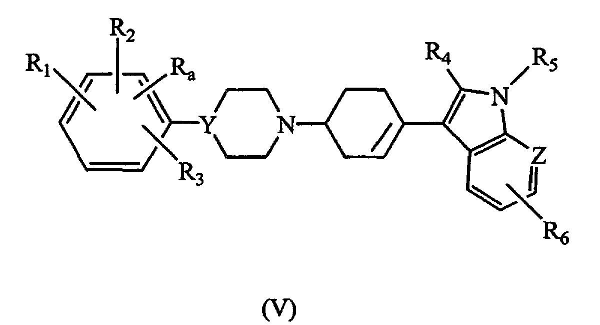 wherein the variables are as defined above to give a compound of formula (I);
or
c) acidifying a basic compound of formula I with a pharmaceutically acceptable acid to give a pharmaceutically acceptable salt;
or
d) separating a mixture of cis and trans isomers of a compound of formula (I) to isolate one isomer substantially free from the other isomer;
or
e) reacting a compound of formula (I) having a reactive substituent group to give a compound of formula (I) having a different substituent group;
or
f) reacting a compound of formula (I) having a reactive site (e.g. NH) to give a compound of formula (I) having a substituent group on the site.

11. Compounds selected from the following:
5-Fluoro-3-{4-[4-(2-methoxy-phenyl)-piperazin-1-yl]cyclohex-1-enyl}-1H-indole;
3-{4-[4-(1H-Indol-4-yl)-piperazin-1-yl]-cyclohex-1-enyl}-1H-indole-5-carbonitrile;

## Patentansprüche

1. Verbindung der Formel: 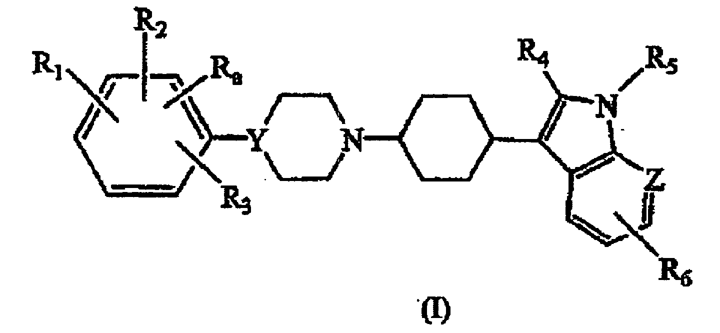 worin:
Rₐ, R₁, R₂ und R₃ jeweils unabhängig Wasserstoff darstellen oder einen Substituenten, ausgewählt aus Halogen, CF₃, Alkyl, Alkoxy, MeSO₂, Amino oder Aminocarbonyl (jedes gegebenenfalls substituiert durch eine oder zwei Gruppen, ausgewählt aus Alkyl und Benzyl) Carboxy oder Alkoxycarbonyl; oder zwei aneinander grenzende von Rₐ und R₁₋₃ zusammen einen 5-7-gliedrigen carbocyclischen oder heterocyclischen Ring bilden können, welcher gegebenenfalls durch einen oben definierten Substituenten substituiert ist;
R₄ für Wasserstoff, Halogen oder Alkyl steht;
R₅ für Wasserstoff, Alkyl, Alkylaryl oder Aryl steht;
R₆ für Wasserstoff, Halogen, CF₃, CN, Carbamid, Alkoxy oder Benzyloxy steht;
Y für CH oder Stickstoff steht; und
Z für Kohlenstoff oder Stickstoff steht; oder ein pharmazeutisch annehmbares Salz davon, wobei jede Alkyl- und Alkoxygruppe einschließlich des Alkylbestandteils von Arylalkyl 1-6 Kohlenstoffatome enthält und jeder Arylrest einschließlich des Arylbestandteils von Arylalkyl 6-12 Kohlenstoffatome enthält.

2. Verbindung wie in Anspruch 1, worin:
Rₐ, R₁, R₂ und R₃ jeweils unabhängig Wasserstoff, Halogen, Alkyl, Alkoxy darstellen oder zusammen einen 5-7-gliedrigen carbocyclischen oder heterocyclischen Ring bilden können.

3. Verbindung wie in Anspruch 1 oder Anspruch 2 beansprucht, worin R₄ für Wasserstoff oder Halogen steht.

4. Verbindung wie in einem der Ansprüche 1 bis 3, worin R₅ für Wasserstoff, Alkyl oder Alkylaryl steht.

5. Verbindung wie in einem der Ansprüche 1 bis 4, worin R₆ für Wasserstoff, Halogen, CN oder Alkoxy steht.

6. Verbindung wie in einem der Ansprüche 1 bis 5 beansprucht, worin Y und Z jeweils Kohlenstoff darstellen.

7. Verbindung nach Anspruch 1, welche ausgewählt wird aus den Folgenden:
3-[cis-4-[4-(1H-Indol-4-yl)-1-piperazinyl]cyclohexyl]-1H-indol;
3-[trans-4-[4-(1H-Indol-4-yl)-1-piperazinyl]cyclohexyl]-1H-indol;
4-Fluor-3-[cis-4-[4-(1H-indol-4-yl)-1-piperazinyl]cyclohexyl]-1H-indol;
4-Fluor-3-[trans-4-[4-(1H-indol-4-yl)-1-piperazinyl]cyclohexyl]-1H-indol;
5-Fluor-3-[cis-4-[4-(1H-indol-4-yl)-1-piperazinyl]cyclohexyl]-1H-indol;
5-Fluor-3-[trans-4-[4-(1H-indol-4-yl)-1-piperazinyl]cyclohexyl]-1H-indol;
6-Fluor-3-[cis-4-[4-(1H-indol-4-yl)-1-piperazinyl]cyclohexyl]-1H-indol;
6-Fluor-3-[trans-4-[4-(1H-indol-4-yl)-1-piperazinyl]cyclohexyl]-1H-indol;
5-Brom-3-[cis-4-[4-(1H-indol-4-yl)-1-piperazinyl]cyclohexyl]-1H-indol;
5-Brom-3-[trans-4-[4-(1H-indol-4-yl)-1-piperazinyl]cyclohexyl]-1H-indol;
5-Chlor-3-[cis-4-[4-(1H-indol-4-yl)-1-piperazinyl]cyclo]-1H-indol;
5-Chlor-3-[trans-4-[4-(1H-indol-4-yl)-1-piperazinyl]cyclohexyl]-1H-indol;
3-{4-[(1,4-cis)-4-(1H-Indol-4-yl)-piperazinyl-1-yl]cyclohexyl)-1H-indol-5-carbonitril;
3-{4-[(1,4-trans)-4-(1H-Indol-4-yl)-piperazinyl-1-yl]cyclohexyl}-1H-indol-5-carbonitril;
5-Methoxy-3-[cis-4-[4-(1H-indol-4-yl)-1-piperazinyl]cyclohexyl]-1H-indol;
5-Methoxy-3-(trans-4-[4-(1H-indol-4-yl)-1-piperazinyl]cyclohexyl]-1H-indol;
3-[cis-4-[4-(1H-Indol-yl)-1-piperazinyl]cyclohexyl]-2-methyl-1H-indol;
3-[trans-4-[4-(1H-Indol-4-yl)-1-piperazinyl]cyclohexyl]-2-methyl-1H-indol;
3-{(1,4-cis)-4-[4-1H-Indol-4-yl)-piperazin-1-yl]-cyclohexyl}-1H-pyrrolo[2,3-b]pyridin;
3-{(1,4-trans)-4-[4-(1H-Indol-4-yl)-piperazin-1-yl]-cyclohexyl}-1H-pyrrolo[2,3-b]pyridin;
6-Fluor-1-methyl-3-{cis-4-[4-(1-methyl-1H-indol-4-yl)-1-piperazinyl]cyclohexyl}-1H-indol;
3-{(1,4-cis)-4-[4-(1H-Indol-4-yl)-piperazin-1-yl]cyclohexyl}-1-methyl-1H-indol-5-carbonitril;
3-{(1,4-trans)-4-[4-(1H-Indol-4-yl)-piperazin-1-yl]cyclohexyl}-1-methyl-1H-indol-5-carbonitril;
1-Ethyl-3-{(1,4-cis)-4-[4-(1H-indol-4-yl)-piperazin-1-yl]-cyclohexyl}-1H-indol-5-carbonitril;
3-{(1,4-cis)-4-[4-(1H-Indol-4-yl)-piperazin-1-yl]-cyclohexyl}-1-propyl-1H-indol-5-carbonitril;
3-{(1,4-trans)-4-[4-(1H-Indol-4-yl)-piperazin-4-yl]-cyclohexyl}-1-propyl-1H-indol-5-carbonitril;
3-{(1,4-cis)-4-[4-(1H-Indol-4-yl)-piperazin-1-yl]-cyclohexyl}-1-isopropyl-1H-indol-5-carbonitril;
3-{(1,4-trans)-4-[4-(1H-Indol-4-yl)-piperazin-1-yl]cyclohexyl}-1-isopropyl-1H-indol-5-carbonitril;
1-Benzyl-3-{(1,4-cis)-4-[4-(1H-indol-4-yl)-piperazin-1-yl]-cyclohexyl}-1H-indol-5-carbonitril;
1-Benzyl-3-{(1,4-trans)-4-[4-(1H-indol-4-yl)-piperazin-2-yl] cyclohexyl}-1H-indol-5-5-carbonitril;
1-Methyl-3-{(1,4-cis)-4-[4-(1-methyl-1H-indol-4-yl)-piperazin-1-yl]-cyclohexyl}-1H-indol-5-carbonitril;
5-Fluor-3-{(cis)-4-[4-(2-methoxy-phenyl)-piperazin-1-yl]-cyclohexyl}-1H-indol;
5-Fluor-3-{(1,4-cis)-4-[4-(2-methoxy-phenyl)-piperidin-1-yl]-cyclohexyl}-1H-indol;
5-Fluor-3-{(1,4-trans)-4-[4-(2-methoxy-phenyl)-piperidin-1-yl]-cyclohexyl}-1H-indol;
5-Methoxy-3-{(1,4-cis)-4-[4-(2-methoxy-phenyl)-piperazin-1-yl]-cyclohexyl}-1H-indol;
5-Methoxy-3-{(1,4-trans)-4-[4-(2-methoxy-phenyl)-piperazin-1-yl]-cyclohexyl}-1H-indol;
3-{(1,4-cis)-4-[4-(2-Methoxy-phenyl)-piperazin-1-yl]-cyclohexyl}-1H-pyrrolo[2,3-b]pyridin;
5-Fluor-3-{(cis)-4-[4-(5-fluor-2-methoxy-phenyl)-piperazin-1-yl]-cyclohexyl}-1H-indol;
5-Fluor-3-{(trans)-4-[4-(5-fluor-2-methoxy-phenyl)-piperazin-1-yl]-cyclohexyl}-1H-indol;
3-{(1,4-cis)-4-[4[(2,3-Dihydro-benzo[1,4]dioxin-5-yl)-piperazin-1-yl]-cyclohexyl}-4-fluor-1H-indol;
3-{(1,4-cis)-4-[4-(2,3-Dihydro-benzo[1,4]dioxin-5-yl)-piperazin-1-yl]-cyclohexyl}-4-fluor-1H-indol;
3-{(1,4-trans)-4-[4-(2,3-Dihydro-benzo[1,4]dioxin-5-yl)-piperazin-1-yl]-cyclohexyl}-5-fluor-1H-indol;
3-{(1,4-trans)-4-[4-(2,3-Dihydro-benzo[1,4]dioxin-5-yl)-piperazin-1-yl]-cyclohexyl}-5-fluor-1H-indol;
3-{(1,4-cis)-4-[4-(2,3-Dihydro-benzo[1,4]dioxin-5-yl)-piperazin-1-yl]-cyclohexyl}-6-fluor-1H-indol;
3-{(1,4-trans)-4-[4-(2,3-Dihydro-benzo[1,4]dioxin-5-yl)-piperazin-1-yl]-cyclohexyl}-6-fluor-1H-indol;
3-{(1,4-cis)-4-[4-(2,3-Dihydro-benzo[1,4]dioxin-5-yl)-piperazin-1-yl]-cyclohexyl}-1H-indol-5-carbonitril;
3-{(1,4-cis)-4-[4-(2,3-Dihydro-benzo[1,4]dioxin-5-yl)-piperazin-1-yl]-cyclohexyl}-1H-indol-5-carbonitril;
3-{(1,4-trans)-4-(4-(2,3-Dihydro-benzo[1,4]dioxin-5-yl)-piperazin-1-yl]-cyclohexyl}-1H-indol-5-carbonitril;
8-{4-[(1,4-cis)-4-(5-Fluor-1H-indol-3-yl)-cyclohexyl]-piperazin-1-yl}chinolin;
8-{4-[(1,4-trans)-4-(5-Fluor-1H-indol-3-yl)-cyclohexyl]-piperazin-1-yl}-chinolin;
8-{4-(1,4-cis)-4-[4-(5-Fluor-1-methyl-1H-indol-3-yl)-cyclohexyl]-piperazin-1-yl}-chinolin;
3-[(1,4-cis)-4-(4-Chinolin-8-yl-piperazin-1-yl)-cyclohexyl]-1H-indol-5-carbonitril;
3-[(1,4-trans)-4-(4-Chinolin-8-yl-piperazin-1-yl)-cyclohexyl]-1H-indol-5-carbonitril;
1-Methyl-3-[(1,4-cis)-4-(4-chinolin-8-yl-piperazin-1-yl)-cyclohexyl]-1H-indol-5-carbonitril;
5-Fluor-3-((1,4-cis)-4-[4-(6-fluor-chroman-8-yl)-piperazin-1-yl]-cyclohexyl}-1H-indol;
5-Fluor-3-{(1,4-trans)-4-[4-(6-fluor-chroman-8-yl)-piperazin-1-yl]-cyclohexyl}-1H-indol;
5-Fluor-3-{(1,4-cis)-4-[4-(5-fluor-2,3-dihydro-benzofuran-7-yl)-piperazin-1-yl]-cyclohexyl}-1H-indol;
5-Fluor-3-((1,4-trans)-4-[4-(5-fluor-2,3-dihydro-benzofuran-7-yl)-piperazin-1-yl]-cyclohexyl}-1H-indol;
3-{(1,4-cis)-4-[4-(5-Fluor-2,3-dihydro-benzofuran-7-yl)-piperazin-1-yl]-cyclohexyl}-1H-indol-5-carbonitril;
3-{(1,4-trans)-4-[4-(5-Fluor-2,3-dihydro-benzofuran-7-yl)-piperazin-1-yl]-cyclohexyl}-1H-indol-5-carbonitril;
3-{(1,4-trans)-4-[4-(5-Fluor-2,3-dihydro-benzofuran-7-yl)-piperazin-1-yl]-cyclohexyl}-1-methyl-1H-indol-5-carbonitril;
3-[(1,4-cis)-4-[4-(Benzofuran-7-yl-piperazin-1-yl)-cyclohexyl]-1H-indol-5-carbonitril;
3-[(1,4-trans)-4-[4-(Benzofuran-7-yl-piperazin-1-yl)-cyclohexyl]-1H-indol-5-carbonitril;
5-Fluor-3-{cis-4-(4-(1H-indol-4-yl)piperazinyl]-cyclohexyl}-1-methyl-1H-indol;
3-{(1,4-cis)-4-[4-(6-Methoxy-chinolin-8-yl)-piperazin-1-yl]-cyclohexyl}-1H-indol-5-carbonitril;
oder ein pharmazeutisch annehmbares Salz davon.

8. Pharmazeutische Zusammensetzung, welche eine Verbindung der Formel (I) umfasst, wie in einem der Ansprüche 1 bis 7 beansprucht, oder ein pharmazeutisch annehmbares Salz davon und einen pharmazeutisch annehmbaren Träger.

9. Verwendung einer Verbindung der Formel (I) wie in einem der Ansprüche 1 bis 7 beansprucht oder ein pharmazeutisch annehmbares Salz davon für die Herstellung eines Medikaments zum Behandeln von Depression.

10. Verfahren zum Herstellen einer Verbindung der Formel (I) wie in Anspruch 1 definiert, welches eines der folgenden umfasst:
a) Umsetzen einer Verbindung der Formel 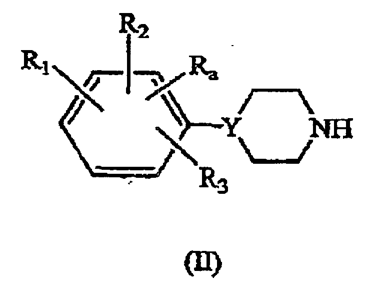 worin Rₐ, R₁₋₃ und Y wie oben definiert sind, mit einer Verbindung der Formel (IV): 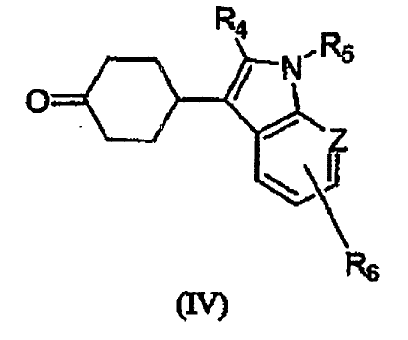 worin Z, R₄, R₅ und R₆ wie oben definiert sind;
oder
b) Reduzieren einer Verbindung der Formel: 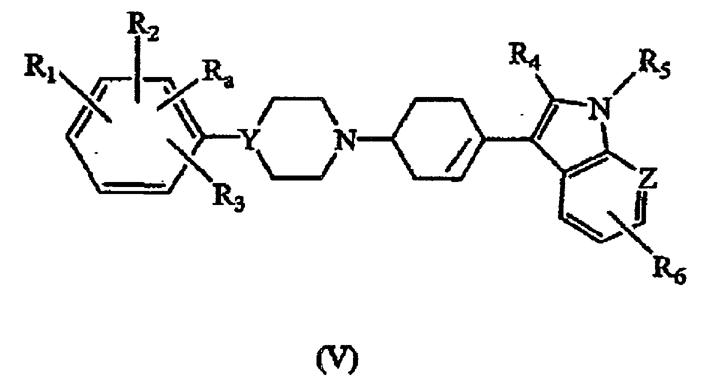 worin die Variablen wie oben definiert sind, um eine Verbindung der Formel (I) zu ergeben;
oder
c) Säuern einer basischen Verbindung der Formel I mit einer pharmazeutisch annehmbaren Säure, um ein pharmazeutisch annehmbares Salz zu ergeben;
oder
d) Trennen eines Gemisches aus cis- und trans-Isomeren einer Verbindung der Formel (I), um ein Isomer im Wesentlichen frei vom anderen Isomer zu isolieren;
oder
e) Umsetzen einer Verbindung der Formel (I) mit einer reaktiven Substituentengruppe, um eine Verbindung der Formel (I) mit einer anderen Substituentengruppe zu ergeben;
oder
f) Umsetzen einer Verbindung der Formel (I) mit einer reaktiven Stelle (z.B. NH), um eine Verbindung der Formel (I) mit einer Substituentengruppe an der Stelle zu ergeben.

11. Verbindungen, ausgewählt aus den Folgenden:
5-Fluor-3-{4-[4-(2-methoxy-phenyl)-piperazin-1-yl]-cyclohex-1-enyl}-1H-indol;
3-{4-[4-1H-Indol-4-yl)-piperazin-1-yl]-cyclohex-1-enyl}-1Hindol-5-carbonitril.

## Revendications

1. Composé de formule: 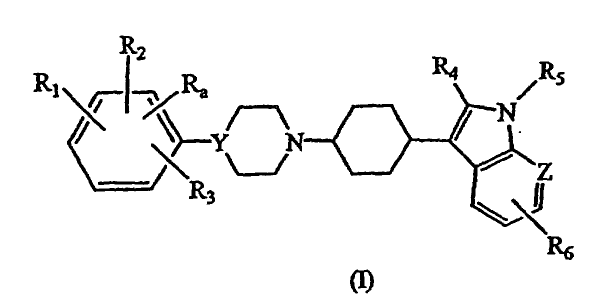 dans laquelle:
Rₐ, R₁, R₂ et R₃ sont chacun, indépendamment, hydrogène ou un substituant choisi parmi halogène, CF₃, alkyle, alkoxy, MeSO₂, amino ou aminocarbonyl (chacun facultativement substitué par un ou deux groupements choisis parmi alkyle et benzyle), carboxy ou alkoxycarbonyl; ou deux adjacents de Rₐ et R₁₋₃ conjointement peuvent former un carbocycle ou un hétérocycle à 5-7 chaînons qui est facultativement substitué par un substituant défini ci-dessus ;
R₄ est hydrogène, halogène ou alkyle;
R₅ est hydrogène, alkyle, alkylaryle, ou aryle;
R₆ est hydrogène, halogène, CF₃, CN, carbamide, alkoxy ou benzyloxy;
Y est CH ou azote ; et
Z est carbone ou azote ; ou un sel pharmaceutiquement acceptable de celui-ci, chaque groupement alkyle et alkoxy incluant le composant alkyle d'arylalkyle contient 1-6 atomes de carbone et chaque radical aryle incluant le composant aryle d'arylalkyle contient 6-12 atomes de carbone.

2. Composé selon la revendication 1, dans lequel:
Rₐ, R₁, R₂ et R₃ sont chacun, indépendamment, hydrogène, halogène, alkyle, alkoxy ou conjointement peuvent former un carbocycle ou hétérocycle à 5-7 chaînons.

3. Composé selon la revendication 1 ou la revendication 2, dans lequel R₄ est hydrogène ou halogène.

4. Composé selon l'une quelconque des revendications 1 à 3 dans lequel R₅ est hydrogène, alkyle ou alkylaryle.

5. Composé selon l'une quelconque des revendications 1 à 4 dans lequel R₆ est hydrogène, halogène, CN ou alkoxy.

6. Composé selon l'une quelconque des revendications 1 à 5 dans lequel Y et Z sont chacun carbone.

7. Composé selon la revendication 1 qui est choisi parmi les suivants :
3-[cis-4-[4-(1H-indol-4-yl)-1-pipérazinyl]-cyclohexyl]-1H-indole;
3-[trans-4-[4-(1H-indol-4-yl)-1-pipérazinyl] cyclohexyl]-1H-indole;
4-fluoro-3-[cis-4-[4-(1H-indol-4-yl)-1-pipérazinyl]cyclohexyl]-1H-indole;
4-fluoro-3-[trans-4-[4-(1H-indol-4-yl)-1-pipérazinyl]cyclohexyl]-1H-indole;
5-fluoro-3-[cis-4-[4-(1H-indol-4-yl)-1-pipérazinyl]cyclohexyl]-1H-indole;
5-fluoro-3-[trans-4-[4-(1H-indol-4-yl)-1-pipérazinyl]cyclohexyl]-1H-indole;
6-fluoro-3-[cis-4-[4-(1H-indol-4-yl)-1-pipérazinyl]cyclohexyl]-1H-indole;
6-fluoro-3-[trans-4-[4-(1H-indol-4-yl)-1-pipérazinyl]cyclohexyl]-1H-indole;
5-bromo-3-[cis-4-[4-(1H-indol-4-yl)-1-pipérazinyl]cyclohexyl]-1H-indole;
5-bromo-3-[trans-4-[4-(1H-indol-4-yl)-1-pipérazinyl]cyclohexyl]-1H-indole;
5-chloro-3-[cis-4-[4-(1H-indol-4-yl)-1-pipérazinyl]cyclohexyl]-1H-indole;
5-chloro-3-[trans-4-[4-(1H-indol-4-yl)-1-pipérazinyl]cyclohexyl]-1H-indole;
3-{4-[(1,4-cis)-4-(1H-indol-4-yl)pipérazinyl-1-yl]cyclohexyl}-1H-indol-5-carbonitrile;
3-{4-[(1,4-trans)-4-(1H-indol-4-yl)-pipérazinyl-1-yl]cyclohexyl}-1H-indol-5-carbonitrile;
5-méthoxy-3-[cis-4-[4-(1H-indol-4-yl)-1-pipérazinyl]cyclohexyl]-1H-indole;
5-méthoxy-3-[trans-4-[4-(1H-indol-4-yl)-1-pipérazinyl]cyclohexyl]-1H-indole;
3-[cis-4-[4-(1H-indol-4-yl)-1-pipérazinyl] cyclohexyl)-2-méthyl-1H-indole;
3-[trans-4-[4-(1H-indol-4-yl)-1-pipérazinyl] cyclohexyl]-2-méthyl-1H-indole;
3-{(1,4-cis)-4-[4-1H-indol-4-yl)pipérazin-1-yl]cyclohexyl]-1H-pyrrolo[2,3-b]pyridine;
3-{(1,4-trans)-4-[4-(1H-indol-4-yl)pipérazin-1-yl]cyclohexyl}-1H-pyrrolo[2,3-b]pyridine;
6-fluoro-1-méthyl-3-{cis-4-[4-(1-méthyl-1H-indol-4-yl)-1-pipérazinyl]cyclohexyl}-1H-indole;
3-{(1,4-cis)-4-[4-(1H-indol-4-yl)pipérazin-1-yl]cyclohexyl}-1-méthyl-1H-indol-5-carbonitrile;
3-{(1,4-trans)-4-[4-(1H-indol-4-yl)pipérazin-1-yl]cyclohexyl}-1-méthyl-1H-indol-5-carbonitrile;
1-éthyl-3-{(1,4-cis)-4-[4-(1H-indol-4-yl)-pipérazin-1-yl]-cyclohexyl}-1H-indol-5-carbonitrile;
3-{(1,4-cis)-4-[4-(1H-indol-4-yl)pipérazin-1-yl]cyclohexyl}-1-propyl-1H-indol-5-carbonitrile;
3-{(1,4-trans)-4-[4-(1H-indol-4-yl)pipérazin-4-yl]cyclohexyl}-1-propyl-1H-indol-5-carbonitrile;
3-{(1,4-cis)-4-[4-(1H-indol-4-yl)pipérazin-1-yl]-cyclohexyl}-1-isopropyl-1H-indol-5-carbonitrile;
3-{(1,4-trans)-4-[4-(1H-indol-4-yl}pipérazin-1-yl]cyclohexyl}-1-isopropyl-1H-indol-5-carbonitrile;
1-benzyl-3-{(1,4-cis)-4-[4-(1H-indol-4-yl)-pipérazin-1-yl]cyclohexyl}-1H-indol-5-carbonitrile;
1-benzyl-3-{(1,4-trans)-4-[4-(1H-indol-4-yl)-pipérazin-1-yl]cyclohexyl}-1H-indol-5-carbonitrile;
1-méthyl-3-{(1,4-cis)-4-[4-(1-méthyl-1H-indol-4-yl)pipérazine-1-yl]-cyclohexyl}1H-indol-5-carbonitrile;
5-fluoro-3-{(cis)-4-[4-(2-méthoxyphényl)-pipérazin-1-yl]cyclohexyl}-1H-indole;
5-fluoro-3-{(1,4-cis)-4-[4-(2-méthoxyphényl)-pipéridin-1-yl]-cyclohexyl}-1H-indole;
5-fluoro-3-{(1,4-trans)-4-[4-(2-méthoxyphényl)pipéridin-1-yl]cyclohexyl}-1H-indole;
5-méthoxy-3-{(1,4-cis)-4-[4-(2-méthoxyphényl)pipérazin-1-yl]cyclohexyl}-1H-indole;
5-méthoxy-3-{(1,4-trans)-4-[4-(2-méthoxyphényl)pipérazin-1-yl]cyclohexyl}-1H-indole;
3-{(1,4-cis)-4-[4-(2-méthoxyphényl)pipérazin-1-yl]cyclohexyl}-1H-pyrrolo[2,3b]pyridine;
5-fluoro-3-{(cis)-4-[4-(5-fluoro-2-méthoxyphényl)pipérazin-1-yl]-cyclohexyl}-1H-indole;
5-fluoro-3-{(trans)-4-[4-(5-fluoro-2-méthoxyphenyl)pipérazin-1-yl)-cyclohexyl}-1H-indole;
3-{(1,4-cis)-4-[4[(2,3-dihydrobenzo[1,4]-dioxin-5-yl)pipérazin-1-yl]cyclohexyl}-4-fluoro-1H-indole;
3-{(1,4-cis)-4-[4-(2,3-dihydrobenzo[1,4]-dioxin-5-yl)pipérazin-1-yl]cyclohexyl}-4-fluoro-1H-indole;
3-{(1,4-trans)-4-[4-(2,3-dihydrobenzo[1,4] dioxin-5-yl)pipérazin-1-yl]-cyclohexyl}-5-fluoro-1H-indole;
3-{(1,4-trans)-4-[4-(2,3-dihydrobenzo[1,4] dioxin-5-yl)pipérazin-1-yl]-cyclohexyl}-5-fluoro-1H-indole;
3-{(1,4-cis)-4-[4-(2,3-dihydrobenzo[1,4]-dioxin-5-yl)pipérazin-1-yl]cyclohexyl}-6-fluoro-1H-indole;
3-{(1,4-trans)-4-[4-(2,3-dihydrobenzo[1,4] dioxin-5-yl)pipérazin-1-yl]cyclohexyl}-6-fluoro-1H-indole;
3-{(1,4-cis)-4-[4-(2,3-dihydrobenzo[1,4]-dioxin-5-yl)pipérazin-1-yl]cyclohexyl}-1H-indol-5-carbonitrile;
3-{(1,4-cis)-4-[4-(2,3-dihydrobenzo[1,4]-dioxin-5-yl)pipérazin-1-yl]cyclohexyl}-1H-indol-5-carbonitrile;
3-{(1,4-trans)-4-(4-(2,3-dihydrobenzo[1,4] dioxin-5-yl)pipérazin-1-yl]cyclohexyl}-1H-indol-5-carbonitrile;
8-{4-[(1,4-cis)-4-(5-fluoro-1H-indol-3-yl)-cyclohexyl]pipérazin-1-yl}quinoléine;
8-{4-[(1,4-trans)-4-(5-fluoro-1H-indol-3-yl)-cyclohexyl]pipérazin-1-yl}quinoléine;
8-{4-(1,4-cis)-4-[4-(5-fluoro-1-méthyl-1H-indol-3-yl)cyclohexyl]pipérazin-1-yl}quinoléine;
3-[(1,4-cis)-4-(4-quinoléin-8-yl-pipérazin-1-yl)cyclohexyl]-1H-indol-5-carbonitrile;
3-[(1,4-trans)-4-(4-quinoléin-8-yl-pipérazin-1-yl)cyclohexyl]-1H-indol-5-carbonitrile;
1-méthyl-3-[(1,4-cis)-4-(4-quinoléin-8-yl-pipérazin-1-yl)cyclohexyl]-1H-indol-5-carbonitrile;
5-fluoro-3-{(1,4-cis)-4-[4-(6-fluorochroman-8-yl)pipérazin-1-yl]-cyclohexyl}-1H-indole;
5-fluoro-3-{(1,4-trans)-4-[4-(6-fluorochroman-8-yl)pipérazin-1-yl]cyclohexyl}-1H-indole;
5-fluoro-3-{(1,4-cis)-4-[4-(5-fluoro-2,3-dihydrobenzofuran-7-yl)pipérazin-1-yl]cyclohexyl}-1H-indole;
5-fluoro-3-{(1,4-trans)-4-[4-(5-fluoro-2,3-dihydrobenzofuran-7-yl)pipérazin-1-yl]cyclohexyl}-1H-indole;
3-{(1,4-cis)-4-[4-(5-fluoro-2,3-dihydrobenzofuran-7-yl)pipérazin-1-yl]cyclohexyl}-1H-indol-5-carbonitrile;
3-{(1,4-trans)-4-[4-(5-fluoro-2,3-dihydrobenzofuran-7-yl)pipérazin-1-yl]cyclohexyl}-1H-indol-5-carbonitrile;
3-{(1,4-trans)-4-[4-(5-fluoro-2,3-dihydrobenzofuran-7-yl)pipérazin-1-yl]cyclohexyl}-1-méthyl-1H-indol-5-carbonitrile;
3-[(1,4-cis)-4-[4-(benzofuran-7-yl-pipérazin-1-yl)-cyclohexyl]-1H-indol-5-carbonitrile;
3-[(1,4-trans)-4-[4-(benzofuran-7-yl-pipérazin-1-yl)cyclohexyl]-1H-indol-5-carbonitrile;
5-fluoro-3-{cis-4-[4-(1H-indol-4-yl) pipérazinyl]cyclohexyl}-1-méthyl-1H-indole;
3-{(1,4-cis)-4-[4-(6-méthoxyquinoléin-8-yl)-pipérazin-1-yl]cyclohexyl}-1H-indol-5-carbonitrile ;
ou un sel pharmaceutiquement acceptable de celui-ci.

8. Composition pharmaceutique comprenant un composé de formule (I) comme revendiqué dans l'une quelconque des revendications 1 à 7 ou un sel pharmaceutiquement acceptable de celui-ci et un excipient pharmaceutiquement acceptable.

9. Utilisation d'un composé de formule (I) comme revendiqué dans l'une quelconque des revendications 1 à 7 ou d'un sel pharmaceutiquement acceptable de celui-ci pour la fabrication d'un médicament pour le traitement de la dépression.

10. Procédé de préparation d'un composé de formule (I) comme défini dans la revendication 1 qui comprend une des suivantes:
a) la réaction d'un composé de formule 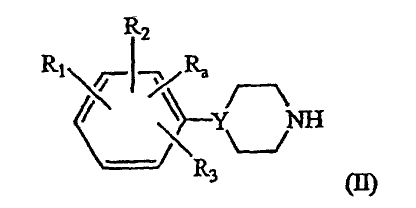 dans laquelle Rₐ, R₁₋₃ et Y sont comme définis ci-dessus, avec un composé de formule (IV): 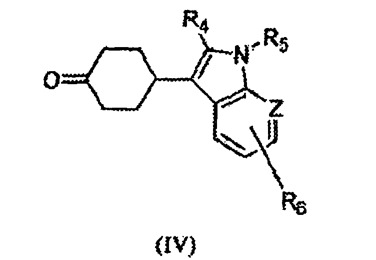 dans laquelle Z, R₄, R₅ et R₆ sont comme définis ci-dessus; ou
b) la réduction d'un composé de formule: 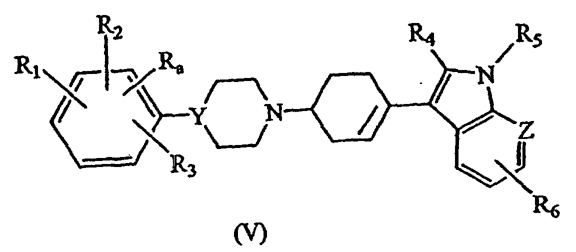 dans laquelle les variables sont comme définies ci-dessus pour donner un composé de formule (I); ou
c) l'acidification d'un composé basique de formule I avec un acide pharmaceutiquement acceptable pour donner un sel pharmaceutiquement acceptable; ou
d) la séparation d'un mélange d'isomères cis et trans d'un composé de formule (I) pour isoler un isomère substantiellement exempt de l'autre isomère; ou
e) la réaction d'un composé de formule (I) ayant un groupement substituant réactif pour donner un composé de formule (I) ayant un groupement substituant différent; ou
f) la réaction d'un composé de formule (I) ayant un site réactif (par exemple, NH) pour donner un composé de formule (I) ayant un groupement substituant sur le site.

11. Composés choisis parmi les suivants :
5-fluoro-3-{4-[4-(2-méthoxyphényl)-pipérazin-1-yl]cyclohex-1-ényl}-1H-indole;
3-{4-[4-(1H-indol-4-yl)pipérazin-1-yl]-cyclohex-1-ényl}-1H-indol-5-carbonitrile.
